# EUROPEAN PATENT APPLICATION

(11) **EP 3 936 517 A1**
(43) Date of publication of application: **12.01.2022**
(21) Application number: 20305789.8
(22) Date of filing: 08.07.2020
(51) Int. Cl.: C07K 14/005, A61K 39/12, C12N 15/86

(54) **AN IMPROVED MEASLES VIRUS VACCINE VECTOR BASED ON MULTIPLE TANDEM ADDITIONAL TRANSCRIPTION UNITS**

(71) Applicant: INSTITUT PASTEUR, 75724 Paris Cedex 15 (FR)
(72) Inventor: Namprachan-Frantz, Phanramphoei, 75724 Paris Cedex 15 (FR); Tangy, Frédéric, 75724 Paris Cedex 15 (FR); Combredet, Chantal, 75724 Paris Cedex 15 (FR); Gracias, Ségolène, 75724 Paris Cedex 15 (FR)
(74) Representative: Ernest Gutmann - Yves Plasseraud S.A.S.

(57) **Abstract**

The application generally relates to enhanced recombinant nucleic acid constructs comprising a cDNA molecule encoding a full length antigenomic (+) RNA strand of a non-segmented negative-sense single-stranded RNA virus for expressing at least one heterologous polypeptide, protein, antigen, or antigenic fragment thereof. The application also relates to the uses of such genetic constructs or viruses, and more particularly their applications for inducing protection against at least one disease).

## Description

### FIELD OF THE INVENTION

The application generally relates to enhanced recombinant genetic constructs comprising a recombinant virus suitable for expressing at least one polypeptide (wherein this term encompasses protein, antigen, or antigenic fragment thereof). The application also relates to the uses of such genetic constructs or viruses, and more particularly their applications for inducing protection against at least one disease, including measles (MV or MV).

The means of the invention are more particularly dedicated to the provision of a recombinant nucleic acid construct allowing the expression of large heterologous polynucleotide(s) inserted, especially as a result of cloning, within a cDNA molecule encoding a full length antigenomic (+) RNA strand of a non-segmented negative-sense single-stranded RNA virus and the expression of a heterologous polypeptide encoded by such an inserted heterologous polynucleotide, in particular by a heterologous polynucleotide or multiple heterologous polynucleotides having or respectively having together a size of at least 6 kb, and more preferably of at least 6,5 kb, and most preferably over 7 kb.

The means of the invention are more particularly dedicated to a recombinant nucleic acid construct allowing the expression of a plurality of heterologous polypeptides e.g. proteins, antigens, or antigenic fragments thereof, encoded within the recombinant nucleic acid construct, wherein the nucleic acid encoding such heterologous polypeptides e.g., proteins, antigens, or antigenic fragments thereof has a size over 6 kb, more particularly over 6,5 kb, and most preferably over 7 kb.

The means of the invention are more particularly dedicated to a recombinant nucleic acid construct allowing a similar expression (in terms of number of RNA copies issued from the recombinant nucleic acid construct) of a plurality of heterologous polypeptides e.g., proteins, antigens, or antigenic fragments thereof, in particular wherein the nucleic acid encoding such heterologous polypeptides e.g., proteins, antigens, or antigenic fragments thereof has a size over 6kb, more particularly over 6.5 kb, and most preferably over 7 kb.

The invention also relates to the use of these genetic constructs, recombinant nucleic acid constructs, expression vectors comprising these constructs, viruses and virus-like particles issued from the expression of these constructs for inducing an immunogenic response within a host, especially a human host, against at least one disease, including but not limited to an immunogenic response against a pathogenic virus such as measles virus, Chikungunya virus, Zika Virus, West Nile Virus, Dengue virus, SARS, MERS, coronavirus, in particular SARS-Cov-2, Influenza virus, Ebola virus, Nipas virus, HIV and/or a non-viral pathogen such as parasites in particular Plasmodium species causing malaria, in particular those infecting humans *P. vivax, P. falciparum, P. malariae, P. ovale, and P. knowlesi.*

The invention also relates to the use of a cDNA molecule encoding a full length antigenomic (+) RNA strand of a non-segmented negative-sense single-stranded RNA virus, in particular a Measles virus, as a vector to express immunogens or epitopes of antigens wherein said immunogens or epitopes encompass or derive from heterologous polypeptides originating from pathogens, in particular viruses or parasites, including in particular immunogens or epitopes thereof of measles virus, Chikungunya virus, Zika Virus, West Nile Virus, Dengue virus, SARS, MERS, coronavirus, in particular SARS-Cov-2, Influenza virus, Lassa virus, Ebola virus, Nipah virus, HIV, and non-viral pathogens such as parasites in particular *Mycobacterium tuberculosis* and Plasmodium species causing malaria, in particular those infecting humans *P. vivax, P. falciparum, P. malariae, P. ovale, and P. knowlesi.*

### BACKGROUND OF THE INVENTION

Measles virus has been isolated in 1954 (Enders, J. F., and T. C. Peebles. 1954. Propagation in tissue cultures of cytopathogenic agents from patients with measles. Proc. Soc. Exp. Biol. Med.86:277-286.). Measles virus is a member of the order mononegavirales, i.e. viruses with a non-segmented negative-strand RNA genome. The non-segmented genome of MV has an antimessage polarity which results in a genomic RNA which is neither translated *in vivo* or *in vitro* nor infectious when purified. Transcription and replication of non-segmented (-) strand RNA viruses and their assembly into virus particles have been studied and reported especially in Fields virology (3rd edition, vol. 1, 1996, Lippincott - Raven publishers - Fields BN *et al*.). Transcription and replication of the measles virus do not involve the nucleus of the infected cells but rather take place in the cytoplasm of host cell. The genome of the MV comprises genes encoding six major structural proteins designated N, P, M, F, H and L, and an additional two non-structural proteins from the P gene, C and V. The gene order is the following: from the 3' end of the genomic RNA; N, P (including C and V), M, F, H and L large polymerase at the 5' end. The genome furthermore comprises non-coding regions in the intergenic region M/F. This non-coding region contains approximatively 100 nucleotides of untranslated RNA. The cited genes respectively encode the proteins of the nucleocapsid of the virus or nucleoprotein (N), the phosphoprotein (P), the large protein (L) which together assemble around the genome RNA to provide the nucleocapsid, the hemagglutinin (H), the fusion protein (F) and the matrix protein (M).

Attenuated viruses have been derived from MV virus to provide vaccine strains and in particular from the Schwarz strain. The Schwarz measles vaccine is a safe and efficient vaccine currently available for preventing measles. Besides providing vaccine, strains attenuated measles virus such as the Schwarz strain have shown to be stable and suitable for the design of efficient delivery vector for immunization against other infections caused by pathogens like but not limited to West Nile Virus, Chikungunya virus, Dengue Virus, Lassa Virus, Zika virus.. Measles vaccine has been administered to billions of children over the last 30 years and have proved both efficiency and safety. It is produced on a large scale in many countries and is distributed at low cost.

Measles vaccine Schwarz/Moraten strain is WHO approved as an efficient and widely used attenuated MV vaccine strain. To generate an infectious clone, its full-length antigenomic viral + strand cDNA was previously cloned into the pTM plasmid under the control of the T7 RNA promoter (Combredet et al, 2003).

Several genetic elements such as hammerhead and hepatitis delta virus ribozymes, and T7 terminator, were added for accurate cleavage to ensure the production of full-length viral RNA. The process of viral recovery consists in transfecting pTM-MVschw together with a plasmid expressing the MV-L gene (MV polymerase) into helper HEK293 cells that constitutively express T7 RNA polymerase, MV-N (Nucleoprotein) and MV-P (Phosphoprotein) (Combredet et al, 2003). Transfected cells are then cocultured with an MV permissive cell line such as Vero cells to amplify virus seed. Rescued virus from the cDNA clone was shown to harbor exactly the same sequence as the parental Schwarz virus strain, in particular the sequence of SEQ ID No. 62. To enable insertion of foreign antigens, several additional transcription units (ATUs) were introduced into pTM-MVschw at various sites in MV genome. Cloning sites can accommodate inserts of up to 6kb in length as multiples of six base pairs to respect the "rule of six" that is essential for measles genome replication (Calain and Roux, 1993).

Recombinant MV vectors generated from attenuated MV vaccine strains are attractive choices for developing vaccine candidates against other diseases. Due to the helicoidal packaging of its genome, MV vector can accommodate foreign genes up to 6kb in size, although this is very difficult to achieve with the current plasmid-based vectors, and no publication has so far demonstrated such capacity. Heterologous proteins can be stably expressed at high levels for more than 12 passages, and rescued recombinant viruses retain growth capabilities similar to the original MV strain (Frantz et al., 2018). The MV delivery platform has hence been developed from preclinical stage to clinical proof-of-concept with a preclinical track record of fast and effective adaptability to a variety of pathogens. Preclinical immunogenicity and protection from lethal challenges have been shown in mice and non- human primates (NHPs). Target pathogens include HIV (Guerbois et al., 2009; Lorin et al., 2012),West Nile Virus (WNV) (Despres et al., 2005; Brandler et al., 2012), dengue virus (DENV) (Brandler et al., 2007; Brandler et al., 2010), hepatitis B virus (HBV) (del Valle et al., 2007), human papillomavirus (HPV) (Cantarella et al., 2009), Chikungunya virus (CHIKV) (Brandler et al., 2013), Nipah virus (NiV) (Yoneda et al., 2013), respiratory syncytia virus (RSV) (Yamaji et al., 2014), SARS-CoV (Escriou et al., 2014), MERS-CoV (Malczyk et al., 2015), H5N1 influenza A virus (IAV) (unpublished), Zika virus (ZIKV) (unpublished), Lassa virus (LASV) (Mateo et al., 2019) and Ebola virus (EBOV) (unpublished).

Immune protection against several diseases has been reached after vaccination with Measles-derived recombinant vaccines, and protection from lethal infectious challenges has been shown in mice and non-human primates (NHPs). Several recombinant MV vaccines reached clinical trials phase I for treating HIV, ZIKA, and Lassa, and phase II for treating chikungunya infections. The recombinant measles-chikungunya (MV-CHIKV) is the most advanced vaccine candidate. It has completed phase I and II clinical trials in adults (Ramsauer et al, 2015, Reisinger et al, 2018) and is now introduced into phase III trials. These clinical studies showed that the vaccine was well tolerated and elicited a robust and functional antibody response after one (90% sero- converters) or two (100% sero-converters) immunizations. Most importantly, the study demonstrated that the immune response to MV and CHIKV was not dampened by previous MV immunization since all volunteers were pre-immune to measles. Moreover, in this phase 2 trial a group of volunteers received a standard MMR vaccination a month before vaccination with the recombinant MV-CHIKV vaccine and did not show statistically different immunization to CHIKV to the others. Thus, MV recombinant vaccines are well suited to immunize measles pre-immune populations. Also, the antibody response to measles was strongly boosted.

As all RNA viruses, and due to the low fidelity of viral RNA polymerases, MV seeds and stocks are composed of a mixture of various individual viral genome sequences named quasispecies (E. Domingo 1985), among which one genomic sequence is usually greatly preponderant and named the mean genomic sequence. The viral genomic sequences of quasispecies may diverge by single nucleotide variations which may account for great variability in growth capacity or stability. As recombinant MV are generated by reverse genetics systems based on the transfection of helper cells with plasmids encoding the full length antigenomic (+)RNA strand of MV, quasispecies are also generated during this process. Therefore, it is of great importance to be able to isolate, clonally select, and identify appropriate virus clonal seeds that grow efficiently and strongly and stably express the additional antigens. For that purpose, the yield of reverse genetics rescue system is critical, as a sufficient number of viral clones must be generated to screen among them the most performant seed in terms of growing capacity, genomic stability, and expression capacity, which are determinant factors for a live vaccine.

However, the current bacterial plasmids used for MV vector, which are usually around 3 kb in size, accommodate the 16 kb of MV genome plus the different additional genetic material, resulting in plasmids that are frequently larger that 20 kb long. This may be the maximum size that currently used bacterial plasmid for the MV delivery platform can accommodate to be stable in bacteria and mammalian cells without recombining. This issue is responsible for the low yield of reverse genetics rescue systems for MV and other mononegavirales currently observed, and for the impossibility to generate recombinant MV harboring more than 5-6 kb additional nucleotides suitable for use in the preparation of seeds and stocks to elaborate vaccines. With the increasing number of vaccine-preventable emerging diseases, single multivalent vaccines inducing protection from several pathogens are desirable. Multivalent vaccines allow reducing the number of administrations and the costs of manufacturing, storage and transportation. MV recombinant vaccines with single immunogen have proven safe and successful in clinical trials. Therefore, MV vector is considered a front-runner platform to create multivalent vaccines against a combination of diseases, particularly diseases that co-circulate in the same epidemic region such as chikungunya, dengue and Zika fevers for example. For all these reasons, alternatives to the backbone plasmid vector currently used to harbor MV antigenome were considered by the inventors.

Moreover, there is a need for an improved recombinant MV vaccine, allowing the provision of larger antigens and/or more antigens within a single construction, while the construct allows to finely tune the expression gradient of each antigen by providing more insertion sites, allowing to express at the same or a different levels the antigens inserted in the construction. The current MV vaccine vectors do not allow the provision of genetic constructs wherein a plurality of antigenic polypeptides originating from a pathogen or from a plurality of pathogens are individually inserted within a transcription unit and are expressed at the same level. The current state of the art provides such plurality of antigenic polypeptides within a fusion protein or allows the expression of these antigenic polypeptides at different expression levels. However, expressing each antigenic polypeptide in a proper immunogenic configuration may benefit from their individual expression rather than in the form of fusion antigens. Allowing the expression of a plurality of antigenic polypeptides, each in an individual form (e.g. not within a fusion protein or the like), in particular at the same quantitative level may lead to the provision of a more efficient vaccine, either easily produced, and/or eliciting a more suitable immune response when administered to a host, in particular a human subject. It will also facilitate the manufacturing of single multivalent vaccines rather than manufacturing several individual vaccines to be administered simultaneously.

### ASPECTS AND EMBODIMENTS OF THE INVENTION

### • Definitions

By "heterologous", it should be understood that the heterologous polypeptide is encoded by a nucleotide sequence provided, especially recombined into the cDNA molecule encoding a full length antigenomic (+) RNA strand of a non-segmented negative-sense single-stranded RNA virus, while it is not naturally present in such cDNA molecule. Heterologous polynucleotides derive from an organism different from the organism from which the cDNA molecule encoding a full length antigenomic (+) RNA strand of a non-segmented negative-sense single-stranded RNA virus is issued. In a particular embodiment, the heterologous polynucleotide encoding the heterologous polypeptide is cloned from or derived from a different virus species from that of the recipient full-length antigenomic (+) RNA; in particular, the heterologous polynucleotide encodes a heterologous polypeptide that is not naturally encoded by a measles virus. Typically in order to elicit in a host, an immune response against the pathogen or the infection caused by the pathogen providing the heterologous polypeptide, the polypeptide itself is not transferred to the expressing cells or administered to the host, but instead the genetic material encoding for the polypeptide (the complementary DNA or cDNA) is inserted within the cDNA molecule encoding a full length antigenomic (+) RNA strand of a non-segmented negative-sense single-stranded RNA virus.

An "expression cassette" is a component of the cDNA comprising a coding sequence, in particular an Open Reading Frame or a gene together with operably linked regulatory sequence(s) for expression, to be expressed by a transfected cell, when the nucleic acid construct comprising the cDNA is present within the transfected cell. The expression cassette allows the transfected cell's machinery to make RNA and protein(s) from the nucleic acid construct of the invention. In addition to the genes of the cDNA molecule and the inserted polynucleotides encoding heterologous polypeptides, an expression cassette usually comprises at least one of the following elements: (inducible) promoters, multiple cloning site(s), an efficient terminator of transcription. An expression cassette may therefore be defined as a polynucleotide present within the cDNA molecule and composed of one or more ORFs or genes and the sequences controlling their expression within the cDNA. An expression cassette comprises at least three components: a promoter sequence, an Open Reading Frame, and a 3' untranslated region that, in eukaryotes, usually contains a polyadenylation site.

A "bacterial artificial chromosome plasmid" (also referenced as "BAC" or BAC plasmid or recombinant BAC plasmid in the present description) is a DNA construct, based on a functional fertility plasmid (or F-plasmid). Recombinant BAC plasmid usually comprises a sequence for plasmid replication and regulation of copy number (repE gene); genes for partitioning the F plasmid into daughter cells during division and to ensure stability of the recombinant BAC plasmid (parA and parB genes); a selectable marker giving antibiotic resistance to cells comprising the BAC, and promoters for transcription of gene(s) possibly inserted therein. BAC plasmids have a high capacity for cloning of large sequences of DNA that can be further modified. The product is a supercoiled, circular molecule that is resistant to rearrangement and shearing. Low-copy BAC replicons were particularly used to clone large DNA or RNA viruses of up to 300 kb (Embers et al.) for example, Human herpes virus HHV-6A (Borenstein et al.), poxviruses (Cottingham et al.; Domi et al.; Meisinger-Henschel et al.; Roth et al.), coronaviruses (Almazan et al. 2000, Almazan et al. 2006; Fan et al.; Rasmussen et al.; Jengarn et al.), or flaviviruses (Pu et al.; Aubry et al.). Moreover, BAC plasmids allow an easier cloning of toxic viral proteins in bacterial cells (O'Connor et al.).

The recombinant BAC plasmid may be selected from the list consisting of the pSMART BAC plasmid or any BAC plasmid issued from the pSMART BAC plasmid, pEZ-BAC plasmid, pBeloBAC11plasmid (from New England Biolabs), pBACe3.6 (from New England Biolabs), pBAC/OriV, which is issued from pBeloBAC11 and is disclosed in Wild et al. Genome Res. 2002. 12: 1434-1444), pBAC-RT (disclosed in Stavropoulos et al, Genomics 2001 Feb 15;72(1):99-104. doi: 10.1006/geno.2000.6481), pHA1 and pHA2 (both disclosed in Adler et al, J Virol. 2000 Aug; 74(15):6964-74. doi: 10.1128/jvi.74.15.6964-6974.2000), pTARBAC2 disclosed in Zeng et al, Genomics. 2001 Sep;77(1/2):27-34.and its derivatives disclosed in Fuentes et al G3 (Bethesda). 2016 Jan 27. doi: 10.1534/g3.115.026344), the pBAC contruct inserted in Transmissible gastroenteritis coronavirus (TGEV) (disclosed in Sola et al., J Virol. 2003 Apr;77(7):4357-69). In a preferred embodiment of the invention, the recombinant BAC plasmid is selected from the list consisting of the pSMART-BAC, the pBeloBAC11 and the pBACe3.6. In a more preferred embodiment, the recombinant BAC plasmid is the pSMART-BAC.

The expression "encodes" used in the present description encompasses the ability of a cDNA molecule to allow transcription of a full length antigenomic (+) RNA, said cDNA serving especially as a template for transcription and where appropriate translation for product expression into cells or cell lines. Hence, when the cDNA is a double stranded molecule, one of the strands has the same nucleotide sequence as the antigenomic (+) RNA of the RNA virus, especially of the measles virus, with the first heterologous polynucleotide cloned within, except "U" nucleotides that are substituted by "T" nucleotides in the cDNA. The nucleic acid construct of the invention may comprise regulatory elements controlling the transcription of the coding sequences, in particular promoters and termination sequences for the transcription, and possibly enhancer and other cis-acting elements. These regulatory elements may be heterologous with respect to the heterologous polynucleotide issued or derived from the polypeptide encoded by the inserted polynucleotide within the cDNA of the MV, and in particular may be the regulatory elements of the measles virus strain, e.g., promoters, enhancers, cis-acting elements of the Measles virus.

The expressions "operatively inserted", "operably inserted" which may be substituted by the expression "operatively/operably linked" or "operatively/operably cloned", refer to the functional cloning, or insertion, of a heterologous polynucleotide within the nucleic acid construct of the invention in particular within the cDNA encoding a full length antigenomic (+) RNA strand of a non-segmented negative-sense single-stranded RNA virus, especially of a measles virus, such that said heterologous polynucleotide and nucleic acid construct are effectively, or efficiently, transcribed and if appropriate translated, in particular in cells, cell line, host cell used as a part of a rescue system for the production of recombinant infectious virus particles, especially recombinant infectious MV particles or MV expressing at least one heterologous polypeptide, e.g., at least one protein, or at least one antigen, or at least an antigenic fragment thereof, of the heterologous polynucleotide inserted within the cDNA molecule encoding a full length antigenomic (+) RNA strand of a non-segmented negative-sense single-stranded RNA virus, especially of the Measles virus. In other words, the nucleic acid construct of the invention allows the production, when placed in appropriate conditions, of an infectious antigenomic (+) RNA capable of producing at least one heterologous polypeptide, e.g. at least one protein, or at least one antigen, or at least an antigenic fragment thereof encoded by the heterologous polynucleotide inserted within the cDNA molecule encoding a full length antigenomic (+) RNA strand of a non-segmented negative-sense single-stranded RNA virus, especially of the Measles virus.

A "pathogen" may be any kind of pathogen according to its broadest definition; a pathogen may be any microorganism that can alter health of a host, in particular produce disease. In particular, pathogens are selected among the pathogens that infect human. A pathogen may also be referred to as an infectious agent, or simply as a germ. A pathogen may be a virus, in particular a virus infecting human, especially a virus selected from the list consisting of measles virus, Chikungunya virus, Zika Virus, West Nile Virus, Dengue virus, SARS, MERS, coronavirus, in particular SARS-Cov-2, Influenza virus, Ebola virus, Nipas virus, HIV. A pathogen may alternatively be a non-viral pathogen such as a parasite in particular *Mycobacterium tuberculosis and Plasmodium* species causing malaria, in particular those infecting humans including *P. vivax, P. falciparum, P. malariae, P. ovale, and P. knowlesi.*

The term "polypeptide" is used to designate for example an "antigen" or a "protein" or "antigenic fragment" and defines a molecule resulting from a concatenation of amino acid residues. In particular, the polypeptides disclosed in the application originate from a pathogen and are antigens, proteins, structural proteins, or antigenic fragments thereof, that may be identical to native proteins or alternatively that may be derived thereof by mutation, including by substitution (in particular by conservative amino acid residues) or by addition of amino acid residues or by secondary modification after translation or by deletion of portions of the native proteins(s) resulting in fragments having a shortened size with respect to the native protein of reference. Fragments are encompassed within the present invention to the extent that they bear epitopes of the native protein suitable for the elicitation of an immune response in a host in particular in a human host, including a child host, preferably a response that enables the protection against an infection from a pathogen or against a pathogen-associated disease. Epitopes are in particular of the type of T epitopes involved in elicitation of Cell Mediated Immune response (CMI response). T epitopes are involved in the stimulation of T cells through presentation of some parts of the T-cell epitope which can bind on MHC class II molecules, leading to the activation of T cells. Epitopes may alternatively be of type B, involved in the activation of the production of antibodies in a host to whom the protein has been administered or in whom it is expressed following administration of the infectious replicative particles of the invention. Fragments may have a size representing more than 50% of the amino-acid sequence size of the native protein of the pathogen wild type strain, preferably at least 90% or at least 95%. Polypeptide may have at least 50% identity with the native protein of the pathogen wild type strain, preferably at least 60%, preferably at least 70%, preferably at least 85%, at least 95%, at least 98% or at least 99% when comparison is carried out after alignment of the fragment or the modified amino acid sequences of the polypeptide with the corresponding region of the native protein.

### • Nucleic acid constructs with multiple Additional Transcription Units (ATUs)

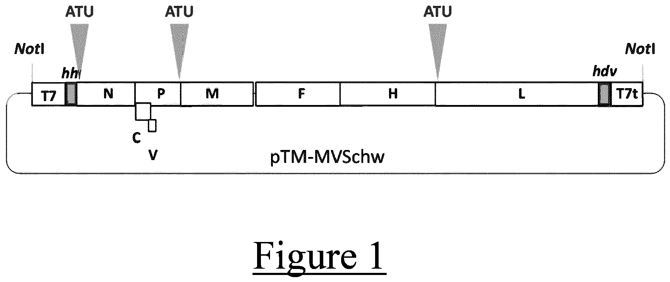

In a first aspect the invention provides a nucleic acid construct which comprises a cDNA molecule encoding a full length antigenomic (+) RNA strand of a non-segmented negative-sense single-stranded RNA virus, especially of a measles virus (MV), wherein the cDNA molecule further comprises inserted therein, at least a first additional transcription unit (ATU) and a second additional transcription unit (ATU), wherein the at least first and second additional transcription units (ATUs) are localized within a single intergenic region of the cDNA encoding the antigenomic (+) RNA strand as a single expression cassette, and wherein each ATU comprises a heterologous polynucleotide operably inserted within the ATU allowing the expression of a heterologous polypeptide encoded by the heterologous polynucleotide. Nucleic acid constructs according to this aspect of the invention are illustrated in Figures 1 to 8 and 9 to 13 for the measles virus.

According to a particular embodiment of the invention, Additional Transcription Units (ATU) comprises cis-acting sequences encompassing a promoter sequence and a terminator sequence originating from intergenic regions of measles virus genome inserted respectively upstream and downstream of additional heterologous sequences and allowing their expression by the measles RNA-dependent RNA polymerase. These cis-acting sequences can be selected from any intergenic sequence of measles virus genome: N-P, P-M, M-F, F-H, H-L. For the purpose of the description of the invention and depending on the context of the disclosure, the term "ATU" is used to designate the herein defined cis-acting sequences and is also used to designate the nucleic acid sequence framed by said cis-acting sequences and obtained after inserting the polynucleotide(s) that encodes the heterologous polypeptide(s).

The nucleic acid construct of the invention is in particular a purified DNA molecule, obtained or obtainable by recombination of at least one polynucleotide of a non-segmented negative-sense single-stranded RNA virus, especially of MV and at least two heterologous polynucleotides of a pathogen, especially a pathogen infecting human, operably cloned or linked together. Accordingly, in an embodiment of the invention, the nucleic acid construct is a recombinant nucleic acid construct.

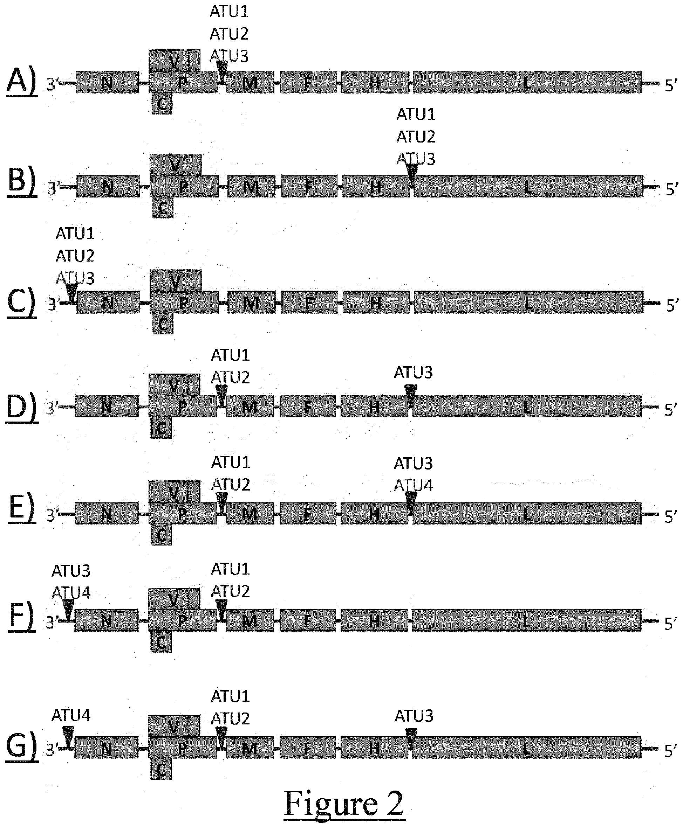

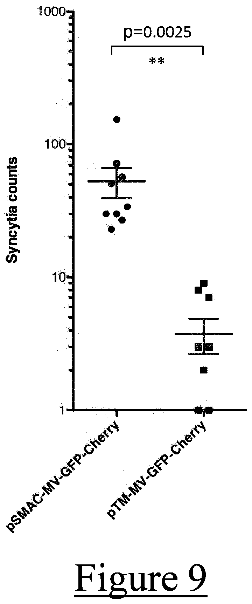

According to the invention, the nucleic acid construct is prepared by cloning two polynucleotides, or several polynucleotides, e.g. more than two polynucleotides, each encoding at least one polypeptide e.g. a protein, an antigen, an antigenic fragment thereof, issued from a pathogen, in the cDNA encoding the full-length antigenomic (+) RNA of the virus, especially of the measles virus, said cloning enabling each ATU to contain at least one coding heterologous polynucleotide. As an example, the genome of a measles virus with ATUs is illustrated on Fig.2 while several nucleic acid constructs according to the invention are illustrated on Fig.1, 3-8 and on Fig. 9-13, and Fig. 18 for the measles virus. Alternatively, a nucleic acid construct of the invention may be prepared using steps of synthesis of nucleic acid fragments or polymerization from a template, including by PCR. The polynucleotide(s) and nucleic acid construct of the invention may be prepared in accordance with any known method in the art and in particular may be cloned, obtained by polymerization especially using PCR methods or may be synthesized.

As illustrated on Figure 2 for the measles virus, different constructions are encompassed within the definition of the invention, wherein within a single intergenic region of the cDNA for the full length antigenomic (+) RNA strand of a non-segmented negative-sense single-stranded RNA genome of a measles virus, two ATUs may be provided between the P gene and the M gene, or three ATUs may be localized between the P gene and the M gene of the measles virus in the cDNA molecule(as illustrated on the Figure 2A); two ATUs may be in the intergenic region localized between the H gene and the L gene of the measles virus in the cDNA molecule; three ATUs may be in the intergenic region localized between the H gene and the L gene of the measles virus in the cDNA molecule (as illustrated on the Figure 2B); two ATUs may be in the intergenic region localized upward the N gene of the measles virus in the cDNA molecule; three ATUs may be localized in the intergenic region upward the N gene of the measles virus in the cDNA molecule (as illustrated on the Figure 2C). In another embodiment of a single construct, ATUs may also be localized in different intergenic regions of the measles virus in the cDNA molecule; for example, two ATUs may be localized in the intergenic region localized between the P gene and the M gene of the measles virus in the cDNA molecule and one ATU may be localized in the intergenic region localized between the H gene and the L gene of the measles virus in the cDNA molecule (illustrated on Figure 2D); two ATUs may be localized in the intergenic region localized between the P gene and the M gene of the measles virus in the cDNA molecule and two ATUs may be localized in the intergenic region localized between the H gene and the L gene of the measles virus in the cDNA molecule (illustrated on Figure 2E); two ATUs may be localized in the intergenic region localized between the P gene and the M gene of the measles virus in the cDNA molecule and two ATUs may be localized in the intergenic region localized upward the N gene of the measles virus in the cDNA molecule (illustrated on Figure 2F); two ATUs may be localized in the intergenic region localized between the P gene and the M gene of the measles virus in the cDNA molecule and one ATU may be localized in the intergenic region localized upward the N gene of the measles virus in the cDNA molecule, and one ATU may be localized in the intergenic region between the H gene and the L gene of the measles virus in the cDNA molecule (illustrated on Figure 2G). These embodiments are only illustrative of the invention. Several other embodiments wherein a different number of ATU regions, and/or different localization of ATUs, are encompassed within the present definition of the invention, provided that at least two ATUs are localized within the same intergenic region of the full length antigenomic (+) RNA strand of a non-segmented negative-sense single-stranded RNA virus and each contains (or each is suitable for containing) a heterologous polynucleotide encoding a heterologous polypeptide issued from a pathogen.

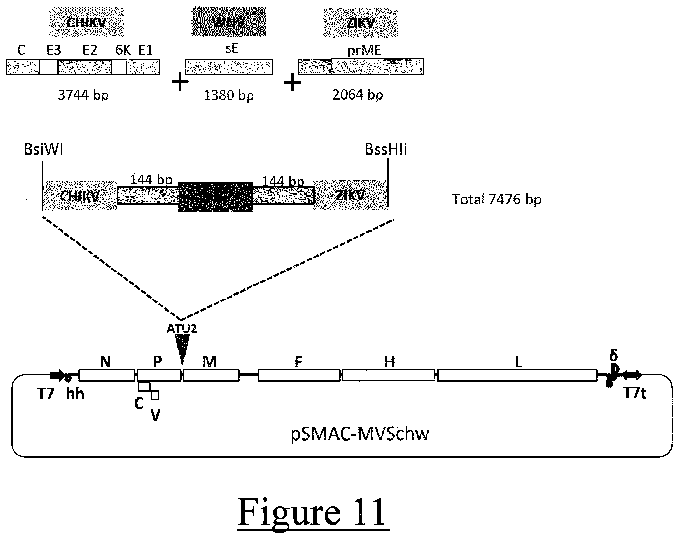

As shown on Fig 2, a plurality of ATUs may be inserted in a single intergenic region of the nucleic acid construct, allowing the insertion within each single ATU of at least one heterologous polynucleotide encoding at least one heterologous polypeptide, thereby allowing the insertion of either one or multiple polynucleotides issued from a single pathogen, or a plurality of heterologous polynucleotides issued from different pathogens. On figure 11, antigens originating from the west Nile virus (WNV), the Chikungunya virus (CHIKV) and the Zika Virus (ZIKV) are each encoded by a polynucleotide inserted within its proper ATU localized within the same single intergenic region of the MV genome, i.e., between the P gene and the M gene of the MV genome in the illustrated example.

In a particular embodiment of the invention, illustrated on Fig. 2, a first ATU and a second ATU are localized: (i) between the P gene and the M gene of the measles virus in the cDNA molecule or (ii) between the H gene and the L gene of the measles virus in the cDNA molecule or (iii) between the N gene of the measles virus and the T7RNA polymerase promoter in the cDNA molecule.

An Additional Transcription Unit according to the invention is hence a DNA unit suitable to be cloned (or inserted) or cloned (or inserted) within a cDNA molecule encoding a full length antigenomic (+) RNA strand of a non-segmented negative-sense single-stranded RNA virus, especially of a measles virus, preferably in an intergenic region of the virus genome. The ATU encodes a heterologous polypeptide as defined herein and contains both a coding sequence, which will be translated into the polypeptide, and regulatory sequence(s), which direct and regulate the synthesis of that polypeptide. ATU may furthermore comprise one, two or several restriction sites to ease the insertion of different polynucleotides in a suitable manner for expressing a polypeptide encoded by each inserted polynucleotide. In a particular embodiment of the invention, the ATUs contain cis-acting sequences necessary for the transcription of the P gene of the measles virus.

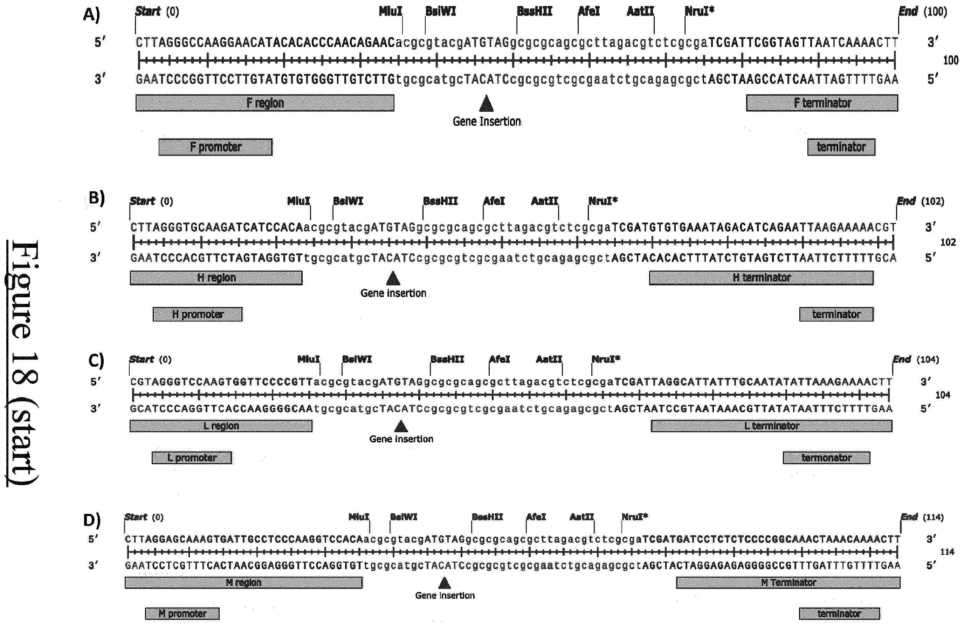

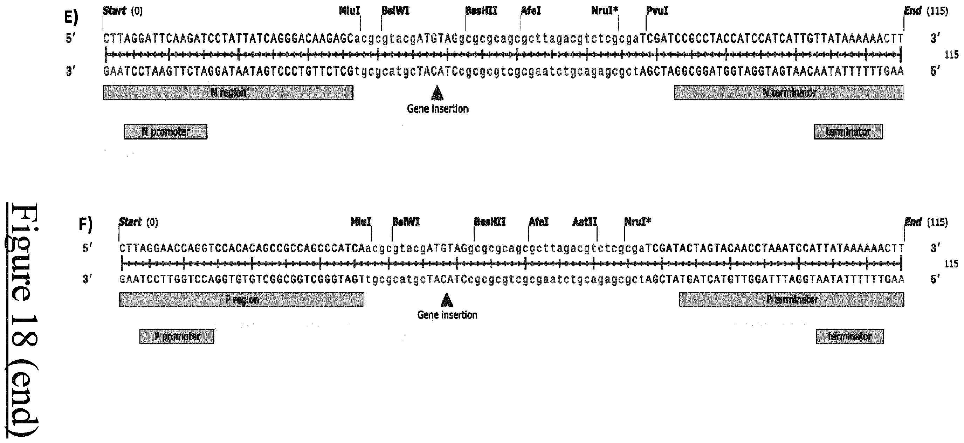

An ATU generally comprises two CTT codons corresponding respectively to the start and stop codons of the virus polymerase. ATUs may further comprise ATG START codon and STOP codon(s), the STOP codon(s) being selected from TAA, TAG or TGA codons, and is in particular TAG codon, corresponding respectively to the start and stop codons for translation of the heterologous polynucleotide comprised within the ATU. In a particular embodiment, ATG and TAG codons corresponding respectively to the start and stop codons for translation of the heterologous polynucleotide cloned within the ATU are part of the ATU. An ATU may also comprise at least one of the following elements: a TATA box, more particularly of sequence "TTATAAAAAA", at least one Start/Stop codon, in particular a "CTT" codon, more particularly two "CTT" codons; at least one "CTT" codon may directly follow the TATA box in the 3' or 5' direction, a promoter (for example a promoter issued from a RNA virus, especially of a measles virus (MV), more particularly the promoter of the P gene of the MV, or the promoter of the F gene of the MV, or the promoter of the H gene of the MV, or the promoter of the L gene of the MV, or the promoter of the M gene of the MV, or the promoter of the N gene of the MV,), more particularly directly following the Start/Stop codon in the 3' direction, of the cDNA molecule encoding a full length antigenomic (+) RNA strand of a non-segmented negative-sense single-stranded RNA virus, especially of the MV wherein an ORF is inserted that encodes a heterologous polypeptide. In a preferred embodiment of the invention, an ATU comprises a CTT codon, a promoter sequence, at least one restriction site, a heterologous polynucleotide encoding a heterologous polypeptide (and comprising a START codon and at least one STOP codon), at least one restriction site, a terminator sequence (which could be a TATA box as defined here above), and a CTT codon; in particular in this order from the 5' end towards the 3' end of the ATU. Several illustrative embodiments are represented in Fig. 18.

In an embodiment of the invention, an ATU comprises from its 5' end to its 3' end:
- a promoter sequence originating from intergenic regions of measles virus genome,
- a heterologous polynucleotide encoding at least one heterologous polypeptide as defined herein, wherein the heterologous polynucleotide is in particular inserted in a multiple cloning site, especially a multiple cloning site containing restriction sites not found in the MV cDNA or rare ly found therein;
- a terminator sequence,
wherein the promoter and terminator sequences are inserted respectively upstream and downstream of additional heterologous sequences in a way to allow their expression by the measles RNA-dependent RNA polymerase and wherein the promoter and terminator sequences can be selected from the functional sequences in any intergenic sequence of measles virus genome: N-P, P-M, M-F, F-H, H-L

In a particular embodiment of the invention, the 5' extremity of an ATU (i.e. the extremity upward the heterologous polynucleotide encoding the heterologous polypeptide) comprises a promoter sequence selected from:
∘ SEQ ID No. 40 (promoter of the gene N of MV); or
∘ SEQ ID No. 41 (promoter of the gene P of MV); or
∘ SEQ ID No. 42 (promoter of the gene M of MV); or
∘ SEQ ID No. 43 (promoter of the gene F of MV); or
∘ SEQ ID No. 44 (promoter of the gene H of MV); or
∘ SEQ ID No. 45 (promoter of the gene L of MV).

In a particular embodiment of the invention, the 3' extremity of an ATU (i.e. the extremity downward the heterologous polynucleotide encoding the heterologous polypeptide) comprises a terminator sequence selected from:
∘ SEQ ID No. 46 (terminator of the N gene of MV); or
∘ SEQ ID No. 47 (terminator of the P gene of MV); or
∘ SEQ ID No. 48 (terminator of the M gene of MV); or
∘ SEQ ID No. 49 (terminator of the F gene of MV); or
∘ SEQ ID No. 50 (terminator of the H gene of MV); or
∘ SEQ ID No. 51 (terminator of the L gene of MV).

In a particular embodiment of the invention, the 5' extremity of an ATU (i.e. the extremity upward the heterologous polynucleotide encoding the heterologous polypeptide) and the 3' extremity of an ATU (i.e. the extremity downward the heterologous polynucleotide encoding the heterologous polypeptide) respectively comprise:
∘ SEQ ID No. 40 (promoter of the N gene of MV) and SEQ ID No. 46 (terminator of the N gene of MV); or
∘ SEQ ID No. 41 (promoter of the P gene of MV) and SEQ ID No. 47 (terminator of the P gene of MV); or
∘ SEQ ID No. 42 (promoter of the M gene of MV) and SEQ ID No. 48 (terminator of the M gene of MV);or
∘ SEQ ID No. 43 (promoter of the F gene of MV) and SEQ ID No. 49 (terminator of the F gene of MV); or
∘ SEQ ID No. 44 (promoter of the H gene of MV) and SEQ ID No. 50 (terminator of the H gene of MV); or
∘ SEQ ID No. 45 (promoter of the L gene of MV) and SEQ ID No. 51 (terminator of the L gene of MV).

In a particular embodiment of the invention, an ATU comprises or consists in the sequence of:
∘ SEQ ID No. 52, wherein the n corresponds to the heterologous polynucleotide encoding a heterologous polypeptide; or
∘ SEQ ID No. 53, wherein the n corresponds to the heterologous polynucleotide encoding a heterologous polypeptide; or
∘ SEQ ID No. 54, wherein the n corresponds to the heterologous polynucleotide encoding a heterologous polypeptide; or
∘ SEQ ID No. 55, wherein the n corresponds to the heterologous polynucleotide encoding a heterologous polypeptide; or
∘ SEQ ID No. 56, wherein the n corresponds to the heterologous polynucleotide encoding a heterologous polypeptide; or
∘ SEQ ID No. 57, wherein the n corresponds to the heterologous polynucleotide encoding a heterologous polypeptide.

The heterologous sequence represented by "n" does not need a START codon, since the START codon is already present upward the "n' in the sequence SEQ ID No. 52, SEQ ID No. 53, SEQ ID No. 54, SEQ ID No. 55, SEQ ID No. 56, SEQ ID No. 57. A STOP codon may or may not be present in the heterologous polynucleotide represented by n since a STOP codon is already present in SEQ ID No. 52 downward the "n" in the sequence; an extra STOP codon may nonetheless be added, in particular for compliance with the rule of 6. The "n" features is not intended to provide indication relative to the number of nucleotides contained in the sequence of the heterologous polynucleotide but rather to indicate the location of the heterologous polynucleotide in the ATU sequence.

In a particular embodiment of the invention, an ATU comprises from its 5' end to its 3' end:
- A polynucleotide (5'ATU) comprising or consisting of (i) SEQ ID No. 16 (5'tataaaaaacttaggaaccaggtccacacagccgccagcccatcaacgcgtacgATG 3'); or (ii) SEQ ID No. 19 (5'tataaaaaacttaggaaccaggtccacacagccgccagcc
- catcaaccatccactcccacgattggaggccggccATG 3'), or (iii) SEQ ID No. 20 (5' tataaaaaacttaggaaccaggtccacacagccgccagcccatcaaccatccactcccacgattg gagcggccgcATG 3');
- A heterologous polynucleotide encoding at least one heterologous polypeptide as defined herein;
- A polynucleotide (3'ATU) comprising or consisting of SEQ ID No. 17 (5' (TAA)TAGgcgcgcagcgct**ta**gacgtctcgcgaTCGATACTAGTACAACCTAAAT CCATTATAAAAAACTTAGGAGCAAAGTGAT **3'**) (characters in bold and in lowercase are extra nucleotide residues added to respect the rule of 6, underlined residues correspond to restriction sites (for BssHII, Afel and Nrul).

The combination of these three polynucleotides forms an ATU, allowing the transcription and the expression of the heterologous polypeptide encoded by the heterologous polynucleotide. The polynucleotide of SEQ ID No. 16 comprises the TATA box, the CTT codon, the P promotor, a restriction site for BsiWI restriction enzyme, and a START codon, while the polynucleotide of SEQ ID No. 17 comprises a STOP codon, and a CTT codon. Additional nucleotides may be present within an ATU, preferentially not between the polynucleotide of SEQ ID No. 16 (or SEQ ID No. 19 or SEQ ID No. 20) and the heterologous polynucleotide and not between the heterologous polynucleotide and the polynucleotide of SEQ ID No. 17. An extra STOP codon TAA may be present towards the 5' end of SEQ ID No. 17, in particular for compliance with the rule of 6. Additional nucleotides may be present at the 5' end and/or at the 3' end of the ATU. Such additional nucleotides may for example be restriction sites or regulation sequences, like but not limited to silencers, enhancers, promoters, terminators.

In a particular embodiment, the nucleic acid construct of the invention comprises a cDNA molecule encoding a full length antigenomic (+) RNA strand of a non-segmented negative-sense single-stranded RNA virus, especially of a measles virus (MV), wherein the cDNA molecule comprises a first ATU and a second ATU localized within a same intergenic region of the cDNA of the virus, wherein the first ATU and the second ATU each comprise or consist from their 5' end towards their 3' end of (i) the nucleotide sequence of SEQ ID No: 16 or SEQ ID No. 19 or SEQ ID No. 20 or SEQ ID No. 40 or SEQ ID No. 41 or SEQ ID No. 42 or SEQ ID No. 43 or SEQ ID No. 44 or SEQ ID No. 45, (ii) a heterologous polynucleotide sequence encoding a heterologous polypeptide, and (iii) the nucleotide sequence of SEQ ID No. 17 or SEQ ID No. 46 or SEQ ID No. 47 or SEQ ID No. 48 or SEQ ID No. 49 or SEQ ID No. 50 or SEQ ID No. 51, wherein the heterologous polynucleotides present within the first ATU and the second ATU are different. The intergenic region comprising the ATUs may in particular be any of those cited herein in respect of the full length antigenomic (+) RNA strand of MV.

In a particular embodiment, the nucleic acid construct of the invention comprises a cDNA molecule encoding a full length antigenomic (+) RNA strand of a non-segmented negative-sense single-stranded RNA virus, especially of a measles virus (MV), wherein the cDNA molecule comprises a first ATU and a second ATU localized within a same intergenic region of the cDNA of the virus, wherein the first ATU and the second ATU each comprise or consist from their 5' end towards their 3' end of (i) the nucleotide sequence of SEQ ID No. 52, SEQ ID No. 53, SEQ ID No. 54, SEQ ID No. 55, SEQ ID No. 56, SEQ ID No. 57, wherein n corresponds to a heterologous polynucleotide sequence encoding a heterologous polypeptide, and, wherein the heterologous polynucleotides present within the first ATU and the second ATU are different. The intergenic region comprising the ATUs may in particular be any of those cited herein in respect of the full length antigenomic (+) RNA strand of MV.

In a particular embodiment of the invention, an ATU is a polynucleotide comprised within SEQ ID No: 1. SEQ ID No: 1 comprises an ATU sequence suitable for cloning within the cDNA molecule encoding a full-length antigenomic (+) RNA strand of a non-segmented negative-sense single-stranded RNA MV virus, wherein a heterologous polynucleotide sequence encoding a heterologous polypeptide issued or derived from a pathogen is inserted between positions 79 and 796 included (*i.e*. comprising the initiation codon ATG present at the 3' end of the sequence SEQ ID No. 16, SEQ ID No. 19 and SEQ ID No. 20, a stop codon TAA is present at positions 794 to 796 of SEQ ID No: 1 and corresponds to the native stop codon of the polynucleotide sequence; another stop codon TAG is also present at positions 797 to 799 of SEQ ID No:1 which corresponds to the STOP codon present in SEQ ID No. 17) or between positions 82 and 796 included (i.e. without the start codon ATG present at the 3' end of the sequence SEQ ID No. 16, SEQ ID No. 19 and SEQ ID No. 20). TATA box and CTT codons encompassing respectively the start and stop codons of the polymerase are in bold. ATG and TAG codons corresponding to the start and stop codons for translation of the heterologous polynucleotide cloned within the ATU are underlined.

ctagcctaccctccatcattgt**tataaaaaactt**aggaaccaggtccacacagccgccagcccatcaacgcg tacg**ATG***TAATAGgcgcgcagcgcttagacgtctcgcgaTCGATACTAGTACAACCTAA ATCCATTATAAAAAA**CTT**AGGAGCAAAGTGAT **(SEQ ID No.16** * **SEQ ID No.17).** The ATG codon in bold corresponds to the start codon ATG of the heterologous polypeptide and is provided at the 3' end of sequence SEQ ID No. 16 in the present example. This start codon ATG corresponds to the first codon of the coding sequence of the heterologous polypeptide. Underlined codons are STOP codons, characters in lowercase correspond to the restriction sites, CTT codons are in bold and underlined.

The * sign in the sequence corresponds to the location of the insert of a heterologous polynucleotide. In the polynucleotide of SEQ ID No.1, * is a sequence encoding Green Fluorescent Protein (GFP) (minus the START codon ATG localized at positions 79 to 81 of SEQ ID No: 1 and already represented here above in bold capital characters) and the STOP codons of the sequence localized at positions 794 to 796 of SEQ ID No: 1 and already represented here above in underlined capital characters) localized between the nucleotide at position 79 and the nucleotide at position 796. In the present invention, this sequence localized between the nucleotide at position 79 and the nucleotide localized at position 796 may be substituted by any heterologous polynucleotide encoding a heterologous polypeptide issued from a pathogen. In other words, the * sign may be substituted by any heterologous polynucleotide encoding a heterologous polypeptide, and an ATU may comprise or consist of the heterologous polynucleotide of SEQ ID No.1 wherein the nucleotide sequence from position 79 to position 796 is substituted by a heterologous polynucleotide sequence encoding a heterologous polypeptide, in particular wherein said heterologous polynucleotides in the first ATU and the second ATU encode heterologous polypeptides that are different from each other and optionally wherein the first and the second heterologous polynucleotides ATUs are separated in the single intergenic region of the cDNA by a spacer sequence such as the sequence of polynucleotide of SEQ ID No: 2 or SEQ ID No: 11; the spacer sequence consisting of the 3' end of the first ATU followed by the 5' end of the subsequent ATU (i.e. the second ATU). As an example, an ATU corresponding to this definition may be retrieved from the polynucleotide of SEQ ID No: 12 which corresponds to an expression vector wherein a heterologous polynucleotide encoding an immunogenic polypeptide of CHIKV is localized between nucleotide residues 3532 and 7275 on SEQ ID No: 12. This heterologous polynucleotide, encoding an immunogenic polypeptide of Chikungunya virus takes place of the * sign in the ATU defined here above and is framed towards its 5' end and its 3' end by the polynucleotide sequence illustrated here above.

Alternatively, an ATU according to the invention may correspond to a polynucleotide sequence wherein ATU localized within SEQ ID No. 1 may be substituted by one of the following ATU: 
tataaaaacttaggaaccaggtccacacagccgccagcccatcaaccatccactcccacgattggaggc
cggccATG*TAATAGgcgcgcagcgcttagacgtctcgcgaTCGATACTAGTACAACCTA
AATCCATTATAAAAAACTTAGGAGCAAAGTGAT (SEQ ID No.19 * SEQ ID
No.17) .
 tataaaaaacttaggaaccaggtccacacagccgccagcccatcaaccatccactcccacgattggagcgg ccgc**ATG***TAATAGgcgcgcagcgcttagacgtctcgcgaTCGATACTAGTACAACCTAA ATCCATTATAAAAAA**CTT**AGGAGCAAAGTGAT **(SEQ ID No.20** * **SEQ ID No.17).** Underlined codons are STOP codons, characters in lowercase correspond to the restriction sites, CTT codons are in bold and underlined.

The nucleotide residues next to the 5' end and the 3' end of each ATU may correspond to the same residues as the nucleotide residues next to the 5' end and the 3' end of the ATU localized within SEQ ID No. 1, or may differ, as illustrated in SEQ ID No. 13, wherein the nucleotide residues next to the 5' end and 3' end of each ATU present within SEQ ID No. 13 are not identical.

In another particular embodiment, within a single intergenic region of the full length antigenomic (+) RNA strand of a non-segmented negative-sense single-stranded RNA virus, especially of MV, comprising several heterologous polynucleotides inserted therein, the first heterologous polynucleotide encoding a first heterologous polypeptide and the second (and/or subsequent) heterologous polynucleotide encoding second (and/or subsequent) heterologous polypeptide may be separated by a polynucleotide of sequence SEQ ID No. 2 or SEQ ID No. 11.

The polynucleotides of SEQ ID No. 2 and SEQ ID No. 11 comprise from their 5' end towards their 3' end a STOP codon, a first CTT codon, a TATA box, a second CTT codon and a P promoter. They comprise elements found at the end of a first ATU and at the beginning of a subsequent ATU. The polynucleotide of SEQ ID No. 2 or of SEQ ID No. 11 may separate two heterologous polynucleotides each encoding a heterologous polypeptide and each pertaining to separate ATUs, as a result of insertion of the sequence of SEQ ID No.2 or 11. In other words, in the nucleic acid construct, and when two heterologous polynucleotides are localized within the same intergenic region of the cDNA of the virus, and when a polynucleotide of SEQ ID No.2 or of SEQ ID No. 11 is inserted between said two heterologous polynucleotides, the 5' end (at least the STOP codon and the first CTT codon) of the polynucleotide of SEQ ID No.2 or of SEQ ID No. 11 belongs to the first ATU, comprising the first heterologous polynucleotide while the 3' end (at least the TATA box, the second CTT codon and the P promoter) of such sequence belongs to the second ATU comprising the second heterologous polynucleotide.

In an embodiment of the invention, the first ATU and the second ATU, both localized within a same intergenic region of the cDNA of the virus, comprise or consist in the nucleotide sequence of SEQ ID No: 16 or SEQ ID No: 19 or SEQ ID No: 20, a heterologous polynucleotide encoding a heterologous polypeptide as defined here in (said sequence comprising a start codon ATG, since SEQ ID No: 16, SEQ ID No: 19 and SEQ ID No: 20 all comprise the start codon ATG at their 3' end), and the nucleotide sequence of SEQ Id No. 17; optionally wherein the first and the second ATUs are in part made of the sequence of polynucleotide of SEQ ID No: 2 or SEQ ID No: 11.

In particular, at least one region separating two heterologous polynucleotides inserted within an intergenic region of the full length antigenomic (+) RNA strand of a non-segmented negative-sense single-stranded RNA virus, especially of the MV, may be derived from the nucleotide sequence of SEQ ID No. 11, wherein each extremity of the sequence corresponds to particular restriction sites, suitable for insertion within a Bacterial Artificial Chromosome (also referenced "BAC" in the present description).

In a particular embodiment, the multiple heterologous polynucleotides (each encoding a heterologous polypeptide issued from a pathogen) inserted within the cDNA molecule encoding a full length antigenomic (+) RNA strand of a non-segmented negative-sense single-stranded RNA virus, especially of a measles virus (MV), are separated from each other by a polynucleotide comprising or consisting of the sequence of SEQ ID No. 2 or SEQ ID No. 11, or a sequence that is issued from SEQ ID No. 2 or SEQ ID No. 11, i.e. shares at least 90% identity, more preferably at least 91% identity, more preferably at least 92% identity, more preferably at least 93% identity, more preferably at least 94% identity, more preferably at least 95% identity, more preferably at least 96% identity, more preferably at least 97% identity more preferably at least 98% identity, more preferably at least 99% identity, and most preferentially 100% identity, with SEQ ID No. 2 or SEQ ID No. 11 and preferably has the same length as one of these sequences.

In a particular embodiment, each ATU presents within the cDNA molecule encoding a full length antigenomic (+) RNA strand of a non-segmented negative-sense single-stranded RNA virus, especially of a measles virus (MV), comprises or consists of (i) the sequence of SEQ ID No. 52, SEQ ID No. 53, SEQ ID No. 54, SEQ ID No. 55, SEQ ID No. 56, SEQ ID No. 57, wherein n represents a polynucleotide sequence encoding a heterologous polypeptide; or (ii) a sequence issued from SEQ ID No. 52, SEQ ID No. 53, SEQ ID No. 54, SEQ ID No. 55, SEQ ID No. 56, SEQ ID No. 57, i.e. shares at least 90% identity, more preferably at least 91% identity, more preferably at least 92% identity, more preferably at least 93% identity, more preferably at least 94% identity, more preferably at least 95% identity, more preferably at least 96% identity, more preferably at least 97% identity more preferably at least 98% identity, more preferably at least 99% identity, and most preferentially 100% identity, with SEQ ID No. 52, SEQ ID No. 53, SEQ ID No. 54, SEQ ID No. 55, SEQ ID No. 56, and SEQ ID No. 57, and preferably has the same length as one of these sequences.

In an embodiment of the invention, it is provided a nucleic acid construct wherein a third ATU is localized within the same intergenic region where the first ATU and the second ATU are localized, the third ATU comprising a heterologous polynucleotide operably linked within the third ATU allowing the expression of a heterologous polypeptide encoded by the heterologous polynucleotide wherein said heterologous polypeptide of the third ATU is different from the heterologous polypeptides encoded by the first and the second ATUs, and wherein the heterologous polynucleotides within a single intergenic region of the cDNA are separated from each other by a sequence comprising the polynucleotide of SEQ ID No. 2 or SEQ ID No. 11.

In a particular embodiment, the nucleic acid construct of the invention comprises a cDNA molecule encoding a full length antigenomic (+) RNA strand of a non-segmented negative-sense single-stranded RNA virus, especially of a measles virus (MV), wherein the cDNA molecule further comprises inserted therein a third ATU, wherein the third ATU (i) comprises or consists in the nucleotide sequence of SEQ ID No: 1 wherein the nucleotide sequence from position 79 to position 796 is substituted by a heterologous polynucleotide sequence encoding a selected heterologous polypeptide (said sequence comprising an start codon ATG, since positions 79 to 81 of SEQ ID No: 1 are respectively A, T and G); in particular the third ATU comprises of consists in the same nucleotide sequence as the first ATU or the second ATU but for the coding heterologous polynucleotide that is contains, and (ii) is separated from another closest ATU in the single intergenic region of the cDNA by the polynucleotide of sequence SEQ ID No.: 2 or SEQ ID No.: 11.

In a particular embodiment, the nucleic acid construct of the invention comprises a cDNA molecule encoding a full length antigenomic (+) RNA strand of a non-segmented negative-sense single-stranded RNA virus, especially of a measles virus (MV), wherein the cDNA molecule further comprises inserted therein a third ATU, wherein the third ATU (i) comprises or consists from its 5' end towards its 3' end in the nucleotide sequence of SEQ ID No: 16 or SEQ ID No: 19 or SEQ ID No: 20 SEQ ID No. 20 or SEQ ID No. 40 or SEQ ID No. 41 or SEQ ID No. 42 or SEQ ID No. 43 or SEQ ID No. 44 or SEQ ID No. 45, heterologous polynucleotide sequence encoding a heterologous polypeptide (without the start codon ATG, since SEQ ID No: 16, SEQ ID No: 19, SEQ ID No: 20, SEQ ID No. 20, SEQ ID No. 40, SEQ ID No. 41, SEQ ID No. 42, SEQ ID No. 43, SEQ ID No. 44 and SEQ ID No. 45 all comprise the start codon ATG at their 3' end), and the nucleotide sequence of SEQ ID No. 17, or SEQ ID No. 46 or SEQ ID No. 47 or SEQ ID No. 48 or SEQ ID No. 49 or SEQ ID No. 50 or SEQ ID No. 51, in particular the third ATU comprises of consists in the same nucleotide sequence as the first ATU or the second ATU but for the coding heterologous polynucleotide that is contains, and (ii) is separated from another closest ATU in the single intergenic region of the cDNA by the polynucleotide of sequence SEQ ID No.: 2 or SEQ ID No.: 11

The nucleic acid construct according to the invention comprises ATUs (a first ATU and a second ATU and when present a third ATU) with a heterologous polynucleotide that encodes a heterologous polypeptide that is different from the heterologous polypeptide encoded by the other ATUs. In other words, each heterologous polynucleotide present within the nucleic acid construct differs from the other heterologous polynucleotide(s) inserted within the same nucleic acid construct, and hence each ATU encodes a particular polypeptide different from the polypeptide(s) encoded by other ATUs. In a more particular embodiment of the invention, the nucleic acid construct of the invention comprises a plurality of heterologous polynucleotides, each inserted within an ATU, each heterologous polynucleotide encoding a heterologous polypeptide different from another heterologous polypeptide encoded by another heterologous polynucleotide.

In an embodiment of the invention, the nucleic acid construct may comprise an additional ATU(s) localized in a different intergenic region of the cDNA molecule encoding a full length antigenomic (+) RNA strand of a non-segmented negative-sense single-stranded RNA virus, especially of a measles virus (MV). Such an embodiment is for example illustrated for a measles virus on Fig. 2D, 2E, 2F and 2G wherein at least one additional ATU is present within the nucleic acid construct in a different intergenic region that the first ATU and the second ATU. In a particular embodiment, the additional ATU comprises or consists in the nucleotide sequence of SEQ ID No: 1 wherein the nucleotide sequence from position 79 to position 796 is substituted by a heterologous polynucleotide sequence encoding at least one heterologous polypeptide (said sequence comprising an start codon ATG, since positions 79 to 81 of SEQ ID No: 1 are respectively A, T and G). In a particular embodiment, the nucleic acid construct of the invention comprises a cDNA molecule encoding a full length antigenomic (+) RNA strand of a non-segmented negative-sense single-stranded RNA virus, especially of a measles virus (MV), wherein the cDNA molecule further comprises inserted therein an additional ATU localized within a different intergenic region than the first and the second ATUs, wherein the additional ATU (i) comprises or consists from its 5' end towards its 3' end in the nucleotide sequence of SEQ ID No: 16 or SEQ ID No. 19 or SEQ ID No. 20 or SEQ ID No. 40 or SEQ ID No. 41 or SEQ ID No. 42 or SEQ ID No. 43 or SEQ ID No. 44 or SEQ ID No. 45, heterologous polynucleotide sequence encoding a heterologous polypeptide (without the start codon ATG which is provided by the the nucleotide sequence of SEQ ID No: 16 or SEQ ID No. 19 or SEQ ID No. 20 or SEQ ID No. 40 or SEQ ID No. 41 or SEQ ID No. 42 or SEQ ID No. 43 or SEQ ID No. 44 or SEQ ID No. 45), and the nucleotide sequence of SEQ ID No. 17 or SEQ ID No. 46 or SEQ ID No. 47 or SEQ ID No. 48 or SEQ ID No. 49 or SEQ ID No. 50 or SEQ ID No. 51, in particular the additional ATU comprises of consists in the same nucleotide sequence as the first ATU or the second ATU but for the coding heterologous polynucleotide that is contains.
- Heterologous polynucleotide inserted within the nucleic acid construct of the invention and encoded heterologous polypeptide

Each heterologous polynucleotide localized within an ATU encodes one heterologous polypeptide or a plurality of heterologous polypeptides issued or derived from one or several pathogens, preferably from one pathogen. In a particular embodiment, each ATU comprises a heterologous polynucleotide issued from a pathogen which is a different pathogen from the one(s) providing the other heterologous polynucleotides inserted in another ATU. Heterologous polypeptides may also comprise different antigens issued from a single pathogen, or may comprise the same antigen repeated several times in a single ATU. Alternatively, for example when the production of a fusion protein comprising antigens issued from a single species or form a plurality of different species may lead to a proper immune response against the antigens present in the fusion protein, the heterologous polynucleotide encoding at least one heterologous polypeptide issued or derived from a single pathogen may result from the fusion of several polynucleotides, each encoding a particular heterologous polypeptide, e.g., a particular protein, antigen or an antigenic fragment thereof, of a pathogen. For example, the heterologous polynucleotide may result from the fusion of heterologous polynucleotides each encoding a single heterologous polypeptide of an antigen of a pathogen and such fusion within the nucleic acid construct may be achieved by a linker sequence, in particular a linker sequence corresponding to an intergenic region, or as a result of cloning or insertion in a particular ATU sequence(s). A linker sequence is well known in the art and can be a short nucleotide sequence comprising or consisting in a regulatory sequence of the measles virus.

Accordingly, within each ATU comprising a heterologous polynucleotide, the heterologous polynucleotide may encode a single heterologous polypeptide, two different heterologous polypeptides, two identical heterologous polypeptides, three different heterologous polypeptides, two identical heterologous polypeptides and another heterologous polypeptide, four different heterologous polypeptides, two identical heterologous polypeptides and two others identical heterologous polypeptides, two identical heterologous polypeptides and two others different heterologous polypeptides, three identical heterologous polypeptides and another different heterologous polypeptide. Any one of these heterologous polynucleotides encoding at least two (identical or different) heterologous polypeptides may be issued from the fusion of several heterologous polynucleotides, or prepared using steps of synthesis of nucleic acid fragments or polymerization from a template, including by PCR. Alternatively, any one of these heterologous polynucleotides may be a cDNA issued from the genomic RNA of at least one pathogen, after retrotranscription, said cDNA being either the full genomic cDNA or a fragment thereof, and encoding a polypeptide of the pathogen. The heterologous polynucleotides contained in the at least one ATUs enable expression of heterologous polypeptides as functional separate polypeptides rather than fusion polypeptides.

In an embodiment, the heterologous polypeptides encoded by the heterologous polynucleotides inserted within the ATUs are immunogenic polypeptides originating from or derived from at least one pathogen as defined herein, in particular pathogens infecting human, in particular from at least one of the following viruses: measles virus, Chikungunya virus, Zika Virus, West Nile Virus, Dengue virus, SARS, MERS, coronavirus, in particular SARS-Cov-2, Influenza virus, Ebola virus, Nipas virus, HIV or from a non-viral pathogen such as a parasite including *Mycobacterium tuberculosis* and Plasmodium species causing malaria, in particular those infecting humans :*P. vivax, P. falciparum, P. malariae, P. ovale, and P. know*/*esi;* in a preferred embodiment, at least one heterologous polypeptide is issued from one of the following viruses: the Chikungunya virus, the West Nile virus, the Zika virus, the SARS virus, the coronavirus, in particular SARS-CoV-2, and/or the HIV. In particular, the pathogen from which the heterologous polypeptide encoded within the genome of the cDNA molecule encoding a full length antigenomic (+) RNA strand of a non-segmented negative-sense single-stranded RNA virus (especially MV) of the invention is issued is a virus, in particular a virus infecting human, especially a virus selected from the list consisting of Chikungunya virus, the West Nile virus, the Zika virus, the SARS virus, the coronavirus, in particular SARS-CoV-2, and/or the HIV. A heterologous polypeptide encoded by the recombinant infectious virus expressed from the nucleic acid construct of the invention may also be the C, E2 and E1 proteins of the Chikungunya virus, the sE protein of the West Nile virus, the prME protein of the Zika virus, the S and M proteins of the SARS (Severe acute respiratory syndrome caused by the SARS Coronavirus), the S and M proteins of the MERS virus (Middle East respiratory syndrome caused by the MERS-Coronavirus) and/or the S and M proteins of the SARS-Cov-2 coronavirus, responsible for the Covid-19 and optionally wherein the heterologous polynucleotides originate or derive from different virus types.

In an alternative embodiment of the invention, a heterologous polypeptide encoded by a heterologous polynucleotide may be a fusion protein as defined in the present description, in particular when the heterologous polypeptide comprises several antigens issued or derived from a single species. In a preferred embodiment of the invention, each heterologous polynucleotide contained in the ATUs enable expression of a heterologous polypeptide comprising antigens issued or derived from a single species.

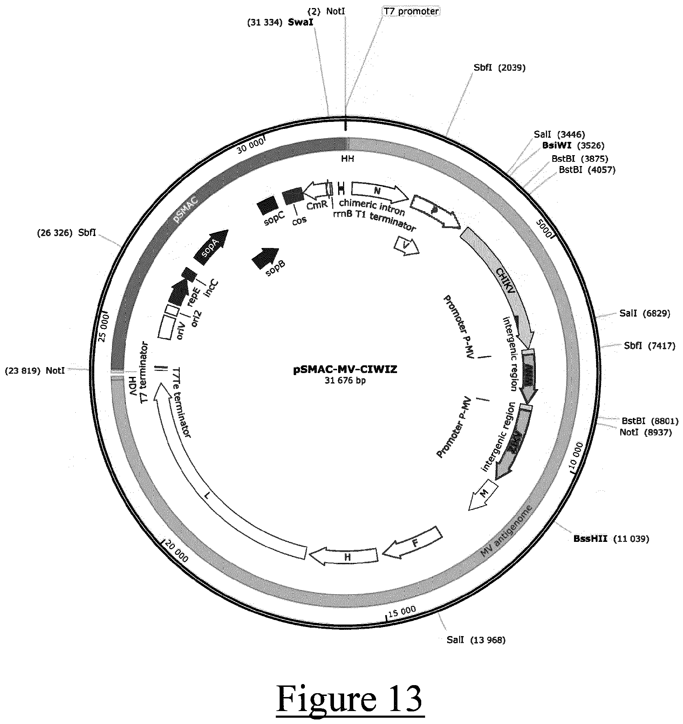

An example is illustrated on Figure 11. The amino acid sequences of SEQ ID No.: 13, SEQ ID No.: 14 and SEQ ID No.: 15 are issued from respectively the C, E2 and E1 proteins of the Chikungunya virus (SEQ ID No.: 13), the sE protein of the West Nile virus (SEQ ID No.: 14), and/or the prME protein of the Zika virus (SEQ ID No.: 15) and are encoded by the polynucleotide localized between the nucleotide at position 3532 and the nucleotide at position 11037 in SEQ ID No. 12. Such a nucleic acid construct is furthermore illustrated on Fig. 13 in combination with a BAC plasmid. The present invention encompasses other constructs, wherein the heterologous polynucleotides localized between positions 3532 and 11037 of the polynucleotide of sequence SEQ ID No.: 12 comprises another heterologous polynucleotide encoding other immunogenic heterologous polypeptides as defined here above.

More particularly, the heterologous polypeptide encoding the C, E2 and E1 proteins of the Chikungunya virus may comprise or consist of the heterologous polypeptide of SEQ ID No. 13.

More particularly, the heterologous polypeptide encoding the sE protein of the West Nile virus may comprise or consist of the heterologous polypeptide of SEQ ID No. 14.

More particularly, the heterologous polypeptide encoding the prME protein of the Zika virus may comprise or consist of the heterologous polypeptide of SEQ ID No. 15.

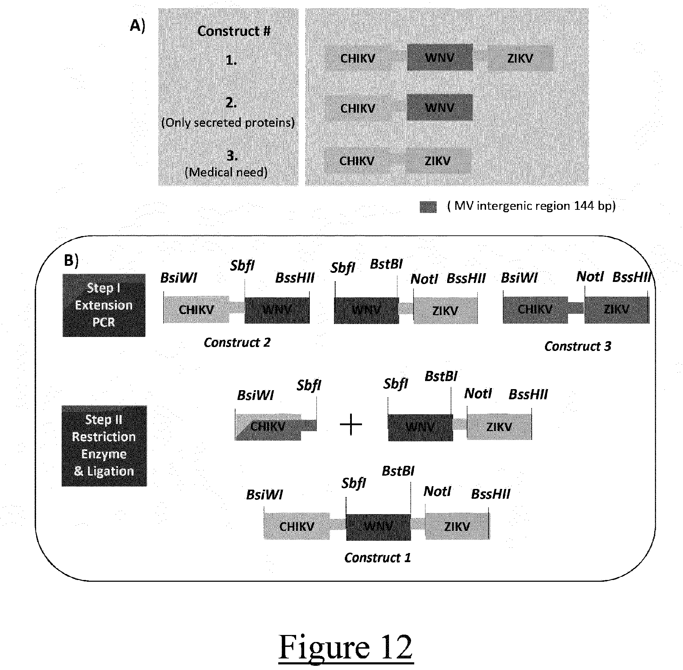

In an embodiment of the invention, illustrated on Fig. 12, the heterologous polypeptides encoded by the heterologous polynucleotides inserted within the ATUs are immunogenic heterologous polypeptides of amino acid sequence of SEQ ID No. 13 for C, E2 and E1 proteins of the Chikungunya virus in one ATU, SEQ ID No. 14 for the sE protein of the West Nile virus in another ATU, SEQ No. 15 for the prME protein of the Zika virus in another ATU. Each heterologous polynucleotides encoding a particular immunogenic polypeptide may be separated from the other heterologous polynucleotide(s) by a sequence comprising or consisting of the polynucleotide of SEQ ID No.: 2 or SEQ ID No.: 11.

In a particular embodiment of the invention, the nucleic acid construct of the invention comprises the polynucleotide of SEQ ID No. 4, which comprises within three distinct ATUs the immunogenic polypeptides C, E2 and E1 proteins of the Chikungunya virus in the first ATU, the sE protein of the West Nile virus in the second ATU and the prME protein of the Zika virus in the third ATU. The polynucleotide of SEQ ID No. 4 may be inserted within any construct disclosed in the present description, in particular within the MV genome of the Schwarz strain, the Zagreb strain, the AIK-C strain, the Moraten strain, the Philips strain, the Beckenham 4A strain, the Beckenham 16 strain, the Edmonston seed A strain, the Edmonston seed B strain, the CAM-70 strain, the TD 97 strain, the Leningrad-16 strain, the Shanghai 191 strain and the Belgrade strain, preferably the Schwarz strain, the AIK-C strain and the Zagreb strain, in particular the Schwarz strain of SEQ ID No. 62, the AIK-C strain of SEQ ID No. 58 and the Zagreb strain of SEQ ID No. 59. The polynucleotide of SEQ ID No. 4 may be inserted within any intergenic region of the MV genome, more particularly (i) between the P gene and the M gene of the measles virus in the cDNA molecule or (ii) between the H gene and the L gene of the measles virus in the cDNA molecule or (iii) between the N gene of the measles virus and the T7RNA polymerase promoter in the cDNA molecule. In a particular embodiment of the invention, the nucleic acid construct of the invention comprises within the intergenic region localized between the P gene and the M gene of the measles virus a heterologous polynucleotide comprising of consisting of the polynucleotide of SEQ ID No. 4.

These nucleic acid constructs comprise encoded in three distinct ATUs the immunogenic polypeptides C, E2 and E1 proteins of the Chikungunya virus in the first ATU, the sE protein of the West Nile virus in the second ATU and the prME protein of the Zika virus in the third ATU, the first and the second polynucleotides encoding respectively the immunogenic polypeptides C, E2 and E1 proteins of the Chikungunya virus and the sE protein of the West Nile virus being separated from each other by a polynucleotide of SEQ ID No. 2 or SEQ ID No. 11, and the second and the third polynucleotides respectively encoding sE protein of the West Nile virus and the prME protein of the Zika virus being separated from each other by a polynucleotide of SEQ ID No. 2 or SEQ ID No. 11. In particular the ATU(s) is (are) built as the sequence of SEQ ID No.16 (or SEQ ID No: 19 or SEQ ID No: 20 or SEQ ID No. 40 or SEQ ID No. 41 or SEQ ID No. 42 or SEQ ID No. 43 or SEQ ID No. 44 or SEQ ID No. 45) - * - SEQ ID No.17 or SEQ ID No. 46 or SEQ ID No. 47 or SEQ ID No. 48 or SEQ ID No. 49 or SEQ ID No. 50 or SEQ ID No. 51 wherein "*" represents one of the above heterologous polynucleotides (without the start codon ATG provided by SEQ ID No. 16, SEQ ID No. 19 or SEQ ID No: 20 and SEQ ID No. 40 to SEQ ID No. 45; a stop codon may or may not be present within the "*" sequence, since SEQ ID No. 17 and SEQ ID No. 46 to SEQ ID No. 51 comprise a stop codon, the presence of an extra STOP codon (or two extra stop codons may be required in order to respect the prescriptions of the rule of 6 as explained here above).

In a particular embodiment, the first ATU and the second ATU comprise or consist of different heterologous polynucleotides that are selected among polynucleotides encoding heterologous polypeptides comprising or consisting in amino acid sequences of SEQ ID No. 13 and SEQ ID No. 14, or selected among heterologous polynucleotides encoding heterologous polypeptides comprising or consisting of amino acid sequences of SEQ ID No. 13 and SEQ ID No. 15, and wherein the first heterologous polynucleotide and the second heterologous polynucleotide are separated by a spacer polynucleotide comprising or consisting in the nucleotide sequence of SEQ ID No: 2 or SEQ ID No:11. In particular the ATU(s) is (are) built as the sequence of SEQ ID No.16 (or SEQ ID No: 19 or SEQ ID No: 20 or SEQ ID No. 40 or SEQ ID No. 41 or SEQ ID No. 42 or SEQ ID No. 43 or SEQ ID No. 44 or SEQ ID No. 45) - * - SEQ ID No.17 or SEQ ID No. 46 or SEQ ID No. 47 or SEQ ID No. 48 or SEQ ID No. 49 or SEQ ID No. 50 or SEQ ID No. 51 wherein "*" represents one of the above heterologous polynucleotides encoding a polypeptide of SEQ ID No.13 or SEQ ID No.15 (the 5' M amino acid of SEQ ID No. 13 or 15 being encoded by the ATG codon provided by SEQ ID No. 16 or SEQ ID No. 19 or SQ ID No. 20).

In a particular embodiment of the invention, each ATU presents within the cDNA molecule encoding a full length antigenomic (+) RNA strand of a non-segmented negative-sense single-stranded RNA virus, especially of a measles virus (MV), comprises or consists of (i) the sequence of SEQ ID No. 52, SEQ ID No. 53, SEQ ID No. 54, SEQ ID No. 55, SEQ ID No. 56, SEQ ID No. 57, wherein n represents a heterologous polynucleotides encoding a polypeptide of SEQ ID No.13, SEQ ID No. 14 or SEQ ID No.15.

In a particular embodiment of the invention, the recombinant nucleic acid molecule or the nucleic acid construct encoding the antigenomic RNA of the virus recombined with the heterologous polynucleotide, which is defined herein is further modified, i.e., comprises additional nucleotide sequences or motifs.

### - Nucleic acid construct comprising a Measles virus cDNA sequence

In an embodiment of the invention, wherein the cDNA molecule encodes a full length antigenomic (+) RNA strand of a measles virus, particular attenuated strains of the measles virus can be used to implement the present invention. Accordingly, in a preferred embodiment of the invention, the non-segmented negative-sense single-stranded RNA virus is a measles virus (MV). In a more particular embodiment, the non-segmented negative-sense single-stranded RNA virus is a measles virus (MV) originates from an attenuated strain.

In a preferred embodiment of the invention, the measles virus is an attenuated virus strain. An "attenuated strain" of measles virus is defined as a strain that is avirulent or less virulent than the parent strain in the same host, while maintaining immunogenicity and possibly adjuvanticity when administered in a host i.e., preserving immunodominant T and B cell epitopes and possibly the adjuvanticity such as the induction of T cell costimulatory proteins or the cytokine IL-12.

An attenuated strain of a MV accordingly refers to a strain which has been serially passaged on selected cells and, possibly, adapted to other cells to produce seed strains suitable for the preparation of vaccine strains, harboring a stable genome which would not allow reversion to pathogenicity nor integration in host chromosomes. As a particular "attenuated strain", an approved strain for a vaccine is an attenuated strain suitable for the invention when it meets the criteria defined by the FDA (US Food and Drug Administration) i.e., it meets safety, efficacy, quality and reproducibility criteria, after rigorous reviews of laboratory and clinical data (www.fda.gov/cber/vaccine/vacappr.htm).

In a more particular embodiment of the invention, the MV is originating from an attenuated virus strain selected from the group consisting of the Schwarz strain, the Zagreb strain, the AIK-C strain, the Moraten strain, the Philips strain, the Beckenham 4A strain, the Beckenham 16 strain, the Edmonston seed A strain, the Edmonston seed B strain, the CAM-70 strain, the TD 97 strain, the Leningrad-16 strain, the Shanghai 191 strain and the Belgrade strain, preferably the Schwarz strain, the Zagreb strain and the AIK-C strain in particular the Schwarz strain of SEQ ID No. 62, the AIK-C strain of SEQ ID No. 58 and the Zagreb strain of SEQ ID No. 59. All these strains have been described in the prior art and access to them is provided in particular as commercial vaccines.

In a preferred embodiment, the nucleic acid construct or the recombinant nucleic acid molecule encoding the antigenomic RNA strand of a non-segmented negative-sense single-stranded RNA virus, especially of the measles virus, recombined with the heterologous polynucleotide according to the invention further comprises, at its 5'-end, adjacent to the first nucleotide of the nucleotide sequence encoding the full-length antigenomic (+)RNA strand of non-segmented negative-sense single-stranded RNA virus, especially of the measles virus, a GGG motif followed by a hammerhead ribozyme sequence and also comprises, at its 3'-end, adjacent to the last nucleotide of said nucleotide sequence encoding the full length anti-genomic (+)RNA strand, the sequence of a ribozyme. The Hepatitis delta virus ribozyme (δ) is advantageously hence provided at the 3'-end, adjacent to the last nucleotide of said nucleotide sequence encoding the full length anti-genomic (+)RNA strand.

The GGG motif placed at the 5' end, adjacent to the first nucleotide of the above coding sequence improves the efficiency of the transcription of said cDNA coding sequence. As a requirement for the proper assembly of measles virus particles is the fact that the cDNA encoding the antigenomic (+)RNA of the nucleic acid construct of the invention complies with the rule of six, when the GGG motif is added, a ribozyme is also added at the 5' end of the coding sequence of the cDNA, 3' from the GGG motif, in order to enable cleavage of the transcript at the first coding nucleotide of the full-length antigenomic (+)RNA strand of MV.

In a particular embodiment of the invention, in order to prepare the nucleic acid construct of the invention, the preparation of a cDNA molecule encoding the full-length antigenomic (+) RNA of a MV disclosed in the prior art is achieved by known methods. Said cDNA provides especially the genome vector when it is inserted in a vector such as a plasmid.

In an embodiment of the invention, the number of consecutive nucleotides in the nucleic acid construct is a multiple of six and/or the number of consecutive nucleotides in the recombinant cDNA molecule is a multiple of six (specified as "the rule of six"). In a particular embodiment of the invention, the nucleic acid construct comprising the cDNA encoding the nucleotides of the full-length infectious antigenomic (+) RNA strand of a non-segmented negative-sense single-stranded RNA virus, especially of a measles virus (MV), but without the operatively cloned heterologous polynucleotide complies with the rule of six (6) of the measles virus genome and the polypeptide encoding a heterologous polypeptide complies with the rule of six.

A particular cDNA molecule suitable for the preparation of the nucleic acid construct of the invention is the one obtained using the Schwarz strain of MV. Accordingly, the cDNA coding for the antigenome of the measles virus used within the present invention may be obtained as disclosed in WO2004/000876 or may be obtained from plasmid pTM-MVSchw deposited by Institut Pasteur at the Collection Nationale de Culture de Microorganismes (CNCM), 28 rue du Dr Roux, 75724 Paris Cedex 15, France, under No I-2889 on June 12, 2002, the sequence of which is disclosed in WO2004/000876 incorporated herein by reference. The plasmid pTM-MVSchw has been obtained from a Bluescript plasmid and comprises the polynucleotide coding for the full-length measles virus (+) RNA strand of the Schwarz strain placed under the control of the promoter of the T7 RNA polymerase. It has 18967 nucleotides and a sequence represented as SEQ ID NO: 5. cDNA molecules (also designated cDNA of the measles virus or MV cDNA for convenience) from other MV strains may be similarly obtained starting from the nucleic acid purified from viral particles of attenuated MV such as those described herein.

The cDNA coding for the antigenome of the measles virus used within the present invention may also be obtained from plasmid pTM2-MVSchw-gfp deposited by Institut Pasteur at the Collection Nationale de Culture de Microorganismes (CNCM), 28 rue du Dr Roux, 75724 Paris Cedex 15, France, under No I-2890 on June 12, 2002. It has 19795 nucleotides and a sequence represented as SEQ ID NO: 6. This plasmid contains the sequence encoding the eGFP marker that may be deleted.

The organization of the genome of measles viruses and their replication and transcription process have been fully identified in the prior art and are especially disclosed in Horikami S.M. and Moyer S.A. (Curr. Top. Microbiol. Immunol. (1995) 191, 35-50 or in Combredet C. et al (Journal of Virology, Nov 2003, p11546-11554) for the Schwarz vaccination strain of the virus or for broadly considered negative-sense RNA viruses, in Neumann G. et al (Journal of General Virology (2002) 83, 2635-2662).

The "rule of six" is expressed in the fact that the total number of nucleotides present in a nucleic acid representing the MV (+) strand RNA genome or in the nucleic acid constructs comprising the same is a multiple of six. The "rule of six" has been acknowledged in the state of the art as a requirement regarding the total number of nucleotides in the genome of the measles virus, which enables efficient or optimized replication of the MV genomic RNA. In the embodiments of the present invention defining a nucleic acid construct that meets the rule of six, said rule applies to the nucleic acid construct specifying the cDNA encoding the full-length MV (+) strand RNA genome. In this regard, the rule of six applies individually to the cDNA encoding the nucleotide sequence of the full-length infectious antigenomic (+) RNA strand of the measles virus, and/or to the nucleic acid construct encompassing the heterologous polynucleotides cloned into ATU(s) in said cDNA and encoding the heterologous polypeptide(s) of pathogen(s).

### - Nucleic acid construct comprising a Bacterial Artificial Chromosome (BAC)

In a particular embodiment of the invention, It is provided a recombinant nucleic acid construct comprising a Bacterial Artificial Chromosome comprising a cDNA molecule encoding a full length antigenomic (+) RNA strand of a measles virus (MV or MV) inserted therein; wherein the cDNA molecule comprises at least one additional transcription unit (ATU), the at least one additional transcription units (ATUs) being localized within an intergenic region of the cDNA, the at least one ATU comprising (i) a heterologous polynucleotide operably linked to enable the expression of a heterologous polypeptide encoded by the heterologous polynucleotide, or (ii) an insertion site suitable for the functional insertion of a heterologous polynucleotide.

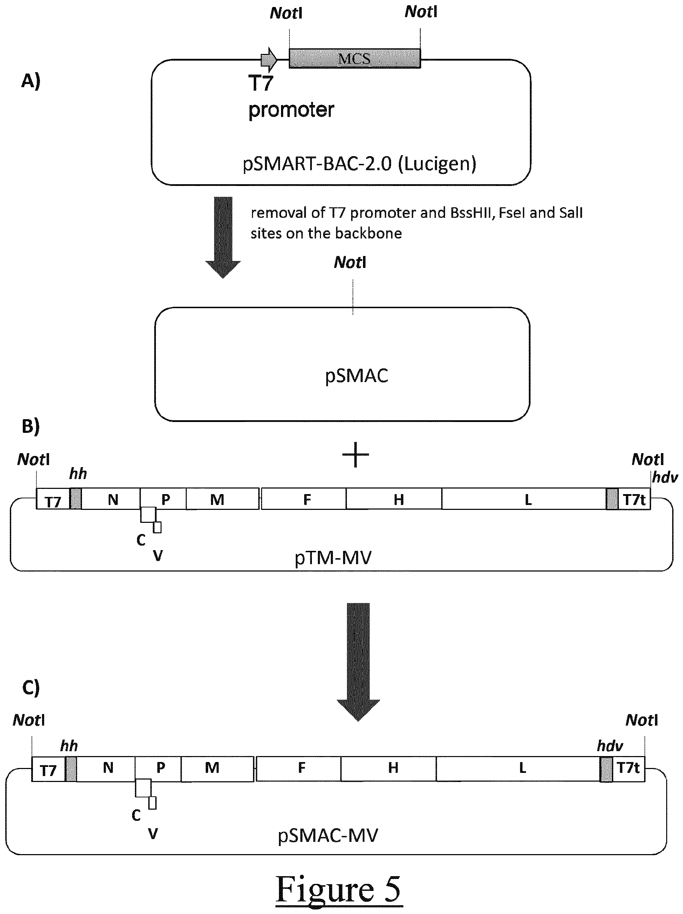

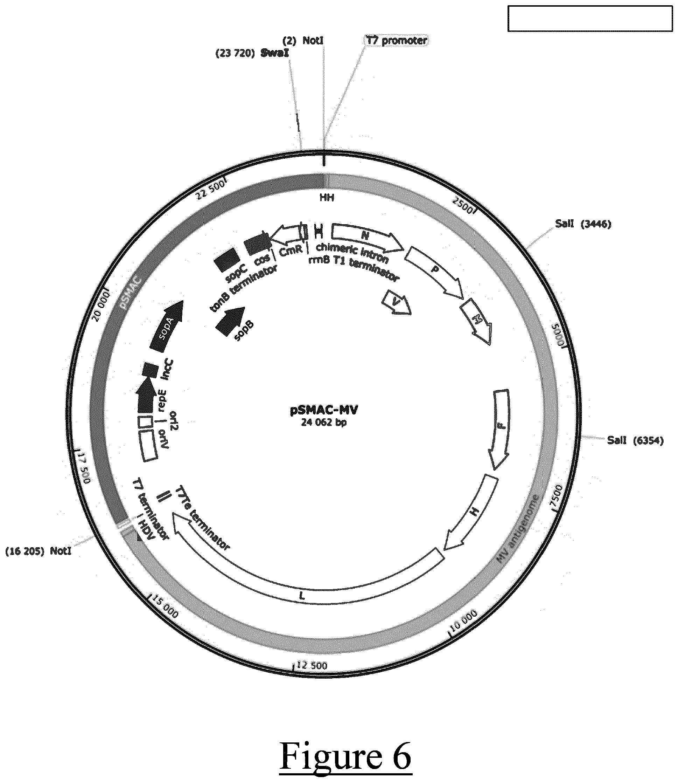

A nucleic acid construct comprising a BAC plasmid is for example illustrated on Fig 5 and 6, for a measles virus, wherein the cDNA molecule is illustrated inserted within a bacterial artificial chromosome plasmid, the pSMART-BAC, while several heterologous polynucleotides encoding antigen(s) from a pathogen or from a plurality of pathogens are or may be inserted within a plurality of ATUs. A nucleic acid construct of the invention may be prepared using steps of synthesis of nucleic acid fragments or polymerization from a template, including by PCR. The heterologous polynucleotide(s) and nucleic acid construct of the invention may rather be prepared in accordance with any known method in the art and in particular may be cloned, obtained by polymerization especially using PCR methods or may be synthesized.

In a particular embodiment, the recombinant nucleic acid construct as disclosed herein in all embodiments is operably inserted within a recombinant Bacterial Artificial Chromosome plasmid, and the cDNA molecule and the at least one ATUs are defined in accordance with any embodiments disclosed herein.

The cDNA molecule encoding a full length antigenomic (+) RNA strand of a virus, in particular MV, may be inserted within the BAC plasmid throughout the use of restriction enzymes which recognize a restriction site on the BAC plasmid on one hand, and on the cDNA molecule encoding a full length antigenomic (+) RNA strand of a virus on the other hand so as to functionally insert (i.e. in a suitable manner for the expression of the genes of the virus and the expression of the heterologous polypeptide encoded by the heterologous polynucleotide inserted within the ATUs localized within the cDNA molecule encoding a full length antigenomic (+) RNA strand of a virus). The BAC plasmid may be any kind of bacterial artificial chromosome plasmid, in particular the pSMART BAC plasmid and its derivatives, pEZ-BAC plasmid, pBeloBAC11plasmid , pBACe3.6, pBAC/OriV, pBAC-RT, pHA1, pHA2, pTARBAC2 and its derivatives, and the pBAC contruct inserted in Transmissible gastroenteritis coronavirus (TGEV), as defined here above. In a preferred embodiment, the bacterial artificial chromosome plasmid is selected from the group consisting of pSMART-BAC, pEZ-BAC and pBeloBAC11. In a more preferred embodiment of the invention, the bacterial artificial chromosome plasmid is the pSMART-BAC plasmid, or is issued from the pSMART-BAC plasmid

In a particular embodiment of the invention, the BAC is devoid of a T7 promoter, when the expression of at least one gene of the virus encoded by the cDNA of its full length antigenomic (+) RNA strand or the heterologous polypeptide encoded by the heterologous polynucleotide inserted within the ATUs, is under control of a T7 promoter. In a particular embodiment of the invention, at least one expression control sequence of the gene of the virus encoded by the cDNA of its full length antigenomic (+) RNA strand or the heterologous polypeptide encoded by the heterologous polynucleotide inserted within the ATUs comprises the T7 promoter and T7 terminator sequences. These sequences are respectively located 5' and 3' of the coding sequence for the full length antigenomic (+)RNA strand of the virus and from the adjacent sequences around this coding sequence. Accordingly, in a particular embodiment the nucleic acid construct of the invention comprises these additional control sequences.

The BAC plasmid with the cDNA molecule encoding a full length antigenomic (+) RNA strand of a non-segmented negative-sense single-stranded RNA virus, especially of a measles virus (MV), may correspond to any recombination of a BAC as described herein and any cDNA molecule encoding a full length antigenomic (+) RNA strand of a non-segmented negative-sense single-stranded RNA virus as described herein, in particular any recombination of a cDNA encoding MV genome with heterologous polynucleotides inserted within ATUs allowing expression of different polypeptides issued from pathogens.

In a particular embodiment of the inventions, the BAC plasmid may be issued or derived from a BAC of SEQ ID No: 3, SEQ ID: 7: SEQ ID No: 8, SEQ ID No. 60 or SEQ ID No. 61 or SEQ ID No: 12, wherein the heterologous polynucleotides inserted within the cDNA molecule encoding a full length antigenomic (+) RNA strand of the measles virus are substituted by other heterologous polynucleotide sequences encoding immunogenic heterologous polypeptides as defined herein. More particularly, the polynucleotide localized between Notl restriction sites of SEQ ID No: 3, or between Notl restriction sites of SEQ ID No: 7, or between Notl restriction sites of SEQ ID No: 8 or SEQ ID No. 60 or SEQ ID No. 61, or between Notl restriction sites of SEQ ID No: 12, may be substituted by any other heterologous polynucleotide sequence(s) encoding immunogenic heterologous polypeptides as defined herein especially according to the specific embodiments disclosed. A Notl restriction site is a nucleotide sequence recognized and cut by the Notl restriction enzyme and it corresponds to the nucleotide sequence of SEQ ID No. 18 (5' GCGGCCGC 3').

In a particular embodiment of the invention, it is provided a nucleic acid construct as described herein or a recombinant BAC plasmid as described herein, wherein a third ATU is localized within the same intergenic region where the first ATU and the second ATU are localized, the third ATU comprising a heterologous polynucleotide operably linked within the third ATU allowing the expression of a heterologous polypeptide encoded by the heterologous polynucleotide wherein the heterologous polypeptide is different form the heterologous polypeptides encoded by the heterologous polynucleotides of the other ATUs, and optionally wherein the heterologous polypeptide(s) encoded by each ATU originate from different virus types. In other particular embodiments, the nucleic acid construct comprises a Measles virus, a BAC plasmid, and three ATUs, and the three ATUs are localized within the same intergenic region of the Measles virus in the cDNA molecule, in particular the three ATUs are localized between the P gene and the M gene of the measles virus in the cDNA molecule; or between the H gene and the L gene of the measles virus in the cDNA molecule, or between the N gene and the T7RNA polymerase promoter.

In another particular embodiment of the invention, it is provided a nucleic acid construct as described herein, in particular a recombinant BAC plasmid as described herein, wherein the intergenic region localized between the P gene and the M gene of the measles virus in the cDNA molecule comprises all the ATUs, and wherein the heterologous polynucleotides inserted in the intergenic region are separated by a spacer polynucleotide comprising or consisting of SEQ ID No: 2 or SEQ ID No. 11.

In another particular embodiment of the invention, it is provided a nucleic acid construct as described herein in particular a recombinant BAC plasmid as described herein, comprising a third ATU, wherein the first ATU, the second ATU and the third ATU comprise(s) or consist(s) of different polynucleotides encoding heterologous polypeptides comprising or consisting in amino acid sequences that are selected among SEQ ID No. 13, SEQ ID No.: 14 and SEQ ID No.: 15, and wherein the heterologous polynucleotide encoding the heterologous polypeptides are separated by a spacer polynucleotide comprising or consisting in the nucleotide sequence of SEQ ID No: 2 or SEQ ID No:11. In a particular embodiment, these heterologous polypeptides comprising or consisting in amino acid sequences that are selected among SEQ ID No. 13, SEQ ID No.: 14 and SEQ ID No.: 15 are all inserted within ATUs localized between the P gene and the M gene of the measles virus in the cDNA molecule.

### • Method for producing a vaccine

The present invention also concerns a method for producing a vaccine based on recombinant infectious viral particles and/or virus-like particles.

To this end, the invention also relates to a process for rescuing recombinant infectious non-segmented negative-sense single-stranded RNA virus, especially recombinant infectious Measles virus, expressing at least one, in particular at least two, heterologous polypeptides or immunogenic fragments thereof or antigenic fragments thereof expressed from at least one, in particular by at least two, heterologous polynucleotide inserted within an ATU localized within a cDNA molecule encoding a full length antigenomic (+) RNA strand of a measles virus (MV), said heterologous polypeptides being in particular issued from a single or a plurality of pathogens as already defined herein, and comprising:
(a) In cells, in particular HEK293 helper cells, stably expressing T7 RNA polymerase and measles virus N and P proteins and/or transfected with an expression vector encoding T7 RNA polymerase and/or transfected with an expression vector encoding measles virus N and P proteins, transfecting any nucleic acid construct according to any embodiment described herein;
(b) Maintaining the transfected cells of step (a) in conditions suitable for the production of recombinant virus and/or virus like particles;
(c) Infecting cells, in particular Vero cells, in conditions enabling the propagation of the recombinant infectious virus by co-cultivating these cells with the cells issued from step (b); in particular at 32 °C;
(d) Harvesting the recombinant infectious virus and/or virus like particles, in particular at 32 °C.

In a particular embodiment, expression is characterized by being maintained over at least 12 passages of the rescued recombinant infectious negative-sense single-stranded RNA virus when amplified on Vero cells, especially of the amplified recombinant infectious measles virus.

According to a particular embodiment, the harvesting of the recombinant virus, in particular recombinant Measles virus, expressing at least one polypeptide of the pathogen or an immunogenic fragment or an antigenic comprises measles viruses expressing the C, E2 and E1 proteins of the Chikungunya virus, the sE protein of the West Nile virus, and/or the prME protein of the Zika virus, and are in particular polypeptides of amino acid sequence of SEQ ID No. 13 for C, E2 and E1 proteins of the Chikungunya virus, SEQ ID No. 14 for the sE protein of the West Nile virus, SEQ ID No. 15 for the prME protein of the Zika virus.

According to a particular embodiment of said process, the transfer vector plasmid has the sequence of SEQ ID NO: 12.
According to a particular embodiment, the harvesting step is performed at 32°C. According to a particular embodiment, the infection step is performed at 32°C.
According to a particular embodiment, the harvesting and the infection steps are both performed at 32 °C. The co-cultivation step between infection and harvesting may be performed at any temperature ranging from 32 °C to 37°C, both extreme temperatures of 32 °C and 37 °C being explicitly included within the range, more preferably either at 37 °C or at 32 ° C, and most preferably at a temperature of 32 °C.

As used herein, the term *"recombining"* applied to cells means introducing at least one polynucleotide into a cell, for example under the form of a vector, said polynucleotide integrating (entirely or partially) or not integrating into the cell genome (such as defined above).

According to a particular embodiment, recombination can be obtained with a first polynucleotide, which is the nucleic acid construct of the invention. Recombination can also, or alternatively, encompass, introducing a polynucleotide, which is a vector encoding a RNA polymerase large protein (L) of a MV, whose definition, nature and stability of expression has been described herein. In accordance with the invention, the cell or cell lines or a culture of cells stably producing a RNA polymerase, a nucleoprotein (N) of a measles virus and a polymerase cofactor phosphoprotein (P) of a measles virus is a cell or cell line as defined in the present specification or a culture of cells as defined in the present specification, i.e., are also recombinant cells to the extent that they have been modified by the introduction of one or more polynucleotides as defined above. In a particular embodiment of the invention, the cell or cell line or culture of cells, stably producing the RNA polymerase, the N and P proteins, does not produce the L protein of a measles virus or does not stably produce the L protein of a measles virus, e.g., enabling its transitory expression or production.

The production of recombinant infectious replicating virus particles of the invention may involve a transfer of cells transformed as described herein. The term *"transfer"* as used herein refers to the plating of the recombinant cells onto a different type of cells, and particularly onto monolayers of a different type of cells. These latter cells are competent to sustain both the replication and the production of infectious virus particles, i.e., respectively the formation of infectious viruses inside the cell and possibly the release of these infectious viruses outside of the cells. This transfer results in the co-culture of the recombinant cells of the invention with competent cells as defined in the previous sentence. The above transfer may be an additional, i.e., optional, step when the recombinant cells are not sufficiently efficient virus-producing culture, i.e., when infectious virus particles cannot be efficiently recovered from these recombinant cells. This step is introduced after further recombination of the recombinant cells of the invention with nucleic acid construct of the invention, and optionally a vector comprising a nucleic acid encoding a RNA polymerase large protein (L) of a measles virus.
In a particular embodiment of the invention, a transfer step is required since the recombinant cells, usually chosen for their capacity to be easily recombined are not efficient enough in the sustaining and production of recombinant infectious virus particles. In said embodiment, the cell or cell line or culture of cells of step 1) of the above-defined methods is a recombinant cell or cell line or culture of recombinant cells according to the invention.

Cells suitable for the preparation of the recombinant cells of the invention are prokaryotic or eukaryotic cells, particularly animal or plant cells, and more particularly mammalian cells such as human cells or non-human mammalian cells or avian cells or yeast cells. In a particular embodiment, cells, before recombination of its genome, are isolated from either a primary culture or a cell line. Cells of the invention may be dividing or non-dividing cells.

According to a preferred embodiment, helper cells are derived from human embryonic kidney cell line 293, which cell line 293 is deposited with the ATCC under No. CRL-1573. Particular cell line 293 is the cell line disclosed in the international application WO2008/078198 and referred to in the following examples.

According to another aspect of this process, the cells suitable for passage are CEF cells. CEF cells can be prepared from fertilized chicken eggs as obtained from EARL Morizeau, 8 rue Moulin, 28190 Dangers, France, or from any other producer of fertilized chicken eggs.

The process which is disclosed according to the present invention is used advantageously for the production of infectious replicative MV particles that may be used in an immunogenic composition.
- Compositions, vaccines and seeds issued or derived from a nucleic acid construction according to the invention

The invention also relates to an immunogenic composition, advantageously a vaccine composition comprising (i) an effective dose of the recombinant measles virus according to the invention, and/or of the recombinant VLPs according to the invention and (ii) a pharmaceutically acceptable vehicle, wherein the composition or the vaccine elicits a humoral, especially a protective, in particular a neutralizing humoral response and/or a cellular response in an animal host, especially in a human being, in particular after a single immunization, against the polypeptide(s) of the pathogen.
Also encompassed by the present invention, it is provided a seed preparation or a stock preparation of a recombinant infectious non-segmented negative-sense single-stranded RNA virus, especially of a recombinant infectious measles virus that consists in an single virus clone or respectively an amplified single virus clone of a recombinant infectious virus rescued by reverse genetics from any nucleic acid construct described herein in particular from any recombinant BAC plasmid described herein.
It is also provided a vaccine which comprises a clonally selected and amplified recombinant infectious negative-sense single-stranded RNA virus, especially a clonally selected and amplified recombinant infectious measles virus obtainable from the rescue by reverse genetics of any nucleic acid construct described herein in particular from the recombinant BAC plasmid according to any one of the recombinant BAC plasmids described herein.
Also encompassed by the present invention is a recombinant infectious non-segmented negative-sense single-stranded RNA virus, especially a recombinant infectious Measles virus, wherein the genome of said recombinant infectious virus comprises or consists in a nucleic acid construct according to any embodiment described herein.
The invention also relates to an immunogenic or vaccine composition comprising (i) an effective dose of the recombinant virus according to the invention, and/or of the recombinant VLPs according to the invention and (ii) a pharmaceutically acceptable vehicle for use in the prevention or treatment of an infection by a pathogen In a particular embodiment, said composition is for administration to children, adolescents or travelers.

The invention also relates to an immunogenic composition which comprises a clonally selected and amplified recombinant infectious negative-sense single-stranded RNA virus, especially a clonally selected and amplified recombinant infectious measles virus obtainable from the rescue by reverse genetics of any nucleic acid construct according to the present description, or from the recombinant BAC plasmid according to any embodiment described herein.
In a particular embodiment, the immunogenic vaccine or composition of the invention enables stable expression of multiple heterologous polypeptides from any nucleic acid construct according to the present description, or from the recombinant BAC plasmid according to any embodiment described herein, wherein such stable expression is characterized by being maintained over at least 12 passages of the rescued recombinant infectious negative-sense single-stranded RNA virus when amplified on Vero cells, especially of the amplified recombinant infectious measles virus.
Said composition may comprise a suitable vehicle for administration e.g. a pharmaceutically acceptable vehicle to a host, especially a human host and may further comprise but not necessarily adjuvant to enhance immune response in a host. The inventors have indeed shown that the administration of the active ingredients of the invention may elicit an immune response without the need for external adjuvantation.
Such a vaccine composition comprises advantageously active principles (active ingredients) which comprise recombinant infectious replicating virus particles rescued from the vector and constructs as defined herein optionally associated with VLPs comprising the (same) pathogen proteins.

The administration scheme and dosage regime may require a unique administration of a selected dose of the recombinant infectious replicating virus particles. Alternatively, it may require administration of multiple doses.

In a particular embodiment the administration is performed in accordance with a prime-boost regimen. Priming and boosting may be achieved with identical active ingredients consisting of said recombinant infectious replicating virus particles. Alternatively, priming and boosting administration may be achieved with different active ingredients, involving said recombinant infectious replicating virus particles in at least one of the administration stages of the prime-boost administrations.

Considering that the currently known doses for human vaccines are in the range of 10³ to 10⁵ TCID50, a suitable dose of recombinant virus to be administered may be in the range of 0.1 to 10ng, in particular 0.2 to 6 ng, and possibly as low as 0.2 to 2 ng.

According to a particular embodiment of the invention, the immunogenic or vaccine composition defined herein may also be used for protection against an infection by the measles virus.

The invention also relates to a method for preventing a pathogen virus related disease said method comprising the immunization of a mammalian, especially a human, in particular a child, by the injection, in particular by subcutaneous injection, of recombinant infectious virus according to the invention.

The invention also relates to a method for treating a pathogen virus related disease, said method comprising the immunization of a mammalian, especially a human, in particular a child, by the injection, in particular subcutaneous injection, of recombinant infectious virus according to the invention.

### - infectious ribonucleoprotein complexes (RNPs)

The invention also relates to infectious ribonucleoprotein complexes (RNPs) also designated ribonucleoproteins, ribonucleocapsids or ribonucleoparticles of the recombinant measles viruses, generated by reverse genetics. The preparation of the RNPs may be achieved using techniques known from the person skilled in the art such as those disclosed in WO 2009/095791.

As defined herein, the term *"RNP"* refers to a structure containing a replicating recombinant MV ribonucleic acid (RNA) obtained according to the invention and the transcriptase complex formed of MV nucleoprotein (N) phosphoprotein (P) and polymerase (L) auto-assembled to form a structure, in particular a rod-shaped structure.

Accordingly, an object of the present invention is infectious RNPs from recombinant measles virus (MV) expressing heterologous polypeptide(s), obtained using the nucleic acid construct defined herein which comprises a cDNA molecule encoding a full length antigenomic (+) RNA strand of a non-segmented negative-sense single-stranded RNA virus, especially of a measles virus (MV), wherein the cDNA molecule further comprises inserted therein, at least a first additional transcription unit (ATU) and a second additional transcription unit (ATU), wherein the at least first and second additional transcription units (ATUs) are localized within a single intergenic region of the cDNA encoding the antigenomic (+) RNA strand as a single expression cassette, and wherein each ATU comprises a heterologous polynucleotide operably inserted within the ATU allowing the expression of a heterologous polypeptide encoded by the heterologous polynucleotide.

According to a particular embodiment of the invention, the RNPs may be obtained using any of the herein disclosed embodiments of this nucleic acid construct.

The invention thus relates to a Ribonucleoprotein of a recombinant measles virus strain which comprises a RNA genome assembled with the transcriptase complex proteins of the measles virus wherein the genome contains the recombinant full length antigenomic (+) RNA strand of the measles virus (MV), and inserted therein, sequences encoding at least a first additional transcription unit (ATU) and a second additional transcription unit (ATU), wherein the at least first and second additional transcription units (ATUs) are localized within a single intergenic region of the recombinant antigenomic (+) RNA strand as a single expression cassette, and wherein each ATU comprises a heterologous polynucleotide operably inserted within the ATU allowing the expression of a heterologous polypeptide encoded by the heterologous polynucleotide

RNPs according to the invention thus express the heterologous polypeptides encoded by the recombinant cDNA of the invention.

RNPs may be obtained using methods known in the art such as methods carried out in yeast, especially budding yeast as illustrated by *Saccharomyces cerevisiae.*

In a particular embodiment of the invention, the recombinant host cell, in particular yeast strain, suitable for the expression of infectious non-segmented negative-strand RNA virus Ribonucleocapsids (RNPs), is obtained after being transformed with at least the following expression vector:
(i) At least one genome vector, comprising, as an insert under the control of regulatory expression sequences functional in the host cell, a cloned DNA molecule which comprises a nucleic acid construct of the invention.
In another particular embodiment of the invention, the recombinant host cell, in particular yeast strain, suitable for the expression of infectious recombinant measles virus Ribonucleocapsids (RNPs), is obtained after being transformed with at least the combination of the following expression vectors:
(i) at least one genome vector, comprising, as an insert under the control of regulatory expression sequences functional in the host cell, a cloned DNA molecule which comprises a nucleic acid construct of the invention,
(ii) one or more trans-complementation vectors comprising, under the control of regulatory expression sequences functional in the host cell, nucleotide sequences which enable said vector(s) to collectively express the proteins necessary for the synthesis of the viral transcriptase complex of said recombinant measles virus used in the genome vector, and enable assembly of the ribonucleocapsid (RNPs) of said virus, which are functional in the host cell for the replication and transcription, said vector(s) further optionally comprising, under the control of regulatory expression sequences functional in the host cell, a selectable marker.

In a particular embodiment, the RNA of the MV used to derive the nucleic acid construct is the RNA of a Schwarz strain, an AIKC strain or a Zagreb strain as disclosed herein.

RNPs can be used in an immunogenic composition or may be used as seeds to reproduce the viral particles or virus-like particles of the said recombinant measles virus, in cell culture in conditions that would allow their use as immunogens, preferably as vaccine components.

### Short description of the drawings

These and further aspects of the invention will be explained in greater detail by way of examples and with reference to the accompanying drawings in which:
**Figure 1****:** Schematic diagram of measles virus and its non-segment negative RNA, genome, structural proteins (N, nucleoprotein; P, phosphoprotein; M, matrix protein; F, fusion protein; H, hemaglutinin; L, Large polymerase), and accessory proteins (C and V protein) in the vector (pTM-MVSchw) containing an additional transcript unit (ATU). T7, T7RNA polymerase promoter; hh, hammerhead ribozyme; δ, hepatitis delta virus (HDV) genome ribozyme; T7t, T7RNA polymerase terminator.
**Figure 2****.** Schematic illustration of measles virus and its non-segment negative RNA wherein several different ATUs have been incorporated. From A) to G), several embodiments are illustrated where the localization of the ATUs are different. ATU references (e.g. ATU1, ATU2 ATU3, etc.) do not involve a particular order, and/or a correspondence to the ATUs disclosed in prior art, wherein ATU1 is usually localized upstream the N gene of the MV genome, ATU2 is localized between genes P and M of the MV genome, and ATU3 is localized between the H and L genes of the MV genome. The numeric reference associated to each ATU has herein only an illustrative purpose, to clearly illustrate that several ATUs may be localized between a single intergenic region of the MV genome, but these ATUs may have a different structure, or share the same structure as detailed in the description of the invention, and may or may not be equivalent to the ATUs disclosed in prior art.

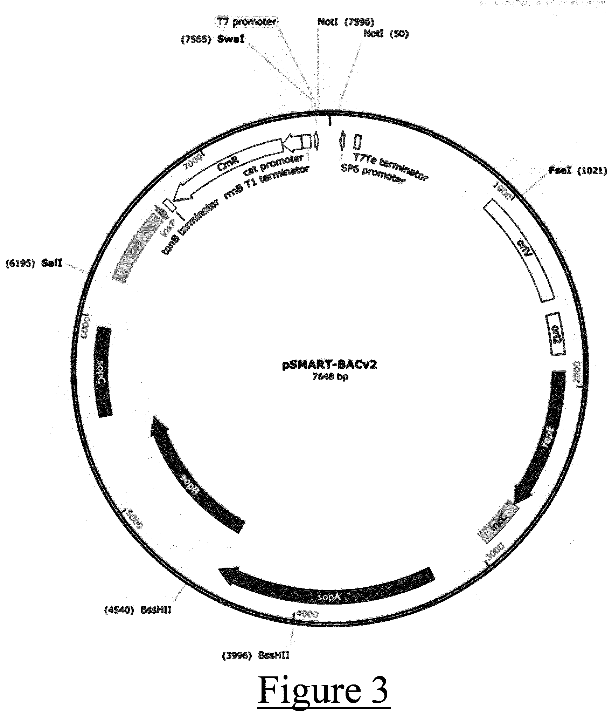

**Figure 3****.** pSMART-BACv2 plasmid (Lucigen) genomic map. The vector incorporates controllable genetic elements that allow the single copy BAC to be amplified in vivo; ori2 (oriS) origin of replication, repE gene, and sopABC partition loci. The vector also carries the oriV medium-copy origin of replication for arabinose inducible promoter. The schematic also indicates the targeted features/restriction sites (underlined in red) that are modified by either removal of the region, T7 promoter, or site-directed mutagenesis to abolish the restriction sites, BssHII, Sall and Fsel, allowing further insertion of targeted genes into ATU2 on MV vector.

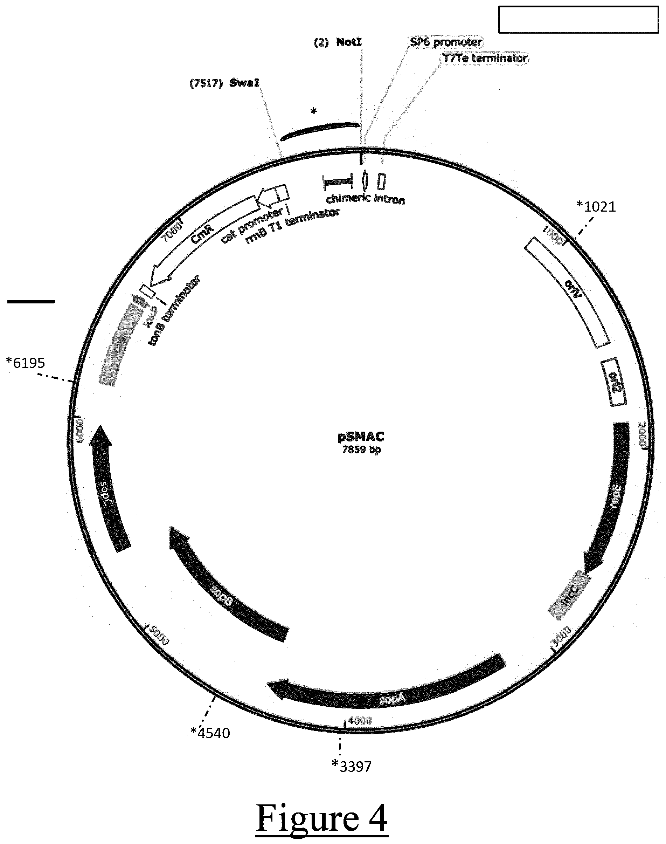

**Figure 4****.** Modified pSMAC-BAC plasmid map with T7 promoter between Swal and Notl sites removed and replaced with chimeric intron region amplified from pCI-Neo. The two sites of BssHII, position 3396 and 4540 were site-directed mutated from C3397 to A and G4542 to A while keeping the same encoding amino acid. To allow insertion of genes in the ATU2 and ATU3 simultaneously, Sall (position 6195) and Fsel (position 1021) restriction sites were also site-directed mutated from T 6196 to A and G1020 to C.
**Figure 5****: A)** pSMART-BAC-2.0 (Lucigen) and its modifications to generate pSMAC-BAC. **B)** pTM-MV map. **C)** pSMAC-MV map.
**Figure 6****.** pSMAC-MV plasmid map; blue box indicates pSMAC vector backbone, grey box indicates the antigenome of Schwarz MV with each measles gene shown in light yellow boxes with translation elements in green boxes.

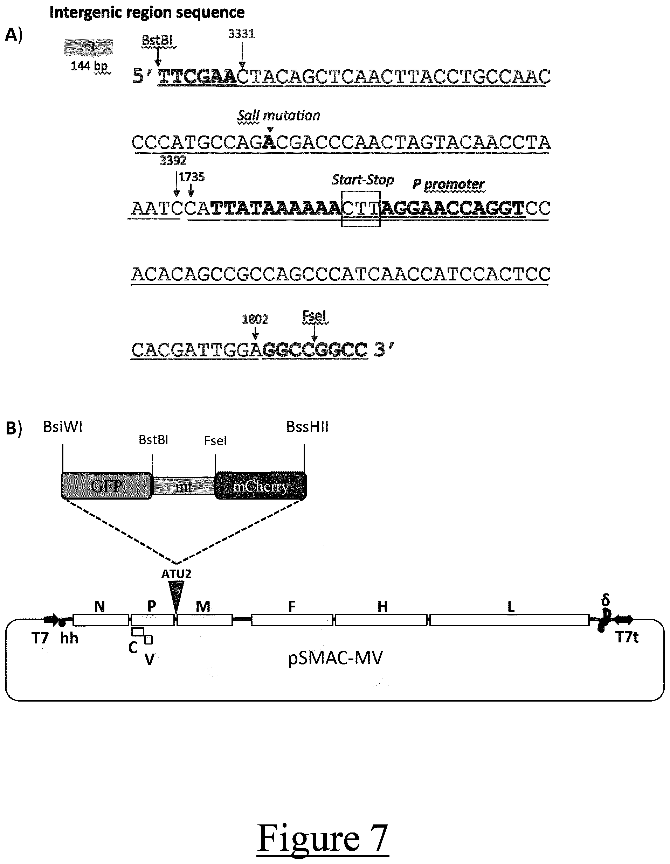

**Figure 7****:** Introduction of fluorescence genes into ATU2 of pSMAC-MV into BsiWI and BssHII restriction sites of ATU2. **A)** Intergenic sequence: fragment 3331-3392 of MV Schwarz genomic sequence with single nucleotide change T to A at position 3365 to mutate Sall restriction site was linked to fragment 1735-1802 of MV Schwarz genomic sequence containing the CTT start-stop signal and P promoter. Two restriction sites (6-base and 8-base respectively) were added at the 5' and 3' end of the intergenic sequence. The unique BstBI and Fsel restriction sites were added to facilitate further insertion of additional genes. **B)** The encoding sequences of fluorescent GFP and mCherry proteins were added on both sides of the intergenic sequence and the whole fragment was introduced into ATU2 of pSMAC-MV to allow GFP and mCherry expression simultaneously from ATU2.

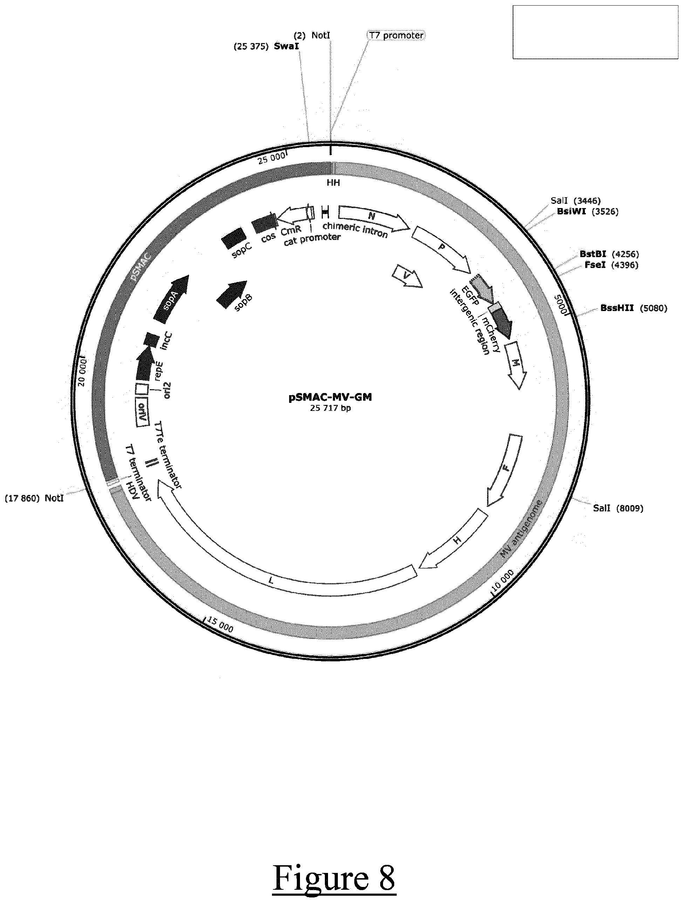

**Figure 8****:** pSMAC-MV-GFP-mCherry (pSMAC-MV-GM) plasmid map. The additional fluorescent genes were added at the ATU2 position between measles virus P and M genes. The GFP gene is indicated in green and the mCherry gene in red with the intergenic region (144 bp) between BstBI and Fsel sites.
**Figure 9****:** Average syncytia counts after recovery of MV-GFP-mCherry comparing between pSMAC and pTM vectors. The experiments were performed in triplicate and 3 biological repeats.

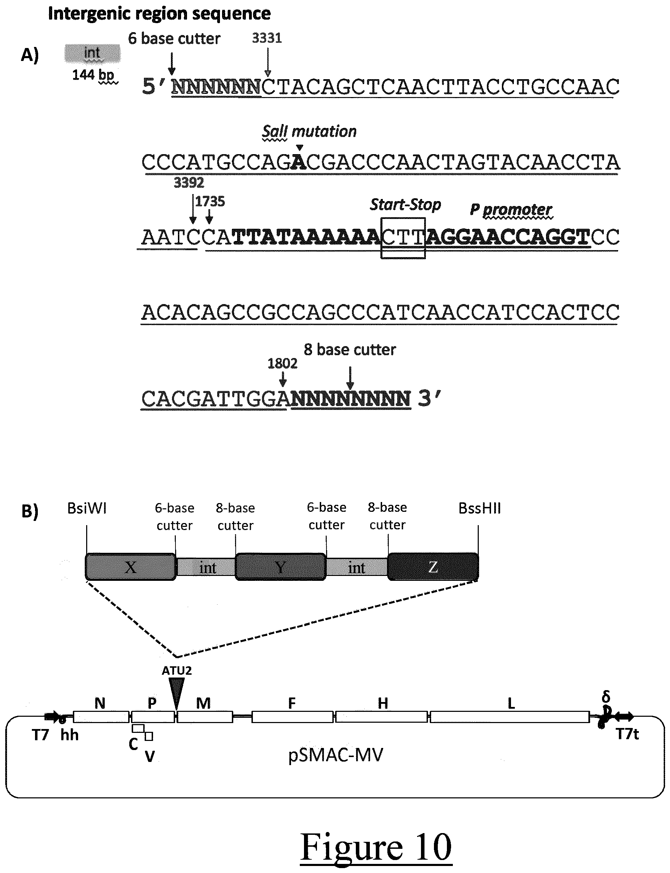

**Figure 10****: A)** Intergenic sequence with possibility of any unique 6- or 8-nucleotide restriction site to facilitate the swapping of genes in the multiple ATU. **B)** Schematic map of pSMAC-MV with multiple ATUs inserted in ATU2 BsiWI / BssHII enabling 3 additional genes expressed separately in the same position of recombinant virus.
**Figure 11****.** Schematic diagram of a measles virus according of Fig. 1. The pSMAC-MV vector contains three antigenic sequences from CHIKV, WNV and ZIKV with intergenic sequences in between, and inserted between the P gene and the M gene of the MV genome, the MV being inserted within a BAC, as illustrated in Fig. 1. CHIKV: Chikungunya Virus - WNV: West Nile Virus - ZIKV: Zika Virus ZIKV with intergenic sequences in between them corresponding to SEQ Id No..2.
**Figure 12****.** Cloning strategy for pSMAC-MV-CIWIZ plasmid containing 3 immunogens of CHIKV, WNV and ZIKV plus intergenic sequences inserted in ATU2. A) Cloning strategy for the multiple antigens to achieve construct 2 (CHIKV and WNV) and construct 3 (CHIKV and ZIKV). This first step was done using extension PCR. B) Cloning steps to achieve the multivalent construct to ligate the CHIKV fragment to the WNV and ZIKV fragment through Sbfl site, giving the triple antigens construct that can be inserted into ATU2 using restriction enzymes, BsiWI and BssHII.
**Figure 13****.** Schematic map of pSMAC-MV-CIWIZ (CHIKV-WNV-ZIKV) vector plasmid. CHIKV, WNV and ZIKV genes are represented in orange, blue and green arrows respectively. The intergenic region or ATU 2.1 and 2.2 are indicated in pink boxes. Total size of pSMAC-MV-CIWIZ is 31676 bp.

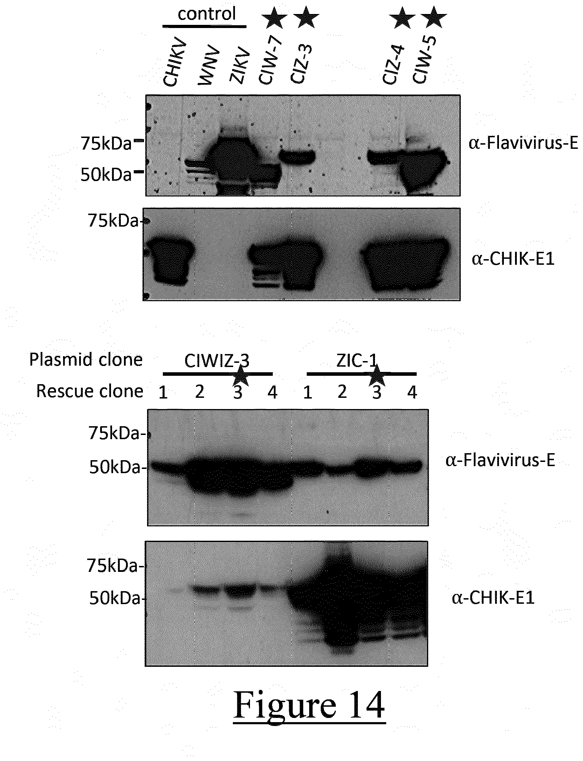

**Figure 14****.** Western Blot analysis of cell lysates from Vero cells infected with bivalent or trivalent recombinant measles viruses. Control cell lysates were from Vero cells infected with MV-CHIK, MV-WNV and MV-ZIKV separately. For CHIKV antigens, anti-CHIK-E1 antibody detecting the E1 envelope protein (50KDa) was used. For WNV and ZIKV antigens, the anti- flavivirus envelope protein (clone 4G2) was used to detect WNV secreted envelope protein (50KDa) and ZIKV surface envelope protein (60KDa). All the clones with the red stars were positive for the antigens expression and were chosen for amplifying viral stocks and further analysis.

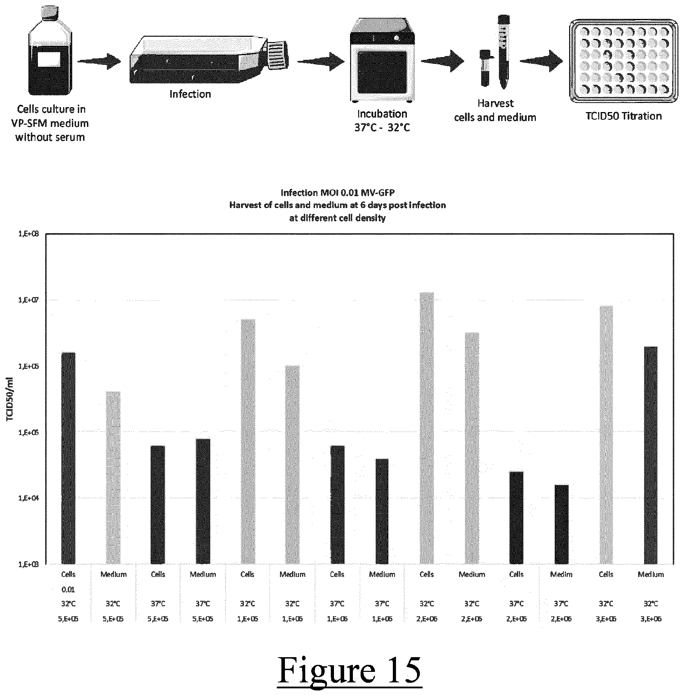

**Figure 15****.** Harvest of MV-GFP grown on Vero CCL-81 cells cultured in VP-SFM medium without serum at 32 or 37 °C. Virus was harvested from cells and medium and tittered by the TCID50 method. Different conditions of cell density were tested.

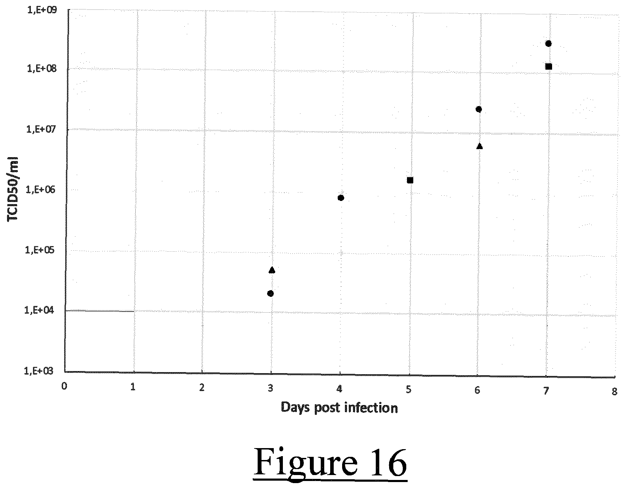

**Figure 16****.** Harvest of MV-GFP grown on Vero CCL-81 cells cultured in VP-SFM medium without serum at 32°C. Virus was harvested from medium and tittered by the TCID50 method.

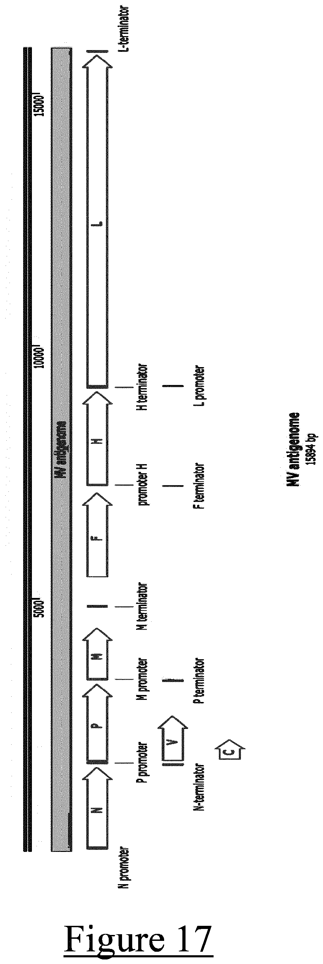

**Figure 17****.** Schematic representation of the MV genome, wherein the promoter sequences and the terminator sequences of the genes of MV are highlighted. Promoter sequences and terminator sequences of (i) the gene N are in red; (ii) the gene P are in green; (iii) the gene M are in orange; (iv) the gene H are in dark blue; (v) of the gene L are in black. These regulatory sequences may be used in the ATUs according to the invention; the promoter sequence of a gene of the MV may be present within an ATU according to the invention, as well as the terminator sequence.
**Figure 18****.** Examples of ATUs suitable for insertion into the genome of a MV genome. Different ATUs are illustrated, and correspond to ATUs designed for the expression of the heterologous polypeptide encoded by the heterologous polynucleotide inserted within each ATU present within the genome of a MV. The "CTT" codons in cyan corresponds to the START/STOP codon of the ATUs; the sequences in purple correspond to the promoter sequences of the ATUs, and are issued from the promoter sequences of the genes of MV; the sequences in orange correspond to restriction sites; the green sequences consists in the START codon and the STOP codon of the heterologous polynucleotides encoding the heterologous polypeptides to be inserted within the ATUs at the "insertion site"; the sequences in red correspond to the terminator sequences of the ATUs, and are issued from the terminator sequences of the genes of MV. (**A**). ATU with F promoter sequence issued from the promoter region of the gene F of MV and F terminator sequence issued from the terminator region of the gene F of MV; (**B**) ATU with H promoter sequence issued from the promoter region of the gene H of MV and H terminator sequence issued from the terminator region of the gene H of MV; (**C**) ATU with L promoter sequence issued from the promoter region of the gene L of MV and L terminator sequence issued from the terminator region of the gene L of MV; (**D**) ATU with M promoter sequence issued from the promoter region of the gene M of MV and M terminator sequence issued from the terminator region of the gene M of MV; (**E**) ATU with N promoter sequence issued from the promoter region of the gene N of MV and N terminator sequence issued from the terminator region of the gene N of MV; (**F**) ATU with P promoter sequence issued from the promoter region of the gene P of MV and M terminator sequence issued from the terminator region of the gene P of MV.

### Examples of the invention

### Modification of pSMART-BAC

Commercially available pBAC or pSMART-BAC were previously used to clone several large non-segmented RNA viruses²⁴. We used pSMART-BAC (Lucigen pSMART-BAC v2.0) to insert the full-length MV genome into Notl restriction sites (Figure 3). However, several modifications of the original pSMART-BAC were first needed in order to allow a successful rescue of measles virus. First, the existing T7 promoter on pSMART-BAC needed to be removed to prevent interfering with the expression from the T7 promoter already present upstream of the MV full-length genome. Second, two BssHII sites on pSMART-BAC were mutated by site directed mutagenesis to abolish BssHII enzyme recognition while maintaining the same amino acid sequence to allow the insertion of heterologous sequences in the additional transcription units (ATUs)³. Similarly, the Sall restriction site was also mutated by site directed mutagenesis to allow insertion of the heterologous genes into ATU2 and ATU3 at the same time. Lastly, the Fsel restriction site was also mutated to enable the multiple gene insertion into a single ATU. The detailed modification of pSMART-BAC is described in the following:

### 1. Removing T7 promoter from the mutated pSMART-BAC plasmid

To remove the T7 promoter from pSMART-BAC-GFP plasmid, a 348 bp linker DNA was amplified from pCl-Neo to replace between Swal and Notl sites (primers are indicated in table 1). The mutated pSMART-BAC-GFP bacmid and the PCR fragment were digested with Swal and Notl (Anza restriction system Invitrogen). After ligation, the pSMART-BAC-GFP plasmid mutated in BssHII sites and deleted of its T7 promoter was generated and named pSMAC-BAC (Figure 4).

**Table 1: primers used to remove T7 promoter from the pSMART-BAC-GFP plasmid**

| Primer name | Sequence 5' - 3' | SEQ ID |
|---|---|---|
| Swal-Neo | TGATTTAAATTCGTTTAGTGAACCGTCAGATC | 25 |
| Notl-Neo | TTGCGGCCGCAGTACTCTAGCCTTAAGAGCTG | 26 |

### 2. Site-directed mutagenesis of two BssHII sites in the pSMART-BAC-GFP plasmid

pSMART plasmid contains two BssHII restriction sites, one in the sopA gene at position 3397 and another in the sopB gene at position 4542. Site-directed mutagenesis was carried out by using GeneART Site-Directed Mutagenesis PLUS kit (ThermoFisher Scientific). The primers were designed to replace nucleotide C3397-A and G4542-A and maintain the same in-frame amino acid (Table 2). Primers were mixed in order of MIX1, 2µl of pSopA-fwd and pSopB-rev for reaction Tube 1, and MIX2, 2µl of pSopB-fwd and pSopA-rev for reaction Tube 2. Site-directed mutagenesis reactions were prepared according to the manual and aliquot 20 µl for each reaction using Q5 High-Fidelity 2X Master Mix (NEB). The amplification step was performed with optimum extension times for Tube1 and tube 2 at, 3:33 min and 16 sec, respectively. The PCR products were analyzed on the gel electrophoresis with 7 kb and 500 bp for Tube1 and 2, respectively. The excised and purified bands were used for a 20-µl recombination reaction by adding 2 µl of PCR product from Tube 1 and 2, plus the addition of 8 µl DNase-RNase free water and GeneArt 2X Enzyme mix for 10 µl. The recombination reaction was stopped by adding 1 µl 0.5M EDTA. The product of this reaction (3 µl) was used to transform DH5aTH-T1R competent cells according to the manual and transformants were screened onto LB plate containing Chloramphenicol. The positive clones were picked for plasmid amplification and purification. The mutated clones were confirmed by BssHII digestion and sequencing.

**Table 2: Primers used for site directed mutagenesis to remove BssHII restriction sites in pSMART**

| Primer name | Sequence 5' - 3' | SEQ ID |
|---|---|---|
| pSopB-fwd | CATTACTCCTACGCGAGCAATTAACGAATCC | 21 |
| pSopB-rev | GGATTCGTTAATTGCTGCCGTAGGAGTAATG | 22 |
| pSopA-fwd | ACCCAGGTTAGGCGCACTGTCAATAACTATG | 23 |
| pSopA-rev | CATAGTTATTGACAGTGCGCCTAACCTGGGT | 24 |

### 3. Site-directed mutagenesis of Sail and Fsel restriction sites in the pSMART-BAC plasmid

To allow cloning genes or ATU cassette in the ATU2 and ATU3 simultaneously, Sall restriction site in pSMART-BAC needed to be abrogated. Because the Sail restriction site is located in a non-coding region of the plasmid, it could be mutated without concern for in-frame amino acid sequence. The mutation changed the T6196-A. Because the Fsel site of pSMART-BAC is located inside the OriV gene, we mutated G1020-C rendering 99.8% homology to the original OriV. Similarly, the site-directed mutagenesis was carried out using GeneART Site-Directed Mutagenesis PLUS kit (ThermoFisher Scientific). The primers were designed to replace nucleotide T6196-A and G1020-C (Table 2). The protocol used was the same as above with different extension times: 1 min 20 sec for TUBE1 and 2 min 35 sec for TUBE2. The mutated clones were confirmed by Sall and Fsel digestions and sequencing.

The resulting plasmid was named pSMAC-BAC.

### Construction of pSMAC-BAC containing full-length MV Schwarz infectious genome

The nucleotide sequence of the full-length antigenomic (+)RNA strand of Schwarz measles virus together with T7 RNA polymerase promoter and hammerhead ribozyme sequences in 5' and hepatitis delta ribozyme and T7 RNA polymerase terminator sequences in 3' was inserted into the pSMAC-BAC plasmid. This sequence was simply excised from plasmid pTM-MVSchw using Notl restriction enzyme and cloned into Notl open pSMAC plasmid after alkaline phosphatase treatment to prevent self-ligation of the vector (Figure 5). The resulting ligation reaction was used to transform NEB10 competent bacteria using manufacturer protocol. The positive clones were screened on LB/Chloramphenicol agar plates. Isolated clones were picked for plasmid amplification and purification and then sequenced to confirm the cloning. The resulting plasmid was named pSMAC-MV (Figure 6).

### Construction of pSMAC-BAC containing full-length MV infectious genome with ATU containing fluorescent proteins GFP and mCherry

To allow an easy observation of rescued virus and comparison between pTM and pSMAC rescue efficiency, we cloned into the pSMAC-BAC plasmid a recombinant MV full-length cDNA antigenomic sequence expressing simultaneously the GFP and the mCherry fluorescent proteins in a single position from ATU2 (Figure 7). We first generated an intergenic sequence allowing the expression of two additional sequences from a single ATU (Figure 7A). This sequence is composed of nucleotides 3331-3392 of MV Schwarz genomic sequence with a single T to A nucleotide change at position 3365 to mutate a Sall restriction site linked to nucleotides 1735-1802 of MV Schwarz genomic sequence containing the P promoter. Two restriction sites (6-base and 8-base respectively) were added at the 5' and 3' end of the intergenic sequence. Unique BstBI and Fsel restriction sites were added to facilitate further insertion of additional genes. The encoding sequences of fluorescent GFP and mCherry proteins were added on both sides of this intergenic sequence and the whole fragment was introduced into BsiWI and BssHII sites of pSMAC-MV ATU2 to allow the simultaneous expression of GFP and mCherry from ATU2 (Figure 7B). The resulting plasmid was named pSMAC-MV-GM (Figure 8).

### Recovery of infectious virus from pSMAC-MV-GM and comparison with pTM-MV-GM

To assess the efficiency of recovery of infectious recombinant virus from pSMAC-BAC compared to pTM, we used the previously described reverse genetics method with the helper cell line HEK293-T7-MV (use of the helper-cell-based rescue system is for example described by Radecke (Radecke et al., EMBO J., 1995, 14:5773-5784) and modified by Parks (Parks et al., J. Virol., 1999, 73:3560-3566)). After co-transfection of HEK293-T7-MV cells with 5 µg of vector plasmids together with 20 ng of pEMC-L plasmid and calcium phosphate in 35 mm dishes and heat shock at 42°C for three hours. After 48-hour incubation, transfected cells were overlaid onto monolayers of Vero cells (ATCC CCL-81) on 10 cm petri dishes. Cells were then gently covered with carboxy-methyl-cellulose (CMC 50% in DMEM and 5% FCS) to allow single clonal syncytia appearance and to avoid spreading and multiplication of clones. pTM-MV-GFP-mCherry (pTM-MV-GM) was used as a comparison control. Rescue experiments were done in triplicate and three separate biological repeats were performed. After three days fluorescent syncytia were counted. Fluorescent infectious recombinant virus was rescued 15-50 times more efficiently from pSMAC-MV-GM than from pTM-MV-GM (Figure 9). This demonstrates that pSMAC strongly enhances the recovery of recombinant MV because the sole difference is the backbone vector between pSMAC-MV-GM and pTM-MV-GM emphasizing the advantage of pBAC in rescue MV more efficiently.

### pSMAC plasmid accommodates larger additional sequences with multiple antigens constructions

The versatility of MV recombinant vaccine vector has demonstrated a solid proof of principle in humans. Clinical trials with a measles-chikungunya vaccine candidate (MV-CHIKV) have shown the safety and the immunogenicity of this vaccine platform in volunteers with preexisting immunity to measles. The use of pSMAC-MV plasmid as MV vector makes it possible to insert larger additional sequences into MV antigenomic sequence as BAC plasmids are known to stably accommodate large amounts of DNA. To prove this concept, we generated a single pSMAC-MV recombinant vector able to express simultaneously three large immunogens from three distinct pathogens: CHIKV, WNV, and ZIKV. We inserted antigens that we previously identified as protective when expressed individually from single MV vectors. The pSMAC-MV-CHIKV-WNV-ZIKV triple-antigen construct (Figure 13) contains 7476 bp additional nucleotides including the intergenic sequences, i.e. more than 3000 bp longer than the largest recombinant MV described so far.

The three antigens were inserted between P and M genes of MV vector as this position allows high expression of the additional transgenes. To express the three transgenes independently from this single position of MV genome, we added between each antigenic sequence an intergenic sequence based on MV cis-acting elements allowing their expression as standard MV genes. The intergenic sequence was designed to contain the essential elements, polyA tail and measles promoter and the CTT start-stop signal for MV polymerase. This intergenic sequence is a combination of N promoter (position 1734-1784) and a copied sequence of P/M intergenic region (position 3331-3409). Several restriction sites were also added to allow easy manipulation of genes within the multiple ATUs. The detailed sequences are depicted in Figures 10, 11 and 12.

### Construction of triple antigens of CHIKV, WNV, and ZIKV with MV intergenic region

The amplification of the triple immunogens with intergenic region (Figure 12) was performed by extension PCR using primers in Table 3. The fragment CHIKV-intergenic-WNV (CIW) was amplified in two-step extension PCR. First, CHIKV fragment was amplified using primers BsiWI-CHIKV and inter-anti-CHIKV using pTM2-CHIKV as template, while WNV fragment was amplified using primers inter-sens-Sbf1-WNV and WNV-BssHII with pTM-WNV as template. The PCR reactions were carried out using Q5 2x mastermix (NEB) protocol recommended by the manufacturer. Both PCR products were analyzed and purified for further use in the second PCR (Figure 12). The final product of CIW was digested with BsiWI and BssHII restriction enzymes, and then ligated into the pSMAC-MV to generate pSMAC-MV-CIW with the double antigenic fragment CIW inserted into ATU2.

**Table 3: Primers used for extension PCR in the construction of a triple antigens (CHIKV, WNV and ZIKV) construct, with intergenic region of MV for individually expression of the antigens**

| Primer name | Sequence 5' - 3' | SEQ ID |
|---|---|---|
| BsiWI-CHIKV | TTACGTACGATGGAGTTCATCCCAACCCAAAC | 27 |
| inter anti-CHIKV | ATAATGGATTTAGGTTGTACTAGTTGGGTCGACTGGCATGGGGTT GGCAGGTAAGTTGAGCTGTAGTTCGAACTATTAGTGCCTGCTGA ACGACAC | 28 |
| inter sens-Sbfl-WNV | AACCTAAATCCATTATAAAAAACTTAGGAACCAGGTCCACACAGC CGCCAGCCCATCAACCATCCACTCCCACGATTGGACCTGCAGGAT GAGAGTTGTGTTTGTC | 29 |
| inter anti-WNV | ATAATGGATTTAGGTTGTACTAGTTGGGTCGACTGGCATGGGGTT GGCAGGTAAGTTGAGCTGTAGTTCGAATTAGACAGCCTTCCCAA C | 30 |
| inter sens-ZIKV | AACCTAAATCCATTATAAAAAACTTAGGAACCAGGTCCACACAGC CGCCAGCCCATCAACCATCCACTCCCACGATTGGAGGCCGGCCA TGGAGAAGAAGCGGAGAG | 31 |
| WNV-BssHII | TGAGCGCGCTTAGACAGCCTTCCCAAC | 32 |
| ZIKV-BssHII | TTAGCGCGCTCATCAGGCAGACACG | 33 |
| Sbfl-WNV | ATTCCTGCAGGATGAGAGTTGTGTTTGTC | 34 |
| Sbfl-ZIKV | TTACCTGCAGGATGGAGAAGAAGCGGAGAG | 35 |
| BsiWI-ZIKV | TTACGTACGATGGAGAAGAAGCGGAGAG | 36 |
| inter anti-ZIKV | ATAATGGATTTAGGTTGTACTAGTTGGGTCGACTGGCATGGGGTT GGCAGGTAAGTTGAGCTGTAGTTCGAATCATCAGGCAGACACG GCGGTGGAC | 37 |
| inter sens-Sbfl-CHIKV | AACCTAAATCCATTATAAAAAACTTAGGAACCAGGTCCACACAGC CGCCAGCCCATCAACCATCCACTCCCACGATTGGACCTGCAGGAT GGAGTTCATCCCAACCCAAAC | 38 |
| CHIKV-BssHII | TTAGCGCGCCTATTAGTGCCTGCTGAACGAC | 39 |

The WNV-intergenic-ZIKV fragment (WIZ) was created in the same manner using the primers Sbf-WNV and inter-anti-WNV with pTM-WNV as template, while primers inter-sens-ZIKV and BssHII-ZIKV using pCDNA-ZIKV as template. The combined WIZ fragment was further digested with Sbfl and BssHII restriction enzymes. The purified WIZ fragment was then ligated to the CIW fragment similarly cut to generate pSMAC-MV-CIWIZ containing the triple antigenic fragment CIWIZ inserted into ATU2 (Figure 12). We have also generated in the same way the bivalent vaccine candidates CHIKV-inter-ZIKV (CIZ) and ZIKV-inter-CHIKV (ZIC) in the same pSMAC-MV vector. Plasmids were fully sequenced to control integrity of cloning.

### Rescue of multivalent recombinant MV

The recovery of infectious recombinant MV expressing multivalent antigens (MV-CIWIZ) from pSMAC-MV-CIWIZ was performed as above³. The recombinant virus clones were picked from the coculture plates, plaque purified and amplified for stock preparation and further characterization. The targeted antigens expression was detected using western blot stained with antibodies directed to CHIKV E1 envelope protein or Flavivirus envelope (4G2) (Figure 14).

The sequence of MV-CIWIZ rescued virus was verified after viral RNA extraction, cDNA synthesis and RT-PCR amplification with specific primers. The sequencing result confirmed that the recombinant MV-CIWIZ contains the exact sequence of the triple antigen CIWIZ.

Therefore, using pSMAC enables constructing and manipulating recombinant infectious MV carrying very large inserted additional genetic material that can still be rescued successfully.

### Culture of recombinant MV on Vero cells CCL-81 approved for human use cultures without fetal calf serum

In order to prepare recombinant MV seeds ready for industrial manufacturing with no risk of contamination by BSE agents we developed a culture method to grow recombinant MV on Vero cells approved for human use and cultured in serum-free medium.

Vero CCL-81 (ATCC) cells seeded at a density of 4 x 10⁴ cells/cm² (3 x 10⁶ cells/T-75 flask) are cultured as monolayers on T-75 flasks in VP-SFM medium (Gibco/Invitrogen, ref 11681-020) supplemented with 4 mM L-glutamine (Invitrogen, ref 25030024) and Penicilin streptomycin (Invitrogen, ref 15140122) by incubation at 37°C, 5% CO2 in humid incubator. Cells are passaged after TrypLE enzyme (Invitrogen, ref 12563011) treatment for 5 minutes at room temperature. Enzyme action is stopped by dilution in 20 ml of VP-SFM medium.

Sub-confluent cells (70-80% confluence) are washed with 10 ml of D-PBS without calcium and magnesium (Invitrogen, ref 14190094) and infected at 32°C with MV-GFP in 2 ml of VP-SFM medium at a multiplicity of infection of 0.01. After 2 hours of adsorption, the inoculum is replaced by 15 - 20 ml of VP-SFM medium. Culture is continued for 7 days. Medium or cells are regularly collected to determine the titer of produced virus by TCID50 titration on Vero cells (Figures 15, 16). The results show that recombinant MV can be produced at titers up to 10⁸ TCID50 in medium after 7 days of production.

### Bibliography

Lund, N., et al., The Effect of Oral Polio Vaccine at Birth on Infant Mortality: A Randomized Trial. Clin Infect Dis, 2015. 61(10): p. 1504-11.
Aaby, P. and C.S. Benn, Stopping live vaccines after disease eradication may increase mortality. Vaccine, 2020. 38(1): p. 10-14.
Combredet, C., et al., A molecularly cloned Schwarz strain of measles virus vaccine induces strong immune responses in macaques and transgenic mice. J Virol, 2003. 77(21): p. 11546-54.
Calain, P. and L. Roux, The rule of six, a basic feature for efficient replication of Sendai virus defective interfering RNA. J Virol, 1993. 67(8): p. 4822-30.
Frantz, P.N., S. Teeravechyan, and F. Tangy, Measles-derived vaccines to prevent emerging viral diseases. Microbes Infect, 2018. 20(9-10): p. 493-500.
Guerbois, M., et al., Live attenuated measles vaccine expressing HIV-1 Gag virus like particles covered with gp160DeltaV1V2 is strongly immunogenic. Virology, 2009. 388(1): p. 191-203.
Lorin, C., et al., Toxicology, biodistribution and shedding profile of a recombinant measles vaccine vector expressing HIV-1 antigens, in cynomolgus macaques. Naunyn Schmiedebergs Arch Pharmacol, 2012. 385(12): p. 1211-25.
Despres, P., et al., Live measles vaccine expressing the secreted form of the West Nile virus envelope glycoprotein protects against West Nile virus encephalitis. J Infect Dis, 2005. 191(2): p. 207-14.
Brandler, S., et al., Measles vaccine expressing the secreted form of West Nile virus envelope glycoprotein induces protective immunity in squirrel monkeys, a new model of West Nile virus infection. J Infect Dis, 2012. 206(2): p. 212-9.
1Brandler, S., et al., Pediatric measles vaccine expressing a dengue antigen induces durable serotype-specific neutralizing antibodies to dengue virus. PLoS Negl Trop Dis, 2007. 1(3): p. e96.
Brandler, S., et al., Pediatric measles vaccine expressing a dengue tetravalent antigen elicits neutralizing antibodies against all four dengue viruses. Vaccine, 2010. 28(41): p. 6730-9.
del Valle, J.R., et al., A vectored measles virus induces hepatitis B surface antigen antibodies while protecting macaques against measles virus challenge. J Virol, 2007. 81(19): p. 10597-605.
Cantarella, G., et al., Recombinant measles virus-HPV vaccine candidates for prevention of cervical carcinoma. Vaccine, 2009. 27(25-26): p. 3385-90.
Brandler, S., et al., A recombinant measles vaccine expressing chikungunya virus-like particles is strongly immunogenic and protects mice from lethal challenge with chikungunya virus. Vaccine, 2013. 31(36): p. 3718-25.
Yoneda, M., et al., Recombinant measles virus vaccine expressing the Nipah virus glycoprotein protects against lethal Nipah virus challenge. PLoS One, 2013. 8(3): p. e58414.
Yamaji, Y. and T. Nakayama, Recombinant measles viruses expressing respiratory syncytial virus proteins induced virus-specific CTL responses in cotton rats. Vaccine, 2014. 32(35): p. 4529-36.
Escriou, N., et al., Protection from SARS coronavirus conferred by live measles vaccine expressing the spike glycoprotein. Virology, 2014. 452-453: p. 32-41.
Malczyk, A.H., et al., A Highly Immunogenic and Protective Middle East Respiratory Syndrome Coronavirus Vaccine Based on a Recombinant Measles Virus Vaccine Platform. J Virol, 2015. 89(22): p. 11654-67.
Mateo, M., et al., Vaccines inducing immunity to Lassa virus glycoprotein and nucleoprotein protect macaques after a single shot. Sci Transl Med, 2019. 11(512).
Ramsauer, K., et al., Immunogenicity, safety, and tolerability of a recombinant measles-virus-based chikungunya vaccine: a randomised, double-blind, placebo-controlled, active-comparator, first-in-man trial. Lancet Infect Dis, 2015. 15(5): p. 519-27.
Reisinger, E.C., et al., Immunogenicity, safety, and tolerability of the measles-vectored chikungunya virus vaccine MV-CHIK: a double-blind, randomised, placebo-controlled and active-controlled phase 2 trial. Lancet, 2019. 392(10165): p. 2718-2727.
Domingo, E., et al., The quasispecies (extremely heterogeneous) nature of viral RNA genome populations: biological relevance--a review. Gene, 1985. 40(1): p. 1-8.
Shi, J. and D.P. Biek, A versatile low-copy-number cloning vector derived from plasmid F. Gene, 1995. 164(1): p. 55-8.
Shizuya, H. and H. Kouros-Mehr, The development and applications of the bacterial artificial chromosome cloning system. Keio J Med, 2001. 50(1): p. 26-30.
Tischer, B.K. and B.B. Kaufer, Viral bacterial artificial chromosomes: generation, mutagenesis, and removal of mini-F sequences. J Biomed Biotechnol, 2012. 2012: p. 472537.
Borenstein, R. and N. Frenkel, Cloning human herpes virus 6A genome into bacterial artificial chromosomes and study of DNA replication intermediates. Proc Natl Acad Sci U S A, 2009. 106(45): p. 19138-43.
Cottingham, M.G., et al., Recombination-mediated genetic engineering of a bacterial artificial chromosome clone of modified vaccinia virus Ankara (MVA). PLoS One, 2008. 3(2): p. e1638.
Domi, A. and B. Moss, Cloning the vaccinia virus genome as a bacterial artificial chromosome in Escherichia coli and recovery of infectious virus in mammalian cells. Proc Natl Acad Sci U S A, 2002. 99(19): p. 12415-20.
Meisinger-Henschel, C., et al., Introduction of the six major genomic deletions of modified vaccinia virus Ankara (MVA) into the parental vaccinia virus is not sufficient to reproduce an MVA-like phenotype in cell culture and in mice. J Virol, 2010. 84(19): p. 9907-19.
Roth, S.J., et al., Recovery of infectious virus from full-length cowpox virus (CPXV) DNA cloned as a bacterial artificial chromosome (BAC). Vet Res, 2011. 42: p. 3.
Almazan, F., et al., Engineering the largest RNA virus genome as an infectious bacterial artificial chromosome. Proc Natl Acad Sci U S A, 2000. 97(10): p. 5516-21.
Almazan, F., et al., Construction of a severe acute respiratory syndrome coronavirus infectious cDNA clone and a replicon to study coronavirus RNA synthesis. J Virol, 2006. 80(21): p. 10900-6.
Fan, Z.C. and R.C. Bird, An improved reverse genetics system for generation of bovine viral diarrhea virus as a BAC cDNA. J Virol Methods, 2008. 149(2): p. 309-15.
Rasmussen, T.B., et al., Generation of recombinant pestiviruses using a full-genome amplification strategy. Vet Microbiol, 2010. 142(1-2): p. 13-7.
Jengarn, J., et al., Genetic manipulation of porcine epidemic diarrhoea virus recovered from a full-length infectious cDNA clone. J Gen Virol, 2015. 96(8): p. 2206-18.
Pu, S.Y., et al., Successful propagation of flavivirus infectious cDNAs by a novel method to reduce the cryptic bacterial promoter activity of virus genomes. J Virol, 2011. 85(6): p. 2927-41.
Aubry, F., et al., Flavivirus reverse genetic systems, construction techniques and applications: a historical perspective. Antiviral Res, 2015. 114: p. 67-85.
O'Connor, M., M. Peifer, and W. Bender, Construction of large DNA segments in Escherichia coli. Science, 1989. 244(4910): p. 1307-12.

## Claims

1. A nucleic acid construct which comprises a cDNA molecule encoding a full length antigenomic (+) RNA strand of a non-segmented negative-sense single-stranded RNA virus, especially of a measles virus (MV), wherein the cDNA molecule further comprises inserted therein, at least a first additional transcription unit (ATU) and a second additional transcription unit (ATU), wherein the at least first and second additional transcription units (ATUs) are localized within a single intergenic region of the cDNA encoding the antigenomic (+) RNA strand as a single expression cassette, and wherein each ATU comprises a heterologous polynucleotide operably inserted within the ATU allowing the expression of a heterologous polypeptide encoded by the heterologous polynucleotide.

2. The nucleic acid construct of claim 1, wherein the non-segmented negative-sense single-stranded RNA virus is a measles virus (MV) originating from an attenuated strain, in particular an attenuated virus strain selected from the group consisting of the Schwarz strain, the Zagreb strain, the AIK-C strain, the Moraten strain, the Philips strain, the Beckenham 4A strain, the Beckenham 16 strain, the Edmonston seed A strain, the Edmonston seed B strain, the CAM-70 strain, the TD 97 strain, the Leningrad-16 strain, the Shanghai 191 strain and the Belgrade strain, preferably the Schwarz strain, the AIK-C strain and the Zagreb strain, in particular the Schwarz strain of SEQ ID No. 62, the AIK-C strain of SEQ ID No. 58 and the Zagreb strain of SEQ ID No. 59.

3. The nucleic acid construct of claim 1 or 2, wherein the first ATU and the second ATU are localized: (i) between the P gene and the M gene of the measles virus in the cDNA molecule or (ii) between the H gene and the L gene of the measles virus in the cDNA molecule or (iii) between the N gene of the measles virus and the T7RNA polymerase promoter in the cDNA molecule, and optionally wherein the number of consecutive nucleotides in the nucleic acid construct is a multiple of six and/or wherein the number of consecutive nucleotides in the recombinant cDNA molecule is a multiple of six.

4. The nucleic acid construct according to any one of claims 1 to 3, wherein the first ATU and/or the second ATU comprises or consists in the nucleotide sequence of SEQ ID No: 1 wherein the nucleotide sequence from position 79 to position 796 is substituted by a heterologous polynucleotide sequence encoding a heterologous polypeptide, in particular wherein said heterologous polynucleotides in the first ATU and the second ATU encode heterologous polypeptides that are different from each other and optionally wherein the first and the second heterologous polynucleotides are separated in the single intergenic region of the cDNA by a spacer sequence such as the sequence of polynucleotide of SEQ ID No: 2 or SEQ ID No: 11.

5. The nucleic acid construct according to any one of claims 1 to 3, wherein the first ATU and/or the second ATU comprises or consists from its 5' end towards its 3' end of (i) the nucleotide sequence of SEQ ID No: 16 or SEQ ID No. 19 or SEQ ID No. 20 or SEQ ID No. 40 or SEQ ID No. 41 or SEQ ID No. 42 or SEQ ID No. 43 or SEQ ID No. 44 or SEQ ID No. 45, (ii) a heterologous polynucleotide sequence encoding a heterologous polypeptide, and (iii) the nucleotide sequence of SEQ ID No. 17 or SEQ ID No. 46 or SEQ ID No. 47 or SEQ ID No. 48 or SEQ ID No. 49 or SEQ ID No. 50 or SEQ ID No. 51; or wherein the first ATU and/or the second ATU comprises or consists of the nucleotide sequence of SEQ ID No. 52, SEQ ID No. 53, SEQ ID No. 54, SEQ ID No. 55, SEQ ID No. 56, SEQ ID No. 57, wherein n corresponds to a heterologous polynucleotide sequence encoding a heterologous polypeptide, and
wherein the heterologous polynucleotides presents within the first ATU is different from the heterologous polynucleotide presents within the second ATU, and optionally wherein the first and the second heterologous polynucleotides are separated in the single intergenic region of the cDNA by a spacer sequence such as the sequence of polynucleotide of SEQ ID No: 2 or SEQ ID No. 11.

6. The nucleic acid construct according to any one of claims 1 to 4, wherein a third ATU is localized within the same intergenic region where the first ATU and the second ATU are localized, the third ATU comprising a heterologous polynucleotide operably linked within the third ATU allowing the expression of a heterologous polypeptide encoded by the heterologous polynucleotide wherein said heterologous polypeptide of the third ATU is different from the heterologous polypeptides encoded by the first and the second ATUs, and wherein heterologous polynucleotides within a single intergenic region of the cDNA are separated from each other by a spacer sequence comprising or consisting of the polynucleotide of SEQ ID No.: 2 or SEQ ID No.: 11.

7. The nucleic acid construct according to claim 5, wherein the third ATU (i) comprises or consists in the nucleotide sequence of SEQ ID No: 1 wherein the nucleotide sequence from position 79 to position 796 is substituted by a heterologous polynucleotide sequence encoding a heterologous polypeptide; in particular the third ATU comprises of consists in the same nucleotide sequence as the first ATU or the second ATU but for the coding heterologous polynucleotide that is contains, and (ii) wherein the heterologous polynucleotide of the third ATU is separated from another heterologous polynucleotide inserted within the closest ATU in the single intergenic region of the cDNA by the polynucleotide of sequence SEQ ID No.: 2 or SEQ ID No.: 11.

8. The nucleic acid construct according to any one of claims 1 to 6, wherein each of the first ATU and the second ATU and when present the third ATU comprises a heterologous polynucleotide that encodes a heterologous polypeptide that is different from the heterologous polypeptide encoded by the other ATUs and optionally wherein the heterologous polynucleotide or at least one of the ATUs, in particular of at least two ATUs or of all the ATUs, encodes multiple heterologous polypeptides.

9. The nucleic acid construct according to any one of claims 1 to 8, wherein the heterologous polypeptides encoded by the heterologous polynucleotides inserted within the ATUs are immunogenic polypeptides originating from or derived from at least one pathogen infecting human, in particular from at least one virus infecting human, in particular the Chikungunya virus, the West Nile virus, the Zika virus, the SARS virus, the coronavirus, and/or the HIV, in particular a heterologous polypeptide comprising the C, E2 and E1 proteins of the Chikungunya virus, the sE protein of the West Nile virus, the prME protein of the Zika virus, the S and M proteins of the SARS virus and/or the S and M proteins of the coronavirus, in particular the S and M proteins of the SARS-CoV-2, and optionally wherein the heterologous polynucleotides originate or derive from different virus types.

10. The nucleic acid construct according to any one of claims 1 to 9, wherein the intergenic region localized between the P gene and the M gene of the measles virus comprises the nucleotide sequence of SEQ ID No: 4.

11. A recombinant bacterial artificial chromosome (BAC) plasmid wherein the nucleic acid construct according to any one of claims 1 to 10 is operably cloned.

12. The recombinant BAC plasmid according to claim 11, wherein the BAC nucleotide sequence is devoid of (i) a T7RNA polymerase promoter and/or of a CMV promoter and/or (ii) cloning sites that are present in the ATU sequences.

13. The recombinant BAC plasmid according to claim 11 or 12, wherein the bacterial artificial chromosome plasmid is selected from the group consisting of the pSMART BAC plasmid and its derivatives, pEZ-BAC plasmid, pBeloBAC11 plasmid , pBACe3.6, pBAC/OriV, pBAC-RT, pHA1, pHA2, pTARBAC2 and its derivatives, and the pBAC contruct inserted in Transmissible gastroenteritis coronavirus (TGEV), in particular is a pSMAC BAC, pEZ-BAC and pBeloBAC11 plasmid,, in particular is the pSMAC-BAC plasmid, in particular comprises or consists of the polynucleotide of SEQ ID No. 3.

14. The recombinant BAC plasmid according to any one of claims 11 to 13, wherein the promoter sequences localized on the recombinant BAC plasmid are different from the promoter sequences localized on the cDNA molecule.

15. The nucleic acid construct according to any one of claims 1 to 11 or the recombinant BAC plasmid according to any one of claims 12 to 15, wherein the first ATU and the second ATU comprise or consist of different polynucleotides that are selected among polynucleotides encoding heterologous polypeptides comprising or consisting in amino acid sequences of to SEQ ID No. 13 and SEQ ID No.: 14, or selected among polynucleotides encoding heterologous polypeptides comprising or consisting of amino acid sequences of SEQ ID No. 13 and SEQ ID No.: 15, and wherein the first heterologous polynucleotide and the second heterologous polynucleotide are separated by a spacer polynucleotide comprising or consisting in the nucleotide sequence of SEQ ID No: 2 or SEQ ID No:11.

16. The nucleic acid construct according to claim 1 to 10 or the recombinant BAC plasmid according to any one of claims 11 to 15 and comprising a third ATU, wherein the first ATU, the second ATU and the third ATU comprise(s) or consist(s) of different polynucleotides encoding heterologous polypeptides comprising or consisting in amino acid sequences that are selected among SEQ ID No. 13, SEQ ID No.: 14 and SEQ ID No.: 15, and wherein the heterologous polynucleotides are separated by a spacer polynucleotide comprising or consisting in the nucleotide sequence of SEQ ID No: 2 or SEQ ID No:11.

17. The nucleic acid construct according to any one of claims 1 to 10 or the recombinant BAC plasmid according to any one of claims 11 to 16, wherein the intergenic region localized between the P gene and the M gene of the measles virus in the cDNA molecule comprises all the ATUs, and wherein the heterologous polynucleotides inserted in the intergenic region are separated by a spacer polynucleotide comprising or consisting of SEQ ID No: 2 or SEQ ID No. 11.

18. A recombinant infectious non-segmented negative-sense single-stranded RNA virus, especially a recombinant infectious Measles virus, wherein the genome of said recombinant infectious virus comprises or consists in a nucleic acid construct according to any one of claims 1 to 10.

19. A process for rescuing recombinant infectious non-segmented negative-sense single-stranded RNA virus, especially recombinant infectious measles virus expressing at least one heterologous polypeptide expressed from a heterologous nucleotide sequence inserted within an ATU localized within a cDNA molecule encoding a full length antigenomic (+) RNA strand of a measles virus (MV); comprising:
(e) In cells, in particular HEK293 helper cells, stably expressing T7 RNA polymerase and measles virus N and P proteins and/or transfected with an expression vector encoding T7 RNA polymerase and/or transfected with an expression vector encoding measles virus N and P proteins, transfecting the nucleic acid construct according to any one of claims 1 to 10;
(f) Maintaining the transfected cells of step (a) in conditions suitable for the production of recombinant infectious virus and/or virus like particles;
(g) Infecting cells, in particular Vero cells, in conditions enabling the propagation of the recombinant infectious virus by co-cultivating these cells with the cells issued from step (b); in particular at 32 °C;
(h) Harvesting the recombinant infectious virus and/or virus like particles, in particular at 32 °C.

20. A seed preparation or a stock preparation of a recombinant infectious non-segmented negative-sense single-stranded RNA virus, especially of a recombinant infectious measles virus that consists in an single virus clone or respectively an amplified single virus clone of a recombinant infectious virus rescued by reverse genetics from the nucleic acid construct according to anyone of claims 1 to 10 or from the recombinant BAC plasmid according to any one of claims 11 to 16.

21. A vaccine which comprises an clonally selected and amplified recombinant infectious negative-sense single-stranded RNA virus, especially a clonally selected and amplified recombinant infectious measles virus obtainable from the rescue by reverse genetics of the nucleic acid construct according to anyone of claims 1 to 10 or from the recombinant BAC plasmid according to any one of claims 11 to 16.

22. A vaccine according to claim 21 which enables stable expression of multiple heterologous polypeptides from the nucleic acid construct according to anyone of claims 1 to 10 or from the recombinant BAC plasmid according to any one of claims 11 to 16, wherein such stable expression is **characterized by** being maintained over at least 12 passages of the rescued recombinant infectious negative-sense single-stranded RNA virus when amplified on Vero cells, especially of the amplified recombinant infectious measles virus.

23. Ribonucleoprotein of a recombinant measles virus strain which comprises a RNA genome assembled with the transcriptase complex proteins of the measles virus wherein the genome contains the recombinant full length antigenomic (+) RNA strand of the measles virus (MV) and inserted therein, sequences encoding at least a first additional transcription unit (ATU) and a second additional transcription unit (ATU), wherein the at least first and second additional transcription units (ATUs) are localized within a single intergenic region of the recombinant antigenomic (+) RNA strand as a single expression cassette, and wherein each ATU comprises a heterologous polynucleotide operably inserted within the ATU allowing the expression of a heterologous polypeptide encoded by the heterologous polynucleotide as disclosed in any one of claims 1 to 10.


SEQUENCE LISTING

<110>  INSTITUT PASTEUR

<120>  AN IMPROVED MEASLES VIRUS VACCINE VECTOR BASED ON MULTIPLE TANDEM
       ADDITIONAL TRANSCRIPTION UNITS (ATUs)

<130>  B14231/AD/HLQ/KN

<140>  EPxxxxx
<141>  2020-07-08

<160>  62

<170>  PatentIn version 3.5

<210>  1
<211>  823
<212>  DNA
<213>  artificial sequence

<220>
<223>  Synthetic sequence

<400>  1
ctagcctacc ctccatcatt gttataaaaa acttaggaac caggtccaca cagccgccag      60

cccatcaacg cgtacgatgg tgagcaaggg cgaggagctg ttcaccgggg tggtgcccat     120

cctggtcgag ctggacggcg acgtaaacgg ccacaagttc agcgtgtccg gcgagggcga     180

gggcgatgcc acctacggca agctgaccct gaagttcatc tgcaccaccg gcaagctgcc     240

cgtgccctgg cccaccctcg tgaccaccct gacctacggc gtgcagtgct tcagccgcta     300

ccccgaccac atgaagcagc acgacttctt caagtccgcc atgcccgaag gctacgtcca     360

ggagcgcacc atcttcttca aggacgacgg caactacaag acccgcgccg aggtgaagtt     420

cgagggcgac accctggtga accgcatcga gctgaagggc atcgacttca aggaggacgg     480

caacatcctg gggcacaagc tggagtacaa ctacaacagc cacaacgtct atatcatggc     540

cgacaagcag aagaacggca tcaaggtgaa cttcaagatc cgccacaaca tcgaggacgg     600

cagcgtgcag ctcgccgacc actaccagca gaacaccccc atcggcgacg gccccgtgct     660

gctgcccgac aaccactacc tgagcaccca gtccgccctg agcaaagacc ccaacgagaa     720

gcgcgatcac atggtcctgc tggagttcgt gaccgccgcc gggatcactc tcggcatgga     780

cgagctgtac aagtaatagt tcgaactaca gctcaactta cct                       823


<210>  2
<211>  144
<212>  DNA
<213>  artificial sequence

<220>
<223>  Synthetic sequence

<400>  2

ttcgaactac agctcaactt acctgccaac cccatgccag acgacccaac tagtacaacc 60

taaatccatt ataaaaaact taggaaccag gtccacacag ccgccagccc atcaaccatc 120

cactcccacg attggaggcc ggcc 144

<210> 3
<211> 7648
<212> DNA
<213> artificial sequence

<220>
<223> Synthetic sequence

<400> 3
ccatcacatt gggatcctaa gcttagaatt ccacgtggac aagttgtcgc ggccgcttct 60

atagtgtcac ctaaatacta gtgactccag cgtaactgga ctggccacag ttaggccgca 120

aatgtaatca cactggctca ccttcgggtg ggcctttctg cgttcgcgag tggacccgat 180

aagctcatgg agcggcgtaa ccgtcgcaca ggaaggacag agaaagcgcg gatctgggaa 240

gtgacggaca gaacggtcag gacctggatt ggggaggcgg ttgccgccgc tgctgctgac 300

ggtgtgacgt tctctgttcc ggtcacacca catacgttcc gccattccta tgcgatgcac 360

atgctgtatg ccggtatacc gctgaaagtt ctgcaaagcc tgatgggaca taagtccatc 420

agttcaacgg aagtctacac gaaggttttt gcgctggatg tggctgcccg gcaccgggtg 480

cagtttgcga tgccggagtc tgatgcggtt gcgatgctga aacaattatc ctgagaataa 540

atgccttggc ctttatatgg aaatgtggaa ctgagtggat atgctgtttt tgtctgttaa 600

acagagaagc tggctgttat ccactgagaa gcgaacgaaa cagtcgggaa aatctcccat 660

tatcgtagag atccgcatta ttaatctcag gagcctgtgt agcgtttata ggaagtagtg 720

ttctgtcatg atgcctgcaa gcggtaacga aaacgatttg aatatgcctt caggaacaat 780

agaaatcttc gtgcggtgtt acgttgaagt ggagcggatt atgtcagcaa tggacagaac 840

aacctaatga acacagaacc atgatgtggt ctgtcctttt acagccagta gtgctcgccg 900

cagtcgagcg acagggcgaa gccctcggct ggttgccctc gccgctgggc tggcggccgt 960

ctatggccct gcaaacgcgc cagaaacgcc gtcgaagccg tgtgcgagac accgcggccg 1020

gccgccggcg ttgtggatac ctcgcggaaa acttggccct cactgacaga tgaggggcgg 1080

acgttgacac ttgaggggcc gactcacccg gcgcggcgtt gacagatgag gggcaggctc 1140

gatttcggcc ggcgacgtgg agctggccag cctcgcaaat cggcgaaaac gcctgatttt 1200

acgcgagttt cccacagatg atgtggacaa gcctggggat aagtgccctg cggtattgac 1260

acttgagggg cgcgactact gacagatgag gggcgcgatc cttgacactt gaggggcaga 1320

gtgctgacag atgaggggcg cacctattga catttgaggg gctgtccaca ggcagaaaat 1380

ccagcatttg caagggtttc cgcccgtttt tcggccaccg ctaacctgtc ttttaacctg 1440

cttttaaacc aatatttata aaccttgttt ttaaccaggg ctgcgccctg tgcgcgtgac 1500

cgcgcacgcc gaaggggggt gccccccctt ctcgaaccct cccggtcgag tgagcgagga 1560

agcaccaggg aacagcactt atatattctg cttacacacg atgcctgaaa aaacttccct 1620

tggggttatc cacttatcca cggggatatt tttataatta ttttttttat agtttttaga 1680

tcttcttttt tagagcgcct tgtaggcctt tatccatgct ggttctagag aaggtgttgt 1740

gacaaattgc cctttcagtg tgacaaatca ccctcaaatg acagtcctgt ctgtgacaaa 1800

ttgcccttaa ccctgtgaca aattgccctc agaagaagct gttttttcac aaagttatcc 1860

ctgcttattg actctttttt atttagtgtg acaatctaaa aacttgtcac acttcacatg 1920

gatctgtcat ggcggaaaca gcggttatca atcacaagaa acgtaaaaat agcccgcgaa 1980

tcgtccagtc aaacgacctc actgaggcgg catatagtct ctcccgggat caaaaacgta 2040

tgctgtatct gttcgttgac cagatcagaa aatctgatgg caccctacag gaacatgacg 2100

gtatctgcga gatccatgtt gctaaatatg ctgaaatatt cggattgacc tctgcggaag 2160

ccagtaagga tatacggcag gcattgaaga gtttcgcggg gaaggaagtg gttttttatc 2220

gccctgaaga ggatgccggc gatgaaaaag gctatgaatc ttttccttgg tttatcaaac 2280

gtgcgcacag tccatccaga gggctttaca gtgtacatat caacccatat ctcattccct 2340

tctttatcgg gttacagaac cggtttacgc agtttcggct tagtgaaaca aaagaaatca 2400

ccaatccgta tgccatgcgt ttatacgaat ccctgtgtca gtatcgtaag ccggatggct 2460

caggcatcgt ctctctgaaa atcgactgga tcatagagcg ttaccagctg cctcaaagtt 2520

accagcgtat gcctgacttc cgccgccgct tcctgcaggt ctgtgttaat gagatcaaca 2580

gcagaactcc aatgcgcctc tcatacattg agaaaaagaa aggccgccag acgactcata 2640

tcgtattttc cttccgcgat atcacttcca tgacgacagg atagtctgag ggttatctgt 2700

cacagatttg agggtggttc gtcacatttg ttctgaccta ctgagggtaa tttgtcacag 2760

ttttgctgtt tccttcagcc tgcatggatt ttctcatact ttttgaactg taatttttaa 2820

ggaagccaaa tttgagggca gtttgtcaca gttgatttcc ttctctttcc cttcgtcatg 2880

tgacctgata tcgggggtta gttcgtcatc attgatgagg gttgattatc acagtttatt 2940

actctgaatt ggctatccgc gtgtgtacct ctacctggag tttttcccac ggtggatatt 3000

tcttcttgcg ctgagcgtaa gagctatctg acagaacagt tcttctttgc ttcctcgcca 3060

gttcgctcgc tatgctcggt tacacggctg cggcgagcgc tagtgataat aagtgactga 3120

ggtatgtgct cttcttatct ccttttgtag tgttgctctt attttaaaca actttgcggt 3180

tttttgatga ctttgcgatt ttgttgttgc tttgcagtaa attgcaagat ttaataaaaa 3240

aacgcaaagc aatgattaaa ggatgttcag aatgaaactc atggaaacac ttaaccagtg 3300

cataaacgct ggtcatgaaa tgacgaaggc tatcgccatt gcacagttta atgatgacag 3360

cccggaagcg aggaaaataa cccggcgctg gagaataggt gaagcagcgg atttagttgg 3420

ggtttcttct caggctatca gagatgccga gaaagcaggg cgactaccgc acccggatat 3480

ggaaattcga ggacgggttg agcaacgtgt tggttataca attgaacaaa ttaatcatat 3540

gcgtgatgtg tttggtacgc gattgcgacg tgctgaagac gtatttccac cggtgatcgg 3600

ggttgctgcc cataaaggag gcgtttacaa aacctcagtt tctgttcatc ttgctcagga 3660

tctggctctg aaggggctac gtgttttgct cgtggaaggt aacgaccccc agggaacagc 3720

ctcaatgtat cacggatggg taccagatct tcatattcat gcagaagaca ctctcctgcc 3780

tttctatctt ggggaaaagg acgatgtcac ttatgcaata aagcccactt gctggccggg 3840

gcttgacatt attccttcct gtctggctct gcaccgtatt gaaactgagt taatgggcaa 3900

atttgatgaa ggtaaactgc ccaccgatcc acacctgatg ctccgactgg ccattgaaac 3960

tgttgctcat gactatgatg tcatagttat tgacagcgcg cctaacctgg gtatcggcac 4020

gattaatgtc gtatgtgctg ctgatgtgct gattgttccc acgcctgctg agttgtttga 4080

ctacacctcc gcactgcagt ttttcgatat gcttcgtgat ctgctcaaga acgttgatct 4140

taaagggttc gagcctgatg tacgtatttt gcttaccaaa tacagcaata gtaatggctc 4200

tcagtccccg tggatggagg agcaaattcg ggatgcctgg ggaagcatgg ttctaaaaaa 4260

tgttgtacgt gaaacggatg aagttggtaa aggtcagatc cggatgagaa ctgtttttga 4320

acaggccatt gatcaacgct cttcaactgg tgcctggaga aatgctcttt ctatttggga 4380

acctgtctgc aatgaaattt tcgatcgtct gattaaacca cgctgggaga ttagataatg 4440

aagcgtgcgc ctgttattcc aaaacatacg ctcaatactc aaccggttga agatacttcg 4500

ttatcgacac cagctgcccc gatggtggat tcgttaattg cgcgcgtagg agtaatggct 4560

cgcggtaatg ccattacttt gcctgtatgt ggtcgggatg tgaagtttac tcttgaagtg 4620

ctccggggtg atagtgttga gaagacctct cgggtatggt caggtaatga acgtgaccag 4680

gagctgctta ctgaggacgc actggatgat ctcatccctt cttttctact gactggtcaa 4740

cagacaccgg cgttcggtcg aagagtatct ggtgtcatag aaattgccga tgggagtcgc 4800

cgtcgtaaag ctgctgcact taccgaaagt gattatcgtg ttctggttgg cgagctggat 4860

gatgagcaga tggctgcatt atccagattg ggtaacgatt atcgcccaac aagtgcttat 4920

gaacgtggtc agcgttatgc aagccgattg cagaatgaat ttgctggaaa tatttctgcg 4980

ctggctgatg cggaaaatat ttcacgtaag attattaccc gctgtatcaa caccgccaaa 5040

ttgcctaaat cagttgttgc tctttttctt caccccggtg aactatctgc ccggtcaggt 5100

gatgcacttc aaaaagcctt tacagataaa gaggaattac ttaagcagca ggcatctaac 5160

cttcatgagc agaaaaaagc tggggtgata tttgaagctg aagaagttat cactctttta 5220

acttctgtgc ttaaaacgtc atctgcatca agaactagtt taagctcacg acatcagttt 5280

gctcctggag cgacagtatt gtataagggc gataaaatgg tgcttaacct ggacaggtct 5340

cgtgttccaa ctgagtgtat agagaaaatt gaggccattc ttaaggaact tgaaaagcca 5400

gcaccctgat gcgaccacgt tttagtctac gtttatctgt ctttacttaa tgtcctttgt 5460

tacaggccag aaagcataac tggcctgaat attctctctg ggcccactgt tccacttgta 5520

tcgtcggtct gataatcaga ctgggaccac ggtcccactc gtatcgtcgg tctgattatt 5580

agtctgggac cacggtccca ctcgtatcgt cggtctgatt attagtctgg gaccacggtc 5640

ccactcgtat cgtcggtctg ataatcagac tgggaccacg gtcccactcg tatcgtcggt 5700

ctgattatta gtctgggacc atggtcccac tcgtatcgtc ggtctgatta ttagtctggg 5760

accacggtcc cactcgtatc gtcggtctga ttattagtct ggaaccacgg tcccactcgt 5820

atcgtcggtc tgattattag tctgggacca cggtcccact cgtatcgtcg gtctgattat 5880

tagtctggga ccacgatccc actcgtgttg tcggtctgat tatcggtctg ggaccacggt 5940

cccacttgta ttgtcgatca gactatcagc gtgagactac gattccatca atgcctgtca 6000

agggcaagta ttgacatggt cgtcgtaacc tgtagaacgg agtaacctcg gtgtgcggtt 6060

gtatgcctgc tgtggattgc tgctgtgtcc tgcttatcca caacattttg cgcacggtta 6120

tgtggacaaa atacctggtt acccaggccg tgccggcacg ttaaccgggc tgcatccgat 6180

gcaagtgtgt cgctgtcgac gagctcgcga gctcggacat gaggttgccc cgtattcagt 6240

gtcgctgatt tgtattgtct gaagttgctt ttacgttaag ttgatgcaga tcaattaata 6300

cgatacctgc gtcataattg attatttgac gtggtttgat ggcctccacg cacgttgtga 6360

tatgtagatg ataatcatta tcactttacg ggtcctttcc ggtgatccga caggttacgg 6420

ggcggcgacc tcgcgggttt tcgctattta tgaaaatttt ccggtttaag gcgtttccgt 6480

tcttcttcgt cataacttaa tgtttttatt taaaataccc tctgaaaaga aaggaaacga 6540

caggtgctga aagcgagctt tttggcctct gtcgtttcct ttctctgttt ttgtccgtgg 6600

aatgaacaat ggaagtccga gctcatcgct aataacttcg tatagcatac attatacgaa 6660

gttatattcg atgcggccga tctagcagaa agtcaaaagc ctccgaccgg aggcttttga 6720

cttctgtcac ctaggttacg ccccgccctg ccactcatcg cagtactgtt gtaattcatt 6780

aagcattctg ccgacatgga agccatcaca aacggcatga tgaacctgaa tcgccagcgg 6840

catcagcacc ttgtcgcctt gcgtataata tttgcccatg gtgaaaacgg gggcgaagaa 6900

gttgtccata ttggccacgt ttaaatcaaa actggtgaaa ctcacccagg gattggctga 6960

gacgaaaaac atattctcaa taaacccttt agggaaatag gccaggtttt caccgtaaca 7020

cgccacatct tgcgaatata tgtgtagaaa ctgccggaaa tcgtcgtggt attcactcca 7080

gagcgatgaa aacgtttcag tttgctcatg gaaaacggtg taacaagggt gaacactatc 7140

ccatatcacc agctcaccgt ctttcattgc catacgaaat tccggatgag cattcatcag 7200

gcgggcaaga atgtgaataa aggccggata aaacttgtgc ttatttttct ttacggtctt 7260

taaaaaggcc gtaatatcca gctgaacggt ctggttatag gtacattgag caactgactg 7320

aaatgcctca aaatgttctt tacgatgcca ttgggatata tcaacggtgg tatatccagt 7380

gatttttttc tccattttag cttccttagc tcctgaaaat ctcgataact caaaaaatac 7440

gcccggtagt gatcttattt cattatggtg aaagttggaa cctcttacgt gccgatcaga 7500

ttaaaacgaa aggcccagtc tttcgactga gcctttcgtt ttatttgacc atgttggtat 7560

gatttaaatt aatacgactc actatagggt cagtgcggcc gcgacttcaa gtcacgtgaa 7620

ttccaagctt cggatcccac tgtggtgg 7648

<210> 4
<211> 7591
<212> DNA
<213> artificial sequence

<220>
<223> Synthetic sequence

<400> 4
ttataaaaaa cttaggaacc aggtccacac agccgccagc ccatcaacgc gtacgatgga 60

gttcatccca acccaaactt tttacaatag gaggtaccag cctcgaccct ggactccgcg 120

ccctactatc caagtcatca ggcccagacc gcgccctcag aggcaagctg ggcaacttgc 180

ccagctgatc tcagcagtta ataaactgac aatgagagcg gtaccccaac agaagccacg 240

caggaatcgg aagaataaga agcaaaagca aaaacaacag gcgccacaaa acaacacaaa 300

tcaaaagaag cagccaccta aaaagaaacc ggctcaaaag aaaaagaagc cgggccgcag 360

agagaggatg tgcatgaaaa tcgaaaatga ttgtattttc gaagtcaagc acgaaggtaa 420

ggtaacaggt tacgcgtgcc tggtggggga caaagtaatg aaaccagcac acgtcaaggg 480

caccatcgat aacgcggacc tggccaaact ggcctttaag cggtcatcta agtatgacct 540

tgaatgcgct cagatacccg tgcacatgaa gtccgacgct tcgaagttca cccatgagaa 600

accggagggg tactacaact ggcaccacgg agcagtacag tactcaggag gccggttcac 660

catccctaca ggtgctggca aaccagggga cagcggcaga ccgatcttcg acaacaaggg 720

acgcgtggtg gccatagtct taggaggagc taatgaagga gcccgtacag ccctctcggt 780

ggtgacctgg aataaagaca ttgtcactaa aatcaccccc gagggggccg aagagtggag 840

tcttgccatc ccagttatgt gcctgttggc aaacaccacg ttcccatgct cccagccacc 900

ttgcacgccc tgctgctacg agaaggaacc ggaggaaacc ctacgcatgc ttgaggacaa 960

cgtcatgaga cctgggtact atcagctgct acaagcatcc ttaacatgtt ctccccaccg 1020

ccagcgacgc agcaccaagg acaacttcaa tgtctataaa gccacaagac catacttagc 1080

tcactgtccc gactgtggag aagggcactc gtgccatagt cccgtagcac tagaacgcat 1140

cagaaatgaa gcgacagacg ggacgctgaa aatccaggtc tccttgcaaa tcggaataaa 1200

gacggatgac agccacgatt ggaccaagct gcgttatatg gacaaccaca tgccagcaga 1260

cgcagagagg gcggggctat ttgtaagaac atcagcaccg tgtacgatta ctggaacaat 1320

gggacacttc atcctggccc gatgtcctaa aggcgaaact ctgacggtgg gattcactga 1380

cagtaggaag attagtcact catgtacgca cccatttcac cacgaccctc ctgtgatagg 1440

tcgggagaaa ttccattccc gaccgcagca cggtaaagag ctaccttgca gcacctacgt 1500

gcagagcacc gccgcaacta ccgaggagat agaggtacac atgcccccag acacccctga 1560

tcgcacatta atgtcacaac agtccggcaa cgtaaagatc acagtcaatg gccagacggt 1620

gcggtacaag tgtaattgcg gtggctcaaa tgaaggacta acaactacag acaaagtgat 1680

taataactgc aaggttgatc aatgtcatgc cgcggtcacc aatcacaaaa agtggcagta 1740

taactcccct ctggtcccgc gtaatgctga acttggggac cggaaaggaa aaattcacat 1800

cccgtttccg ctggcaaatg taacatgcag ggtgcctaaa gcaaggaacc ccaccgtgac 1860

gtatggcaaa aaccaagtca tcatgctact gtatcctgac cacccaacac tcctgtccta 1920

ccggaatatg ggagaagaac caaactatca agaagagtgg gtgatgcata agaaggaagt 1980

cgtgctaacc gtgccgactg aagggctcga ggtcacgtgg ggcaacaacg agccgtataa 2040

gtattggccg cagttatcta caaacggtac agcccatggc cacccgcacg agataattct 2100

gtattattat gagctgtacc ccactatgac tgtagtagtt gtgtcagtgg ccacgttcat 2160

actcctgtcg atggtgggta tggcagcggg gatgtgcatg tgtgcacgac gcagatgcat 2220

cacaccgtat gaactgacac caggagctac cgtcccattc ctgcttagcc taatatgctg 2280

catcagaaca gctaaagcgg ccacatacca agaggctgcg atatacctgt ggaacgagca 2340

gcaacctttg ttttggctac aagcccttat tccgctggca gccctgattg ttctatgcaa 2400

ctgtctgaga ctcttaccat gctgctgtaa aacgttggct tttttagccg taatgagcgt 2460

cggtgcccac actgtgagcg cttacgaaca cgtaacagtg atcccgaaca cggtgggagt 2520

accgtataag actctagtca atagacctgg ctacagcccc atggtattgg agatggaact 2580

actgtcagtc actttggagc caacactatc gcttgattac atcacgtgcg agtacaaaac 2640

cgtcatcccg tctccttacg tgaagtgctg cggtacagca gagtgcaagg acaaaaacct 2700

acctgactac agctgtaagg tcttcaccgg cgtctaccca tttatgtggg gcggcgccta 2760

ctgcttctgc gacgctgaaa acacgcagtt gagcgaagca cacgtggaga agtccgaatc 2820

atgcaaaaca gaatttgcat cagcatacag ggctcatacc gcatctgcat cagctaagct 2880

ccgcgtcctt taccaaggaa ataacatcac tgtaactgcc tatgcaaacg gcgaccatgc 2940

cgtcacagtt aaggacgcca aattcattgt ggggccaatg tcttcagcct ggacaccttt 3000

cgacaacaaa attgtggtgt acaaaggtga cgtctataac atggactacc cgcccttcgg 3060

cgcaggaaga ccaggacaat ttggcgatat ccaaagtcgc acacctgaga gtaaagacgt 3120

ctatgctaat acacaactgg tactgcagag accggctgtg ggtacggtac acgtgccata 3180

ctctcaggca ccatctggct ttaagtattg gctaaaagaa cgcggggcgt cgctgcagca 3240

cacagcacca tttggctgcc aaatagcaac aaacccggta agagcggtga actgcgccgt 3300

agggaacatg cccatctcca tcgacatacc ggaagcggcc ttcactaggg tcgtcgacgc 3360

gccctcttta acggacatgt cgtgcgaggt accagcctgc acccattcct cagactttgg 3420

gggcgtcgcc attattaaat atgcagccag caagaaaggc aagtgtgcgg tgcattcgat 3480

gactaacgcc gtcactattc gggaagctga gatagaagtt gaagggaatt ctcagctgca 3540

aatctctttc tcgacggcct tagccagcgc cgaattccgc gtacaagtct gttctacaca 3600

agtacactgt gcagccgagt gccacccccc gaaggaccac atagtcaact acccggcgtc 3660

acataccacc ctcggggtcc aggacatctc cgctacggcg atgtcatggg tgcagaagat 3720

cacgggaggt gtgggactgg ttgttgctgt tgccgcactg attctaatcg tggtgctatg 3780

cgtgtcgttc agcaggcact aatagctaca gctcaactta cctgccaacc ccatgccaga 3840

cgacccaact agtacaacct aaatccatta taaaaaactt aggaaccagg tccacacagc 3900

cgccagccca tcaaccatcc actcccacga ttggaggccg gccatgagag ttgtgtttgt 3960

cgtgctattg cttttggtgg ccccagctta cagcttcaac tgccttggaa tgagcaacag 4020

agacttcttg gaaggagtgt ctggagcaac atgggtggat ttggttctcg aaggcgacag 4080

ctgcgtgact atcatgtcta aggacaagcc taccatcgat gtgaagatga tgaatatgga 4140

ggcggtcaac ctggcagagg tccgcagtta ttgctatttg gctaccgtca gcgatctctc 4200

caccaaagct gcgtgcccga ccatgggaga agctcacaat gacaaacgtg ctgacccagc 4260

ttttgtgtgc agacaaggag tggtggacag gggctggggc aacggctgcg gattatttgg 4320

caaaggaagc attgacacat gcgccaaatt tgcctgctct accaaggcaa taggaagaac 4380

catcttgaaa gagaatatca agtacgaagt ggccattttt gtccatggac caactactgt 4440

ggagtcgcac ggaaactact ccacacaggt tggagccact caggcaggga gattcagcat 4500

cactcctgcg gcgccttcat acacactaaa gcttggagaa tatggagagg tgacagtgga 4560

ctgtgaacca cggtcaggga ttgacaccaa tgcatactac gtgatgactg ttggaacaaa 4620

gacgttcttg gtccatcgtg agtggttcat ggacctcaac ctcccttgga gcagtgctgg 4680

aagtactgtg tggaggaaca gagagacgtt aatggagttt gaggaaccac acgccacgaa 4740

gcagtctgtg atagcattgg gctcacaaga gggagctctg catcaagctt tggctggagc 4800

cattcctgtg gaattttcaa gcaacactgt caagttgacg tcgggtcatt tgaagtgtag 4860

agtgaagatg gaaaaattgc agttgaaggg aacaacctat ggcgtctgtt caaaggcttt 4920

caagtttctt gggactcccg cagacacagg tcacggcact gtggtgttgg aattgcagta 4980

cactggcacg gatggacctt gcaaagttcc tatctcgtca gtggcttcat tgaacgacct 5040

aacgccagtg ggcagattgg tcactgtcaa cccttttgtt tcagtggcca cggccaacgc 5100

taaggtcctg attgaattgg aaccaccctt tggagactca tacatagtgg tgggcagagg 5160

agaacaacag atcaatcacc attggcacaa gtctggaagc agcattggca aagcctttac 5220

aaccaccctc aaaggagcgc agagactagc cgctctagga gacacagctt gggactttgg 5280

atcagttgga ggggtgttca cctcagttgg gaaggctgtc taattcgaac tacagctcaa 5340

cttacctgcc aaccccatgc cagacgaccc aactagtaca acctaaatcc attataaaaa 5400

acttaggaac caggtccaca cagccgccag cccatcaacc atccactccc acgattggag 5460

cggccgcatg gagaagaagc ggagaggagc agacacaagc gtgggaatcg tgggcctgct 5520

gctgaccaca gcaatggcag cagaggtgac caggagagga agcgcctact atatgtacct 5580

ggacaggaat gatgccggcg aggccatctc cttcccaacc acactgggca tgaacaagtg 5640

ctacatccag atcatggacc tgggccacat gtgcgatgcc accatgtcct atgagtgtcc 5700

aatgctggac gagggcgtgg agcccgacga tgtggattgc tggtgtaata ccacatctac 5760

atgggtggtg tacggcacct gtcaccacaa gaagggagag gcccggcgga gccggcgggc 5820

cgtgacactg ccttcccact ctaccaggaa gctgcagaca cgcagccaga cctggctgga 5880

gtccagagag tataccaagc acctgatcag ggtggagaac tggatctttc gcaatccagg 5940

attcgcactg gcagcagcag caatcgcatg gctgctggga agctccacca gccagaaagt 6000

gatctacctg gtcatgatcc tgctgatcgc tcctgcctat tctatccggt gcatcggcgt 6060

gagcaataga gacttcgtgg agggaatgtc cggaggaacc tgggtggatg tggtgctgga 6120

gcacggcggc tgcgtgacag tgatggccca ggacaagcca accgtggata tcgagctggt 6180

gaccacaacc gtgtccaaca tggccgaggt gaggtcttac tgctatgagg ccagcatctc 6240

cgacatggcc tctgatagca ggtgtccaac ccagggagag gcatacctgg acaagcagtc 6300

cgatacacag tacgtgtgca agcggaccct ggtggacaga ggctggggca atggctgtgg 6360

cctgtttggc aagggctctc tggtgacatg cgccaagttc gcctgtagca agaagatgac 6420

cggcaagtcc atccagccag agaacctgga gtaccggatc atgctgtctg tgcacggctc 6480

ccagcactct ggcatgatcg tgaacgacac aggccacgag acagatgaga atcgggccaa 6540

ggtggagatc acacctaact ctccaagagc cgaggccacc ctgggaggat ttggctctct 6600

gggcctggac tgcgagccta gaacaggcct ggacttctcc gatctgtact atctgaccat 6660

gaacaataag cactggctgg tgcacaagga gtggtttcac gacatcccac tgccatggca 6720

cgcaggagca gatacaggaa caccacactg gaacaataag gaggccctgg tggagttcaa 6780

ggatgcccac gccaagcggc agacagtggt ggtgctgggc agccaggagg gagcagtgca 6840

caccgccctg gcaggcgccc tggaggcaga gatggacgga gctaagggca gactgtctag 6900

cggccacctg aagtgcaggc tgaagatgga taagctgcgc ctgaagggcg tgtcctactc 6960

tctgtgcaca gccgccttca ccttcaccaa gatccctgcc gagacactgc acggcacagt 7020

gaccgtggag gtgcagtatg ccggcacaga cggaccctgt aaggtgcctg cccagatggc 7080

cgtggatatg cagacactga cacctgtggg caggctgatc accgccaatc cagtgatcac 7140

agagtctacc gagaacagca agatgatgct ggagctggac ccaccatttg gcgatagcta 7200

tatcgtgatc ggcgtgggcg agaagaagat cacacaccac tggcaccgca gcggctccac 7260

aatcggcaag gcctttgagg caaccgtgcg cggagcaaag agaatggccg tgctgggcga 7320

caccgcatgg gatttcggat ctgtgggagg cgccctgaac agcctgggca agggcatcca 7380

ccagatcttc ggcgccgcct ttaagtccct gttcggcggc atgagctggt tctcacagat 7440

cctgatcggc acactgctga tgtggctggg cctgaacacc aagaatggct ctatcagcct 7500

gatgtgcctg gccctgggag gcgtgctgat cttcctgtcc accgccgtgt ctgcctgatg 7560

ataatagttc gaactacagc tcaacttacc t 7591

<210> 5
<211> 18967
<212> DNA
<213> artificial sequence

<220>
<223> Synthetic sequence

<400> 5
gcggccgcta atacgactca ctatagggcc aactttgttt ggtctgatga gtccgtgagg 60

acgaaacccg gagtcccggg tcaccaaaca aagttgggta aggatagttc aatcaatgat 120

catcttctag tgcacttagg attcaagatc ctattatcag ggacaagagc aggattaggg 180

atatccgaga tggccacact tttaaggagc ttagcattgt tcaaaagaaa caaggacaaa 240

ccacccatta catcaggatc cggtggagcc atcagaggaa tcaaacacat tattatagta 300

ccaatccctg gagattcctc aattaccact cgatccagac ttctggaccg gttggtgagg 360

ttaattggaa acccggatgt gagcgggccc aaactaacag gggcactaat aggtatatta 420

tccttatttg tggagtctcc aggtcaattg attcagagga tcaccgatga ccctgacgtt 480

agcataaggc tgttagaggt tgtccagagt gaccagtcac aatctggcct taccttcgca 540

tcaagaggta ccaacatgga ggatgaggcg gaccaatact tttcacatga tgatccaatt 600

agtagtgatc aatccaggtt cggatggttc gggaacaagg aaatctcaga tattgaagtg 660

caagaccctg agggattcaa catgattctg ggtaccatcc tagcccaaat ttgggtcttg 720

ctcgcaaagg cggttacggc cccagacacg gcagctgatt cggagctaag aaggtggata 780

aagtacaccc aacaaagaag ggtagttggt gaatttagat tggagagaaa atggttggat 840

gtggtgagga acaggattgc cgaggacctc tccttacgcc gattcatggt cgctctaatc 900

ctggatatca agagaacacc cggaaacaaa cccaggattg ctgaaatgat atgtgacatt 960

gatacatata tcgtagaggc aggattagcc agttttatcc tgactattaa gtttgggata 1020

gaaactatgt atcctgctct tggactgcat gaatttgctg gtgagttatc cacacttgag 1080

tccttgatga acctttacca gcaaatgggg gaaactgcac cctacatggt aatcctggag 1140

aactcaattc agaacaagtt cagtgcagga tcataccctc tgctctggag ctatgccatg 1200

ggagtaggag tggaacttga aaactccatg ggaggtttga actttggccg atcttacttt 1260

gatccagcat attttagatt agggcaagag atggtaagga ggtcagctgg aaaggtcagt 1320

tccacattgg catctgaact cggtatcact gccgaggatg caaggcttgt ttcagagatt 1380

gcaatgcata ctactgagga caagatcagt agagcggttg gacccagaca agcccaagta 1440

tcatttctac acggtgatca aagtgagaat gagctaccga gattgggggg caaggaagat 1500

aggagggtca aacagagtcg aggagaagcc agggagagct acagagaaac cgggcccagc 1560

agagcaagtg atgcgagagc tgcccatctt ccaaccggca cacccctaga cattgacact 1620

gcaacggagt ccagccaaga tccgcaggac agtcgaaggt cagctgacgc cctgcttagg 1680

ctgcaagcca tggcaggaat ctcggaagaa caaggctcag acacggacac ccctatagtg 1740

tacaatgaca gaaatcttct agactaggtg cgagaggccg agggccagaa caacatccgc 1800

ctaccatcca tcattgttat aaaaaactta ggaaccaggt ccacacagcc gccagcccat 1860

caaccatcca ctcccacgat tggagccaat ggcagaagag caggcacgcc atgtcaaaaa 1920

cggactggaa tgcatccggg ctctcaaggc cgagcccatc ggctcactgg ccatcgagga 1980

agctatggca gcatggtcag aaatatcaga caacccagga caggagcgag ccacctgcag 2040

ggaagagaag gcaggcagtt cgggtctcag caaaccatgc ctctcagcaa ttggatcaac 2100

tgaaggcggt gcacctcgca tccgcggtca gggacctgga gagagcgatg acgacgctga 2160

aactttggga atccccccaa gaaatctcca ggcatcaagc actgggttac agtgttatta 2220

cgtttatgat cacagcggtg aagcggttaa gggaatccaa gatgctgact ctatcatggt 2280

tcaatcaggc cttgatggtg atagcaccct ctcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cccccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tgtgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agctaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaactagt acaacctaaa tccattataa 3480

aaaacttagg agcaaagtga ttgcctccca aggtccacaa tgacagagac ctacgacttc 3540

gacaagtcgg catgggacat caaagggtcg atcgctccga tacaacccac cacctacagt 3600

gatggcaggc tggtgcccca ggtcagagtc atagatcctg gtctaggcga caggaaggat 3660

gaatgcttta tgtacatgtt tctgctgggg gttgttgagg acagcgattc cctagggcct 3720

ccaatcgggc gagcatttgg gttcctgccc ttaggtgttg gcagatccac agcaaagccc 3780

gaaaaactcc tcaaagaggc cactgagctt gacatagttg ttagacgtac agcagggctc 3840

aatgaaaaac tggtgttcta caacaacacc ccactaactc tcctcacacc ttggagaaag 3900

gtcctaacaa cagggagtgt cttcaacgca aaccaagtgt gcaatgcggt taatctgata 3960

ccgctcgata ccccgcagag gttccgtgtt gtttatatga gcatcacccg tctttcggat 4020

aacgggtatt acaccgttcc tagaagaatg ctggaattca gatcggtcaa tgcagtggcc 4080

ttcaacctgc tggtgaccct taggattgac aaggcgatag gccctgggaa gatcatcgac 4140

aatacagagc aacttcctga ggcaacattt atggtccaca tcgggaactt caggagaaag 4200

aagagtgaag tctactctgc cgattattgc aaaatgaaaa tcgaaaagat gggcctggtt 4260

tttgcacttg gtgggatagg gggcaccagt cttcacatta gaagcacagg caaaatgagc 4320

aagactctcc atgcacaact cgggttcaag aagaccttat gttacccgct gatggatatc 4380

aatgaagacc ttaatcgatt actctggagg agcagatgca agatagtaag aatccaggca 4440

gttttgcagc catcagttcc tcaagaattc cgcatttacg acgacgtgat cataaatgat 4500

gaccaaggac tattcaaagt tctgtagacc gtagtgccca gcaatgcccg aaaacgaccc 4560

ccctcacaat gacagccaga aggcccggac aaaaaagccc cctccgaaag actccacgga 4620

ccaagcgaga ggccagccag cagccgacgg caagcgcgaa caccaggcgg ccccagcaca 4680

gaacagccct gacacaaggc caccaccagc caccccaatc tgcatcctcc tcgtgggacc 4740

cccgaggacc aacccccaag gctgcccccg atccaaacca ccaaccgcat ccccaccacc 4800

cccgggaaag aaacccccag caattggaag gcccctcccc ctcttcctca acacaagaac 4860

tccacaaccg aaccgcacaa gcgaccgagg tgacccaacc gcaggcatcc gactccctag 4920

acagatcctc tctccccggc aaactaaaca aaacttaggg ccaaggaaca tacacaccca 4980

acagaaccca gaccccggcc cacggcgccg cgcccccaac ccccgacaac cagagggagc 5040

ccccaaccaa tcccgccggc tcccccggtg cccacaggca gggacaccaa cccccgaaca 5100

gacccagcac ccaaccatcg acaatccaag acgggggggc ccccccaaaa aaaggccccc 5160

aggggccgac agccagcacc gcgaggaagc ccacccaccc cacacacgac cacggcaacc 5220

aaaccagaac ccagaccacc ctgggccacc agctcccaga ctcggccatc accccgcaga 5280

aaggaaaggc cacaacccgc gcaccccagc cccgatccgg cggggagcca cccaacccga 5340

accagcaccc aagagcgatc cccgaaggac ccccgaaccg caaaggacat cagtatccca 5400

cagcctctcc aagtcccccg gtctcctcct cttctcgaag ggaccaaaag atcaatccac 5460

cacacccgac gacactcaac tccccacccc taaaggagac accgggaatc ccagaatcaa 5520

gactcatcca atgtccatca tgggtctcaa ggtgaacgtc tctgccatat tcatggcagt 5580

actgttaact ctccaaacac ccaccggtca aatccattgg ggcaatctct ctaagatagg 5640

ggtggtagga ataggaagtg caagctacaa agttatgact cgttccagcc atcaatcatt 5700

agtcataaaa ttaatgccca atataactct cctcaataac tgcacgaggg tagagattgc 5760

agaatacagg agactactga gaacagtttt ggaaccaatt agagatgcac ttaatgcaat 5820

gacccagaat ataagaccgg ttcagagtgt agcttcaagt aggagacaca agagatttgc 5880

gggagtagtc ctggcaggtg cggccctagg cgttgccaca gctgctcaga taacagccgg 5940

cattgcactt caccagtcca tgctgaactc tcaagccatc gacaatctga gagcgagcct 6000

ggaaactact aatcaggcaa ttgagacaat cagacaagca gggcaggaga tgatattggc 6060

tgttcagggt gtccaagact acatcaataa tgagctgata ccgtctatga accaactatc 6120

ttgtgattta atcggccaga agctcgggct caaattgctc agatactata cagaaatcct 6180

gtcattattt ggccccagtt tacgggaccc catatctgcg gagatatcta tccaggcttt 6240

gagctatgcg cttggaggag acatcaataa ggtgttagaa aagctcggat acagtggagg 6300

tgatttactg ggcatcttag agagcggagg aataaaggcc cggataactc acgtcgacac 6360

agagtcctac ttcattgtcc tcagtatagc ctatccgacg ctgtccgaga ttaagggggt 6420

gattgtccac cggctagagg gggtctcgta caacataggc tctcaagagt ggtataccac 6480

tgtgcccaag tatgttgcaa cccaagggta ccttatctcg aattttgatg agtcatcgtg 6540

tactttcatg ccagagggga ctgtgtgcag ccaaaatgcc ttgtacccga tgagtcctct 6600

gctccaagaa tgcctccggg ggtacaccaa gtcctgtgct cgtacactcg tatccgggtc 6660

ttttgggaac cggttcattt tatcacaagg gaacctaata gccaattgtg catcaatcct 6720

ttgcaagtgt tacacaacag gaacgatcat taatcaagac cctgacaaga tcctaacata 6780

cattgctgcc gatcactgcc cggtagtcga ggtgaacggc gtgaccatcc aagtcgggag 6840

caggaggtat ccagacgctg tgtacttgca cagaattgac ctcggtcctc ccatatcatt 6900

ggagaggttg gacgtaggga caaatctggg gaatgcaatt gctaagttgg aggatgccaa 6960

ggaattgttg gagtcatcgg accagatatt gaggagtatg aaaggtttat cgagcactag 7020

catagtctac atcctgattg cagtgtgtct tggagggttg atagggatcc ccgctttaat 7080

atgttgctgc agggggcgtt gtaacaaaaa gggagaacaa gttggtatgt caagaccagg 7140

cctaaagcct gatcttacgg gaacatcaaa atcctatgta aggtcgctct gatcctctac 7200

aactcttgaa acacaaatgt cccacaagtc tcctcttcgt catcaagcaa ccaccgcacc 7260

cagcatcaag cccacctgaa attatctccg gcttccctct ggccgaacaa tatcggtagt 7320

taatcaaaac ttagggtgca agatcatcca caatgtcacc acaacgagac cggataaatg 7380

ccttctacaa agataacccc catcccaagg gaagtaggat agtcattaac agagaacatc 7440

ttatgattga tagaccttat gttttgctgg ctgttctgtt tgtcatgttt ctgagcttga 7500

tcgggttgct agccattgca ggcattagac ttcatcgggc agccatctac accgcagaga 7560

tccataaaag cctcagcacc aatctagatg taactaactc aatcgagcat caggtcaagg 7620

acgtgctgac accactcttc aaaatcatcg gtgatgaagt gggcctgagg acacctcaga 7680

gattcactga cctagtgaaa ttaatctctg acaagattaa attccttaat ccggataggg 7740

agtacgactt cagagatctc acttggtgta tcaacccgcc agagagaatc aaattggatt 7800

atgatcaata ctgtgcagat gtggctgctg aagagctcat gaatgcattg gtgaactcaa 7860

ctctactgga gaccagaaca accaatcagt tcctagctgt ctcaaaggga aactgctcag 7920

ggcccactac aatcagaggt caattctcaa acatgtcgct gtccctgtta gacttgtatt 7980

taggtcgagg ttacaatgtg tcatctatag tcactatgac atcccaggga atgtatgggg 8040

gaacttacct agtggaaaag cctaatctga gcagcaaaag gtcagagttg tcacaactga 8100

gcatgtaccg agtgtttgaa gtaggtgtta tcagaaatcc gggtttgggg gctccggtgt 8160

tccatatgac aaactatctt gagcaaccag tcagtaatga tctcagcaac tgtatggtgg 8220

ctttggggga gctcaaactc gcagcccttt gtcacgggga agattctatc acaattccct 8280

atcagggatc agggaaaggt gtcagcttcc agctcgtcaa gctaggtgtc tggaaatccc 8340

caaccgacat gcaatcctgg gtccccttat caacggatga tccagtgata gacaggcttt 8400

acctctcatc tcacagaggt gttatcgctg acaatcaagc aaaatgggct gtcccgacaa 8460

cacgaacaga tgacaagttg cgaatggaga catgcttcca acaggcgtgt aagggtaaaa 8520

tccaagcact ctgcgagaat cccgagtggg caccattgaa ggataacagg attccttcat 8580

acggggtctt gtctgttgat ctgagtctga cagttgagct taaaatcaaa attgcttcgg 8640

gattcgggcc attgatcaca cacggttcag ggatggacct atacaaatcc aaccacaaca 8700

atgtgtattg gctgactatc ccgccaatga agaacctagc cttaggtgta atcaacacat 8760

tggagtggat accgagattc aaggttagtc cctacctctt cactgtccca attaaggaag 8820

caggcgaaga ctgccatgcc ccaacatacc tacctgcgga ggtggatggt gatgtcaaac 8880

tcagttccaa tctggtgatt ctacctggtc aagatctcca atatgttttg gcaacctacg 8940

atacttccag ggttgaacat gctgtggttt attacgttta cagcccaagc cgctcatttt 9000

cttactttta tccttttagg ttgcctataa agggggtccc catcgaatta caagtggaat 9060

gcttcacatg ggaccaaaaa ctctggtgcc gtcacttctg tgtgcttgcg gactcagaat 9120

ctggtggaca tatcactcac tctgggatgg tgggcatggg agtcagctgc acagtcaccc 9180

gggaagatgg aaccaatcgc agatagggct gctagtgaac caatcacatg atgtcaccca 9240

gacatcaggc atacccacta gtgtgaaata gacatcagaa ttaagaaaaa cgtagggtcc 9300

aagtggttcc ccgttatgga ctcgctatct gtcaaccaga tcttataccc tgaagttcac 9360

ctagatagcc cgatagttac caataagata gtagccatcc tggagtatgc tcgagtccct 9420

cacgcttaca gcctggagga ccctacactg tgtcagaaca tcaagcaccg cctaaaaaac 9480

ggattttcca accaaatgat tataaacaat gtggaagttg ggaatgtcat caagtccaag 9540

cttaggagtt atccggccca ctctcatatt ccatatccaa attgtaatca ggatttattt 9600

aacatagaag acaaagagtc aacgaggaag atccgtgaac tcctcaaaaa ggggaattcg 9660

ctgtactcca aagtcagtga taaggttttc caatgcttaa gggacactaa ctcacggctt 9720

ggcctaggct ccgaattgag ggaggacatc aaggagaaag ttattaactt gggagtttac 9780

atgcacagct cccagtggtt tgagcccttt ctgttttggt ttacagtcaa gactgagatg 9840

aggtcagtga ttaaatcaca aacccatact tgccatagga ggagacacac acctgtattc 9900

ttcactggta gttcagttga gttgctaatc tctcgtgacc ttgttgctat aatcagtaaa 9960

gagtctcaac atgtatatta cctgacattt gaactggttt tgatgtattg tgatgtcata 10020

gaggggaggt taatgacaga gaccgctatg actattgatg ctaggtatac agagcttcta 10080

ggaagagtca gatacatgtg gaaactgata gatggtttct tccctgcact cgggaatcca 10140

acttatcaaa ttgtagccat gctggagcct ctttcacttg cttacctgca gctgagggat 10200

ataacagtag aactcagagg tgctttcctt aaccactgct ttactgaaat acatgatgtt 10260

cttgaccaaa acgggttttc tgatgaaggt acttatcatg agttaactga agctctagat 10320

tacattttca taactgatga catacatctg acaggggaga ttttctcatt tttcagaagt 10380

ttcggccacc ccagacttga agcagtaacg gctgctgaaa atgttaggaa atacatgaat 10440

cagcctaaag tcattgtgta tgagactctg atgaaaggtc atgccatatt ttgtggaatc 10500

ataatcaacg gctatcgtga caggcacgga ggcagttggc caccgctgac cctccccctg 10560

catgctgcag acacaatccg gaatgctcaa gcttcaggtg aagggttaac acatgagcag 10620

tgcgttgata actggaaatc ttttgctgga gtgaaatttg gctgctttat gcctcttagc 10680

ctggatagtg atctgacaat gtacctaaag gacaaggcac ttgctgctct ccaaagggaa 10740

tgggattcag tttacccgaa agagttcctg cgttacgacc ctcccaaggg aaccgggtca 10800

cggaggcttg tagatgtttt ccttaatgat tcgagctttg acccatatga tgtgataatg 10860

tatgttgtaa gtggagctta cctccatgac cctgagttca acctgtctta cagcctgaaa 10920

gaaaaggaga tcaaggaaac aggtagactt tttgctaaaa tgacttacaa aatgaggcca 10980

tgccaagtga ttgctgaaaa tctaatctca aacgggattg gcaaatattt taaggacaat 11040

gggatggcca aggatgagca cgatttgact aaggcactcc acactctagc tgtctcagga 11100

gtccccaaag atctcaaaga aagtcacagg ggggggccag tcttaaaaac ctactcccga 11160

agcccagtcc acacaagtac caggaacgtg agagcagcaa aagggtttat agggttccct 11220

caagtaattc ggcaggacca agacactgat catccggaga atatggaagc ttacgagaca 11280

gtcagtgcat ttatcacgac tgatctcaag aagtactgcc ttaattggag atatgagacc 11340

atcagcttgt ttgcacagag gctaaatgag atttacggat tgccctcatt tttccagtgg 11400

ctgcataaga ggcttgagac ctctgtcctg tatgtaagtg accctcattg cccccccgac 11460

cttgacgccc atatcccgtt atataaagtc cccaatgatc aaatcttcat taagtaccct 11520

atgggaggta tagaagggta ttgtcagaag ctgtggacca tcagcaccat tccctatcta 11580

tacctggctg cttatgagag cggagtaagg attgcttcgt tagtgcaagg ggacaatcag 11640

accatagccg taacaaaaag ggtacccagc acatggccct acaaccttaa gaaacgggaa 11700

gctgctagag taactagaga ttactttgta attcttaggc aaaggctaca tgatattggc 11760

catcacctca aggcaaatga gacaattgtt tcatcacatt tttttgtcta ttcaaaagga 11820

atatattatg atgggctact tgtgtcccaa tcactcaaga gcatcgcaag atgtgtattc 11880

tggtcagaga ctatagttga tgaaacaagg gcagcatgca gtaatattgc tacaacaatg 11940

gctaaaagca tcgagagagg ttatgaccgt taccttgcat attccctgaa cgtcctaaaa 12000

gtgatacagc aaattctgat ctctcttggc ttcacaatca attcaaccat gacccgggat 12060

gtagtcatac ccctcctcac aaacaacgac ctcttaataa ggatggcact gttgcccgct 12120

cctattgggg ggatgaatta tctgaatatg agcaggctgt ttgtcagaaa catcggtgat 12180

ccagtaacat catcaattgc tgatctcaag agaatgattc tcgcctcact aatgcctgaa 12240

gagaccctcc atcaagtaat gacacaacaa ccgggggact cttcattcct agactgggct 12300

agcgaccctt actcagcaaa tcttgtatgt gtccagagca tcactagact cctcaagaac 12360

ataactgcaa ggtttgtcct gatccatagt ccaaacccaa tgttaaaagg attattccat 12420

gatgacagta aagaagagga cgagggactg gcggcattcc tcatggacag gcatattata 12480

gtacctaggg cagctcatga aatcctggat catagtgtca caggggcaag agagtctatt 12540

gcaggcatgc tggataccac aaaaggcttg attcgagcca gcatgaggaa gggggggtta 12600

acctctcgag tgataaccag attgtccaat tatgactatg aacaattcag agcagggatg 12660

gtgctattga caggaagaaa gagaaatgtc ctcattgaca aagagtcatg ttcagtgcag 12720

ctggcgagag ctctaagaag ccatatgtgg gcgaggctag ctcgaggacg gcctatttac 12780

ggccttgagg tccctgatgt actagaatct atgcgaggcc accttattcg gcgtcatgag 12840

acatgtgtca tctgcgagtg tggatcagtc aactacggat ggttttttgt cccctcgggt 12900

tgccaactgg atgatattga caaggaaaca tcatccttga gagtcccata tattggttct 12960

accactgatg agagaacaga catgaagctt gccttcgtaa gagccccaag tcgatccttg 13020

cgatctgctg ttagaatagc aacagtgtac tcatgggctt acggtgatga tgatagctct 13080

tggaacgaag cctggttgtt ggctaggcaa agggccaatg tgagcctgga ggagctaagg 13140

gtgatcactc ccatctcaac ttcgactaat ttagcgcata ggttgaggga tcgtagcact 13200

caagtgaaat actcaggtac atcccttgtc cgagtggcga ggtataccac aatctccaac 13260

gacaatctct catttgtcat atcagataag aaggttgata ctaactttat ataccaacaa 13320

ggaatgcttc tagggttggg tgttttagaa acattgtttc gactcgagaa agataccgga 13380

tcatctaaca cggtattaca tcttcacgtc gaaacagatt gttgcgtgat cccgatgata 13440

gatcatccca ggatacccag ctcccgcaag ctagagctga gggcagagct atgtaccaac 13500

ccattgatat atgataatgc acctttaatt gacagagatg caacaaggct atacacccag 13560

agccatagga ggcaccttgt ggaatttgtt acatggtcca caccccaact atatcacatt 13620

ttagctaagt ccacagcact atctatgatt gacctggtaa caaaatttga gaaggaccat 13680

atgaatgaaa tttcagctct cataggggat gacgatatca atagtttcat aactgagttt 13740

ctgctcatag agccaagatt attcactatc tacttgggcc agtgtgcggc catcaattgg 13800

gcatttgatg tacattatca tagaccatca gggaaatatc agatgggtga gctgttgtca 13860

tcgttccttt ctagaatgag caaaggagtg tttaaggtgc ttgtcaatgc tctaagccac 13920

ccaaagatct acaagaaatt ctggcattgt ggtattatag agcctatcca tggtccttca 13980

cttgatgctc aaaacttgca cacaactgtg tgcaacatgg tttacacatg ctatatgacc 14040

tacctcgacc tgttgttgaa tgaagagtta gaagagttca catttctctt gtgtgaaagc 14100

gacgaggatg tagtaccgga cagattcgac aacatccagg caaaacactt atgtgttctg 14160

gcagatttgt actgtcaacc agggacctgc ccaccaattc gaggtctaag accggtagag 14220

aaatgtgcag ttctaaccga ccatatcaag gcagaggcta tgttatctcc agcaggatct 14280

tcgtggaaca taaatccaat tattgtagac cattactcat gctctctgac ttatctccgg 14340

cgaggatcga tcaaacagat aagattgaga gttgatccag gattcatttt cgacgccctc 14400

gctgaggtaa atgtcagtca gccaaagatc ggcagcaaca acatctcaaa tatgagcatc 14460

aaggctttca gacccccaca cgatgatgtt gcaaaattgc tcaaagatat caacacaagc 14520

aagcacaatc ttcccatttc agggggcaat ctcgccaatt atgaaatcca tgctttccgc 14580

agaatcgggt tgaactcatc tgcttgctac aaagctgttg agatatcaac attaattagg 14640

agatgccttg agccagggga ggacggcttg ttcttgggtg agggatcggg ttctatgttg 14700

atcacttata aagagatact taaactaaac aagtgcttct ataatagtgg ggtttccgcc 14760

aattctagat ctggtcaaag ggaattagca ccctatccct ccgaagttgg ccttgtcgaa 14820

cacagaatgg gagtaggtaa tattgtcaaa gtgctcttta acggaggccc cgaagtcacg 14880

tgggtaggca gtgtagattg cttcaatttc atagttagta atatccctac ctctagtgtg 14940

gggtttatcc attcagatat agagaccttg cctgacaaag atactataga gaagctagag 15000

gaattggcag ccatcttatc gatggctctg ctcctgggca aaataggatc aatactggtg 15060

attaagctta tgcctttcag cggggatttt gttcagggat ttataagtta tgtagggtct 15120

cattatagag aagtgaacct tgtataccct agatacagca acttcatctc tactgaatct 15180

tatttggtta tgacagatct caaggctaac cggctaatga atcctgaaaa gattaagcag 15240

cagataattg aatcatctgt gaggacttca cctggactta taggtcacat cctatccatt 15300

aagcaactaa gctgcataca agcaattgtg ggagacgcag ttagtagagg tgatatcaat 15360

cctactctga aaaaacttac acctatagag caggtgctga tcaattgcgg gttggcaatt 15420

aacggaccta agctgtgcaa agaattgatc caccatgatg ttgcctcagg gcaagatgga 15480

ttgcttaatt ctatactcat cctctacagg gagttggcaa gattcaaaga caaccaaaga 15540

agtcaacaag ggatgttcca cgcttacccc gtattggtaa gtagcaggca acgagaactt 15600

atatctagga tcacccgcaa attctggggg cacattcttc tttactccgg gaacaaaaag 15660

ttgataaata agtttatcca gaatctcaag tccggctatc tgatactaga cttacaccag 15720

aatatcttcg ttaagaatct atccaagtca gagaaacaga ttattatgac ggggggtttg 15780

aaacgtgagt gggtttttaa ggtaacagtc aaggagacca aagaatggta taagttagtc 15840

ggatacagtg ccctgattaa ggactaattg gttgaactcc ggaaccctaa tcctgcccta 15900

ggtggttagg cattatttgc aatatattaa agaaaacttt gaaaatacga agtttctatt 15960

cccagctttg tctggtggcc ggcatggtcc cagcctcctc gctggcgccg gctgggcaac 16020

attccgaggg gaccgtcccc tcggtaatgg cgaatgggac gcggccgatc cggctgctaa 16080

caaagcccga aaggaagctg agttggctgc tgccaccgct gagcaataac tagcataacc 16140

ccttggggcc tctaaacggg tcttgagggg ttttttgctg aaaggaggaa ctatatccgg 16200

atgcggccgc gggccctatg gtacccagct tttgttccct ttagtgaggg ttaattccga 16260

gcttggcgta atcatggtca tagctgtttc ctgtgtgaaa ttgttatccg ctcacaattc 16320

cacacaacat aggagccgga agcataaagt gtaaagcctg gggtgcctaa tgagtgaggt 16380

aactcacatt aattgcgttg cgctcactgc ccgctttcca gtcgggaaac ctgtcgtgcc 16440

agctgcatta atgaatcggc caacgcgcgg ggagaggcgg tttgcgtatt gggcgctctt 16500

ccgcttcctc gctcactgac tcgctgcgct cggtcgttcg gctgcggcga gcggtatcag 16560

ctcactcaaa ggcggtaata cggttatcca cagaatcagg ggataacgca ggaaagaaca 16620

tgtgagcaaa aggccagcaa aaggccagga accgtaaaaa ggccgcgttg ctggcgtttt 16680

tccataggct ccgcccccct gacgagcatc acaaaaatcg acgctcaagt cagaggtggc 16740

gaaacccgac aggactataa agataccagg cgtttccccc tggaagctcc ctcgtgcgct 16800

ctcctgttcc gaccctgccg cttaccggat acctgtccgc ctttctccct tcgggaagcg 16860

tggcgctttc tcaatgctca cgctgtaggt atctcagttc ggtgtaggtc gttcgctcca 16920

agctgggctg tgtgcacgaa ccccccgttc agcccgaccg ctgcgcctta tccggtaact 16980

atcgtcttga gtccaacccg gtaagacacg acttatcgcc actggcagca gccactggta 17040

acaggattag cagagcgagg tatgtaggcg gtgctacaga gttcttgaag tggtggccta 17100

actacggcta cactagaagg acagtatttg gtatctgcgc tctgctgaag ccagttacct 17160

tcggaaaaag agttggtagc tcttgatccg gcaaacaaac caccgctggt agcggtggtt 17220

tttttgtttg caagcagcag attacgcgca gaaaaaaagg atctcaagaa gatcctttga 17280

tcttttctac ggggtctgac gctcagtgga acgaaaactc acgttaaggg attttggtca 17340

tgagattatc aaaaaggatc ttcacctaga tccttttaaa ttaaaaatga agttttaaat 17400

caatctaaag tatatatgag taaacttggt ctgacagtta ccaatgctta atcagtgagg 17460

cacctatctc agcgatctgt ctatttcgtt catccatagt tgcctgactg cccgtcgtgt 17520

agataactac gatacgggag ggcttaccat ctggccccag tgctgcaatg ataccgcgag 17580

acccacgctc accggctcca gatttatcag caataaacca gccagccgga agggccgagc 17640

gcagaagtgg tcctgcaact ttatccgcct ccatccagtc tattaattgt tgccgggaag 17700

ctagagtaag tagttcgcca gttaatagtt tgcgcaacgt tgttgccatt gctacaggca 17760

tcgtggtgtc acgctcgtcg tttggtatgg cttcattcag ctccggttcc caacgatcaa 17820

ggcgagttac atgatccccc atgttgtgaa aaaaagcggt tagctccttc ggtcctccga 17880

tcgttgtcag aagtaagttg gccgcagtgt tatcactcat gcttatggca gcactgcata 17940

attctcttac tgtcatgcca tccgtaagat gcttttctgt gactggtgag tactcaacca 18000

agtcattctg agaatagtgt atgcggcgac cgagttgctc ttgcccggcg tcaatacggg 18060

ataataccgc gccacatagc agaactttaa aagtgctcat cattggaaaa cgttcttcgg 18120

ggcgaaaact ctcaaggatc ttaccgctgt tgagatccag ttcgatgtaa cccactcgtg 18180

cacccaactg atcttcagca tcttttactt tcaccagcgt ttctgggtga gcaaaaacag 18240

gaaggcaaaa tgccgcaaaa aagggaataa gggcgacacg gaaatgttga atactcatac 18300

tcttcctttt tcaatattat tgaagcattt atcagggtta ttgtctcatg agcggataca 18360

tatttgaatg tatttagaaa aataaacaaa taggggttcc gcgcacattt ccccgaaaag 18420

tgccacctga aattgtaaac gttaatattt tgttaaaatt cgcgttaaat ttttgttaaa 18480

tcagctcatt ttttaaccaa taggccgaaa tcggcaaaat cccttataaa tcaaaagaat 18540

agaccgagat agggttgagt gttgttccag tttggaacaa gagtccacta ttaaagaacg 18600

tggactccaa cgtcaaaggg cgaaaaaccg tctatcaggg cgatggccca ctacgtgaac 18660

catcacccta atcaagtttt ttggggtcga ggtgccgtaa agcactaaat cggaacccta 18720

aagggagccc ccgatttaga gcttgacggg gaaagccggc gaacgtggcg agaaaggaag 18780

ggaagaaagc gaaaggagcg ggcgctaggg cgctggcaag tgtagcggtc acgctgcgcg 18840

taaccaccac acccgccgcg cttaatgcgc cgctacaggg cgcgtcccat tcgccattca 18900

ggctgcgcaa ctgttgggaa gggcgatcgg tgcgggcctc ttcgctatta cgccagccac 18960

cgcggtg 18967

<210> 6
<211> 19795
<212> DNA
<213> artificial sequence

<220>
<223> Synthetic sequence

<400> 6
gcggccgcta atacgactca ctatagggcc aactttgttt ggtctgatga gtccgtgagg 60

acgaaacccg gagtcccggg tcaccaaaca aagttgggta aggatagttc aatcaatgat 120

catcttctag tgcacttagg attcaagatc ctattatcag ggacaagagc aggattaggg 180

atatccgaga tggccacact tttaaggagc ttagcattgt tcaaaagaaa caaggacaaa 240

ccacccatta catcaggatc cggtggagcc atcagaggaa tcaaacacat tattatagta 300

ccaatccctg gagattcctc aattaccact cgatccagac ttctggaccg gttggtgagg 360

ttaattggaa acccggatgt gagcgggccc aaactaacag gggcactaat aggtatatta 420

tccttatttg tggagtctcc aggtcaattg attcagagga tcaccgatga ccctgacgtt 480

agcataaggc tgttagaggt tgtccagagt gaccagtcac aatctggcct taccttcgca 540

tcaagaggta ccaacatgga ggatgaggcg gaccaatact tttcacatga tgatccaatt 600

agtagtgatc aatccaggtt cggatggttc gggaacaagg aaatctcaga tattgaagtg 660

caagaccctg agggattcaa catgattctg ggtaccatcc tagcccaaat ttgggtcttg 720

ctcgcaaagg cggttacggc cccagacacg gcagctgatt cggagctaag aaggtggata 780

aagtacaccc aacaaagaag ggtagttggt gaatttagat tggagagaaa atggttggat 840

gtggtgagga acaggattgc cgaggacctc tccttacgcc gattcatggt cgctctaatc 900

ctggatatca agagaacacc cggaaacaaa cccaggattg ctgaaatgat atgtgacatt 960

gatacatata tcgtagaggc aggattagcc agttttatcc tgactattaa gtttgggata 1020

gaaactatgt atcctgctct tggactgcat gaatttgctg gtgagttatc cacacttgag 1080

tccttgatga acctttacca gcaaatgggg gaaactgcac cctacatggt aatcctggag 1140

aactcaattc agaacaagtt cagtgcagga tcataccctc tgctctggag ctatgccatg 1200

ggagtaggag tggaacttga aaactccatg ggaggtttga actttggccg atcttacttt 1260

gatccagcat attttagatt agggcaagag atggtaagga ggtcagctgg aaaggtcagt 1320

tccacattgg catctgaact cggtatcact gccgaggatg caaggcttgt ttcagagatt 1380

gcaatgcata ctactgagga caagatcagt agagcggttg gacccagaca agcccaagta 1440

tcatttctac acggtgatca aagtgagaat gagctaccga gattgggggg caaggaagat 1500

aggagggtca aacagagtcg aggagaagcc agggagagct acagagaaac cgggcccagc 1560

agagcaagtg atgcgagagc tgcccatctt ccaaccggca cacccctaga cattgacact 1620

gcaacggagt ccagccaaga tccgcaggac agtcgaaggt cagctgacgc cctgcttagg 1680

ctgcaagcca tggcaggaat ctcggaagaa caaggctcag acacggacac ccctatagtg 1740

tacaatgaca gaaatcttct agactaggtg cgagaggccg agggccagaa caacatccgc 1800

ctaccatcca tcattgttat aaaaaactta ggaaccaggt ccacacagcc gccagcccat 1860

caaccatcca ctcccacgat tggagccaat ggcagaagag caggcacgcc atgtcaaaaa 1920

cggactggaa tgcatccggg ctctcaaggc cgagcccatc ggctcactgg ccatcgagga 1980

agctatggca gcatggtcag aaatatcaga caacccagga caggagcgag ccacctgcag 2040

ggaagagaag gcaggcagtt cgggtctcag caaaccatgc ctctcagcaa ttggatcaac 2100

tgaaggcggt gcacctcgca tccgcggtca gggacctgga gagagcgatg acgacgctga 2160

aactttggga atccccccaa gaaatctcca ggcatcaagc actgggttac agtgttatta 2220

cgtttatgat cacagcggtg aagcggttaa gggaatccaa gatgctgact ctatcatggt 2280

tcaatcaggc cttgatggtg atagcaccct ctcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cccccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tgtgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agctaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaactagc ctaccctcca tcattgttat 3480

aaaaaactta ggaaccaggt ccacacagcc gccagcccat caacgcgtac gatggtgagc 3540

aagggcgagg agctgttcac cggggtggtg cccatcctgg tcgagctgga cggcgacgta 3600

aacggccaca agttcagcgt gtccggcgag ggcgagggcg atgccaccta cggcaagctg 3660

accctgaagt tcatctgcac caccggcaag ctgcccgtgc cctggcccac cctcgtgacc 3720

accctgacct acggcgtgca gtgcttcagc cgctaccccg accacatgaa gcagcacgac 3780

ttcttcaagt ccgccatgcc cgaaggctac gtccaggagc gcaccatctt cttcaaggac 3840

gacggcaact acaagacccg cgccgaggtg aagttcgagg gcgacaccct ggtgaaccgc 3900

atcgagctga agggcatcga cttcaaggag gacggcaaca tcctggggca caagctggag 3960

tacaactaca acagccacaa cgtctatatc atggccgaca agcagaagaa cggcatcaag 4020

gtgaacttca agatccgcca caacatcgag gacggcagcg tgcagctcgc cgaccactac 4080

cagcagaaca cccccatcgg cgacggcccc gtgctgctgc ccgacaacca ctacctgagc 4140

acccagtccg ccctgagcaa agaccccaac gagaagcgcg atcacatggt cctgctggag 4200

ttcgtgaccg ccgccgggat cactctcggc atggacgagc tgtacaagta ggcgcgcagc 4260

gcttagacgt ctcgcgatcg atactagtac aacctaaatc cattataaaa aacttaggag 4320

caaagtgatt gcctcccaag gtccacaatg acagagacct acgacttcga caagtcggca 4380

tgggacatca aagggtcgat cgctccgata caacccacca cctacagtga tggcaggctg 4440

gtgccccagg tcagagtcat agatcctggt ctaggcgaca ggaaggatga atgctttatg 4500

tacatgtttc tgctgggggt tgttgaggac agcgattccc tagggcctcc aatcgggcga 4560

gcatttgggt tcctgccctt aggtgttggc agatccacag caaagcccga aaaactcctc 4620

aaagaggcca ctgagcttga catagttgtt agacgtacag cagggctcaa tgaaaaactg 4680

gtgttctaca acaacacccc actaactctc ctcacacctt ggagaaaggt cctaacaaca 4740

gggagtgtct tcaacgcaaa ccaagtgtgc aatgcggtta atctgatacc gctcgatacc 4800

ccgcagaggt tccgtgttgt ttatatgagc atcacccgtc tttcggataa cgggtattac 4860

accgttccta gaagaatgct ggaattcaga tcggtcaatg cagtggcctt caacctgctg 4920

gtgaccctta ggattgacaa ggcgataggc cctgggaaga tcatcgacaa tacagagcaa 4980

cttcctgagg caacatttat ggtccacatc gggaacttca ggagaaagaa gagtgaagtc 5040

tactctgccg attattgcaa aatgaaaatc gaaaagatgg gcctggtttt tgcacttggt 5100

gggatagggg gcaccagtct tcacattaga agcacaggca aaatgagcaa gactctccat 5160

gcacaactcg ggttcaagaa gaccttatgt tacccgctga tggatatcaa tgaagacctt 5220

aatcgattac tctggaggag cagatgcaag atagtaagaa tccaggcagt tttgcagcca 5280

tcagttcctc aagaattccg catttacgac gacgtgatca taaatgatga ccaaggacta 5340

ttcaaagttc tgtagaccgt agtgcccagc aatgcccgaa aacgaccccc ctcacaatga 5400

cagccagaag gcccggacaa aaaagccccc tccgaaagac tccacggacc aagcgagagg 5460

ccagccagca gccgacggca agcgcgaaca ccaggcggcc ccagcacaga acagccctga 5520

cacaaggcca ccaccagcca ccccaatctg catcctcctc gtgggacccc cgaggaccaa 5580

cccccaaggc tgcccccgat ccaaaccacc aaccgcatcc ccaccacccc cgggaaagaa 5640

acccccagca attggaaggc ccctccccct cttcctcaac acaagaactc cacaaccgaa 5700

ccgcacaagc gaccgaggtg acccaaccgc aggcatccga ctccctagac agatcctctc 5760

tccccggcaa actaaacaaa acttagggcc aaggaacata cacacccaac agaacccaga 5820

ccccggccca cggcgccgcg cccccaaccc ccgacaacca gagggagccc ccaaccaatc 5880

ccgccggctc ccccggtgcc cacaggcagg gacaccaacc cccgaacaga cccagcaccc 5940

aaccatcgac aatccaagac gggggggccc ccccaaaaaa aggcccccag gggccgacag 6000

ccagcaccgc gaggaagccc acccacccca cacacgacca cggcaaccaa accagaaccc 6060

agaccaccct gggccaccag ctcccagact cggccatcac cccgcagaaa ggaaaggcca 6120

caacccgcgc accccagccc cgatccggcg gggagccacc caacccgaac cagcacccaa 6180

gagcgatccc cgaaggaccc ccgaaccgca aaggacatca gtatcccaca gcctctccaa 6240

gtcccccggt ctcctcctct tctcgaaggg accaaaagat caatccacca cacccgacga 6300

cactcaactc cccaccccta aaggagacac cgggaatccc agaatcaaga ctcatccaat 6360

gtccatcatg ggtctcaagg tgaacgtctc tgccatattc atggcagtac tgttaactct 6420

ccaaacaccc accggtcaaa tccattgggg caatctctct aagatagggg tggtaggaat 6480

aggaagtgca agctacaaag ttatgactcg ttccagccat caatcattag tcataaaatt 6540

aatgcccaat ataactctcc tcaataactg cacgagggta gagattgcag aatacaggag 6600

actactgaga acagttttgg aaccaattag agatgcactt aatgcaatga cccagaatat 6660

aagaccggtt cagagtgtag cttcaagtag gagacacaag agatttgcgg gagtagtcct 6720

ggcaggtgcg gccctaggcg ttgccacagc tgctcagata acagccggca ttgcacttca 6780

ccagtccatg ctgaactctc aagccatcga caatctgaga gcgagcctgg aaactactaa 6840

tcaggcaatt gagacaatca gacaagcagg gcaggagatg atattggctg ttcagggtgt 6900

ccaagactac atcaataatg agctgatacc gtctatgaac caactatctt gtgatttaat 6960

cggccagaag ctcgggctca aattgctcag atactataca gaaatcctgt cattatttgg 7020

ccccagttta cgggacccca tatctgcgga gatatctatc caggctttga gctatgcgct 7080

tggaggagac atcaataagg tgttagaaaa gctcggatac agtggaggtg atttactggg 7140

catcttagag agcggaggaa taaaggcccg gataactcac gtcgacacag agtcctactt 7200

cattgtcctc agtatagcct atccgacgct gtccgagatt aagggggtga ttgtccaccg 7260

gctagagggg gtctcgtaca acataggctc tcaagagtgg tataccactg tgcccaagta 7320

tgttgcaacc caagggtacc ttatctcgaa ttttgatgag tcatcgtgta ctttcatgcc 7380

agaggggact gtgtgcagcc aaaatgcctt gtacccgatg agtcctctgc tccaagaatg 7440

cctccggggg tacaccaagt cctgtgctcg tacactcgta tccgggtctt ttgggaaccg 7500

gttcatttta tcacaaggga acctaatagc caattgtgca tcaatccttt gcaagtgtta 7560

cacaacagga acgatcatta atcaagaccc tgacaagatc ctaacataca ttgctgccga 7620

tcactgcccg gtagtcgagg tgaacggcgt gaccatccaa gtcgggagca ggaggtatcc 7680

agacgctgtg tacttgcaca gaattgacct cggtcctccc atatcattgg agaggttgga 7740

cgtagggaca aatctgggga atgcaattgc taagttggag gatgccaagg aattgttgga 7800

gtcatcggac cagatattga ggagtatgaa aggtttatcg agcactagca tagtctacat 7860

cctgattgca gtgtgtcttg gagggttgat agggatcccc gctttaatat gttgctgcag 7920

ggggcgttgt aacaaaaagg gagaacaagt tggtatgtca agaccaggcc taaagcctga 7980

tcttacggga acatcaaaat cctatgtaag gtcgctctga tcctctacaa ctcttgaaac 8040

acaaatgtcc cacaagtctc ctcttcgtca tcaagcaacc accgcaccca gcatcaagcc 8100

cacctgaaat tatctccggc ttccctctgg ccgaacaata tcggtagtta atcaaaactt 8160

agggtgcaag atcatccaca atgtcaccac aacgagaccg gataaatgcc ttctacaaag 8220

ataaccccca tcccaaggga agtaggatag tcattaacag agaacatctt atgattgata 8280

gaccttatgt tttgctggct gttctgtttg tcatgtttct gagcttgatc gggttgctag 8340

ccattgcagg cattagactt catcgggcag ccatctacac cgcagagatc cataaaagcc 8400

tcagcaccaa tctagatgta actaactcaa tcgagcatca ggtcaaggac gtgctgacac 8460

cactcttcaa aatcatcggt gatgaagtgg gcctgaggac acctcagaga ttcactgacc 8520

tagtgaaatt aatctctgac aagattaaat tccttaatcc ggatagggag tacgacttca 8580

gagatctcac ttggtgtatc aacccgccag agagaatcaa attggattat gatcaatact 8640

gtgcagatgt ggctgctgaa gagctcatga atgcattggt gaactcaact ctactggaga 8700

ccagaacaac caatcagttc ctagctgtct caaaggaaaa ctgctcaggg cccactacaa 8760

tcagaggtca attctcaaac atgtcgctgt ccctgttaga cttgtattta ggtcgaggtt 8820

acaatgtgtc atctatagtc actatgacat cccagggaat gtatggggga acttacctag 8880

tggaaaagcc taatctgagc agcaaaaggt cagagttgtc acaactgagc atgtaccgag 8940

tgtttgaagt aggtgttatc agaaatccgg gtttgggggc tccggtgttc catatgacaa 9000

actatcttga gcaaccagtc agtaatgatc tcagcaactg tatggtggct ttgggggagc 9060

tcaaactcgc agccctttgt cacggggaag attctatcac aattccctat cagggatcag 9120

ggaaaggtgt cagcttccag ctcgtcaagc taggtgtctg gaaatcccca accgacatgc 9180

aatcctgggt ccccttatca acggatgatc cagtgataga caggctttac ctctcatctc 9240

acagaggtgt tatcgctgac aatcaagcaa aatgggctgt cccgacaaca cgaacagatg 9300

acaagttgcg aatggagaca tgcttccaac aggcgtgtaa gggtaaaatc caagcactct 9360

gcgagaatcc cgagtgggca ccattgaagg ataacaggat tccttcatac ggggtcttgt 9420

ctgttgatct gagtctgaca gttgagctta aaatcaaaat tgcttcggga ttcgggccat 9480

tgatcacaca cggttcaggg atggacctat acaaatccaa ccacaacaat gtgtattggc 9540

tgactatccc gccaatgaag aacctagcct taggtgtaat caacacattg gagtggatac 9600

cgagattcaa ggttagtccc tacctcttca ctgtcccaat taaggaagca ggcgaagact 9660

gccatgcccc aacataccta cctgcggagg tggatggtga tgtcaaactc agttccaatc 9720
tggtgattct acctggtcaa gatctccaat atgttttggc aacctacgat acttccaggg 9780
ttgaacatgc tgtggtttat tacgtttaca gcccaagccg ctcattttct tacttttatc 9840
cttttaggtt gcctataaag gggtccccca tcgaattaca agtggaatgc ttcacatggg 9900
accaaaaact ctggtgccgt cacttctgtg tgcttgcgga ctcagaatct ggtggacata 9960
tcactcactc tgggatggtg ggcatgggag tcagctgcac agtcacccgg gaagatggaa 10020
ccaatcgcag atagggctgc tagtgaacca atcacatgat gtcacccaga catcaggcat 10080
acccactagt gtgaaataga catcagaatt aagaaaaacg tagggtccaa gtggttcccc 10140
gttatggact cgctatctgt caaccagatc ttataccctg aagttcacct agatagcccg 10200
atagttacca ataagatagt agccatcctg gagtatgctc gagtccctca cgcttacagc 10260
ctggaggacc ctacactgtg tcagaacatc aagcaccgcc taaaaaacgg attttccaac 10320
caaatgatta taaacaatgt ggaagttggg aatgtcatca agtccaagct taggagttat 10380
ccggcccact ctcatattcc atatccaaat tgtaatcagg atttatttaa catagaagac 10440
aaagagtcaa cgaggaagat ccgtgaactc ctcaaaaagg ggaattcgct gtactccaaa 10500
gtcagtgata aggttttcca atgcttaagg gacactaact cacggcttgg cctaggctcc 10560
gaattgaggg aggacatcaa ggagaaagtt attaacttgg gagtttacat gcacagctcc 10620
cagtggtttg agccctttct gttttggttt acagtcaaga ctgagatgag gtcagtgatt 10680
aaatcacaaa cccatacttg ccataggagg agacacacac ctgtattctt cactggtagt 10740
tcagttgagt tgctaatctc tcgtgacctt gttgctataa tcagtaaaga gtctcaacat 10800
gtatattacc tgacatttga actggttttg atgtattgtg atgtcataga ggggaggtta 10860
atgacagaga ccgctatgac tattgatgct aggtatacag agcttctagg aagagtcaga 10920
tacatgtgga aactgataga tggtttcttc cctgcactcg ggaatccaac ttatcaaatt 10980
gtagccatgc tggagcctct ttcacttgct tacctgcagc tgagggatat aacagtagaa 11040
ctcagaggtg ctttccttaa ccactgcttt actgaaatac atgatgttct tgaccaaaac 11100
gggttttctg atgaaggtac ttatcatgag ttaactgaag ctctagatta cattttcata 11160
actgatgaca tacatctgac aggggagatt ttctcatttt tcagaagttt cggccacccc 11220
agacttgaag cagtaacggc tgctgaaaat gttaggaaat acatgaatca gcctaaagtc 11280
attgtgtatg agactctgat gaaaggtcat gccatatttt gtggaatcat aatcaacggc 11340
tatcgtgaca ggcacggagg cagttggcca ccgctgaccc tccccctgca tgctgcagac 11400
acaatccgga atgctcaagc ttcaggtgaa gggttaacac atgagcagtg cgttgataac 11460
tggaaatctt ttgctggagt gaaatttggc tgctttatgc ctcttagcct ggatagtgat 11520
ctgacaatgt acctaaagga caaggcactt gctgctctcc aaagggaatg ggattcagtt 11580

tacccgaaag agttcctgcg ttacgaccct cccaagggaa ccgggtcacg gaggcttgta 11640

gatgttttcc ttaatgattc gagctttgac ccatatgatg tgataatgta tgttgtaagt 11700

ggagcttacc tccatgaccc tgagttcaac ctgtcttaca gcctgaaaga aaaggagatc 11760

aaggaaacag gtagactttt tgctaaaatg acttacaaaa tgagggcatg ccaagtgatt 11820

gctgaaaatc taatctcaaa cgggattggc aaatatttta aggacaatgg gatggccaag 11880

gatgagcacg atttgactaa ggcactccac actctagctg tctcaggagt ccccaaagat 11940

ctcaaagaaa gtcacagggg ggggccagtc ttaaaaacct actcccgaag cccagtccac 12000

acaagtacca ggaacgtgag agcagcaaaa gggtttatag ggttccctca agtaattcgg 12060

caggaccaag acactgatca tccggagaat atggaagctt acgagacagt cagtgcattt 12120

atcacgactg atctcaagaa gtactgcctt aattggagat atgagaccat cagcttgttt 12180

gcacagaggc taaatgagat ttacggattg ccctcatttt tccagtggct gcataagagg 12240

cttgagacct ctgtcctgta tgtaagtgac cctcattgcc cccccgacct tgacgcccat 12300

atcccgttat ataaagtccc caatgatcaa atcttcatta agtaccctat gggaggtata 12360

gaagggtatt gtcagaagct gtggaccatc agcaccattc cctatctata cctggctgct 12420

tatgagagcg gagtaaggat tgcttcgtta gtgcaagggg acaatcagac catagccgta 12480

acaaaaaggg tacccagcac atggccctac aaccttaaga aacgggaagc tgctagagta 12540

actagagatt actttgtaat tcttaggcaa aggctacatg atattggcca tcacctcaag 12600

gcaaatgaga caattgtttc atcacatttt tttgtctatt caaaaggaat atattatgat 12660

gggctacttg tgtcccaatc actcaagagc atcgcaagat gtgtattctg gtcagagact 12720

atagttgatg aaacaagggc agcatgcagt aatattgcta caacaatggc taaaagcatc 12780

gagagaggtt atgaccgtta ccttgcatat tccctgaacg tcctaaaagt gatacagcaa 12840

attctgatct ctcttggctt cacaatcaat tcaaccatga cccgggatgt agtcataccc 12900

ctcctcacaa acaacgacct cttaataagg atggcactgt tgcccgctcc tattgggggg 12960

atgaattatc tgaatatgag caggctgttt gtcagaaaca tcggtgatcc agtaacatca 13020

tcaattgctg atctcaagag aatgattctc gcctcactaa tgcctgaaga gaccctccat 13080

caagtaatga cacaacaacc gggggactct tcattcctag actgggctag cgacccttac 13140

tcagcaaatc ttgtatgtgt ccagagcatc actagactcc tcaagaacat aactgcaagg 13200

tttgtcctga tccatagtcc aaacccaatg ttaaaaggat tattccatga tgacagtaaa 13260

gaagaggacg agggactggc ggcattcctc atggacaggc atattatagt acctagggca 13320

gctcatgaaa tcctggatca tagtgtcaca ggggcaagag agtctattgc aggcatgctg 13380

gataccacaa aaggcttgat tcgagccagc atgaggaagg gggggttaac ctctcgagtg 13440

ataaccagat tgtccaatta tgactatgaa caattcagag cagggatggt gctattgaca 13500

ggaagaaaga gaaatgtcct cattgacaaa gagtcatgtt cagtgcagct ggcgagagct 13560

ctaagaagcc atatgtgggc gaggctagct cgaggacggc ctatttacgg ccttgaggtc 13620

cctgatgtac tagaatctat gcgaggccac cttattcggc gtcatgagac atgtgtcatc 13680

tgcgagtgtg gatcagtcaa ctacggatgg ttttttgtcc cctcgggttg ccaactggat 13740

gatattgaca aggaaacatc atccttgaga gtcccatata ttggttctac cactgatgag 13800

agaacagaca tgaagcttgc cttcgtaaga gccccaagtc gatccttgcg atctgctgtt 13860

agaatagcaa cagtgtactc atgggcttac ggtgatgatg atagctcttg gaacgaagcc 13920

tggttgttgg ctaggcaaag ggccaatgtg agcctggagg agctaagggt gatcactccc 13980

atctcaactt cgactaattt agcgcatagg ttgagggatc gtagcactca agtgaaatac 14040

tcaggtacat cccttgtccg agtggcgagg tataccacaa tctccaacga caatctctca 14100

tttgtcatat cagataagaa ggttgatact aactttatat accaacaagg aatgcttcta 14160

gggttgggtg ttttagaaac attgtttcga ctcgagaaag ataccggatc atctaacacg 14220

gtattacatc ttcacgtcga aacagattgt tgcgtgatcc cgatgataga tcatcccagg 14280

atacccagct cccgcaagct agagctgagg gcagagctat gtaccaaccc attgatatat 14340

gataatgcac ctttaattga cagagatgca acaaggctat acacccagag ccataggagg 14400

caccttgtgg aatttgttac atggtccaca ccccaactat atcacatttt agctaagtcc 14460

acagcactat ctatgattga cctggtaaca aaatttgaga aggaccatat gaatgaaatt 14520

tcagctctca taggggatga cgatatcaat agtttcataa ctgagtttct gctcatagag 14580

ccaagattat tcactatcta cttgggccag tgtgcggcca tcaattgggc atttgatgta 14640

cattatcata gaccatcagg gaaatatcag atgggtgagc tgttgtcatc gttcctttct 14700

agaatgagca aaggagtgtt taaggtgctt gtcaatgctc taagccaccc aaagatctac 14760

aagaaattct ggcattgtgg tattatagag cctatccatg gtccttcact tgatgctcaa 14820

aacttgcaca caactgtgtg caacatggtt tacacatgct atatgaccta cctcgacctg 14880

ttgttgaatg aagagttaga agagttcaca tttctcttgt gtgaaagcga cgaggatgta 14940

gtaccggaca gattcgacaa catccaggca aaacacttat gtgttctggc agatttgtac 15000

tgtcaaccag ggacctgccc accaattcga ggtctaagac cggtagagaa atgtgcagtt 15060

ctaaccgacc atatcaaggc agaggctatg ttatctccag caggatcttc gtggaacata 15120

aatccaatta ttgtagacca ttactcatgc tctctgactt atctccggcg aggatcgatc 15180

aaacagataa gattgagagt tgatccagga ttcattttcg acgccctcgc tgaggtaaat 15240

gtcagtcagc caaagatcgg cagcaacaac atctcaaata tgagcatcaa ggctttcaga 15300

cccccacacg atgatgttgc aaaattgctc aaagatatca acacaagcaa gcacaatctt 15360

cccatttcag ggggcaatct cgccaattat gaaatccatg ctttccgcag aatcgggttg 15420

aactcatctg cttgctacaa agctgttgag atatcaacat taattaggag atgccttgag 15480

ccaggggagg acggcttgtt cttgggtgag ggatcgggtt ctatgttgat cacttataaa 15540

gagatactta aactaaacaa gtgcttctat aatagtgggg tttccgccaa ttctagatct 15600

ggtcaaaggg aattagcacc ctatccctcc gaagttggcc ttgtcgaaca cagaatggga 15660

gtaggtaata ttgtcaaagt gctctttaac gggaggcccg aagtcacgtg ggtaggcagt 15720

gtagattgct tcaatttcat agttagtaat atccctacct ctagtgtggg gtttatccat 15780

tcagatatag agaccttgcc tgacaaagat actatagaga agctagagga attggcagcc 15840

atcttatcga tggctctgct cctgggcaaa ataggatcaa tactggtgat taagcttatg 15900

cctttcagcg gggattttgt tcagggattt ataagttatg tagggtctca ttatagagaa 15960

gtgaaccttg tataccctag atacagcaac ttcatctcta ctgaatctta tttggttatg 16020

acagatctca aggctaaccg gctaatgaat cctgaaaaga ttaagcagca gataattgaa 16080

tcatctgtga ggacttcacc tggacttata ggtcacatcc tatccattaa gcaactaagc 16140

tgcatacaag caattgtggg agacgcagtt agtagaggtg atatcaatcc tactctgaaa 16200

aaacttacac ctatagagca ggtgctgatc aattgcgggt tggcaattaa cggacctaag 16260

ctgtgcaaag aattgatcca ccatgatgtt gcctcagggc aagatggatt gcttaattct 16320

atactcatcc tctacaggga gttggcaaga ttcaaagaca accaaagaag tcaacaaggg 16380

atgttccacg cttaccccgt attggtaagt agcaggcaac gagaacttat atctaggatc 16440

acccgcaaat tctgggggca cattcttctt tactccggga acaaaaagtt gataaataag 16500

tttatccaga atctcaagtc cggctatctg atactagact tacaccagaa tatcttcgtt 16560

aagaatctat ccaagtcaga gaaacagatt attatgacgg ggggtttgaa acgtgagtgg 16620

gtttttaagg taacagtcaa ggagaccaaa gaatggtata agttagtcgg atacagtgcc 16680

ctgattaagg actaattggt tgaactccgg aaccctaatc ctgccctagg tggttaggca 16740

ttatttgcaa tatattaaag aaaactttga aaatacgaag tttctattcc cagctttgtc 16800

tggtggccgg catggtccca gcctcctcgc tggcgccggc tgggcaacat tccgagggga 16860

ccgtcccctc ggtaatggcg aatgggacgc ggccgatccg gctgctaaca aagcccgaaa 16920

ggaagctgag ttggctgctg ccaccgctga gcaataacta gcataacccc ttggggcctc 16980

taaacgggtc ttgaggggtt ttttgctgaa aggaggaact atatccggat gcggccgcgg 17040

gccctatggt acccagcttt tgttcccttt agtgagggtt aattccgagc ttggcgtaat 17100

catggtcata gctgtttcct gtgtgaaatt gttatccgct cacaattcca cacaacatag 17160

gagccggaag cataaagtgt aaagcctggg gtgcctaatg agtgaggtaa ctcacattaa 17220

ttgcgttgcg ctcactgccc gctttccagt cgggaaacct gtcgtgccag ctgcattaat 17280

gaatcggcca acgcgcgggg agaggcggtt tgcgtattgg gcgctcttcc gcttcctcgc 17340

tcactgactc gctgcgctcg gtcgttcggc tgcggcgagc ggtatcagct cactcaaagg 17400

cggtaatacg gttatccaca gaatcagggg ataacgcagg aaagaacatg tgagcaaaag 17460

gccagcaaaa ggccaggaac cgtaaaaagg ccgcgttgct ggcgtttttc cataggctcg 17520

gcccccctga cgagcatcac aaaaatcgac gctcaagtca gaggtggcga aacccgacag 17580

gactataaag ataccaggcg tttccccctg gaagctccct cgtgcgctct cctgttccga 17640

ccctgccgct taccggatac ctgtccgcct ttctcccttc gggaagcgtg gcgctttctc 17700

aatgctcacg ctgtaggtat ctcagttcgg tgtaggtcgt tcgctccaag ctgggctgtg 17760

tgcacgaacc ccccgttcag cccgaccgct gcgccttatc cggtaactat cgtcttgagt 17820

ccaacccggt aagacacgac ttatcgccac tggcagcagc cactggtaac aggattagca 17880

gagcgaggta tgtaggcggt gctacagagt tcttgaagtg gtggcctaac tacggctaca 17940

ctagaaggac agtatttggt atctgcgctc tgctgaagcc agttaccttc ggaaaaagag 18000

ttggtagctc ttgatccggc aaacaaacca ccgctggtag cggtggtttt tttgtttgca 18060

agcagcagat tacgcgcaga aaaaaaggat ctcaagaaga tcctttgatc ttttctacgg 18120

ggtctgacgc tcagtggaac gaaaactcac gttaagggat tttggtcatg agattatcaa 18180

aaaggatctt cacctagatc cttttaaatt aaaaatgaag ttttaaatca atctaaagta 18240

tatatgagta aacttggtct gacagttacc aatgcttaat cagtgaggca cctatctcag 18300

cgatctgtct atttcgttca tccatagttg cctgactgcc cgtcgtgtag ataactacga 18360

tacgggaggg cttaccatct ggccccagtg ctgcaatgat accgcgagac ccacgctcac 18420

cggctccaga tttatcagca ataaaccagc cagccggaag ggccgagcgc agaagtggtc 18480

ctgcaacttt atccgcctcc atccagtcta ttaattgttg ccgggaagct agagtaagta 18540

gttcgccagt taatagtttg cgcaacgttg ttgccattgc tacaggcatc gtggtgtcac 18600

gctcgtcgtt tggtatggct tcattcagct ccggttccca acgatcaagg cgagttacat 18660

gatcccccat gttgtgaaaa aaagcggtta gctccttcgg tcctccgatc gttgtcagaa 18720

gtaagttggc cgcagtgtta tcactcatgc ttatggcagc actgcataat tctcttactg 18780

tcatgccatc cgtaagatgc ttttctgtga ctggtgagta ctcaaccaag tcattctgag 18840

aatagtgtat gcggcgaccg agttgctctt gcccggcgtc aatacgggat aataccgcgc 18900

cacatagcag aactttaaaa gtgctcatca ttggaaaacg ttcttcgggg cgaaaactct 18960

caaggatctt accgctgttg agatccagtt cgatgtaacc cactcgtgca cccaactgat 19020

cttcagcatc ttttactttc accagcgttt ctgggtgagc aaaaacagga aggcaaaatg 19080

ccgcaaaaaa gggaataagg gcgacacgga aatgttgaat actcatactc ttcctttttc 19140

aatattattg aagcatttat cagggttatt gtctcatgag cggatacata tttgaatgta 19200

tttagaaaaa taaacaaata ggggttccgc gcacatttcc ccgaaaagtg ccacctgaaa 19260

ttgtaaacgt taatattttg ttaaaattcg cgttaaattt ttgttaaatc agctcatttt 19320

ttaaccaata ggccgaaatc ggcaaaatcc cttataaatc aaaagaatag accgagatag 19380

ggttgagtgt tgttccagtt tggaacaaga gtccactatt aaagaacgtg gactccaacg 19440

tcaaagggcg aaaaaccgtc tatcagggcg atggcccact acgtgaacca tcaccctaat 19500

caagtttttt ggggtcgagg tgccgtaaag cactaaatcg gaaccctaaa gggagccccc 19560

gatttagagc ttgacgggga aagccggcga acgtggcgag aaaggaaggg aagaaagcga 19620

aaggagcggg cgctagggcg ctggcaagtg tagcggtcac gctgcgcgta accaccacac 19680

ccgccgcgct taatgcgccg ctacagggcg cgtcccattc gccattcagg ctgcgcaact 19740

gttgggaagg gcgatcggtg cgggcctctt cgctattacg ccagccaccg cggtg 19795

<210> 7
<211> 25717
<212> DNA
<213> artificial sequence

<220>
<223> Synthetic sequence

<400> 7
gcggccgcta atacgactca ctatagggcc aactttgttt ggtctgatga gtccgtgagg 60

acgaaacccg gagtcccggg tcaccaaaca aagttgggta aggatagttc aatcaatgat 120

catcttctag tgcacttagg attcaagatc ctattatcag ggacaagagc aggattaggg 180

atatccgaga tggccacact tttaaggagc ttagcattgt tcaaaagaaa caaggacaaa 240

ccacccatta catcaggatc cggtggagcc atcagaggaa tcaaacacat tattatagta 300

ccaatccctg gagattcctc aattaccact cgatccagac ttctggaccg gttggtgagg 360

ttaattggaa acccggatgt gagcgggccc aaactaacag gggcactaat aggtatatta 420

tccttatttg tggagtctcc aggtcaattg attcagagga tcaccgatga ccctgacgtt 480

agcataaggc tgttagaggt tgtccagagt gaccagtcac aatctggcct taccttcgca 540

tcaagaggta ccaacatgga ggatgaggcg gaccaatact tttcacatga tgatccaatt 600

agtagtgatc aatccaggtt cggatggttc gggaacaagg aaatctcaga tattgaagtg 660

caagaccctg agggattcaa catgattctg ggtaccatcc tagcccaaat ttgggtcttg 720

ctcgcaaagg cggttacggc cccagacacg gcagctgatt cggagctaag aaggtggata 780

aagtacaccc aacaaagaag ggtagttggt gaatttagat tggagagaaa atggttggat 840

gtggtgagga acaggattgc cgaggacctc tccttacgcc gattcatggt cgctctaatc 900

ctggatatca agagaacacc cggaaacaaa cccaggattg ctgaaatgat atgtgacatt 960

gatacatata tcgtagaggc aggattagcc agttttatcc tgactattaa gtttgggata 1020

gaaactatgt atcctgctct tggactgcat gaatttgctg gtgagttatc cacacttgag 1080

tccttgatga acctttacca gcaaatgggg gaaactgcac cctacatggt aatcctggag 1140

aactcaattc agaacaagtt cagtgcagga tcataccctc tgctctggag ctatgccatg 1200

ggagtaggag tggaacttga aaactccatg ggaggtttga actttggccg atcttacttt 1260

gatccagcat attttagatt agggcaagag atggtaagga ggtcagctgg aaaggtcagt 1320

tccacattgg catctgaact cggtatcact gccgaggatg caaggcttgt ttcagagatt 1380

gcaatgcata ctactgagga caagatcagt agagcggttg gacccagaca agcccaagta 1440

tcatttctac acggtgatca aagtgagaat gagctaccga gattgggggg caaggaagat 1500

aggagggtca aacagagtcg aggagaagcc agggagagct acagagaaac cgggcccagc 1560

agagcaagtg atgcgagagc tgcccatctt ccaaccggca cacccctaga cattgacact 1620

gcaacggagt ccagccaaga tccgcaggac agtcgaaggt cagctgacgc cctgcttagg 1680

ctgcaagcca tggcaggaat ctcggaagaa caaggctcag acacggacac ccctatagtg 1740

tacaatgaca gaaatcttct agactaggtg cgagaggccg agggccagaa caacatccgc 1800

ctaccatcca tcattgttat aaaaaactta ggaaccaggt ccacacagcc gccagcccat 1860

caaccatcca ctcccacgat tggagccaat ggcagaagag caggcacgcc atgtcaaaaa 1920

cggactggaa tgcatccggg ctctcaaggc cgagcccatc ggctcactgg ccatcgagga 1980

agctatggca gcatggtcag aaatatcaga caacccagga caggagcgag ccacctgcag 2040

ggaagagaag gcaggcagtt cgggtctcag caaaccatgc ctctcagcaa ttggatcaac 2100

tgaaggcggt gcacctcgca tccgcggtca gggacctgga gagagcgatg acgacgctga 2160

aactttggga atccccccaa gaaatctcca ggcatcaagc actgggttac agtgttatta 2220

cgtttatgat cacagcggtg aagcggttaa gggaatccaa gatgctgact ctatcatggt 2280

tcaatcaggc cttgatggtg atagcaccct ctcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cccccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tgtgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agctaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaactagc ctaccctcca tcattgttat 3480

aaaaaactta ggaaccaggt ccacacagcc gccagcccat caacgcgtac gatggtgagc 3540

aagggcgagg agctgttcac cggggtggtg cccatcctgg tcgagctgga cggcgacgta 3600

aacggccaca agttcagcgt gtccggcgag ggcgagggcg atgccaccta cggcaagctg 3660

accctgaagt tcatctgcac caccggcaag ctgcccgtgc cctggcccac cctcgtgacc 3720

accctgacct acggcgtgca gtgcttcagc cgctaccccg accacatgaa gcagcacgac 3780

ttcttcaagt ccgccatgcc cgaaggctac gtccaggagc gcaccatctt cttcaaggac 3840

gacggcaact acaagacccg cgccgaggtg aagttcgagg gcgacaccct ggtgaaccgc 3900

atcgagctga agggcatcga cttcaaggag gacggcaaca tcctggggca caagctggag 3960

tacaactaca acagccacaa cgtctatatc atggccgaca agcagaagaa cggcatcaag 4020

gtgaacttca agatccgcca caacatcgag gacggcagcg tgcagctcgc cgaccactac 4080

cagcagaaca cccccatcgg cgacggcccc gtgctgctgc ccgacaacca ctacctgagc 4140

acccagtccg ccctgagcaa agaccccaac gagaagcgcg atcacatggt cctgctggag 4200

ttcgtgaccg ccgccgggat cactctcggc atggacgagc tgtacaagta atagttcgaa 4260

ctacagctca acttacctgc caaccccatg ccagacgacc caactagtac aacctaaatc 4320

cattataaaa aacttaggaa ccaggtccac acagccgcca gcccatcaac catccactcc 4380

cacgattgga ggccggccat ggtgagcaag ggcgaggagg ataacatggc catcatcaag 4440

gagttcatgc gcttcaaggt gcacatggag ggctccgtga acggccacga gttcgagatc 4500

gagggcgagg gcgagggccg cccctacgag ggcacccaga ccgccaagct gaaggtgacc 4560

aagggtggcc ccctgccctt cgcctgggac atcctgtccc ctcagttcat gtacggctcc 4620

aaggcctacg tgaagcaccc cgccgacatc cccgactact tgaagctgtc cttccccgag 4680

ggcttcaagt gggagcgcgt gatgaacttc gaggacggcg gcgtggtgac cgtgacccag 4740

gactcctccc tgcaggacgg cgagttcatc tacaaggtga agctgcgcgg caccaacttc 4800

ccctccgacg gccccgtaat gcagaagaag accatgggct gggaggcctc ctccgagcgg 4860

atgtaccccg aggacggcgc cctgaagggc gagatcaagc agaggctgaa gctgaaggac 4920

ggcggccact acgacgctga ggtcaagacc acctacaagg ccaagaagcc cgtgcagctg 4980

cccggcgcct acaacgtcaa catcaagttg gacatcacct cccacaacga ggactacacc 5040

atcgtggaac agtacgaacg cgccgagggc cgtagtaaag cgcgcagcgc ttagacgtct 5100

cgcgatcgat actagtacaa cctaaatcca ttataaaaaa cttaggagca aagtgattgc 5160

ctcccaaggt ccacaatgac agagacctac gacttcgaca agtcggcatg ggacatcaaa 5220

gggtcgatcg ctccgataca acccaccacc tacagtgatg gcaggctggt gccccaggtc 5280

agagtcatag atcctggtct aggcgacagg aaggatgaat gctttatgta catgtttctg 5340

ctgggggttg ttgaggacag cgattcccta gggcctccaa tcgggcgagc atttgggttc 5400

ctgcccttag gtgttggcag atccacagca aagcccgaaa aactcctcaa agaggccact 5460

gagcttgaca tagttgttag acgtacagca gggctcaatg aaaaactggt gttctacaac 5520

aacaccccac taactctcct cacaccttgg agaaaggtcc taacaacagg gagtgtcttc 5580

aacgcaaacc aagtgtgcaa tgcggttaat ctgataccgc tcgatacccc gcagaggttc 5640

cgtgttgttt atatgagcat cacccgtctt tcggataacg ggtattacac cgttcctaga 5700

agaatgctgg aattcagatc ggtcaatgca gtggccttca acctgctggt gacccttagg 5760

attgacaagg cgataggccc tgggaagatc atcgacaata cagagcaact tcctgaggca 5820

acatttatgg tccacatcgg gaacttcagg agaaagaaga gtgaagtcta ctctgccgat 5880

tattgcaaaa tgaaaatcga aaagatgggc ctggtttttg cacttggtgg gatagggggc 5940

accagtcttc acattagaag cacaggcaaa atgagcaaga ctctccatgc acaactcggg 6000

ttcaagaaga ccttatgtta cccgctgatg gatatcaatg aagaccttaa tcgattactc 6060

tggaggagca gatgcaagat agtaagaatc caggcagttt tgcagccatc agttcctcaa 6120

gaattccgca tttacgacga cgtgatcata aatgatgacc aaggactatt caaagttctg 6180

tagaccgtag tgcccagcaa tgcccgaaaa cgacccccct cacaatgaca gccagaaggc 6240

ccggacaaaa aagccccctc cgaaagactc cacggaccaa gcgagaggcc agccagcagc 6300

cgacggcaag cgcgaacacc aggcggcccc agcacagaac agccctgaca caaggccacc 6360

accagccacc ccaatctgca tcctcctcgt gggacccccg aggaccaacc cccaaggctg 6420

cccccgatcc aaaccaccaa ccgcatcccc accacccccg ggaaagaaac ccccagcaat 6480

tggaaggccc ctccccctct tcctcaacac aagaactcca caaccgaacc gcacaagcga 6540

ccgaggtgac ccaaccgcag gcatccgact ccctagacag atcctctctc cccggcaaac 6600

taaacaaaac ttagggccaa ggaacataca cacccaacag aacccagacc ccggcccacg 6660

gcgccgcgcc cccaaccccc gacaaccaga gggagccccc aaccaatccc gccggctccc 6720

ccggtgccca caggcaggga caccaacccc cgaacagacc cagcacccaa ccatcgacaa 6780

tccaagacgg ggggcccccc ccaaaaaaag gcccccaggg gccgacagcc agcaccgcga 6840

ggaagcccac ccaccccaca cacgaccacg gcaaccaaac cagaacccag accaccctgg 6900

gccaccagct cccagactcg gccatcaccc cgcagaaagg aaaggccaca acccgcgcac 6960

cccagccccg atccggcggg gagccaccca acccgaacca gcacccaaga gcgatccccg 7020

aaggaccccc gaaccgcaaa ggacatcagt atcccacagc ctctccaagt cccccggtct 7080

cctcctcttc tcgaagggac caaaagatca atccaccaca cccgacgaca ctcaactccc 7140

cacccctaaa ggagacaccg ggaatcccag aatcaagact catccaatgt ccatcatggg 7200

tctcaaggtg aacgtctctg ccatattcat ggcagtactg ttaactctcc aaacacccac 7260

cggtcaaatc cattggggca atctctctaa gataggggtg gtaggaatag gaagtgcaag 7320

ctacaaagtt atgactcgtt ccagccatca atcattagtc ataaaattaa tgcccaatat 7380

aactctcctc aataactgca cgagggtaga gattgcagaa tacaggagac tactgagaac 7440

agttttggaa ccaattagag atgcacttaa tgcaatgacc cagaatataa gaccggttca 7500

gagtgtagct tcaagtagga gacacaagag atttgcggga gtagtcctgg caggtgcggc 7560

cctaggcgtt gccacagctg ctcagataac agccggcatt gcacttcacc agtccatgct 7620

gaactctcaa gccatcgaca atctgagagc gagcctggaa actactaatc aggcaattga 7680

gacaatcaga caagcagggc aggagatgat attggctgtt cagggtgtcc aagactacat 7740

caataatgag ctgataccgt ctatgaacca actatcttgt gatttaatcg gccagaagct 7800

cgggctcaaa ttgctcagat actatacaga aatcctgtca ttatttggcc ccagtttacg 7860

ggaccccata tctgcggaga tatctatcca ggctttgagc tatgcgcttg gaggagacat 7920

caataaggtg ttagaaaagc tcggatacag tggaggtgat ttactgggca tcttagagag 7980

cggaggaata aaggcccgga taactcacgt cgacacagag tcctacttca ttgtcctcag 8040

tatagcctat ccgacgctgt ccgagattaa gggggtgatt gtccaccggc tagagggggt 8100

ctcgtacaac ataggctctc aagagtggta taccactgtg cccaagtatg ttgcaaccca 8160

agggtacctt atctcgaatt ttgatgagtc atcgtgtact ttcatgccag aggggactgt 8220

gtgcagccaa aatgccttgt acccgatgag tcctctgctc caagaatgcc tccgggggta 8280

caccaagtcc tgtgctcgta cactcgtatc cgggtctttt gggaaccggt tcattttatc 8340

acaagggaac ctaatagcca attgtgcatc aatcctttgc aagtgttaca caacaggaac 8400

gatcattaat caagaccctg acaagatcct aacatacatt gctgccgatc actgcccggt 8460

agtcgaggtg aacggcgtga ccatccaagt cgggagcagg aggtatccag acgctgtgta 8520

cttgcacaga attgacctcg gtcctcccat atcattggag aggttggacg tagggacaaa 8580

tctggggaat gcaattgcta agttggagga tgccaaggaa ttgttggagt catcggacca 8640

gatattgagg agtatgaaag gtttatcgag cactagcata gtctacatcc tgattgcagt 8700

gtgtcttgga gggttgatag ggatccccgc tttaatatgt tgctgcaggg ggcgttgtaa 8760

caaaaaggga gaacaagttg gtatgtcaag accaggccta aagcctgatc ttacgggaac 8820

atcaaaatcc tatgtaaggt cgctctgatc ctctacaact cttgaaacac aaatgtccca 8880

caagtctcct cttcgtcatc aagcaaccac cgcacccagc atcaagccca cctgaaatta 8940

tctccggctt ccctctggcc gaacaatatc ggtagttaat caaaacttag ggtgcaagat 9000

catccacaat gtcaccacaa cgagaccgga taaatgcctt ctacaaagat aacccccatc 9060

ccaagggaag taggatagtc attaacagag aacatcttat gattgataga ccttatgttt 9120

tgctggctgt tctgtttgtc atgtttctga gcttgatcgg gttgctagcc attgcaggca 9180

ttagacttca tcgggcagcc atctacaccg cagagatcca taaaagcctc agcaccaatc 9240

tagatgtaac taactcaatc gagcatcagg tcaaggacgt gctgacacca ctcttcaaaa 9300

tcatcggtga tgaagtgggc ctgaggacac ctcagagatt cactgaccta gtgaaattaa 9360

tctctgacaa gattaaattc cttaatccgg atagggagta cgacttcaga gatctcactt 9420

ggtgtatcaa cccgccagag agaatcaaat tggattatga tcaatactgt gcagatgtgg 9480

ctgctgaaga gctcatgaat gcattggtga actcaactct actggagacc agaacaacca 9540

atcagttcct agctgtctca aagggaaact gctcagggcc cactacaatc agaggtcaat 9600

tctcaaacat gtcgctgtcc ctgttagact tgtatttagg tcgaggttac aatgtgtcat 9660

ctatagtcac tatgacatcc cagggaatgt atgggggaac ttacctagtg gaaaagccta 9720

atctgagcag caaaaggtca gagttgtcac aactgagcat gtaccgagtg tttgaagtag 9780

gtgttatcag aaatccgggt ttgggggctc cggtgttcca tatgacaaac tatcttgagc 9840

aaccagtcag taatgatctc agcaactgta tggtggcttt gggggagctc aaactcgcag 9900

ccctttgtca cggggaagat tctatcacaa ttccctatca gggatcaggg aaaggtgtca 9960

gcttccagct cgtcaagcta ggtgtctgga aatccccaac cgacatgcaa tcctgggtcc 10020

ccttatcaac ggatgatcca gtgatagaca ggctttacct ctcatctcac agaggtgtta 10080

tcgctgacaa tcaagcaaaa tgggctgtcc cgacaacacg aacagatgac aagttgcgaa 10140

tggagacatg cttccaacag gcgtgtaagg gtaaaatcca agcactctgc gagaatcccg 10200

agtgggcacc attgaaggat aacaggattc cttcatacgg ggtcttgtct gttgatctga 10260

gtctgacagt tgagcttaaa atcaaaattg cttcgggatt cgggccattg atcacacacg 10320

gttcagggat ggacctatac aaatccaacc acaacaatgt gtattggctg actatcccgc 10380

caatgaagaa cctagcctta ggtgtaatca acacattgga gtggataccg agattcaagg 10440

ttagtcccta cctcttcact gtcccaatta aggaagcagg cgaagactgc catgccccaa 10500

catacctacc tgcggaggtg gatggtgatg tcaaactcag ttccaatctg gtgattctac 10560

ctggtcaaga tctccaatat gttttggcaa cctacgatac ttccagggtt gaacatgctg 10620

tggtttatta cgtttacagc ccaagccgct cattttctta cttttatcct tttaggttgc 10680

ctataaaggg ggtccccatc gaattacaag tggaatgctt cacatgggac caaaaactct 10740

ggtgccgtca cttctgtgtg cttgcggact cagaatctgg tggacatatc actcactctg 10800

ggatggtggg catgggagtc agctgcacag tcacccggga agatggaacc aatcgcagat 10860

agggctgcta gtgaaccaat cacatgatgt cacccagaca tcaggcatac ccactagtgt 10920

gaaatagaca tcagaattaa gaaaaacgta gggtccaagt ggttccccgt tatggactcg 10980

ctatctgtca accagatctt ataccctgaa gttcacctag atagcccgat agttaccaat 11040

aagatagtag ccatcctgga gtatgctcga gtccctcacg cttacagcct ggaggaccct 11100

acactgtgtc agaacatcaa gcaccgccta aaaaacggat tttccaacca aatgattata 11160

aacaatgtgg aagttgggaa tgtcatcaag tccaagctta ggagttatcc ggcccactct 11220

catattccat atccaaattg taatcaggat ttatttaaca tagaagacaa agagtcaacg 11280

aggaagatcc gtgaactcct caaaaagggg aattcgctgt actccaaagt cagtgataag 11340

gttttccaat gcttaaggga cactaactca cggcttggcc taggctccga attgagggag 11400

gacatcaagg agaaagttat taacttggga gtttacatgc acagctccca gtggtttgag 11460

ccctttctgt tttggtttac agtcaagact gagatgaggt cagtgattaa atcacaaacc 11520

catacttgcc ataggaggag acacacacct gtattcttca ctggtagttc agttgagttg 11580

ctaatctctc gtgaccttgt tgctataatc agtaaagagt ctcaacatgt atattacctg 11640

acatttgaac tggttttgat gtattgtgat gtcatagagg ggaggttaat gacagagacc 11700

gctatgacta ttgatgctag gtatacagag cttctaggaa gagtcagata catgtggaaa 11760

ctgatagatg gtttcttccc tgcactcggg aatccaactt atcaaattgt agccatgctg 11820

gagcctcttt cacttgctta cctgcagctg agggatataa cagtagaact cagaggtgct 11880

ttccttaacc actgctttac tgaaatacat gatgttcttg accaaaacgg gttttctgat 11940

gaaggtactt atcatgagtt aactgaagct ctagattaca ttttcataac tgatgacata 12000

catctgacag gggagatttt ctcatttttc agaagtttcg gccaccccag acttgaagca 12060

gtaacggctg ctgaaaatgt taggaaatac atgaatcagc ctaaagtcat tgtgtatgag 12120

actctgatga aaggtcatgc catattttgt ggaatcataa tcaacggcta tcgtgacagg 12180

cacggaggca gttggccacc gctgaccctc cccctgcatg ctgcagacac aatccggaat 12240

gctcaagctt caggtgaagg gttaacacat gagcagtgcg ttgataactg gaaatctttt 12300
gctggagtga aatttggctg ctttatgcct cttagcctgg atagtgatct gacaatgtac 12360
ctaaaggaca aggcacttgc tgctctccaa agggaatggg attcagttta cccgaaagag 12420
ttcctgcgtt acgaccctcc caagggaacc gggtcacgga ggcttgtaga tgttttcctt 12480
aatgattcga gctttgaccc atatgatgtg ataatgtatg ttgtaagtgg agcttacctc 12540
catgaccctg agttcaacct gtcttacagc ctgaaagaaa aggagatcaa ggaaacaggt 12600
agactttttg ctaaaatgac ttacaaaatg agggcatgcc aagtgattgc tgaaaatcta 12660
atctcaaacg ggattggcaa atattttaag gacaatggga tggccaagga tgagcacgat 12720
ttgactaagg cactccacac tctagctgtc tcaggagtcc ccaaagatct caaagaaagt 12780
cacagggggg ggccagtctt aaaaacctac tcccgaagcc cagtccacac aagtaccagg 12840
aacgtgagag cagcaaaagg gtttataggg ttccctcaag taattcggca ggaccaagac 12900
actgatcatc cggagaatat ggaagcttac gagacagtca gtgcatttat cacgactgat 12960
ctcaagaagt actgccttaa ttggagatat gagaccatca gcttgtttgc acagaggcta 13020
aatgagattt acggattgcc ctcatttttc cagtggctgc ataagaggct tgagacctct 13080
gtcctgtatg taagtgaccc tcattgcccc cccgaccttg acgcccatat cccgttatat 13140
aaagtcccca atgatcaaat cttcattaag taccctatgg gaggtataga agggtattgt 13200
cagaagctgt ggaccatcag caccattccc tatctatacc tggctgctta tgagagcgga 13260
gtaaggattg cttcgttagt gcaaggggac aatcagacca tagccgtaac aaaaagggta 13320
cccagcacat ggccctacaa ccttaagaaa cgggaagctg ctagagtaac tagagattac 13380
tttgtaattc ttaggcaaag gctacatgat attggccatc acctcaaggc aaatgagaca 13440
attgtttcat cacatttttt tgtctattca aaaggaatat attatgatgg gctacttgtg 13500
tcccaatcac tcaagagcat cgcaagatgt gtattctggt cagagactat agttgatgaa 13560
acaagggcag catgcagtaa tattgctaca acaatggcta aaagcatcga gagaggttat 13620
gaccgttacc ttgcatattc cctgaacgtc ctaaaagtga tacagcaaat tctgatctct 13680
cttggcttca caatcaattc aaccatgacc cgggatgtag tcatacccct cctcacaaac 13740
aacgacctct taataaggat ggcactgttg cccgctccta ttggggggat gaattatctg 13800
aatatgagca ggctgtttgt cagaaacatc ggtgatccag taacatcatc aattgctgat 13860
ctcaagagaa tgattctcgc ctcactaatg cctgaagaga ccctccatca agtaatgaca 13920
caacaaccgg gggactcttc attcctagac tgggctagcg acccttactc agcaaatctt 13980
gtatgtgtcc agagcatcac tagactcctc aagaacataa ctgcaaggtt tgtcctgatc 14040
catagtccaa acccaatgtt aaaaggatta ttccatgatg acagtaaaga agaggacgag 14100
ggactggcgg cattcctcat ggacaggcat attatagtac ctagggcagc tcatgaaatc 14160

ctggatcata gtgtcacagg ggcaagagag tctattgcag gcatgctgga taccacaaaa 14220

ggcttgattc gagccagcat gaggaagggg gggttaacct ctcgagtgat aaccagattg 14280

tccaattatg actatgaaca attcagagca gggatggtgc tattgacagg aagaaagaga 14340

aatgtcctca ttgacaaaga gtcatgttca gtgcagctgg cgagagctct aagaagccat 14400

atgtgggcga ggctagctcg aggacggcct atttacggcc ttgaggtccc tgatgtacta 14460

gaatctatgc gaggccacct tattcggcgt catgagacat gtgtcatctg cgagtgtgga 14520

tcagtcaact acggatggtt ttttgtcccc tcgggttgcc aactggatga tattgacaag 14580

gaaacatcat ccttgagagt cccatatatt ggttctacca ctgatgagag aacagacatg 14640

aagcttgcct tcgtaagagc cccaagtcga tccttgcgat ctgctgttag aatagcaaca 14700

gtgtactcat gggcttacgg tgatgatgat agctcttgga acgaagcctg gttgttggct 14760

aggcaaaggg ccaatgtgag cctggaggag ctaagggtga tcactcccat ctcaacttcg 14820

actaatttag cgcataggtt gagggatcgt agcactcaag tgaaatactc aggtacatcc 14880

cttgtccgag tggcgaggta taccacaatc tccaacgaca atctctcatt tgtcatatca 14940

gataagaagg ttgatactaa ctttatatac caacaaggaa tgcttctagg gttgggtgtt 15000

ttagaaacat tgtttcgact cgagaaagat accggatcat ctaacacggt attacatctt 15060

cacgtcgaaa cagattgttg cgtgatcccg atgatagatc atcccaggat acccagctcc 15120

cgcaagctag agctgagggc agagctatgt accaacccat tgatatatga taatgcacct 15180

ttaattgaca gagatgcaac aaggctatac acccagagcc ataggaggca ccttgtggaa 15240

tttgttacat ggtccacacc ccaactatat cacattttag ctaagtccac agcactatct 15300

atgattgacc tggtaacaaa atttgagaag gaccatatga atgaaatttc agctctcata 15360

ggggatgacg atatcaatag tttcataact gagtttctgc tcatagagcc aagattattc 15420

actatctact tgggccagtg tgcggccatc aattgggcat ttgatgtaca ttatcataga 15480

ccatcaggga aatatcagat gggtgagctg ttgtcatcgt tcctttctag aatgagcaaa 15540

ggagtgttta aggtgcttgt caatgctcta agccacccaa agatctacaa gaaattctgg 15600

cattgtggta ttatagagcc tatccatggt ccttcacttg atgctcaaaa cttgcacaca 15660

actgtgtgca acatggttta cacatgctat atgacctacc tcgacctgtt gttgaatgaa 15720

gagttagaag agttcacatt tctcttgtgt gaaagcgacg aggatgtagt accggacaga 15780

ttcgacaaca tccaggcaaa acacttatgt gttctggcag atttgtactg tcaaccaggg 15840

acctgcccac caattcgagg tctaagaccg gtagagaaat gtgcagttct aaccgaccat 15900

atcaaggcag aggctatgtt atctccagca ggatcttcgt ggaacataaa tccaattatt 15960

gtagaccatt actcatgctc tctgacttat ctccggcgag gatcgatcaa acagataaga 16020

ttgagagttg atccaggatt cattttcgac gccctcgctg aggtaaatgt cagtcagcca 16080

aagatcggca gcaacaacat ctcaaatatg agcatcaagg ctttcagacc cccacacgat 16140

gatgttgcaa aattgctcaa agatatcaac acaagcaagc acaatcttcc catttcaggg 16200

ggcaatctcg ccaattatga aatccatgct ttccgcagaa tcgggttgaa ctcatctgct 16260

tgctacaaag ctgttgagat atcaacatta attaggagat gccttgagcc aggggaggac 16320

ggcttgttct tgggtgaggg atcgggttct atgttgatca cttataaaga gatacttaaa 16380

ctaaacaagt gcttctataa tagtggggtt tccgccaatt ctagatctgg tcaaagggaa 16440

ttagcaccct atccctccga agttggcctt gtcgaacaca gaatgggagt aggtaatatt 16500

gtcaaagtgc tctttaacgg gaggcccgaa gtcacgtggg taggcagtgt agattgcttc 16560

aatttcatag ttagtaatat ccctacctct agtgtggggt ttatccattc agatatagag 16620

accttgcctg acaaagatac tatagagaag ctagaggaat tggcagccat cttatcgatg 16680

gctctgctcc tgggcaaaat aggatcaata ctggtgatta agcttatgcc tttcagcggg 16740

gattttgttc agggatttat aagttatgta gggtctcatt atagagaagt gaaccttgta 16800

taccctagat acagcaactt catctctact gaatcttatt tggttatgac agatctcaag 16860

gctaaccggc taatgaatcc tgaaaagatt aagcagcaga taattgaatc atctgtgagg 16920

acttcacctg gacttatagg tcacatccta tccattaagc aactaagctg catacaagca 16980

attgtgggag acgcagttag tagaggtgat atcaatccta ctctgaaaaa acttacacct 17040

atagagcagg tgctgatcaa ttgcgggttg gcaattaacg gacctaagct gtgcaaagaa 17100

ttgatccacc atgatgttgc ctcagggcaa gatggattgc ttaattctat actcatcctc 17160

tacagggagt tggcaagatt caaagacaac caaagaagtc aacaagggat gttccacgct 17220

taccccgtat tggtaagtag caggcaacga gaacttatat ctaggatcac ccgcaaattc 17280

tgggggcaca ttcttcttta ctccgggaac aaaaagttga taaataagtt tatccagaat 17340

ctcaagtccg gctatctgat actagactta caccagaata tcttcgttaa gaatctatcc 17400

aagtcagaga aacagattat tatgacgggg ggtttgaaac gtgagtgggt ttttaaggta 17460

acagtcaagg agaccaaaga atggtataag ttagtcggat acagtgccct gattaaggac 17520

taattggttg aactccggaa ccctaatcct gccctaggtg gttaggcatt atttgcaata 17580

tattaaagaa aactttgaaa atacgaagtt tctattccca gctttgtctg gtggccggca 17640

tggtcccagc ctcctcgctg gcgccggctg ggcaacattc cgaggggacc gtcccctcgg 17700

taatggcgaa tgggacgcgg ccgatccggc tgctaacaaa gcccgaaagg aagctgagtt 17760

ggctgctgcc accgctgagc aataactagc ataacccctt ggggcctcta aacgggtctt 17820

gaggggtttt ttgctgaaag gaggaactat atccggatgc ggccgcttct atagtgtcac 17880

ctaaatacta gtgactccag cgtaactgga ctggccacag ttaggccgca aatgtaatca 17940

cactggctca ccttcgggtg ggcctttctg cgttcgcgag tggacccgat aagctcatgg 18000

agcggcgtaa ccgtcgcaca ggaaggacag agaaagcgcg gatctgggaa gtgacggaca 18060

gaacggtcag gacctggatt ggggaggcgg ttgccgccgc tgctgctgac ggtgtgacgt 18120

tctctgttcc ggtcacacca catacgttcc gccattccta tgcgatgcac atgctgtatg 18180

ccggtatacc gctgaaagtt ctgcaaagcc tgatgggaca taagtccatc agttcaacgg 18240

aagtctacac gaaggttttt gcgctggatg tggctgcccg gcaccgggtg cagtttgcga 18300

tgccggagtc tgatgcggtt gcgatgctga aacaattatc ctgagaataa atgccttggc 18360

ctttatatgg aaatgtggaa ctgagtggat atgctgtttt tgtctgttaa acagagaagc 18420

tggctgttat ccactgagaa gcgaacgaaa cagtcgggaa aatctcccat tatcgtagag 18480

atccgcatta ttaatctcag gagcctgtgt agcgtttata ggaagtagtg ttctgtcatg 18540

atgcctgcaa gcggtaacga aaacgatttg aatatgcctt caggaacaat agaaatcttc 18600

gtgcggtgtt acgttgaagt ggagcggatt atgtcagcaa tggacagaac aacctaatga 18660

acacagaacc atgatgtggt ctgtcctttt acagccagta gtgctcgccg cagtcgagcg 18720

acagggcgaa gccctcggct ggttgccctc gccgctgggc tggcggccgt ctatggccct 18780

gcaaacgcgc cagaaacgcc gtcgaagccg tgtgcgagac accgcggccc gccgccggcg 18840

ttgtggatac ctcgcggaaa acttggccct cactgacaga tgaggggcgg acgttgacac 18900

ttgaggggcc gactcacccg gcgcggcgtt gacagatgag gggcaggctc gatttcggcc 18960

ggcgacgtgg agctggccag cctcgcaaat cggcgaaaac gcctgatttt acgcgagttt 19020

cccacagatg atgtggacaa gcctggggat aagtgccctg cggtattgac acttgagggg 19080

cgcgactact gacagatgag gggcgcgatc cttgacactt gaggggcaga gtgctgacag 19140

atgaggggcg cacctattga catttgaggg gctgtccaca ggcagaaaat ccagcatttg 19200

caagggtttc cgcccgtttt tcggccaccg ctaacctgtc ttttaacctg cttttaaacc 19260

aatatttata aaccttgttt ttaaccaggg ctgcgccctg tgcgcgtgac cgcgcacgcc 19320

gaaggggggt gccccccctt ctcgaaccct cccggtcgag tgagcgagga agcaccaggg 19380

aacagcactt atatattctg cttacacacg atgcctgaaa aaacttccct tggggttatc 19440

cacttatcca cggggatatt tttataatta ttttttttat agtttttaga tcttcttttt 19500

tagagcgcct tgtaggcctt tatccatgct ggttctagag aaggtgttgt gacaaattgc 19560

cctttcagtg tgacaaatca ccctcaaatg acagtcctgt ctgtgacaaa ttgcccttaa 19620

ccctgtgaca aattgccctc agaagaagct gttttttcac aaagttatcc ctgcttattg 19680

actctttttt atttagtgtg acaatctaaa aacttgtcac acttcacatg gatctgtcat 19740

ggcggaaaca gcggttatca atcacaagaa acgtaaaaat agcccgcgaa tcgtccagtc 19800

aaacgacctc actgaggcgg catatagtct ctcccgggat caaaaacgta tgctgtatct 19860

gttcgttgac cagatcagaa aatctgatgg caccctacag gaacatgacg gtatctgcga 19920

gatccatgtt gctaaatatg ctgaaatatt cggattgacc tctgcggaag ccagtaagga 19980

tatacggcag gcattgaaga gtttcgcggg gaaggaagtg gttttttatc gccctgaaga 20040

ggatgccggc gatgaaaaag gctatgaatc ttttccttgg tttatcaaac gtgcgcacag 20100

tccatccaga gggctttaca gtgtacatat caacccatat ctcattccct tctttatcgg 20160

gttacagaac cggtttacgc agtttcggct tagtgaaaca aaagaaatca ccaatccgta 20220

tgccatgcgt ttatacgaat ccctgtgtca gtatcgtaag ccggatggct caggcatcgt 20280

ctctctgaaa atcgactgga tcatagagcg ttaccagctg cctcaaagtt accagcgtat 20340

gcctgacttc cgccgccgct tcctgcaggt ctgtgttaat gagatcaaca gcagaactcc 20400

aatgcgcctc tcatacattg agaaaaagaa aggccgccag acgactcata tcgtattttc 20460

cttccgcgat atcacttcca tgacgacagg atagtctgag ggttatctgt cacagatttg 20520

agggtggttc gtcacatttg ttctgaccta ctgagggtaa tttgtcacag ttttgctgtt 20580

tccttcagcc tgcatggatt ttctcatact ttttgaactg taatttttaa ggaagccaaa 20640

tttgagggca gtttgtcaca gttgatttcc ttctctttcc cttcgtcatg tgacctgata 20700

tcgggggtta gttcgtcatc attgatgagg gttgattatc acagtttatt actctgaatt 20760

ggctatccgc gtgtgtacct ctacctggag tttttcccac ggtggatatt tcttcttgcg 20820

ctgagcgtaa gagctatctg acagaacagt tcttctttgc ttcctcgcca gttcgctcgc 20880

tatgctcggt tacacggctg cggcgagcgc tagtgataat aagtgactga ggtatgtgct 20940

cttcttatct ccttttgtag tgttgctctt attttaaaca actttgcggt tttttgatga 21000

ctttgcgatt ttgttgttgc tttgcagtaa attgcaagat ttaataaaaa aacgcaaagc 21060

aatgattaaa ggatgttcag aatgaaactc atggaaacac ttaaccagtg cataaacgct 21120

ggtcatgaaa tgacgaaggc tatcgccatt gcacagttta atgatgacag cccggaagcg 21180

aggaaaataa cccggcgctg gagaataggt gaagcagcgg atttagttgg ggtttcttct 21240

caggctatca gagatgccga gaaagcaggg cgactaccgc acccggatat ggaaattcga 21300

ggacgggttg agcaacgtgt tggttataca attgaacaaa ttaatcatat gcgtgatgtg 21360

tttggtacgc gattgcgacg tgctgaagac gtatttccac cggtgatcgg ggttgctgcc 21420

cataaaggag gcgtttacaa aacctcagtt tctgttcatc ttgctcagga tctggctctg 21480

aaggggctac gtgttttgct cgtggaaggt aacgaccccc agggaacagc ctcaatgtat 21540

cacggatggg taccagatct tcatattcat gcagaagaca ctctcctgcc tttctatctt 21600

ggggaaaagg acgatgtcac ttatgcaata aagcccactt gctggccggg gcttgacatt 21660

attccttcct gtctggctct gcaccgtatt gaaactgagt taatgggcaa atttgatgaa 21720

ggtaaactgc ccaccgatcc acacctgatg ctccgactgg ccattgaaac tgttgctcat 21780

gactatgatg tcatagttat tgacagtgcg cctaacctgg gtatcggcac gattaatgtc 21840

gtatgtgctg ctgatgtgct gattgttccc acgcctgctg agttgtttga ctacacctcc 21900

gcactgcagt ttttcgatat gcttcgtgat ctgctcaaga acgttgatct taaagggttc 21960

gagcctgatg tacgtatttt gcttaccaaa tacagcaata gtaatggctc tcagtccccg 22020

tggatggagg agcaaattcg ggatgcctgg ggaagcatgg ttctaaaaaa tgttgtacgt 22080

gaaacggatg aagttggtaa aggtcagatc cggatgagaa ctgtttttga acaggccatt 22140

gatcaacgct cttcaactgg tgcctggaga aatgctcttt ctatttggga acctgtctgc 22200

aatgaaattt tcgatcgtct gattaaacca cgctgggaga ttagataatg aagcgtgcgc 22260

ctgttattcc aaaacatacg ctcaatactc aaccggttga agatacttcg ttatcgacac 22320

cagctgcccc gatggtggat tcgttaattg ctcgcgtagg agtaatggct cgcggtaatg 22380

ccattacttt gcctgtatgt ggtcgggatg tgaagtttac tcttgaagtg ctccggggtg 22440

atagtgttga gaagacctct cgggtatggt caggtaatga acgtgaccag gagctgctta 22500

ctgaggacgc actggatgat ctcatccctt cttttctact gactggtcaa cagacaccgg 22560

cgttcggtcg aagagtatct ggtgtcatag aaattgccga tgggagtcgc cgtcgtaaag 22620

ctgctgcact taccgaaagt gattatcgtg ttctggttgg cgagctggat gatgagcaga 22680

tggctgcatt atccagattg ggtaacgatt atcgcccaac aagtgcttat gaacgtggtc 22740

agcgttatgc aagccgattg cagaatgaat ttgctggaaa tatttctgcg ctggctgatg 22800

cggaaaatat ttcacgtaag attattaccc gctgtatcaa caccgccaaa ttgcctaaat 22860

cagttgttgc tcttttttct caccccggtg aactatctgc ccggtcaggt gatgcacttc 22920

aaaaagcctt tacagataaa gaggaattac ttaagcagca ggcatctaac cttcatgagc 22980

agaaaaaagc tggggtgata tttgaagctg aagaagttat cactctttta acttctgtgc 23040

ttaaaacgtc atctgcatca agaactagtt taagctcacg acatcagttt gctcctggag 23100

cgacagtatt gtataagggc gataaaatgg tgcttaacct ggacaggtct cgtgttccaa 23160

ctgagtgtat agagaaaatt gaggccattc ttaaggaact tgaaaagcca gcaccctgat 23220

gcgaccacgt tttagtctac gtttatctgt ctttacttaa tgtcctttgt tacaggccag 23280

aaagcataac tggcctgaat attctctctg ggcccactgt tccacttgta tcgtcggtct 23340

gataatcaga ctgggaccac ggtcccactc gtatcgtcgg tctgattatt agtctgggac 23400

cacggtccca ctcgtatcgt cggtctgatt attagtctgg gaccacggtc ccactcgtat 23460

cgtcggtctg ataatcagac tgggaccacg gtcccactcg tatcgtcggt ctgattatta 23520

gtctgggacc atggtcccac tcgtatcgtc ggtctgatta ttagtctggg accacggtcc 23580

cactcgtatc gtcggtctga ttattagtct ggaaccacgg tcccactcgt atcgtcggtc 23640

tgattattag tctgggacca cggtcccact cgtatcgtcg gtctgattat tagtctggga 23700

ccacgatccc actcgtgttg tcggtctgat tatcggtctg ggaccacggt cccacttgta 23760

ttgtcgatca gactatcagc gtgagactac gattccatca atgcctgtca agggcaagta 23820

ttgacatggt cgtcgtaacc tgtagaacgg agtaacctcg gtgtgcggtt gtatgcctgc 23880

tgtggattgc tgctgtgtcc tgcttatcca caacattttg cgcacggtta tgtggacaaa 23940

atacctggtt acccaggccg tgccggcacg ttaaccgggc tgcatccgat gcaagtgtgt 24000

cgctgacgac gagctcgcga gctcggacat gaggttgccc cgtattcagt gtcgctgatt 24060

tgtattgtct gaagttgctt ttacgttaag ttgatgcaga tcaattaata cgatacctgc 24120

gtcataattg attatttgac gtggtttgat ggcctccacg cacgttgtga tatgtagatg 24180

ataatcatta tcactttacg ggtcctttcc ggtgatccga caggttacgg ggcggcgacc 24240

tcgcgggttt tcgctattta tgaaaatttt ccggtttaag gcgtttccgt tcttcttcgt 24300

cataacttaa tgtttttatt taaaataccc tctgaaaaga aaggaaacga caggtgctga 24360

aagcgagctt tttggcctct gtcgtttcct ttctctgttt ttgtccgtgg aatgaacaat 24420

ggaagtccga gctcatcgct aataacttcg tatagcatac attatacgaa gttatattcg 24480

atgcggccga tctagcagaa agtcaaaagc ctccgaccgg aggcttttga cttctgtcac 24540

ctaggttacg ccccgccctg ccactcatcg cagtactgtt gtaattcatt aagcattctg 24600

ccgacatgga agccatcaca aacggcatga tgaacctgaa tcgccagcgg catcagcacc 24660

ttgtcgcctt gcgtataata tttgcccatg gtgaaaacgg gggcgaagaa gttgtccata 24720

ttggccacgt ttaaatcaaa actggtgaaa ctcacccagg gattggctga gacgaaaaac 24780

atattctcaa taaacccttt agggaaatag gccaggtttt caccgtaaca cgccacatct 24840

tgcgaatata tgtgtagaaa ctgccggaaa tcgtcgtggt attcactcca gagcgatgaa 24900

aacgtttcag tttgctcatg gaaaacggtg taacaagggt gaacactatc ccatatcacc 24960

agctcaccgt ctttcattgc catacgaaat tccggatgag cattcatcag gcgggcaaga 25020

atgtgaataa aggccggata aaacttgtgc ttatttttct ttacggtctt taaaaaggcc 25080

gtaatatcca gctgaacggt ctggttatag gtacattgag caactgactg aaatgcctca 25140

aaatgttctt tacgatgcca ttgggatata tcaacggtgg tatatccagt gatttttttc 25200

tccattttag cttccttagc tcctgaaaat ctcgataact caaaaaatac gcccggtagt 25260

gatcttattt cattatggtg aaagttggaa cctcttacgt gccgatcaga ttaaaacgaa 25320

aggcccagtc tttcgactga gcctttcgtt ttatttgacc atgttggtat gatttaaatt 25380

cgtttagtga accgtcagat cactagaagc tttattgcgg tagtttatca cagttaaatt 25440

gctaacgcag tcagtgcttc tgacacaaca gtctcgaact taagctgcag tgactctctt 25500

aaggtagcct tgcagaagtt ggtcgtgagg cactgggcag gtaagtatca aggttacaag 25560

acaggtttaa ggagaccaat agaaactggg cttgtcgaga cagagaagac tcttgcgttt 25620

ctgataggca cctattggtc ttactgacat ccactttgcc tttctctcca caggtgtcca 25680

ctcccagttc aattacagct cttaaggcta gagtact 25717

<210> 8
<211> 24062
<212> DNA
<213> artificial sequence

<220>
<223> Synthetic sequence

<400> 8
gcggccgcta atacgactca ctatagggcc aactttgttt ggtctgatga gtccgtgagg 60

acgaaacccg gagtcccggg tcaccaaaca aagttgggta aggatagttc aatcaatgat 120

catcttctag tgcacttagg attcaagatc ctattatcag ggacaagagc aggattaggg 180

atatccgaga tggccacact tttaaggagc ttagcattgt tcaaaagaaa caaggacaaa 240

ccacccatta catcaggatc cggtggagcc atcagaggaa tcaaacacat tattatagta 300

ccaatccctg gagattcctc aattaccact cgatccagac ttctggaccg gttggtgagg 360

ttaattggaa acccggatgt gagcgggccc aaactaacag gggcactaat aggtatatta 420

tccttatttg tggagtctcc aggtcaattg attcagagga tcaccgatga ccctgacgtt 480

agcataaggc tgttagaggt tgtccagagt gaccagtcac aatctggcct taccttcgca 540

tcaagaggta ccaacatgga ggatgaggcg gaccaatact tttcacatga tgatccaatt 600

agtagtgatc aatccaggtt cggatggttc gggaacaagg aaatctcaga tattgaagtg 660

caagaccctg agggattcaa catgattctg ggtaccatcc tagcccaaat ttgggtcttg 720

ctcgcaaagg cggttacggc cccagacacg gcagctgatt cggagctaag aaggtggata 780

aagtacaccc aacaaagaag ggtagttggt gaatttagat tggagagaaa atggttggat 840

gtggtgagga acaggattgc cgaggacctc tccttacgcc gattcatggt cgctctaatc 900

ctggatatca agagaacacc cggaaacaaa cccaggattg ctgaaatgat atgtgacatt 960

gatacatata tcgtagaggc aggattagcc agttttatcc tgactattaa gtttgggata 1020

gaaactatgt atcctgctct tggactgcat gaatttgctg gtgagttatc cacacttgag 1080

tccttgatga acctttacca gcaaatgggg gaaactgcac cctacatggt aatcctggag 1140

aactcaattc agaacaagtt cagtgcagga tcataccctc tgctctggag ctatgccatg 1200

ggagtaggag tggaacttga aaactccatg ggaggtttga actttggccg atcttacttt 1260

gatccagcat attttagatt agggcaagag atggtaagga ggtcagctgg aaaggtcagt 1320

tccacattgg catctgaact cggtatcact gccgaggatg caaggcttgt ttcagagatt 1380

gcaatgcata ctactgagga caagatcagt agagcggttg gacccagaca agcccaagta 1440

tcatttctac acggtgatca aagtgagaat gagctaccga gattgggggg caaggaagat 1500

aggagggtca aacagagtcg aggagaagcc agggagagct acagagaaac cgggcccagc 1560

agagcaagtg atgcgagagc tgcccatctt ccaaccggca cacccctaga cattgacact 1620

gcaacggagt ccagccaaga tccgcaggac agtcgaaggt cagctgacgc cctgcttagg 1680

ctgcaagcca tggcaggaat ctcggaagaa caaggctcag acacggacac ccctatagtg 1740

tacaatgaca gaaatcttct agactaggtg cgagaggccg agggccagaa caacatccgc 1800

ctaccatcca tcattgttat aaaaaactta ggaaccaggt ccacacagcc gccagcccat 1860

caaccatcca ctcccacgat tggagccaat ggcagaagag caggcacgcc atgtcaaaaa 1920

cggactggaa tgcatccggg ctctcaaggc cgagcccatc ggctcactgg ccatcgagga 1980

agctatggca gcatggtcag aaatatcaga caacccagga caggagcgag ccacctgcag 2040

ggaagagaag gcaggcagtt cgggtctcag caaaccatgc ctctcagcaa ttggatcaac 2100

tgaaggcggt gcacctcgca tccgcggtca gggacctgga gagagcgatg acgacgctga 2160

aactttggga atccccccaa gaaatctcca ggcatcaagc actgggttac agtgttatta 2220

cgtttatgat cacagcggtg aagcggttaa gggaatccaa gatgctgact ctatcatggt 2280

tcaatcaggc cttgatggtg atagcaccct ctcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cccccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tgtgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agctaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaaactag tacaacctaa atccattata 3480

aaaaacttag gagcaaagtg attgcctccc aaggtccaca atgacagaga cctacgactt 3540

cgacaagtcg gcatgggaca tcaaagggtc gatcgctccg atacaaccca ccacctacag 3600

tgatggcagg ctggtgcccc aggtcagagt catagatcct ggtctaggcg acaggaagga 3660

tgaatgcttt atgtacatgt ttctgctggg ggttgttgag gacagcgatt ccctagggcc 3720

tccaatcggg cgagcatttg ggttcctgcc cttaggtgtt ggcagatcca cagcaaagcc 3780

cgaaaaactc ctcaaagagg ccactgagct tgacatagtt gttagacgta cagcagggct 3840

caatgaaaaa ctggtgttct acaacaacac cccactaact ctcctcacac cttggagaaa 3900

ggtcctaaca acagggagtg tcttcaacgc aaaccaagtg tgcaatgcgg ttaatctgat 3960

accgctcgat accccgcaga ggttccgtgt tgtttatatg agcatcaccc gtctttcgga 4020

taacgggtat tacaccgttc ctagaagaat gctggaattc agatcggtca atgcagtggc 4080

cttcaacctg ctggtgaccc ttaggattga caaggcgata ggccctggga agatcatcga 4140

caatacagag caacttcctg aggcaacatt tatggtccac atcgggaact tcaggagaaa 4200

gaagagtgaa gtctactctg ccgattattg caaaatgaaa atcgaaaaga tgggcctggt 4260

ttttgcactt ggtgggatag ggggcaccag tcttcacatt agaagcacag gcaaaatgag 4320

caagactctc catgcacaac tcgggttcaa gaagacctta tgttacccgc tgatggatat 4380

caatgaagac cttaatcgat tactctggag gagcagatgc aagatagtaa gaatccaggc 4440

agttttgcag ccatcagttc ctcaagaatt ccgcatttac gacgacgtga tcataaatga 4500

tgaccaagga ctattcaaag ttctgtagac cgtagtgccc agcaatgccc gaaaacgacc 4560

cccctcacaa tgacagccag aaggcccgga caaaaaagcc ccctccgaaa gactccacgg 4620

accaagcgag aggccagcca gcagccgacg gcaagcgcga acaccaggcg gccccagcac 4680

agaacagccc tgacacaagg ccaccaccag ccaccccaat ctgcatcctc ctcgtgggac 4740

ccccgaggac caacccccaa ggctgccccc gatccaaacc accaaccgca tccccaccac 4800

ccccgggaaa gaaaccccca gcaattggaa ggcccctccc cctcttcctc aacacaagaa 4860

ctccacaacc gaaccgcaca agcgaccgag gtgacccaac cgcaggcatc cgactcccta 4920

gacagatcct ctctccccgg caaactaaac aaaacttagg gccaaggaac atacacaccc 4980

aacagaaccc agaccccggc ccacggcgcc gcgcccccaa cccccgacaa ccagagggag 5040

cccccaacca atcccgccgg ctcccccggt gcccacaggc agggacacca acccccgaac 5100

agacccagca cccaaccatc gacaatccaa gacggggggg cccccccaaa aaaaggcccc 5160

caggggccga cagccagcac cgcgaggaag cccacccacc ccacacacga ccacggcaac 5220

caaaccagaa cccagaccac cctgggccac cagctcccag actcggccat caccccgcag 5280

aaaggaaagg ccacaacccg cgcaccccag ccccgatccg gcggggagcc acccaacccg 5340

aaccagcacc caagagcgat ccccgaagga cccccgaacc gcaaaggaca tcagtatccc 5400

acagcctctc caagtccccc ggtctcctcc tcttctcgaa gggaccaaaa gatcaatcca 5460

ccacacccga cgacactcaa ctccccaccc ctaaaggaga caccgggaat cccagaatca 5520

agactcatcc aatgtccatc atgggtctca aggtgaacgt ctctgccata ttcatggcag 5580

tactgttaac tctccaaaca cccaccggtc aaatccattg gggcaatctc tctaagatag 5640

gggtggtagg aataggaagt gcaagctaca aagttatgac tcgttccagc catcaatcat 5700

tagtcataaa attaatgccc aatataactc tcctcaataa ctgcacgagg gtagagattg 5760

cagaatacag gagactactg agaacagttt tggaaccaat tagagatgca cttaatgcaa 5820

tgacccagaa tataagaccg gttcagagtg tagcttcaag taggagacac aagagatttg 5880

cgggagtagt cctggcaggt gcggccctag gcgttgccac agctgctcag ataacagccg 5940

gcattgcact tcaccagtcc atgctgaact ctcaagccat cgacaatctg agagcgagcc 6000

tggaaactac taatcaggca attgagacaa tcagacaagc agggcaggag atgatattgg 6060

ctgttcaggg tgtccaagac tacatcaata atgagctgat accgtctatg aaccaactat 6120

cttgtgattt aatcggccag aagctcgggc tcaaattgct cagatactat acagaaatcc 6180

tgtcattatt tggccccagt ttacggaccc ccatatctgc ggagatatct atccaggctt 6240

tgagctatgc gcttggagga gacatcaata aggtgttaga aaagctcgga tacagtggag 6300

gtgatttact gggcatctta gagagcggag gaataaaggc ccggataact cacgtcgaca 6360

cagagtccta cttcattgtc ctcagtatag cctatccgac gctgtccgag attaaggggg 6420

tgattgtcca ccggctagag ggggtctcgt acaacatagg ctctcaagag tggtatacca 6480

ctgtgcccaa gtatgttgca acccaagggt accttatctc gaattttgat gagtcatcgt 6540

gtactttcat gccagagggg actgtgtgca gccaaaatgc cttgtacccg atgagtcctc 6600

tgctccaaga atgcctccgg gggtacacca agtcctgtgc tcgtacactc gtatccgggt 6660

cttttgggaa ccggttcatt ttatcacaag ggaacctaat agccaattgt gcatcaatcc 6720

tttgcaagtg ttacacaaca ggaacgatca ttaatcaaga ccctgacaag atcctaacat 6780

acattgctgc cgatcactgc ccggtagtcg aggtgaacgg cgtgaccatc caagtcggga 6840

gcaggaggta tccagacgct gtgtacttgc acagaattga cctcggtcct cccatatcat 6900

tggagaggtt ggacgtaggg acaaatctgg ggaatgcaat tgctaagttg gaggatgcca 6960

aggaattgtt ggagtcatcg gaccagatat tgaggagtat gaaaggttta tcgagcacta 7020

gcatagtcta catcctgatt gcagtgtgtc ttggagggtt gatagggatc cccgctttaa 7080

tatgttgctg cagggggcgt tgtaacaaaa agggagaaca agttggtatg tcaagaccag 7140

gcctaaagcc tgatcttacg ggaacatcaa aatcctatgt aaggtcgctc tgatcctcta 7200

caactcttga aacacaaatg tcccacaagt ctcctcttcg tcatcaagca accaccgcac 7260

ccagcatcaa gcccacctga aattatctcc ggcttccctc tggccgaaca atatcggtag 7320

ttaatcaaaa cttagggtgc aagatcatcc acaatgtcac cacaacgaga ccggataaat 7380

gccttctaca aagataaccc ccatcccaag ggaagtagga tagtcattaa cagagaacat 7440

cttatgattg atagacctta tgttttgctg gctgttctgt ttgtcatgtt tctgagcttg 7500

atcgggttgc tagccattgc aggcattaga cttcatcggg cagccatcta caccgcagag 7560

atccataaaa gcctcagcac caatctagat gtaactaact caatcgagca tcaggtcaag 7620

gacgtgctga caccactctt caaaatcatc ggtgatgaag tgggcctgag gacacctcag 7680

agattcactg acctagtgaa attaatctct gacaagatta aattccttaa tccggatagg 7740

gagtacgact tcagagatct cacttggtgt atcaacccgc cagagagaat caaattggat 7800

tatgatcaat actgtgcaga tgtggctgct gaagagctca tgaatgcatt ggtgaactca 7860

actctactgg agaccagaac aaccaatcag ttcctagctg tctcaaaggg aaactgctca 7920

gggcccacta caatcagagg tcaattctca aacatgtcgc tgtccctgtt agacttgtat 7980

ttaggtcgag gttacaatgt gtcatctata gtcactatga catcccaggg aatgtatggg 8040

ggaacttacc tagtggaaaa gcctaatctg agcagcaaaa ggtcagagtt gtcacaactg 8100

agcatgtacc gagtgtttga agtaggtgtt atcagaaatc cgggtttggg ggctccggtg 8160

ttccatatga caaactatct tgagcaacca gtcagtaatg atctcagcaa ctgtatggtg 8220

gctttggggg agctcaaact cgcagccctt tgtcacgggg aagattctat cacaattccc 8280

tatcagggat cagggaaagg tgtcagcttc cagctcgtca agctaggtgt ctggaaatcc 8340

ccaaccgaca tgcaatcctg ggtcccctta tcaacggatg atccagtgat agacaggctt 8400

tacctctcat ctcacagagg tgttatcgct gacaatcaag caaaatgggc tgtcccgaca 8460

acacgaacag atgacaagtt gcgaatggag acatgcttcc aacaggcgtg taagggtaaa 8520

atccaagcac tctgcgagaa tcccgagtgg gcaccattga aggataacag gattccttca 8580

tacggggtct tgtctgttga tctgagtctg acagttgagc ttaaaatcaa aattgcttcg 8640

ggattcgggc cattgatcac acacggttca gggatggacc tatacaaatc caaccacaac 8700

aatgtgtatt ggctgactat cccgccaatg aagaacctag ccttaggtgt aatcaacaca 8760

ttggagtgga taccgagatt caaggttagt ccctacctct tcactgtccc aattaaggaa 8820

gcaggcgaag actgccatgc cccaacatac ctacctgcgg aggtggatgg tgatgtcaaa 8880

ctcagttcca atctggtgat tctacctggt caagatctcc aatatgtttt ggcaacctac 8940

gatacttcca gggttgaaca tgctgtggtt tattacgttt acagcccaag ccgctcattt 9000

tcttactttt atccttttag gttgcctata aagggggtcc ccatcgaatt acaagtggaa 9060

tgcttcacat gggaccaaaa actctggtgc cgtcacttct gtgtgcttgc ggactcagaa 9120

tctggtggac atatcactca ctctgggatg gtgggcatgg gagtcagctg cacagtcacc 9180

cgggaagatg gaaccaatcg cagatagggc tgctagtgaa ccaatcacat gatgtcaccc 9240

agacatcagg catacccact agtgtgaaat agacatcaga attaagaaaa acgtagggtc 9300

caagtggttc cccgttatgg actcgctatc tgtcaaccag atcttatacc ctgaagttca 9360

cctagatagc ccgatagtta ccaataagat agtagccatc ctggagtatg ctcgagtccc 9420

tcacgcttac agcctggagg accctacact gtgtcagaac atcaagcacc gcctaaaaaa 9480

cggattttcc aaccaaatga ttataaacaa tgtggaagtt gggaatgtca tcaagtccaa 9540

gcttaggagt tatccggccc actctcatat tccatatcca aattgtaatc aggatttatt 9600

taacatagaa gacaaagagt caacgaggaa gatccgtgaa ctcctcaaaa aggggaattc 9660

gctgtactcc aaagtcagtg ataaggtttt ccaatgctta agggacacta actcacggct 9720

tggcctaggc tccgaattga gggaggacat caaggagaaa gttattaact tgggagttta 9780

catgcacagc tcccagtggt ttgagccctt tctgttttgg tttacagtca agactgagat 9840

gaggtcagtg attaaatcac aaacccatac ttgccatagg aggagacaca cacctgtatt 9900

cttcactggt agttcagttg agttgctaat ctctcgtgac cttgttgcta taatcagtaa 9960

agagtctcaa catgtatatt acctgacatt tgaactggtt ttgatgtatt gtgatgtcat 10020

agaggggagg ttaatgacag agaccgctat gactattgat gctaggtata cagagcttct 10080

aggaagagtc agatacatgt ggaaactgat agatggtttc ttccctgcac tcgggaatcc 10140

aacttatcaa attgtagcca tgctggagcc tctttcactt gcttacctgc agctgaggga 10200

tataacagta gaactcagag gtgctttcct taaccactgc tttactgaaa tacatgatgt 10260

tcttgaccaa aacgggtttt ctgatgaagg tacttatcat gagttaactg aagctctaga 10320

ttacattttc ataactgatg acatacatct gacaggggag attttctcat ttttcagaag 10380

tttcggccac cccagacttg aagcagtaac ggctgctgaa aatgttagga aatacatgaa 10440

tcagcctaaa gtcattgtgt atgagactct gatgaaaggt catgccatat tttgtggaat 10500

cataatcaac ggctatcgtg acaggcacgg aggcagttgg ccaccgctga ccctccccct 10560

gcatgctgca gacacaatcc ggaatgctca agcttcaggt gaagggttaa cacatgagca 10620

gtgcgttgat aactggaaat cttttgctgg agtgaaattt ggctgcttta tgcctcttag 10680

cctggatagt gatctgacaa tgtacctaaa ggacaaggca cttgctgctc tccaaaggga 10740

atgggattca gtttacccga aagagttcct gcgttacgac cctcccaagg gaaccgggtc 10800

acggaggctt gtagatgttt tccttaatga ttcgagcttt gacccatatg atgtgataat 10860

gtatgttgta agtggagctt acctccatga ccctgagttc aacctgtctt acagcctgaa 10920

agaaaaggag atcaaggaaa caggtagact ttttgctaaa atgacttaca aaatgagggc 10980

atgccaagtg attgctgaaa atctaatctc aaacgggatt ggcaaatatt ttaaggacaa 11040

tgggatggcc aaggatgagc acgatttgac taaggcactc cacactctag ctgtctcagg 11100

agtccccaaa gatctcaaag aaagtcacag gggggggcca gtcttaaaaa cctactcccg 11160

aagcccagtc cacacaagta ccaggaacgt gagagcagca aaagggttta tagggttccc 11220

tcaagtaatt cggcaggacc aagacactga tcatccggag aatatggaag cttacgagac 11280

agtcagtgca tttatcacga ctgatctcaa gaagtactgc cttaattgga gatatgagac 11340

catcagcttg tttgcacaga ggctaaatga gatttacgga ttgccctcat ttttccagtg 11400

gctgcataag aggcttgaga cctctgtcct gtatgtaagt gaccctcatt gcccccccga 11460

ccttgacgcc catatcccgt tatataaagt ccccaatgat caaatcttca ttaagtaccc 11520

tatgggaggt atagaagggt attgtcagaa gctgtggacc atcagcacca ttccctatct 11580

atacctggct gcttatgaga gcggagtaag gattgcttcg ttagtgcaag gggacaatca 11640

gaccatagcc gtaacaaaaa gggtacccag cacatggccc tacaacctta agaaacggga 11700

agctgctaga gtaactagag attactttgt aattcttagg caaaggctac atgatattgg 11760

ccatcacctc aaggcaaatg agacaattgt ttcatcacat ttttttgtct attcaaaagg 11820

aatatattat gatgggctac ttgtgtccca atcactcaag agcatcgcaa gatgtgtatt 11880

ctggtcagag actatagttg atgaaacaag ggcagcatgc agtaatattg ctacaacaat 11940

ggctaaaagc atcgagagag gttatgaccg ttaccttgca tattccctga acgtcctaaa 12000

agtgatacag caaattctga tctctcttgg cttcacaatc aattcaacca tgacccggga 12060

tgtagtcata cccctcctca caaacaacga cctcttaata aggatggcac tgttgcccgc 12120

tcctattggg gggatgaatt atctgaatat gagcaggctg tttgtcagaa acatcggtga 12180

tccagtaaca tcatcaattg ctgatctcaa gagaatgatt ctcgcctcac taatgcctga 12240

agagaccctc catcaagtaa tgacacaaca accgggggac tcttcattcc tagactgggc 12300

tagcgaccct tactcagcaa atcttgtatg tgtccagagc atcactagac tcctcaagaa 12360

cataactgca aggtttgtcc tgatccatag tccaaaccca atgttaaaag gattattcca 12420

tgatgacagt aaagaagagg acgagggact ggcggcattc ctcatggaca ggcatattat 12480

agtacctagg gcagctcatg aaatcctgga tcatagtgtc acaggggcaa gagagtctat 12540

tgcaggcatg ctggatacca caaaaggctt gattcgagcc agcatgagga agggggggtt 12600

aacctctcga gtgataacca gattgtccaa ttatgactat gaacaattca gagcagggat 12660

ggtgctattg acaggaagaa agagaaatgt cctcattgac aaagagtcat gttcagtgca 12720

gctggcgaga gctctaagaa gccatatgtg ggcgaggcta gctcgaggac ggcctattta 12780

cggccttgag gtccctgatg tactagaatc tatgcgaggc caccttattc ggcgtcatga 12840

gacatgtgtc atctgcgagt gtggatcagt caactacgga tggttttttg tcccctcggg 12900

ttgccaactg gatgatattg acaaggaaac atcatccttg agagtcccat atattggttc 12960

taccactgat gagagaacag acatgaagct tgccttcgta agagccccaa gtcgatcctt 13020

gcgatctgct gttagaatag caacagtgta ctcatgggct tacggtgatg atgatagctc 13080

ttggaacgaa gcctggttgt tggctaggca aagggccaat gtgagcctgg aggagctaag 13140

ggtgatcact cccatctcaa cttcgactaa tttagcgcat aggttgaggg atcgtagcac 13200

tcaagtgaaa tactcaggta catcccttgt ccgagtggcg aggtatacca caatctccaa 13260

cgacaatctc tcatttgtca tatcagataa gaaggttgat actaacttta tataccaaca 13320

aggaatgctt ctagggttgg gtgttttaga aacattgttt cgactcgaga aagataccgg 13380

atcatctaac acggtattac atcttcacgt cgaaacagat tgttgcgtga tcccgatgat 13440

agatcatccc aggataccca gctcccgcaa gctagagctg agggcagagc tatgtaccaa 13500

cccattgata tatgataatg cacctttaat tgacagagat gcaacaaggc tatacaccca 13560

gagccatagg aggcaccttg tggaatttgt tacatggtcc acaccccaac tatatcacat 13620

tttagctaag tccacagcac tatctatgat tgacctggta acaaaatttg agaaggacca 13680

tatgaatgaa atttcagctc tcatagggga tgacgatatc aatagtttca taactgagtt 13740

tctgctcata gagccaagat tattcactat ctacttgggc cagtgtgcgg ccatcaattg 13800

ggcatttgat gtacattatc atagaccatc agggaaatat cagatgggtg agctgttgtc 13860

atcgttcctt tctagaatga gcaaaggagt gtttaaggtg cttgtcaatg ctctaagcca 13920

cccaaagatc tacaagaaat tctggcattg tggtattata gagcctatcc atggtccttc 13980

acttgatgct caaaacttgc acacaactgt gtgcaacatg gtttacacat gctatatgac 14040

ctacctcgac ctgttgttga atgaagagtt agaagagttc acatttctct tgtgtgaaag 14100

cgacgaggat gtagtaccgg acagattcga caacatccag gcaaaacact tatgtgttct 14160

ggcagatttg tactgtcaac cagggacctg cccaccaatt cgaggtctaa gaccggtaga 14220

gaaatgtgca gttctaaccg accatatcaa ggcagaggct atgttatctc cagcaggatc 14280

ttcgtggaac ataaatccaa ttattgtaga ccattactca tgctctctga cttatctccg 14340

gcgaggatcg atcaaacaga taagattgag agttgatcca ggattcattt tcgacgccct 14400

cgctgaggta aatgtcagtc agccaaagat cggcagcaac aacatctcaa atatgagcat 14460

caaggctttc agacccccac acgatgatgt tgcaaaattg ctcaaagata tcaacacaag 14520

caagcacaat cttcccattt cagggggcaa tctcgccaat tatgaaatcc atgctttccg 14580

cagaatcggg ttgaactcat ctgcttgcta caaagctgtt gagatatcaa cattaattag 14640

gagatgcctt gagccagggg aggacggctt gttcttgggt gagggatcgg gttctatgtt 14700

gatcacttat aaagagatac ttaaactaaa caagtgcttc tataatagtg gggtttccgc 14760

caattctaga tctggtcaaa gggaattagc accctatccc tccgaagttg gccttgtcga 14820

acacagaatg ggagtaggta atattgtcaa agtgctcttt aacgggaggc ccgaagtcac 14880

gtgggtaggc agtgtagatt gcttcaattt catagttagt aatatcccta cctctagtgt 14940

ggggtttatc cattcagata tagagacctt gcctgacaaa gatactatag agaagctaga 15000

ggaattggca gccatcttat cgatggctct gctcctgggc aaaataggat caatactggt 15060

gattaagctt atgcctttca gcggggattt tgttcaggga tttataagtt atgtagggtc 15120

tcattataga gaagtgaacc ttgtataccc tagatacagc aacttcatct ctactgaatc 15180

ttatttggtt atgacagatc tcaaggctaa ccggctaatg aatcctgaaa agattaagca 15240

gcagataatt gaatcatctg tgaggacttc acctggactt ataggtcaca tcctatccat 15300

taagcaacta agctgcatac aagcaattgt gggagacgca gttagtagag gtgatatcaa 15360

tcctactctg aaaaaactta cacctataga gcaggtgctg atcaattgcg ggttggcaat 15420

taacggacct aagctgtgca aagaattgat ccaccatgat gttgcctcag ggcaagatgg 15480

attgcttaat tctatactca tcctctacag ggagttggca agattcaaag acaaccaaag 15540

aagtcaacaa gggatgttcc acgcttaccc cgtattggta agtagcaggc aacgagaact 15600

tatatctagg atcacccgca aattctgggg gcacattctt ctttactccg ggaacaaaaa 15660

gttgataaat aagtttatcc agaatctcaa gtccggctat ctgatactag acttacacca 15720

gaatatcttc gttaagaatc tatccaagtc agagaaacag attattatga cggggggttt 15780

gaaacgtgag tgggttttta aggtaacagt caaggagacc aaagaatggt ataagttagt 15840

cggatacagt gccctgatta aggactaatt ggttgaactc cggaacccta atcctgccct 15900

aggtggttag gcattatttg caatatatta aagaaaactt tgaaaatacg aagtttctat 15960

tcccagcttt gtctggtggc cggcatggtc ccagcctcct cgctggcgcc ggctgggcaa 16020

cattccgagg ggaccgtccc ctcggtaatg gcgaatggga cgcggccgat ccggctgcta 16080

acaaagcccg aaaggaagct gagttggctg ctgccaccgc tgagcaataa ctagcataac 16140

cccttggggc ctctaaacgg gtcttgaggg gttttttgct gaaaggagga actatatccg 16200

gatgcggccg cttctatagt gtcacctaaa tactagtgac tccagcgtaa ctggactggc 16260

cacagttagg ccgcaaatgt aatcacactg gctcaccttc gggtgggcct ttctgcgttc 16320

gcgagtggac ccgataagct catggagcgg cgtaaccgtc gcacaggaag gacagagaaa 16380

gcgcggatct gggaagtgac ggacagaacg gtcaggacct ggattgggga ggcggttgcc 16440

gccgctgctg ctgacggtgt gacgttctct gttccggtca caccacatac gttccgccat 16500

tcctatgcga tgcacatgct gtatgccggt ataccgctga aagttctgca aagcctgatg 16560

ggacataagt ccatcagttc aacggaagtc tacacgaagg tttttgcgct ggatgtggct 16620

gcccggcacc gggtgcagtt tgcgatgccg gagtctgatg cggttgcgat gctgaaacaa 16680

ttatcctgag aataaatgcc ttggccttta tatggaaatg tggaactgag tggatatgct 16740

gtttttgtct gttaaacaga gaagctggct gttatccact gagaagcgaa cgaaacagtc 16800

gggaaaatct cccattatcg tagagatccg cattattaat ctcaggagcc tgtgtagcgt 16860

ttataggaag tagtgttctg tcatgatgcc tgcaagcggt aacgaaaacg atttgaatat 16920

gccttcagga acaatagaaa tcttcgtgcg gtgttacgtt gaagtggagc ggattatgtc 16980

agcaatggac agaacaacct aatgaacaca gaaccatgat gtggtctgtc cttttacagc 17040

cagtagtgct cgccgcagtc gagcgacagg gcgaagccct cggctggttg ccctcgccgc 17100

tgggctggcg gccgtctatg gccctgcaaa cgcgccagaa acgccgtcga agccgtgtgc 17160

gagacaccgc ggcccgccgc cggcgttgtg gatacctcgc ggaaaacttg gccctcactg 17220

acagatgagg ggcggacgtt gacacttgag gggccgactc acccggcgcg gcgttgacag 17280

atgaggggca ggctcgattt cggccggcga cgtggagctg gccagcctcg caaatcggcg 17340

aaaacgcctg attttacgcg agtttcccac agatgatgtg gacaagcctg gggataagtg 17400

ccctgcggta ttgacacttg aggggcgcga ctactgacag atgaggggcg cgatccttga 17460

cacttgaggg gcagagtgct gacagatgag gggcgcacct attgacattt gaggggctgt 17520

ccacaggcag aaaatccagc atttgcaagg gtttccgccc gtttttcggc caccgctaac 17580

ctgtctttta acctgctttt aaaccaatat ttataaacct tgtttttaac cagggctgcg 17640

ccctgtgcgc gtgaccgcgc acgccgaagg ggggtgcccc cccttctcga accctcccgg 17700

tcgagtgagc gaggaagcac cagggaacag cacttatata ttctgcttac acacgatgcc 17760

tgaaaaaact tcccttgggg ttatccactt atccacgggg atatttttat aattattttt 17820

tttatagttt ttagatcttc ttttttagag cgccttgtag gcctttatcc atgctggttc 17880

tagagaaggt gttgtgacaa attgcccttt cagtgtgaca aatcaccctc aaatgacagt 17940

cctgtctgtg acaaattgcc cttaaccctg tgacaaattg ccctcagaag aagctgtttt 18000

ttcacaaagt tatccctgct tattgactct tttttattta gtgtgacaat ctaaaaactt 18060

gtcacacttc acatggatct gtcatggcgg aaacagcggt tatcaatcac aagaaacgta 18120

aaaatagccc gcgaatcgtc cagtcaaacg acctcactga ggcggcatat agtctctccc 18180

gggatcaaaa acgtatgctg tatctgttcg ttgaccagat cagaaaatct gatggcaccc 18240

tacaggaaca tgacggtatc tgcgagatcc atgttgctaa atatgctgaa atattcggat 18300

tgacctctgc ggaagccagt aaggatatac ggcaggcatt gaagagtttc gcggggaagg 18360

aagtggtttt ttatcgccct gaagaggatg ccggcgatga aaaaggctat gaatcttttc 18420

cttggtttat caaacgtgcg cacagtccat ccagagggct ttacagtgta catatcaacc 18480

catatctcat tcccttcttt atcgggttac agaaccggtt tacgcagttt cggcttagtg 18540

aaacaaaaga aatcaccaat ccgtatgcca tgcgtttata cgaatccctg tgtcagtatc 18600

gtaagccgga tggctcaggc atcgtctctc tgaaaatcga ctggatcata gagcgttacc 18660

agctgcctca aagttaccag cgtatgcctg acttccgccg ccgcttcctg caggtctgtg 18720

ttaatgagat caacagcaga actccaatgc gcctctcata cattgagaaa aagaaaggcc 18780

gccagacgac tcatatcgta ttttccttcc gcgatatcac ttccatgacg acaggatagt 18840

ctgagggtta tctgtcacag atttgagggt ggttcgtcac atttgttctg acctactgag 18900

ggtaatttgt cacagttttg ctgtttcctt cagcctgcat ggattttctc atactttttg 18960

aactgtaatt tttaaggaag ccaaatttga gggcagtttg tcacagttga tttccttctc 19020

tttcccttcg tcatgtgacc tgatatcggg ggttagttcg tcatcattga tgagggttga 19080

ttatcacagt ttattactct gaattggcta tccgcgtgtg tacctctacc tggagttttt 19140

cccacggtgg atatttcttc ttgcgctgag cgtaagagct atctgacaga acagttcttc 19200

tttgcttcct cgccagttcg ctcgctatgc tcggttacac ggctgcggcg agcgctagtg 19260

ataataagtg actgaggtat gtgctcttct tatctccttt tgtagtgttg ctcttatttt 19320

aaacaacttt gcggtttttt gatgactttg cgattttgtt gttgctttgc agtaaattgc 19380

aagatttaat aaaaaaacgc aaagcaatga ttaaaggatg ttcagaatga aactcatgga 19440

aacacttaac cagtgcataa acgctggtca tgaaatgacg aaggctatcg ccattgcaca 19500

gtttaatgat gacagcccgg aagcgaggaa aataacccgg cgctggagaa taggtgaagc 19560

agcggattta gttggggttt cttctcaggc tatcagagat gccgagaaag cagggcgact 19620

accgcacccg gatatggaaa ttcgaggacg ggttgagcaa cgtgttggtt atacaattga 19680

acaaattaat catatgcgtg atgtgtttgg tacgcgattg cgacgtgctg aagacgtatt 19740

tccaccggtg atcggggttg ctgcccataa aggaggcgtt tacaaaacct cagtttctgt 19800

tcatcttgct caggatctgg ctctgaaggg gctacgtgtt ttgctcgtgg aaggtaacga 19860

cccccaggga acagcctcaa tgtatcacgg atgggtacca gatcttcata ttcatgcaga 19920

agacactctc ctgcctttct atcttgggga aaaggacgat gtcacttatg caataaagcc 19980

cacttgctgg ccggggcttg acattattcc ttcctgtctg gctctgcacc gtattgaaac 20040

tgagttaatg ggcaaatttg atgaaggtaa actgcccacc gatccacacc tgatgctccg 20100

actggccatt gaaactgttg ctcatgacta tgatgtcata gttattgaca gtgcgcctaa 20160

cctgggtatc ggcacgatta atgtcgtatg tgctgctgat gtgctgattg ttcccacgcc 20220

tgctgagttg tttgactaca cctccgcact gcagtttttc gatatgcttc gtgatctgct 20280

caagaacgtt gatcttaaag ggttcgagcc tgatgtacgt attttgctta ccaaatacag 20340

caatagtaat ggctctcagt ccccgtggat ggaggagcaa attcgggatg cctggggaag 20400

catggttcta aaaaatgttg tacgtgaaac ggatgaagtt ggtaaaggtc agatccggat 20460

gagaactgtt tttgaacagg ccattgatca acgctcttca actggtgcct ggagaaatgc 20520

tctttctatt tgggaacctg tctgcaatga aattttcgat cgtctgatta aaccacgctg 20580

ggagattaga taatgaagcg tgcgcctgtt attccaaaac atacgctcaa tactcaaccg 20640

gttgaagata cttcgttatc gacaccagct gccccgatgg tggattcgtt aattgctcgc 20700

gtaggagtaa tggctcgcgg taatgccatt actttgcctg tatgtggtcg ggatgtgaag 20760

tttactcttg aagtgctccg gggtgatagt gttgagaaga cctctcgggt atggtcaggt 20820

aatgaacgtg accaggagct gcttactgag gacgcactgg atgatctcat cccttctttt 20880

ctactgactg gtcaacagac accggcgttc ggtcgaagag tatctggtgt catagaaatt 20940

gccgatggga gtcgccgtcg taaagctgct gcacttaccg aaagtgatta tcgtgttctg 21000

gttggcgagc tggatgatga gcagatggct gcattatcca gattgggtaa cgattatcgc 21060

ccaacaagtg cttatgaacg tggtcagcgt tatgcaagcc gattgcagaa tgaatttgct 21120

ggaaatattt ctgcgctggc tgatgcggaa aatatttcac gtaagattat tacccgctgt 21180

atcaacaccg ccaaattgcc taaatcagtt gttgctcttt tttctcaccc cggtgaacta 21240

tctgcccggt caggtgatgc acttcaaaaa gcctttacag ataaagagga attacttaag 21300

cagcaggcat ctaaccttca tgagcagaaa aaagctgggg tgatatttga agctgaagaa 21360

gttatcactc ttttaacttc tgtgcttaaa acgtcatctg catcaagaac tagtttaagc 21420

tcacgacatc agtttgctcc tggagcgaca gtattgtata agggcgataa aatggtgctt 21480

aacctggaca ggtctcgtgt tccaactgag tgtatagaga aaattgaggc cattcttaag 21540

gaacttgaaa agccagcacc ctgatgcgac cacgttttag tctacgttta tctgtcttta 21600

cttaatgtcc tttgttacag gccagaaagc ataactggcc tgaatattct ctctgggccc 21660

actgttccac ttgtatcgtc ggtctgataa tcagactggg accacggtcc cactcgtatc 21720

gtcggtctga ttattagtct gggaccacgg tcccactcgt atcgtcggtc tgattattag 21780

tctgggacca cggtcccact cgtatcgtcg gtctgataat cagactggga ccacggtccc 21840

actcgtatcg tcggtctgat tattagtctg ggaccatggt cccactcgta tcgtcggtct 21900

gattattagt ctgggaccac ggtcccactc gtatcgtcgg tctgattatt agtctggaac 21960

cacggtccca ctcgtatcgt cggtctgatt attagtctgg gaccacggtc ccactcgtat 22020

cgtcggtctg attattagtc tgggaccacg atcccactcg tgttgtcggt ctgattatcg 22080

gtctgggacc acggtcccac ttgtattgtc gatcagacta tcagcgtgag actacgattc 22140

catcaatgcc tgtcaagggc aagtattgac atggtcgtcg taacctgtag aacggagtaa 22200

cctcggtgtg cggttgtatg cctgctgtgg attgctgctg tgtcctgctt atccacaaca 22260

ttttgcgcac ggttatgtgg acaaaatacc tggttaccca ggccgtgccg gcacgttaac 22320

cgggctgcat ccgatgcaag tgtgtcgctg acgacgagct cgcgagctcg gacatgaggt 22380

tgccccgtat tcagtgtcgc tgatttgtat tgtctgaagt tgcttttacg ttaagttgat 22440

gcagatcaat taatacgata cctgcgtcat aattgattat ttgacgtggt ttgatggcct 22500

ccacgcacgt tgtgatatgt agatgataat cattatcact ttacgggtcc tttccggtga 22560

tccgacaggt tacggggcgg cgacctcgcg ggttttcgct atttatgaaa attttccggt 22620

ttaaggcgtt tccgttcttc ttcgtcataa cttaatgttt ttatttaaaa taccctctga 22680

aaagaaagga aacgacaggt gctgaaagcg agctttttgg cctctgtcgt ttcctttctc 22740

tgtttttgtc cgtggaatga acaatggaag tccgagctca tcgctaataa cttcgtatag 22800

catacattat acgaagttat attcgatgcg gccgatctag cagaaagtca aaagcctccg 22860

accggaggct tttgacttct gtcacctagg ttacgccccg ccctgccact catcgcagta 22920

ctgttgtaat tcattaagca ttctgccgac atggaagcca tcacaaacgg catgatgaac 22980

ctgaatcgcc agcggcatca gcaccttgtc gccttgcgta taatatttgc ccatggtgaa 23040

aacgggggcg aagaagttgt ccatattggc cacgtttaaa tcaaaactgg tgaaactcac 23100

ccagggattg gctgagacga aaaacatatt ctcaataaac cctttaggga aataggccag 23160

gttttcaccg taacacgcca catcttgcga atatatgtgt agaaactgcc ggaaatcgtc 23220

gtggtattca ctccagagcg atgaaaacgt ttcagtttgc tcatggaaaa cggtgtaaca 23280

agggtgaaca ctatcccata tcaccagctc accgtctttc attgccatac gaaattccgg 23340

atgagcattc atcaggcggg caagaatgtg aataaaggcc ggataaaact tgtgcttatt 23400

tttctttacg gtctttaaaa aggccgtaat atccagctga acggtctggt tataggtaca 23460

ttgagcaact gactgaaatg cctcaaaatg ttctttacga tgccattggg atatatcaac 23520

ggtggtatat ccagtgattt ttttctccat tttagcttcc ttagctcctg aaaatctcga 23580

taactcaaaa aatacgcccg gtagtgatct tatttcatta tggtgaaagt tggaacctct 23640

tacgtgccga tcagattaaa acgaaaggcc cagtctttcg actgagcctt tcgttttatt 23700

tgaccatgtt ggtatgattt aaattcgttt agtgaaccgt cagatcacta gaagctttat 23760

tgcggtagtt tatcacagtt aaattgctaa cgcagtcagt gcttctgaca caacagtctc 23820

gaacttaagc tgcagtgact ctcttaaggt agccttgcag aagttggtcg tgaggcactg 23880

ggcaggtaag tatcaaggtt acaagacagg tttaaggaga ccaatagaaa ctgggcttgt 23940

cgagacagag aagactcttg cgtttctgat aggcacctat tggtcttact gacatccact 24000

ttgcctttct ctccacaggt gtccactccc agttcaatta cagctcttaa ggctagagta 24060

ct 24062

<210> 9
<211> 7648
<212> DNA
<213> artificial sequence

<220>
<223> Synthetic sequence

<400> 9
ccatcacatt gggatcctaa gcttagaatt ccacgtggac aagttgtcgc ggccgcttct 60

atagtgtcac ctaaatacta gtgactccag cgtaactgga ctggccacag ttaggccgca 120

aatgtaatca cactggctca ccttcgggtg ggcctttctg cgttcgcgag tggacccgat 180

aagctcatgg agcggcgtaa ccgtcgcaca ggaaggacag agaaagcgcg gatctgggaa 240

gtgacggaca gaacggtcag gacctggatt ggggaggcgg ttgccgccgc tgctgctgac 300

ggtgtgacgt tctctgttcc ggtcacacca catacgttcc gccattccta tgcgatgcac 360

atgctgtatg ccggtatacc gctgaaagtt ctgcaaagcc tgatgggaca taagtccatc 420

agttcaacgg aagtctacac gaaggttttt gcgctggatg tggctgcccg gcaccgggtg 480

cagtttgcga tgccggagtc tgatgcggtt gcgatgctga aacaattatc ctgagaataa 540

atgccttggc ctttatatgg aaatgtggaa ctgagtggat atgctgtttt tgtctgttaa 600

acagagaagc tggctgttat ccactgagaa gcgaacgaaa cagtcgggaa aatctcccat 660

tatcgtagag atccgcatta ttaatctcag gagcctgtgt agcgtttata ggaagtagtg 720

ttctgtcatg atgcctgcaa gcggtaacga aaacgatttg aatatgcctt caggaacaat 780

agaaatcttc gtgcggtgtt acgttgaagt ggagcggatt atgtcagcaa tggacagaac 840

aacctaatga acacagaacc atgatgtggt ctgtcctttt acagccagta gtgctcgccg 900

cagtcgagcg acagggcgaa gccctcggct ggttgccctc gccgctgggc tggcggccgt 960

ctatggccct gcaaacgcgc cagaaacgcc gtcgaagccg tgtgcgagac accgcggccg 1020

gccgccggcg ttgtggatac ctcgcggaaa acttggccct cactgacaga tgaggggcgg 1080

acgttgacac ttgaggggcc gactcacccg gcgcggcgtt gacagatgag gggcaggctc 1140

gatttcggcc ggcgacgtgg agctggccag cctcgcaaat cggcgaaaac gcctgatttt 1200

acgcgagttt cccacagatg atgtggacaa gcctggggat aagtgccctg cggtattgac 1260

acttgagggg cgcgactact gacagatgag gggcgcgatc cttgacactt gaggggcaga 1320

gtgctgacag atgaggggcg cacctattga catttgaggg gctgtccaca ggcagaaaat 1380

ccagcatttg caagggtttc cgcccgtttt tcggccaccg ctaacctgtc ttttaacctg 1440

cttttaaacc aatatttata aaccttgttt ttaaccaggg ctgcgccctg tgcgcgtgac 1500

cgcgcacgcc gaaggggggt gccccccctt ctcgaaccct cccggtcgag tgagcgagga 1560

agcaccaggg aacagcactt atatattctg cttacacacg atgcctgaaa aaacttccct 1620

tggggttatc cacttatcca cggggatatt tttataatta ttttttttat agtttttaga 1680

tcttcttttt tagagcgcct tgtaggcctt tatccatgct ggttctagag aaggtgttgt 1740

gacaaattgc cctttcagtg tgacaaatca ccctcaaatg acagtcctgt ctgtgacaaa 1800

ttgcccttaa ccctgtgaca aattgccctc agaagaagct gttttttcac aaagttatcc 1860

ctgcttattg actctttttt atttagtgtg acaatctaaa aacttgtcac acttcacatg 1920

gatctgtcat ggcggaaaca gcggttatca atcacaagaa acgtaaaaat agcccgcgaa 1980

tcgtccagtc aaacgacctc actgaggcgg catatagtct ctcccgggat caaaaacgta 2040

tgctgtatct gttcgttgac cagatcagaa aatctgatgg caccctacag gaacatgacg 2100

gtatctgcga gatccatgtt gctaaatatg ctgaaatatt cggattgacc tctgcggaag 2160

ccagtaagga tatacggcag gcattgaaga gtttcgcggg gaaggaagtg gttttttatc 2220

gccctgaaga ggatgccggc gatgaaaaag gctatgaatc ttttccttgg tttatcaaac 2280

gtgcgcacag tccatccaga gggctttaca gtgtacatat caacccatat ctcattccct 2340

tctttatcgg gttacagaac cggtttacgc agtttcggct tagtgaaaca aaagaaatca 2400

ccaatccgta tgccatgcgt ttatacgaat ccctgtgtca gtatcgtaag ccggatggct 2460

caggcatcgt ctctctgaaa atcgactgga tcatagagcg ttaccagctg cctcaaagtt 2520

accagcgtat gcctgacttc cgccgccgct tcctgcaggt ctgtgttaat gagatcaaca 2580

gcagaactcc aatgcgcctc tcatacattg agaaaaagaa aggccgccag acgactcata 2640

tcgtattttc cttccgcgat atcacttcca tgacgacagg atagtctgag ggttatctgt 2700

cacagatttg agggtggttc gtcacatttg ttctgaccta ctgagggtaa tttgtcacag 2760

ttttgctgtt tccttcagcc tgcatggatt ttctcatact ttttgaactg taatttttaa 2820

ggaagccaaa tttgagggca gtttgtcaca gttgatttcc ttctctttcc cttcgtcatg 2880

tgacctgata tcgggggtta gttcgtcatc attgatgagg gttgattatc acagtttatt 2940

actctgaatt ggctatccgc gtgtgtacct ctacctggag tttttcccac ggtggatatt 3000

tcttcttgcg ctgagcgtaa gagctatctg acagaacagt tcttctttgc ttcctcgcca 3060

gttcgctcgc tatgctcggt tacacggctg cggcgagcgc tagtgataat aagtgactga 3120

ggtatgtgct cttcttatct ccttttgtag tgttgctctt attttaaaca actttgcggt 3180

tttttgatga ctttgcgatt ttgttgttgc tttgcagtaa attgcaagat ttaataaaaa 3240

aacgcaaagc aatgattaaa ggatgttcag aatgaaactc atggaaacac ttaaccagtg 3300

cataaacgct ggtcatgaaa tgacgaaggc tatcgccatt gcacagttta atgatgacag 3360

cccggaagcg aggaaaataa cccggcgctg gagaataggt gaagcagcgg atttagttgg 3420

ggtttcttct caggctatca gagatgccga gaaagcaggg cgactaccgc acccggatat 3480

ggaaattcga ggacgggttg agcaacgtgt tggttataca attgaacaaa ttaatcatat 3540

gcgtgatgtg tttggtacgc gattgcgacg tgctgaagac gtatttccac cggtgatcgg 3600

ggttgctgcc cataaaggag gcgtttacaa aacctcagtt tctgttcatc ttgctcagga 3660

tctggctctg aaggggctac gtgttttgct cgtggaaggt aacgaccccc agggaacagc 3720

ctcaatgtat cacggatggg taccagatct tcatattcat gcagaagaca ctctcctgcc 3780

tttctatctt ggggaaaagg acgatgtcac ttatgcaata aagcccactt gctggccggg 3840

gcttgacatt attccttcct gtctggctct gcaccgtatt gaaactgagt taatgggcaa 3900

atttgatgaa ggtaaactgc ccaccgatcc acacctgatg ctccgactgg ccattgaaac 3960

tgttgctcat gactatgatg tcatagttat tgacagcgcg cctaacctgg gtatcggcac 4020

gattaatgtc gtatgtgctg ctgatgtgct gattgttccc acgcctgctg agttgtttga 4080

ctacacctcc gcactgcagt ttttcgatat gcttcgtgat ctgctcaaga acgttgatct 4140

taaagggttc gagcctgatg tacgtatttt gcttaccaaa tacagcaata gtaatggctc 4200

tcagtccccg tggatggagg agcaaattcg ggatgcctgg ggaagcatgg ttctaaaaaa 4260

tgttgtacgt gaaacggatg aagttggtaa aggtcagatc cggatgagaa ctgtttttga 4320

acaggccatt gatcaacgct cttcaactgg tgcctggaga aatgctcttt ctatttggga 4380

acctgtctgc aatgaaattt tcgatcgtct gattaaacca cgctgggaga ttagataatg 4440

aagcgtgcgc ctgttattcc aaaacatacg ctcaatactc aaccggttga agatacttcg 4500

ttatcgacac cagctgcccc gatggtggat tcgttaattg cgcgcgtagg agtaatggct 4560

cgcggtaatg ccattacttt gcctgtatgt ggtcgggatg tgaagtttac tcttgaagtg 4620

ctccggggtg atagtgttga gaagacctct cgggtatggt caggtaatga acgtgaccag 4680

gagctgctta ctgaggacgc actggatgat ctcatccctt cttttctact gactggtcaa 4740

cagacaccgg cgttcggtcg aagagtatct ggtgtcatag aaattgccga tgggagtcgc 4800

cgtcgtaaag ctgctgcact taccgaaagt gattatcgtg ttctggttgg cgagctggat 4860

gatgagcaga tggctgcatt atccagattg ggtaacgatt atcgcccaac aagtgcttat 4920

gaacgtggtc agcgttatgc aagccgattg cagaatgaat ttgctggaaa tatttctgcg 4980

ctggctgatg cggaaaatat ttcacgtaag attattaccc gctgtatcaa caccgccaaa 5040

ttgcctaaat cagttgttgc tcttttttct caccccggtg aactatctgc ccggtcaggt 5100

gatgcacttc aaaaagcctt tacagataaa gaggaattac ttaagcagca ggcatctaac 5160

cttcatgagc agaaaaaagc tggggtgata tttgaagctg aagaagttat cactctttta 5220

acttctgtgc ttaaaacgtc atctgcatca agaactagtt taagctcacg acatcagttt 5280

gctcctggag cgacagtatt gtataagggc gataaaatgg tgcttaacct ggacaggtct 5340

cgtgttccaa ctgagtgtat agagaaaatt gaggccattc ttaaggaact tgaaaagcca 5400

gcaccctgat gcgaccacgt tttagtctac gtttatctgt ctttacttaa tgtcctttgt 5460

tacaggccag aaagcataac tggcctgaat attctctctg ggcccactgt tccacttgta 5520

tcgtcggtct gataatcaga ctgggaccac ggtcccactc gtatcgtcgg tctgattatt 5580

agtctgggac cacggtccca ctcgtatcgt cggtctgatt attagtctgg gaccacggtc 5640

ccactcgtat cgtcggtctg ataatcagac tgggaccacg gtcccactcg tatcgtcggt 5700

ctgattatta gtctgggacc atggtcccac tcgtatcgtc ggtctgatta ttagtctggg 5760

accacggtcc cactcgtatc gtcggtctga ttattagtct ggaaccacgg tcccactcgt 5820

atcgtcggtc tgattattag tctgggacca cggtcccact cgtatcgtcg gtctgattat 5880

tagtctggga ccacgatccc actcgtgttg tcggtctgat tatcggtctg ggaccacggt 5940

cccacttgta ttgtcgatca gactatcagc gtgagactac gattccatca atgcctgtca 6000

agggcaagta ttgacatggt cgtcgtaacc tgtagaacgg agtaacctcg gtgtgcggtt 6060

gtatgcctgc tgtggattgc tgctgtgtcc tgcttatcca caacattttg cgcacggtta 6120

tgtggacaaa atacctggtt acccaggccg tgccggcacg ttaaccgggc tgcatccgat 6180

gcaagtgtgt cgctgtcgac gagctcgcga gctcggacat gaggttgccc cgtattcagt 6240

gtcgctgatt tgtattgtct gaagttgctt ttacgttaag ttgatgcaga tcaattaata 6300

cgatacctgc gtcataattg attatttgac gtggtttgat ggcctccacg cacgttgtga 6360

tatgtagatg ataatcatta tcactttacg ggtcctttcc ggtgatccga caggttacgg 6420

ggcggcgacc tcgcgggttt tcgctattta tgaaaatttt ccggtttaag gcgtttccgt 6480

tcttcttcgt cataacttaa tgtttttatt taaaataccc tctgaaaaga aaggaaacga 6540

caggtgctga aagcgagctt tttggcctct gtcgtttcct ttctctgttt ttgtccgtgg 6600

aatgaacaat ggaagtccga gctcatcgct aataacttcg tatagcatac attatacgaa 6660

gttatattcg atgcggccga tctagcagaa agtcaaaagc ctccgaccgg aggcttttga 6720

cttctgtcac ctaggttacg ccccgccctg ccactcatcg cagtactgtt gtaattcatt 6780

aagcattctg ccgacatgga agccatcaca aacggcatga tgaacctgaa tcgccagcgg 6840

catcagcacc ttgtcgcctt gcgtataata tttgcccatg gtgaaaacgg gggcgaagaa 6900

gttgtccata ttggccacgt ttaaatcaaa actggtgaaa ctcacccagg gattggctga 6960

gacgaaaaac atattctcaa taaacccttt agggaaatag gccaggtttt caccgtaaca 7020

cgccacatct tgcgaatata tgtgtagaaa ctgccggaaa tcgtcgtggt attcactcca 7080

gagcgatgaa aacgtttcag tttgctcatg gaaaacggtg taacaagggt gaacactatc 7140

ccatatcacc agctcaccgt ctttcattgc catacgaaat tccggatgag cattcatcag 7200

gcgggcaaga atgtgaataa aggccggata aaacttgtgc ttatttttct ttacggtctt 7260

taaaaaggcc gtaatatcca gctgaacggt ctggttatag gtacattgag caactgactg 7320

aaatgcctca aaatgttctt tacgatgcca ttgggatata tcaacggtgg tatatccagt 7380

gatttttttc tccattttag cttccttagc tcctgaaaat ctcgataact caaaaaatac 7440

gcccggtagt gatcttattt cattatggtg aaagttggaa cctcttacgt gccgatcaga 7500

ttaaaacgaa aggcccagtc tttcgactga gcctttcgtt ttatttgacc atgttggtat 7560

gatttaaatt aatacgactc actatagggt cagtgcggcc gcgacttcaa gtcacgtgaa 7620

ttccaagctt cggatcccac tgtggtgg 7648

<210> 10
<211> 7859
<212> DNA
<213> artificial sequence

<220>
<223> Synthetic sequence

<400> 10
gcggccgctt ctatagtgtc acctaaatac tagtgactcc agcgtaactg gactggccac 60

agttaggccg caaatgtaat cacactggct caccttcggg tgggcctttc tgcgttcgcg 120

agtggacccg ataagctcat ggagcggcgt aaccgtcgca caggaaggac agagaaagcg 180

cggatctggg aagtgacgga cagaacggtc aggacctgga ttggggaggc ggttgccgcc 240

gctgctgctg acggtgtgac gttctctgtt ccggtcacac cacatacgtt ccgccattcc 300

tatgcgatgc acatgctgta tgccggtata ccgctgaaag ttctgcaaag cctgatggga 360

cataagtcca tcagttcaac ggaagtctac acgaaggttt ttgcgctgga tgtggctgcc 420

cggcaccggg tgcagtttgc gatgccggag tctgatgcgg ttgcgatgct gaaacaatta 480

tcctgagaat aaatgccttg gcctttatat ggaaatgtgg aactgagtgg atatgctgtt 540

tttgtctgtt aaacagagaa gctggctgtt atccactgag aagcgaacga aacagtcggg 600

aaaatctccc attatcgtag agatccgcat tattaatctc aggagcctgt gtagcgttta 660

taggaagtag tgttctgtca tgatgcctgc aagcggtaac gaaaacgatt tgaatatgcc 720

ttcaggaaca atagaaatct tcgtgcggtg ttacgttgaa gtggagcgga ttatgtcagc 780

aatggacaga acaacctaat gaacacagaa ccatgatgtg gtctgtcctt ttacagccag 840

tagtgctcgc cgcagtcgag cgacagggcg aagccctcgg ctggttgccc tcgccgctgg 900

gctggcggcc gtctatggcc ctgcaaacgc gccagaaacg ccgtcgaagc cgtgtgcgag 960

acaccgcggc ccgccgccgg cgttgtggat acctcgcgga aaacttggcc ctcactgaca 1020

gatgaggggc ggacgttgac acttgagggg ccgactcacc cggcgcggcg ttgacagatg 1080

aggggcaggc tcgatttcgg ccggcgacgt ggagctggcc agcctcgcaa atcggcgaaa 1140

acgcctgatt ttacgcgagt ttcccacaga tgatgtggac aagcctgggg ataagtgccc 1200

tgcggtattg acacttgagg ggcgcgacta ctgacagatg aggggcgcga tccttgacac 1260

ttgaggggca gagtgctgac agatgagggg cgcacctatt gacatttgag gggctgtcca 1320

caggcagaaa atccagcatt tgcaagggtt tccgcccgtt tttcggccac cgctaacctg 1380

tcttttaacc tgcttttaaa ccaatattta taaaccttgt ttttaaccag ggctgcgccc 1440

tgtgcgcgtg accgcgcacg ccgaaggggg gtgccccccc ttctcgaacc ctcccggtcg 1500

agtgagcgag gaagcaccag ggaacagcac ttatatattc tgcttacaca cgatgcctga 1560

aaaaacttcc cttggggtta tccacttatc cacggggata tttttataat tatttttttt 1620

atagttttta gatcttcttt tttagagcgc cttgtaggcc tttatccatg ctggttctag 1680

agaaggtgtt gtgacaaatt gccctttcag tgtgacaaat caccctcaaa tgacagtcct 1740

gtctgtgaca aattgccctt aaccctgtga caaattgccc tcagaagaag ctgttttttc 1800

acaaagttat ccctgcttat tgactctttt ttatttagtg tgacaatcta aaaacttgtc 1860

acacttcaca tggatctgtc atggcggaaa cagcggttat caatcacaag aaacgtaaaa 1920

atagcccgcg aatcgtccag tcaaacgacc tcactgaggc ggcatatagt ctctcccggg 1980

atcaaaaacg tatgctgtat ctgttcgttg accagatcag aaaatctgat ggcaccctac 2040

aggaacatga cggtatctgc gagatccatg ttgctaaata tgctgaaata ttcggattga 2100

cctctgcgga agccagtaag gatatacggc aggcattgaa gagtttcgcg gggaaggaag 2160

tggtttttta tcgccctgaa gaggatgccg gcgatgaaaa aggctatgaa tcttttcctt 2220

ggtttatcaa acgtgcgcac agtccatcca gagggcttta cagtgtacat atcaacccat 2280

atctcattcc cttctttatc gggttacaga accggtttac gcagtttcgg cttagtgaaa 2340

caaaagaaat caccaatccg tatgccatgc gtttatacga atccctgtgt cagtatcgta 2400

agccggatgg ctcaggcatc gtctctctga aaatcgactg gatcatagag cgttaccagc 2460

tgcctcaaag ttaccagcgt atgcctgact tccgccgccg cttcctgcag gtctgtgtta 2520

atgagatcaa cagcagaact ccaatgcgcc tctcatacat tgagaaaaag aaaggccgcc 2580

agacgactca tatcgtattt tccttccgcg atatcacttc catgacgaca ggatagtctg 2640

agggttatct gtcacagatt tgagggtggt tcgtcacatt tgttctgacc tactgagggt 2700

aatttgtcac agttttgctg tttccttcag cctgcatgga ttttctcata ctttttgaac 2760

tgtaattttt aaggaagcca aatttgaggg cagtttgtca cagttgattt ccttctcttt 2820

cccttcgtca tgtgacctga tatcgggggt tagttcgtca tcattgatga gggttgatta 2880

tcacagttta ttactctgaa ttggctatcc gcgtgtgtac ctctacctgg agtttttccc 2940

acggtggata tttcttcttg cgctgagcgt aagagctatc tgacagaaca gttcttcttt 3000

gcttcctcgc cagttcgctc gctatgctcg gttacacggc tgcggcgagc gctagtgata 3060

ataagtgact gaggtatgtg ctcttcttat ctccttttgt agtgttgctc ttattttaaa 3120

caactttgcg gttttttgat gactttgcga ttttgttgtt gctttgcagt aaattgcaag 3180

atttaataaa aaaacgcaaa gcaatgatta aaggatgttc agaatgaaac tcatggaaac 3240

acttaaccag tgcataaacg ctggtcatga aatgacgaag gctatcgcca ttgcacagtt 3300

taatgatgac agcccggaag cgaggaaaat aacccggcgc tggagaatag gtgaagcagc 3360

ggatttagtt ggggtttctt ctcaggctat cagagatgcc gagaaagcag ggcgactacc 3420

gcacccggat atggaaattc gaggacgggt tgagcaacgt gttggttata caattgaaca 3480

aattaatcat atgcgtgatg tgtttggtac gcgattgcga cgtgctgaag acgtatttcc 3540

accggtgatc ggggttgctg cccataaagg aggcgtttac aaaacctcag tttctgttca 3600

tcttgctcag gatctggctc tgaaggggct acgtgttttg ctcgtggaag gtaacgaccc 3660

ccagggaaca gcctcaatgt atcacggatg ggtaccagat cttcatattc atgcagaaga 3720

cactctcctg cctttctatc ttggggaaaa ggacgatgtc acttatgcaa taaagcccac 3780

ttgctggccg gggcttgaca ttattccttc ctgtctggct ctgcaccgta ttgaaactga 3840

gttaatgggc aaatttgatg aaggtaaact gcccaccgat ccacacctga tgctccgact 3900

ggccattgaa actgttgctc atgactatga tgtcatagtt attgacagtg cgcctaacct 3960

gggtatcggc acgattaatg tcgtatgtgc tgctgatgtg ctgattgttc ccacgcctgc 4020

tgagttgttt gactacacct ccgcactgca gtttttcgat atgcttcgtg atctgctcaa 4080

gaacgttgat cttaaagggt tcgagcctga tgtacgtatt ttgcttacca aatacagcaa 4140

tagtaatggc tctcagtccc cgtggatgga ggagcaaatt cgggatgcct ggggaagcat 4200

ggttctaaaa aatgttgtac gtgaaacgga tgaagttggt aaaggtcaga tccggatgag 4260

aactgttttt gaacaggcca ttgatcaacg ctcttcaact ggtgcctgga gaaatgctct 4320

ttctatttgg gaacctgtct gcaatgaaat tttcgatcgt ctgattaaac cacgctggga 4380

gattagataa tgaagcgtgc gcctgttatt ccaaaacata cgctcaatac tcaaccggtt 4440

gaagatactt cgttatcgac accagctgcc ccgatggtgg attcgttaat tgctcgcgta 4500

ggagtaatgg ctcgcggtaa tgccattact ttgcctgtat gtggtcggga tgtgaagttt 4560

actcttgaag tgctccgggg tgatagtgtt gagaagacct ctcgggtatg gtcaggtaat 4620

gaacgtgacc aggagctgct tactgaggac gcactggatg atctcatccc ttcttttcta 4680

ctgactggtc aacagacacc ggcgttcggt cgaagagtat ctggtgtcat agaaattgcc 4740

gatgggagtc gccgtcgtaa agctgctgca cttaccgaaa gtgattatcg tgttctggtt 4800

ggcgagctgg atgatgagca gatggctgca ttatccagat tgggtaacga ttatcgccca 4860

acaagtgctt atgaacgtgg tcagcgttat gcaagccgat tgcagaatga atttgctgga 4920

aatatttctg cgctggctga tgcggaaaat atttcacgta agattattac ccgctgtatc 4980

aacaccgcca aattgcctaa atcagttgtt gctctttttt ctcaccccgg tgaactatct 5040

gcccggtcag gtgatgcact tcaaaaagcc tttacagata aagaggaatt acttaagcag 5100

caggcatcta accttcatga gcagaaaaaa gctggggtga tatttgaagc tgaagaagtt 5160

atcactcttt taacttctgt gcttaaaacg tcatctgcat caagaactag tttaagctca 5220

cgacatcagt ttgctcctgg agcgacagta ttgtataagg gcgataaaat ggtgcttaac 5280

ctggacaggt ctcgtgttcc aactgagtgt atagagaaaa ttgaggccat tcttaaggaa 5340

cttgaaaagc cagcaccctg atgcgaccac gttttagtct acgtttatct gtctttactt 5400

aatgtccttt gttacaggcc agaaagcata actggcctga atattctctc tgggcccact 5460

gttccacttg tatcgtcggt ctgataatca gactgggacc acggtcccac tcgtatcgtc 5520

ggtctgatta ttagtctggg accacggtcc cactcgtatc gtcggtctga ttattagtct 5580

gggaccacgg tcccactcgt atcgtcggtc tgataatcag actgggacca cggtcccact 5640

cgtatcgtcg gtctgattat tagtctggga ccatggtccc actcgtatcg tcggtctgat 5700

tattagtctg ggaccacggt cccactcgta tcgtcggtct gattattagt ctggaaccac 5760

ggtcccactc gtatcgtcgg tctgattatt agtctgggac cacggtccca ctcgtatcgt 5820

cggtctgatt attagtctgg gaccacgatc ccactcgtgt tgtcggtctg attatcggtc 5880

tgggaccacg gtcccacttg tattgtcgat cagactatca gcgtgagact acgattccat 5940

caatgcctgt caagggcaag tattgacatg gtcgtcgtaa cctgtagaac ggagtaacct 6000

cggtgtgcgg ttgtatgcct gctgtggatt gctgctgtgt cctgcttatc cacaacattt 6060

tgcgcacggt tatgtggaca aaatacctgg ttacccaggc cgtgccggca cgttaaccgg 6120

gctgcatccg atgcaagtgt gtcgctgacg acgagctcgc gagctcggac atgaggttgc 6180

cccgtattca gtgtcgctga tttgtattgt ctgaagttgc ttttacgtta agttgatgca 6240

gatcaattaa tacgatacct gcgtcataat tgattatttg acgtggtttg atggcctcca 6300

cgcacgttgt gatatgtaga tgataatcat tatcacttta cgggtccttt ccggtgatcc 6360

gacaggttac ggggcggcga cctcgcgggt tttcgctatt tatgaaaatt ttccggttta 6420

aggcgtttcc gttcttcttc gtcataactt aatgttttta tttaaaatac cctctgaaaa 6480

gaaaggaaac gacaggtgct gaaagcgagc tttttggcct ctgtcgtttc ctttctctgt 6540

ttttgtccgt ggaatgaaca atggaagtcc gagctcatcg ctaataactt cgtatagcat 6600

acattatacg aagttatatt cgatgcggcc gatctagcag aaagtcaaaa gcctccgacc 6660

ggaggctttt gacttctgtc acctaggtta cgccccgccc tgccactcat cgcagtactg 6720

ttgtaattca ttaagcattc tgccgacatg gaagccatca caaacggcat gatgaacctg 6780

aatcgccagc ggcatcagca ccttgtcgcc ttgcgtataa tatttgccca tggtgaaaac 6840

gggggcgaag aagttgtcca tattggccac gtttaaatca aaactggtga aactcaccca 6900

gggattggct gagacgaaaa acatattctc aataaaccct ttagggaaat aggccaggtt 6960

ttcaccgtaa cacgccacat cttgcgaata tatgtgtaga aactgccgga aatcgtcgtg 7020

gtattcactc cagagcgatg aaaacgtttc agtttgctca tggaaaacgg tgtaacaagg 7080

gtgaacacta tcccatatca ccagctcacc gtctttcatt gccatacgaa attccggatg 7140

agcattcatc aggcgggcaa gaatgtgaat aaaggccgga taaaacttgt gcttattttt 7200

ctttacggtc tttaaaaagg ccgtaatatc cagctgaacg gtctggttat aggtacattg 7260

agcaactgac tgaaatgcct caaaatgttc tttacgatgc cattgggata tatcaacggt 7320

ggtatatcca gtgatttttt tctccatttt agcttcctta gctcctgaaa atctcgataa 7380

ctcaaaaaat acgcccggta gtgatcttat ttcattatgg tgaaagttgg aacctcttac 7440

gtgccgatca gattaaaacg aaaggcccag tctttcgact gagcctttcg ttttatttga 7500

ccatgttggt atgatttaaa ttcgtttagt gaaccgtcag atcactagaa gctttattgc 7560

ggtagtttat cacagttaaa ttgctaacgc agtcagtgct tctgacacaa cagtctcgaa 7620

cttaagctgc agtgactctc ttaaggtagc cttgcagaag ttggtcgtga ggcactgggc 7680

aggtaagtat caaggttaca agacaggttt aaggagacca atagaaactg ggcttgtcga 7740

gacagagaag actcttgcgt ttctgatagg cacctattgg tcttactgac atccactttg 7800

cctttctctc cacaggtgtc cactcccagt tcaattacag ctcttaaggc tagagtact 7859

<210> 11
<211> 144
<212> DNA
<213> artificial sequence

<220>
<223> Synthetic sequence


<220>
<221> misc_feature
<222> (1)..(6)
<223> n is a, c, g, or t

<220>
<221> misc_feature
<222> (137)..(144)
<223> n is a, c, g, or t

<400> 11
nnnnnnctac agctcaactt acctgccaac cccatgccag acgacccaac tagtacaacc 60

taaatccatt ataaaaaact taggaaccag gtccacacag ccgccagccc atcaaccatc 120

cactcccacg attggannnn nnnn                                          144

<210>  12
<211>  31676
<212>  DNA
<213>  artificial sequence

<220>
<223>  Synthetic sequence

<400>  12
gcggccgcta atacgactca ctatagggcc aactttgttt ggtctgatga gtccgtgagg      60

acgaaacccg gagtcccggg tcaccaaaca aagttgggta aggatagttc aatcaatgat     120

catcttctag tgcacttagg attcaagatc ctattatcag ggacaagagc aggattaggg     180

atatccgaga tggccacact tttaaggagc ttagcattgt tcaaaagaaa caaggacaaa     240

ccacccatta catcaggatc cggtggagcc atcagaggaa tcaaacacat tattatagta     300

ccaatccctg gagattcctc aattaccact cgatccagac ttctggaccg gttggtgagg     360

ttaattggaa acccggatgt gagcgggccc aaactaacag gggcactaat aggtatatta     420

tccttatttg tggagtctcc aggtcaattg attcagagga tcaccgatga ccctgacgtt     480

agcataaggc tgttagaggt tgtccagagt gaccagtcac aatctggcct taccttcgca     540

tcaagaggta ccaacatgga ggatgaggcg gaccaatact tttcacatga tgatccaatt     600

agtagtgatc aatccaggtt cggatggttc gggaacaagg aaatctcaga tattgaagtg     660

caagaccctg agggattcaa catgattctg ggtaccatcc tagcccaaat ttgggtcttg     720

ctcgcaaagg cggttacggc cccagacacg gcagctgatt cggagctaag aaggtggata     780

aagtacaccc aacaaagaag ggtagttggt gaatttagat tggagagaaa atggttggat     840

gtggtgagga acaggattgc cgaggacctc tccttacgcc gattcatggt cgctctaatc     900

ctggatatca agagaacacc cggaaacaaa cccaggattg ctgaaatgat atgtgacatt     960

gatacatata tcgtagaggc aggattagcc agttttatcc tgactattaa gtttgggata    1020

gaaactatgt atcctgctct tggactgcat gaatttgctg gtgagttatc cacacttgag    1080

tccttgatga acctttacca gcaaatgggg gaaactgcac cctacatggt aatcctggag    1140

aactcaattc agaacaagtt cagtgcagga tcataccctc tgctctggag ctatgccatg    1200

ggagtaggag tggaacttga aaactccatg ggaggtttga actttggccg atcttacttt    1260

gatccagcat attttagatt agggcaagag atggtaagga ggtcagctgg aaaggtcagt    1320

tccacattgg catctgaact cggtatcact gccgaggatg caaggcttgt ttcagagatt    1380

gcaatgcata ctactgagga caagatcagt agagcggttg gacccagaca agcccaagta    1440

tcatttctac acggtgatca aagtgagaat gagctaccga gattgggggg caaggaagat    1500

aggagggtca aacagagtcg aggagaagcc agggagagct acagagaaac cgggcccagc    1560

agagcaagtg atgcgagagc tgcccatctt ccaaccggca cacccctaga cattgacact 1620

gcaacggagt ccagccaaga tccgcaggac agtcgaaggt cagctgacgc cctgcttagg 1680

ctgcaagcca tggcaggaat ctcggaagaa caaggctcag acacggacac ccctatagtg 1740

tacaatgaca gaaatcttct agactaggtg cgagaggccg agggccagaa caacatccgc 1800

ctaccatcca tcattgttat aaaaaactta ggaaccaggt ccacacagcc gccagcccat 1860

caaccatcca ctcccacgat tggagccaat ggcagaagag caggcacgcc atgtcaaaaa 1920

cggactggaa tgcatccggg ctctcaaggc cgagcccatc ggctcactgg ccatcgagga 1980

agctatggca gcatggtcag aaatatcaga caacccagga caggagcgag ccacctgcag 2040

ggaagagaag gcaggcagtt cgggtctcag caaaccatgc ctctcagcaa ttggatcaac 2100

tgaaggcggt gcacctcgca tccgcggtca gggacctgga gagagcgatg acgacgctga 2160

aactttggga atccccccaa gaaatctcca ggcatcaagc actgggttac agtgttatta 2220

cgtttatgat cacagcggtg aagcggttaa gggaatccaa gatgctgact ctatcatggt 2280

tcaatcaggc cttgatggtg atagcaccct ctcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cccccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tgtgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agctaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaactagc ctaccctcca tcattgttat 3480

aaaaaactta ggaaccaggt ccacacagcc gccagcccat caacgcgtac gatggagttc 3540

atcccaaccc aaacttttta caataggagg taccagcctc gaccctggac tccgcgccct 3600

actatccaag tcatcaggcc cagaccgcgc cctcagaggc aagctgggca acttgcccag 3660

ctgatctcag cagttaataa actgacaatg agagcggtac cccaacagaa gccacgcagg 3720

aatcggaaga ataagaagca aaagcaaaaa caacaggcgc cacaaaacaa cacaaatcaa 3780

aagaagcagc cacctaaaaa gaaaccggct caaaagaaaa agaagccggg ccgcagagag 3840

aggatgtgca tgaaaatcga aaatgattgt attttcgaag tcaagcacga aggtaaggta 3900

acaggttacg cgtgcctggt gggggacaaa gtaatgaaac cagcacacgt caagggcacc 3960

atcgataacg cggacctggc caaactggcc tttaagcggt catctaagta tgaccttgaa 4020

tgcgctcaga tacccgtgca catgaagtcc gacgcttcga agttcaccca tgagaaaccg 4080

gaggggtact acaactggca ccacggagca gtacagtact caggaggccg gttcaccatc 4140

cctacaggtg ctggcaaacc aggggacagc ggcagaccga tcttcgacaa caagggacgc 4200

gtggtggcca tagtcttagg aggagctaat gaaggagccc gtacagccct ctcggtggtg 4260

acctggaata aagacattgt cactaaaatc acccccgagg gggccgaaga gtggagtctt 4320

gccatcccag ttatgtgcct gttggcaaac accacgttcc catgctccca gccaccttgc 4380

acgccctgct gctacgagaa ggaaccggag gaaaccctac gcatgcttga ggacaacgtc 4440

atgagacctg ggtactatca gctgctacaa gcatccttaa catgttctcc ccaccgccag 4500

cgacgcagca ccaaggacaa cttcaatgtc tataaagcca caagaccata cttagctcac 4560

tgtcccgact gtggagaagg gcactcgtgc catagtcccg tagcactaga acgcatcaga 4620

aatgaagcga cagacgggac gctgaaaatc caggtctcct tgcaaatcgg aataaagacg 4680

gatgacagcc acgattggac caagctgcgt tatatggaca accacatgcc agcagacgca 4740

gagagggcgg ggctatttgt aagaacatca gcaccgtgta cgattactgg aacaatggga 4800

cacttcatcc tggcccgatg tcctaaaggc gaaactctga cggtgggatt cactgacagt 4860

aggaagatta gtcactcatg tacgcaccca tttcaccacg accctcctgt gataggtcgg 4920

gagaaattcc attcccgacc gcagcacggt aaagagctac cttgcagcac ctacgtgcag 4980

agcaccgccg caactaccga ggagatagag gtacacatgc ccccagacac ccctgatcgc 5040

acattaatgt cacaacagtc cggcaacgta aagatcacag tcaatggcca gacggtgcgg 5100

tacaagtgta attgcggtgg ctcaaatgaa ggactaacaa ctacagacaa agtgattaat 5160

aactgcaagg ttgatcaatg tcatgccgcg gtcaccaatc acaaaaagtg gcagtataac 5220

tcccctctgg tcccgcgtaa tgctgaactt ggggaccgga aaggaaaaat tcacatcccg 5280

tttccgctgg caaatgtaac atgcagggtg cctaaagcaa ggaaccccac cgtgacgtat 5340

ggcaaaaacc aagtcatcat gctactgtat cctgaccacc caacactcct gtcctaccgg 5400

aatatgggag aagaaccaaa ctatcaagaa gagtgggtga tgcataagaa ggaagtcgtg 5460

ctaaccgtgc cgactgaagg gctcgaggtc acgtggggca acaacgagcc gtataagtat 5520

tggccgcagt tatctacaaa cggtacagcc catggccacc cgcacgagat aattctgtat 5580

tattatgagc tgtaccccac tatgactgta gtagttgtgt cagtggccac gttcatactc 5640

ctgtcgatgg tgggtatggc agcggggatg tgcatgtgtg cacgacgcag atgcatcaca 5700

ccgtatgaac tgacaccagg agctaccgtc ccattcctgc ttagcctaat atgctgcatc 5760

agaacagcta aagcggccac ataccaagag gctgcgatat acctgtggaa cgagcagcaa 5820

cctttgtttt ggctacaagc ccttattccg ctggcagccc tgattgttct atgcaactgt 5880

ctgagactct taccatgctg ctgtaaaacg ttggcttttt tagccgtaat gagcgtcggt 5940

gcccacactg tgagcgctta cgaacacgta acagtgatcc cgaacacggt gggagtaccg 6000

tataagactc tagtcaatag acctggctac agccccatgg tattggagat ggaactactg 6060

tcagtcactt tggagccaac actatcgctt gattacatca cgtgcgagta caaaaccgtc 6120

atcccgtctc cttacgtgaa gtgctgcggt acagcagagt gcaaggacaa aaacctacct 6180

gactacagct gtaaggtctt caccggcgtc tacccattta tgtggggcgg cgcctactgc 6240

ttctgcgacg ctgaaaacac gcagttgagc gaagcacacg tggagaagtc cgaatcatgc 6300

aaaacagaat ttgcatcagc atacagggct cataccgcat ctgcatcagc taagctccgc 6360

gtcctttacc aaggaaataa catcactgta actgcctatg caaacggcga ccatgccgtc 6420

acagttaagg acgccaaatt cattgtgggg ccaatgtctt cagcctggac acctttcgac 6480

aacaaaattg tggtgtacaa aggtgacgtc tataacatgg actacccgcc cttcggcgca 6540

ggaagaccag gacaatttgg cgatatccaa agtcgcacac ctgagagtaa agacgtctat 6600

gctaatacac aactggtact gcagagaccg gctgtgggta cggtacacgt gccatactct 6660

caggcaccat ctggctttaa gtattggcta aaagaacgcg gggcgtcgct gcagcacaca 6720

gcaccatttg gctgccaaat agcaacaaac ccggtaagag cggtgaactg cgccgtaggg 6780

aacatgccca tctccatcga cataccggaa gcggccttca ctagggtcgt cgacgcgccc 6840

tctttaacgg acatgtcgtg cgaggtacca gcctgcaccc attcctcaga ctttgggggc 6900

gtcgccatta ttaaatatgc agccagcaag aaaggcaagt gtgcggtgca ttcgatgact 6960

aacgccgtca ctattcggga agctgagata gaagttgaag ggaattctca gctgcaaatc 7020

tctttctcga cggccttagc cagcgccgaa ttccgcgtac aagtctgttc tacacaagta 7080

cactgtgcag ccgagtgcca ccccccgaag gaccacatag tcaactaccc ggcgtcacat 7140

accaccctcg gggtccagga catctccgct acggcgatgt catgggtgca gaagatcacg 7200

ggaggtgtgg gactggttgt tgctgttgcc gcactgattc taatcgtggt gctatgcgtg 7260

tcgttcagca ggcactaata gctacagctc aacttacctg ccaaccccat gccagacgac 7320

ccaactagta caacctaaat ccattataaa aaacttagga accaggtcca cacagccgcc 7380

agcccatcaa ccatccactc ccacgattgg aggccggcca tgagagttgt gtttgtcgtg 7440

ctattgcttt tggtggcccc agcttacagc ttcaactgcc ttggaatgag caacagagac 7500

ttcttggaag gagtgtctgg agcaacatgg gtggatttgg ttctcgaagg cgacagctgc 7560

gtgactatca tgtctaagga caagcctacc atcgatgtga agatgatgaa tatggaggcg 7620

gtcaacctgg cagaggtccg cagttattgc tatttggcta ccgtcagcga tctctccacc 7680

aaagctgcgt gcccgaccat gggagaagct cacaatgaca aacgtgctga cccagctttt 7740

gtgtgcagac aaggagtggt ggacaggggc tggggcaacg gctgcggatt atttggcaaa 7800

ggaagcattg acacatgcgc caaatttgcc tgctctacca aggcaatagg aagaaccatc 7860

ttgaaagaga atatcaagta cgaagtggcc atttttgtcc atggaccaac tactgtggag 7920

tcgcacggaa actactccac acaggttgga gccactcagg cagggagatt cagcatcact 7980

cctgcggcgc cttcatacac actaaagctt ggagaatatg gagaggtgac agtggactgt 8040

gaaccacggt cagggattga caccaatgca tactacgtga tgactgttgg aacaaagacg 8100

ttcttggtcc atcgtgagtg gttcatggac ctcaacctcc cttggagcag tgctggaagt 8160

actgtgtgga ggaacagaga gacgttaatg gagtttgagg aaccacacgc cacgaagcag 8220

tctgtgatag cattgggctc acaagaggga gctctgcatc aagctttggc tggagccatt 8280

cctgtggaat tttcaagcaa cactgtcaag ttgacgtcgg gtcatttgaa gtgtagagtg 8340

aagatggaaa aattgcagtt gaagggaaca acctatggcg tctgttcaaa ggctttcaag 8400

tttcttggga ctcccgcaga cacaggtcac ggcactgtgg tgttggaatt gcagtacact 8460

ggcacggatg gaccttgcaa agttcctatc tcgtcagtgg cttcattgaa cgacctaacg 8520

ccagtgggca gattggtcac tgtcaaccct tttgtttcag tggccacggc caacgctaag 8580

gtcctgattg aattggaacc accctttgga gactcataca tagtggtggg cagaggagaa 8640

caacagatca atcaccattg gcacaagtct ggaagcagca ttggcaaagc ctttacaacc 8700

accctcaaag gagcgcagag actagccgct ctaggagaca cagcttggga ctttggatca 8760

gttggagggg tgttcacctc agttgggaag gctgtctaat tcgaactaca gctcaactta 8820

cctgccaacc ccatgccaga cgacccaact agtacaacct aaatccatta taaaaaactt 8880

aggaaccagg tccacacagc cgccagccca tcaaccatcc actcccacga ttggagcggc 8940

cgcatggaga agaagcggag aggagcagac acaagcgtgg gaatcgtggg cctgctgctg 9000

accacagcaa tggcagcaga ggtgaccagg agaggaagcg cctactatat gtacctggac 9060

aggaatgatg ccggcgaggc catctccttc ccaaccacac tgggcatgaa caagtgctac 9120

atccagatca tggacctggg ccacatgtgc gatgccacca tgtcctatga gtgtccaatg 9180

ctggacgagg gcgtggagcc cgacgatgtg gattgctggt gtaataccac atctacatgg 9240

gtggtgtacg gcacctgtca ccacaagaag ggagaggccc ggcggagccg gcgggccgtg 9300

acactgcctt cccactctac caggaagctg cagacacgca gccagacctg gctggagtcc 9360

agagagtata ccaagcacct gatcagggtg gagaactgga tctttcgcaa tccaggattc 9420

gcactggcag cagcagcaat cgcatggctg ctgggaagct ccaccagcca gaaagtgatc 9480

tacctggtca tgatcctgct gatcgctcct gcctattcta tccggtgcat cggcgtgagc 9540

aatagagact tcgtggaggg aatgtccgga ggaacctggg tggatgtggt gctggagcac 9600

ggcggctgcg tgacagtgat ggcccaggac aagccaaccg tggatatcga gctggtgacc 9660

acaaccgtgt ccaacatggc cgaggtgagg tcttactgct atgaggccag catctccgac 9720

atggcctctg atagcaggtg tccaacccag ggagaggcat acctggacaa gcagtccgat 9780

acacagtacg tgtgcaagcg gaccctggtg gacagaggct ggggcaatgg ctgtggcctg 9840

tttggcaagg gctctctggt gacatgcgcc aagttcgcct gtagcaagaa gatgaccggc 9900

aagtccatcc agccagagaa cctggagtac cggatcatgc tgtctgtgca cggctcccag 9960

cactctggca tgatcgtgaa cgacacaggc cacgagacag atgagaatcg ggccaaggtg 10020

gagatcacac ctaactctcc aagagccgag gccaccctgg gaggatttgg ctctctgggc 10080

ctggactgcg agcctagaac aggcctggac ttctccgatc tgtactatct gaccatgaac 10140

aataagcact ggctggtgca caaggagtgg tttcacgaca tcccactgcc atggcacgca 10200

ggagcagata caggaacacc acactggaac aataaggagg ccctggtgga gttcaaggat 10260

gcccacgcca agcggcagac agtggtggtg ctgggcagcc aggagggagc agtgcacacc 10320

gccctggcag gcgccctgga ggcagagatg gacggagcta agggcagact gtctagcggc 10380

cacctgaagt gcaggctgaa gatggataag ctgcgcctga agggcgtgtc ctactctctg 10440

tgcacagccg ccttcacctt caccaagatc cctgccgaga cactgcacgg cacagtgacc 10500

gtggaggtgc agtatgccgg cacagacgga ccctgtaagg tgcctgccca gatggccgtg 10560

gatatgcaga cactgacacc tgtgggcagg ctgatcaccg ccaatccagt gatcacagag 10620

tctaccgaga acagcaagat gatgctggag ctggacccac catttggcga tagctatatc 10680

gtgatcggcg tgggcgagaa gaagatcaca caccactggc accgcagcgg ctccacaatc 10740

ggcaaggcct ttgaggcaac cgtgcgcgga gcaaagagaa tggccgtgct gggcgacacc 10800

gcatgggatt tcggatctgt gggaggcgcc ctgaacagcc tgggcaaggg catccaccag 10860

atcttcggcg ccgcctttaa gtccctgttc ggcggcatga gctggttctc acagatcctg 10920

atcggcacac tgctgatgtg gctgggcctg aacaccaaga atggctctat cagcctgatg 10980

tgcctggccc tgggaggcgt gctgatcttc ctgtccaccg ccgtgtctgc ctgatgaagc 11040

gcgcagcgct tagacgtctc gcgatcgata ctagtacaac ctaaatccat tataaaaaac 11100

ttaggagcaa agtgattgcc tcccaaggtc cacaatgaca gagacctacg acttcgacaa 11160

gtcggcatgg gacatcaaag ggtcgatcgc tccgatacaa cccaccacct acagtgatgg 11220

caggctggtg ccccaggtca gagtcataga tcctggtcta ggcgacagga aggatgaatg 11280

ctttatgtac atgtttctgc tgggggttgt tgaggacagc gattccctag ggcctccaat 11340

cgggcgagca tttgggttcc tgcccttagg tgttggcaga tccacagcaa agcccgaaaa 11400

actcctcaaa gaggccactg agcttgacat agttgttaga cgtacagcag ggctcaatga 11460

aaaactggtg ttctacaaca acaccccact aactctcctc acaccttgga gaaaggtcct 11520

aacaacaggg agtgtcttca acgcaaacca agtgtgcaat gcggttaatc tgataccgct 11580

cgataccccg cagaggttcc gtgttgttta tatgagcatc acccgtcttt cggataacgg 11640

gtattacacc gttcctagaa gaatgctgga attcagatcg gtcaatgcag tggccttcaa 11700

cctgctggtg acccttagga ttgacaaggc gataggccct gggaagatca tcgacaatac 11760

agagcaactt cctgaggcaa catttatggt ccacatcggg aacttcagga gaaagaagag 11820

tgaagtctac tctgccgatt attgcaaaat gaaaatcgaa aagatgggcc tggtttttgc 11880

acttggtggg atagggggca ccagtcttca cattagaagc acaggcaaaa tgagcaagac 11940

tctccatgca caactcgggt tcaagaagac cttatgttac ccgctgatgg atatcaatga 12000

agaccttaat cgattactct ggaggagcag atgcaagata gtaagaatcc aggcagtttt 12060

gcagccatca gttcctcaag aattccgcat ttacgacgac gtgatcataa atgatgacca 12120

aggactattc aaagttctgt agaccgtagt gcccagcaat gcccgaaaac gacccccctc 12180

acaatgacag ccagaaggcc cggacaaaaa agccccctcc gaaagactcc acggaccaag 12240

cgagaggcca gccagcagcc gacggcaagc gcgaacacca ggcggcccca gcacagaaca 12300

gccctgacac aaggccacca ccagccaccc caatctgcat cctcctcgtg ggacccccga 12360

ggaccaaccc ccaaggctgc ccccgatcca aaccaccaac cgcatcccca ccacccccgg 12420

gaaagaaacc cccagcaatt ggaaggcccc tccccctctt cctcaacaca agaactccac 12480

aaccgaaccg cacaagcgac cgaggtgacc caaccgcagg catccgactc cctagacaga 12540

tcctctctcc ccggcaaact aaacaaaact tagggccaag gaacatacac acccaacaga 12600

acccagaccc cggcccacgg cgccgcgccc ccaacccccg acaaccagag ggagccccca 12660

accaatcccg ccggctcccc cggtgcccac aggcagggac accaaccccc gaacagaccc 12720

agcacccaac catcgacaat ccaagacggg ggggcccccc caaaaaaagg cccccagggg 12780

ccgacagcca gcaccgcgag gaagcccacc caccccacac acgaccacgg caaccaaacc 12840

agaacccaga ccaccctggg ccaccagctc ccagactcgg ccatcacccc gcagaaagga 12900

aaggccacaa cccgcgcacc ccagccccga tccggcgggg agccacccaa cccgaaccag 12960

cacccaagag cgatccccga aggacccccg aaccgcaaag gacatcagta tcccacagcc 13020

tctccaagtc ccccggtctc ctcctcttct cgaagggacc aaaagatcaa tccaccacac 13080

ccgacgacac tcaactcccc acccctaaag gagacaccgg gaatcccaga atcaagactc 13140

atccaatgtc catcatgggt ctcaaggtga acgtctctgc catattcatg gcagtactgt 13200

taactctcca aacacccacc ggtcaaatcc attggggcaa tctctctaag ataggggtgg 13260

taggaatagg aagtgcaagc tacaaagtta tgactcgttc cagccatcaa tcattagtca 13320

taaaattaat gcccaatata actctcctca ataactgcac gagggtagag attgcagaat 13380

acaggagact actgagaaca gttttggaac caattagaga tgcacttaat gcaatgaccc 13440

agaatataag accggttcag agtgtagctt caagtaggag acacaagaga tttgcgggag 13500

tagtcctggc aggtgcggcc ctaggcgttg ccacagctgc tcagataaca gccggcattg 13560

cacttcacca gtccatgctg aactctcaag ccatcgacaa tctgagagcg agcctggaaa 13620

ctactaatca ggcaattgag acaatcagac aagcagggca ggagatgata ttggctgttc 13680

agggtgtcca agactacatc aataatgagc tgataccgtc tatgaaccaa ctatcttgtg 13740

atttaatcgg ccagaagctc gggctcaaat tgctcagata ctatacagaa atcctgtcat 13800

tatttggccc cagtttacgg gaccccatat ctgcggagat atctatccag gctttgagct 13860

atgcgcttgg aggagacatc aataaggtgt tagaaaagct cggatacagt ggaggtgatt 13920

tactgggcat cttagagagc ggaggaataa aggcccggat aactcacgtc gacacagagt 13980

cctacttcat tgtcctcagt atagcctatc cgacgctgtc cgagattaag ggggtgattg 14040

tccaccggct agagggggtc tcgtacaaca taggctctca agagtggtat accactgtgc 14100

ccaagtatgt tgcaacccaa gggtacctta tctcgaattt tgatgagtca tcgtgtactt 14160

tcatgccaga ggggactgtg tgcagccaaa atgccttgta cccgatgagt cctctgctcc 14220

aagaatgcct ccgggggtac accaagtcct gtgctcgtac actcgtatcc gggtcttttg 14280

ggaaccggtt cattttatca caagggaacc taatagccaa ttgtgcatca atcctttgca 14340

agtgttacac aacaggaacg atcattaatc aagaccctga caagatccta acatacattg 14400

ctgccgatca ctgcccggta gtcgaggtga acggcgtgac catccaagtc gggagcagga 14460

ggtatccaga cgctgtgtac ttgcacagaa ttgacctcgg tcctcccata tcattggaga 14520

ggttggacgt agggacaaat ctggggaatg caattgctaa gttggaggat gccaaggaat 14580

tgttggagtc atcggaccag atattgagga gtatgaaagg tttatcgagc actagcatag 14640

tctacatcct gattgcagtg tgtcttggag ggttgatagg gatccccgct ttaatatgtt 14700

gctgcagggg gcgttgtaac aaaaagggag aacaagttgg tatgtcaaga ccaggcctaa 14760

agcctgatct tacgggaaca tcaaaatcct atgtaaggtc gctctgatcc tctacaactc 14820

ttgaaacaca aatgtcccac aagtctcctc ttcgtcatca agcaaccacc gcacccagca 14880

tcaagcccac ctgaaattat ctccggcttc cctctggccg aacaatatcg gtagttaatc 14940

aaaacttagg gtgcaagatc atccacaatg tcaccacaac gagaccggat aaatgccttc 15000

tacaaagata acccccatcc caagggaagt aggatagtca ttaacagaga acatcttatg 15060

attgatagac cttatgtttt gctggctgtt ctgtttgtca tgtttctgag cttgatcggg 15120

ttgctagcca ttgcaggcat tagacttcat cgggcagcca tctacaccgc agagatccat 15180

aaaagcctca gcaccaatct agatgtaact aactcaatcg agcatcaggt caaggacgtg 15240

ctgacaccac tcttcaaaat catcggtgat gaagtgggcc tgaggacacc tcagagattc 15300

actgacctag tgaaattaat ctctgacaag attaaattcc ttaatccgga tagggagtac 15360

gacttcagag atctcacttg gtgtatcaac ccgccagaga gaatcaaatt ggattatgat 15420

caatactgtg cagatgtggc tgctgaagag ctcatgaatg cattggtgaa ctcaactcta 15480

ctggagacca gaacaaccaa tcagttccta gctgtctcaa agggaaactg ctcagggccc 15540

actacaatca gaggtcaatt ctcaaacatg tcgctgtccc tgttagactt gtatttaggt 15600

cgaggttaca atgtgtcatc tatagtcact atgacatccc agggaatgta tgggggaact 15660

tacctagtgg aaaagcctaa tctgagcagc aaaaggtcag agttgtcaca actgagcatg 15720

taccgagtgt ttgaagtagg tgttatcaga aatccgggtt tgggggctcc ggtgttccat 15780

atgacaaact atcttgagca accagtcagt aatgatctca gcaactgtat ggtggctttg 15840

ggggagctca aactcgcagc cctttgtcac ggggaagatt ctatcacaat tccctatcag 15900

ggatcaggga aaggtgtcag cttccagctc gtcaagctag gtgtctggaa atccccaacc 15960

gacatgcaat cctgggtccc cttatcaacg gatgatccag tgatagacag gctttacctc 16020

tcatctcaca gaggtgttat cgctgacaat caagcaaaat gggctgtccc gacaacacga 16080

acagatgaca agttgcgaat ggagacatgc ttccaacagg cgtgtaaggg taaaatccaa 16140

gcactctgcg agaatcccga gtgggcacca ttgaaggata acaggattcc ttcatacggg 16200

gtcttgtctg ttgatctgag tctgacagtt gagcttaaaa tcaaaattgc ttcgggattc 16260

gggccattga tcacacacgg ttcagggatg gacctataca aatccaacca caacaatgtg 16320

tattggctga ctatcccgcc aatgaagaac ctagccttag gtgtaatcaa cacattggag 16380

tggataccga gattcaaggt tagtccctac ctcttcactg tcccaattaa ggaagcaggc 16440

gaagactgcc atgccccaac atacctacct gcggaggtgg atggtgatgt caaactcagt 16500

tccaatctgg tgattctacc tggtcaagat ctccaatatg ttttggcaac ctacgatact 16560

tccagggttg aacatgctgt ggtttattac gtttacagcc caagccgctc attttcttac 16620

ttttatcctt ttaggttgcc tataaagggg gtccccatcg aattacaagt ggaatgcttc 16680

acatgggacc aaaaactctg gtgccgtcac ttctgtgtgc ttgcggactc agaatctggt 16740

ggacatatca ctcactctgg gatggtgggc atgggagtca gctgcacagt cacccgggaa 16800

gatggaacca atcgcagata gggctgctag tgaaccaatc acatgatgtc acccagacat 16860

caggcatacc cactagtgtg aaatagacat cagaattaag aaaaacgtag ggtccaagtg 16920

gttccccgtt atggactcgc tatctgtcaa ccagatctta taccctgaag ttcacctaga 16980

tagcccgata gttaccaata agatagtagc catcctggag tatgctcgag tccctcacgc 17040

ttacagcctg gaggacccta cactgtgtca gaacatcaag caccgcctaa aaaacggatt 17100

ttccaaccaa atgattataa acaatgtgga agttgggaat gtcatcaagt ccaagcttag 17160

gagttatccg gcccactctc atattccata tccaaattgt aatcaggatt tatttaacat 17220

agaagacaaa gagtcaacga ggaagatccg tgaactcctc aaaaagggga attcgctgta 17280

ctccaaagtc agtgataagg ttttccaatg cttaagggac actaactcac ggcttggcct 17340

aggctccgaa ttgagggagg acatcaagga gaaagttatt aacttgggag tttacatgca 17400

cagctcccag tggtttgagc cctttctgtt ttggtttaca gtcaagactg agatgaggtc 17460

agtgattaaa tcacaaaccc atacttgcca taggaggaga cacacacctg tattcttcac 17520

tggtagttca gttgagttgc taatctctcg tgaccttgtt gctataatca gtaaagagtc 17580

tcaacatgta tattacctga catttgaact ggttttgatg tattgtgatg tcatagaggg 17640

gaggttaatg acagagaccg ctatgactat tgatgctagg tatacagagc ttctaggaag 17700

agtcagatac atgtggaaac tgatagatgg tttcttccct gcactcggga atccaactta 17760

tcaaattgta gccatgctgg agcctctttc acttgcttac ctgcagctga gggatataac 17820

agtagaactc agaggtgctt tccttaacca ctgctttact gaaatacatg atgttcttga 17880

ccaaaacggg ttttctgatg aaggtactta tcatgagtta actgaagctc tagattacat 17940

tttcataact gatgacatac atctgacagg ggagattttc tcatttttca gaagtttcgg 18000

ccaccccaga cttgaagcag taacggctgc tgaaaatgtt aggaaataca tgaatcagcc 18060

taaagtcatt gtgtatgaga ctctgatgaa aggtcatgcc atattttgtg gaatcataat 18120

caacggctat cgtgacaggc acggaggcag ttggccaccg ctgaccctcc ccctgcatgc 18180

tgcagacaca atccggaatg ctcaagcttc aggtgaaggg ttaacacatg agcagtgcgt 18240

tgataactgg aaatcttttg ctggagtgaa atttggctgc tttatgcctc ttagcctgga 18300

tagtgatctg acaatgtacc taaaggacaa ggcacttgct gctctccaaa gggaatggga 18360

ttcagtttac ccgaaagagt tcctgcgtta cgaccctccc aagggaaccg ggtcacggag 18420

gcttgtagat gttttcctta atgattcgag ctttgaccca tatgatgtga taatgtatgt 18480

tgtaagtgga gcttacctcc atgaccctga gttcaacctg tcttacagcc tgaaagaaaa 18540

ggagatcaag gaaacaggta gactttttgc taaaatgact tacaaaatga gggcatgcca 18600

agtgattgct gaaaatctaa tctcaaacgg gattggcaaa tattttaagg acaatgggat 18660

ggccaaggat gagcacgatt tgactaaggc actccacact ctagctgtct caggagtccc 18720

caaagatctc aaagaaagtc acaggggggg gccagtctta aaaacctact cccgaagccc 18780

agtccacaca agtaccagga acgtgagagc agcaaaaggg tttatagggt tccctcaagt 18840

aattcggcag gaccaagaca ctgatcatcc ggagaatatg gaagcttacg agacagtcag 18900

tgcatttatc acgactgatc tcaagaagta ctgccttaat tggagatatg agaccatcag 18960

cttgtttgca cagaggctaa atgagattta cggattgccc tcatttttcc agtggctgca 19020

taagaggctt gagacctctg tcctgtatgt aagtgaccct cattgccccc ccgaccttga 19080

cgcccatatc ccgttatata aagtccccaa tgatcaaatc ttcattaagt accctatggg 19140

aggtatagaa gggtattgtc agaagctgtg gaccatcagc accattccct atctatacct 19200

ggctgcttat gagagcggag taaggattgc ttcgttagtg caaggggaca atcagaccat 19260

agccgtaaca aaaagggtac ccagcacatg gccctacaac cttaagaaac gggaagctgc 19320

tagagtaact agagattact ttgtaattct taggcaaagg ctacatgata ttggccatca 19380

cctcaaggca aatgagacaa ttgtttcatc acattttttt gtctattcaa aaggaatata 19440

ttatgatggg ctacttgtgt cccaatcact caagagcatc gcaagatgtg tattctggtc 19500

agagactata gttgatgaaa caagggcagc atgcagtaat attgctacaa caatggctaa 19560

aagcatcgag agaggttatg accgttacct tgcatattcc ctgaacgtcc taaaagtgat 19620

acagcaaatt ctgatctctc ttggcttcac aatcaattca accatgaccc gggatgtagt 19680

catacccctc ctcacaaaca acgacctctt aataaggatg gcactgttgc ccgctcctat 19740

tggggggatg aattatctga atatgagcag gctgtttgtc agaaacatcg gtgatccagt 19800

aacatcatca attgctgatc tcaagagaat gattctcgcc tcactaatgc ctgaagagac 19860

cctccatcaa gtaatgacac aacaaccggg ggactcttca ttcctagact gggctagcga 19920

cccttactca gcaaatcttg tatgtgtcca gagcatcact agactcctca agaacataac 19980

tgcaaggttt gtcctgatcc atagtccaaa cccaatgtta aaaggattat tccatgatga 20040

cagtaaagaa gaggacgagg gactggcggc attcctcatg gacaggcata ttatagtacc 20100

tagggcagct catgaaatcc tggatcatag tgtcacaggg gcaagagagt ctattgcagg 20160

catgctggat accacaaaag gcttgattcg agccagcatg aggaaggggg ggttaacctc 20220

tcgagtgata accagattgt ccaattatga ctatgaacaa ttcagagcag ggatggtgct 20280

attgacagga agaaagagaa atgtcctcat tgacaaagag tcatgttcag tgcagctggc 20340

gagagctcta agaagccata tgtgggcgag gctagctcga ggacggccta tttacggcct 20400

tgaggtccct gatgtactag aatctatgcg aggccacctt attcggcgtc atgagacatg 20460

tgtcatctgc gagtgtggat cagtcaacta cggatggttt tttgtcccct cgggttgcca 20520

actggatgat attgacaagg aaacatcatc cttgagagtc ccatatattg gttctaccac 20580

tgatgagaga acagacatga agcttgcctt cgtaagagcc ccaagtcgat ccttgcgatc 20640

tgctgttaga atagcaacag tgtactcatg ggcttacggt gatgatgata gctcttggaa 20700

cgaagcctgg ttgttggcta ggcaaagggc caatgtgagc ctggaggagc taagggtgat 20760

cactcccatc tcaacttcga ctaatttagc gcataggttg agggatcgta gcactcaagt 20820

gaaatactca ggtacatccc ttgtccgagt ggcgaggtat accacaatct ccaacgacaa 20880

tctctcattt gtcatatcag ataagaaggt tgatactaac tttatatacc aacaaggaat 20940

gcttctaggg ttgggtgttt tagaaacatt gtttcgactc gagaaagata ccggatcatc 21000

taacacggta ttacatcttc acgtcgaaac agattgttgc gtgatcccga tgatagatca 21060

tcccaggata cccagctccc gcaagctaga gctgagggca gagctatgta ccaacccatt 21120

gatatatgat aatgcacctt taattgacag agatgcaaca aggctataca cccagagcca 21180

taggaggcac cttgtggaat ttgttacatg gtccacaccc caactatatc acattttagc 21240

taagtccaca gcactatcta tgattgacct ggtaacaaaa tttgagaagg accatatgaa 21300

tgaaatttca gctctcatag gggatgacga tatcaatagt ttcataactg agtttctgct 21360

catagagcca agattattca ctatctactt gggccagtgt gcggccatca attgggcatt 21420

tgatgtacat tatcatagac catcagggaa atatcagatg ggtgagctgt tgtcatcgtt 21480

cctttctaga atgagcaaag gagtgtttaa ggtgcttgtc aatgctctaa gccacccaaa 21540

gatctacaag aaattctggc attgtggtat tatagagcct atccatggtc cttcacttga 21600

tgctcaaaac ttgcacacaa ctgtgtgcaa catggtttac acatgctata tgacctacct 21660

cgacctgttg ttgaatgaag agttagaaga gttcacattt ctcttgtgtg aaagcgacga 21720

ggatgtagta ccggacagat tcgacaacat ccaggcaaaa cacttatgtg ttctggcaga 21780

tttgtactgt caaccaggga cctgcccacc aattcgaggt ctaagaccgg tagagaaatg 21840

tgcagttcta accgaccata tcaaggcaga ggctatgtta tctccagcag gatcttcgtg 21900

gaacataaat ccaattattg tagaccatta ctcatgctct ctgacttatc tccggcgagg 21960

atcgatcaaa cagataagat tgagagttga tccaggattc attttcgacg ccctcgctga 22020

ggtaaatgtc agtcagccaa agatcggcag caacaacatc tcaaatatga gcatcaaggc 22080

tttcagaccc ccacacgatg atgttgcaaa attgctcaaa gatatcaaca caagcaagca 22140

caatcttccc atttcagggg gcaatctcgc caattatgaa atccatgctt tccgcagaat 22200

cgggttgaac tcatctgctt gctacaaagc tgttgagata tcaacattaa ttaggagatg 22260

ccttgagcca ggggaggacg gcttgttctt gggtgaggga tcgggttcta tgttgatcac 22320

ttataaagag atacttaaac taaacaagtg cttctataat agtggggttt ccgccaattc 22380

tagatctggt caaagggaat tagcacccta tccctccgaa gttggccttg tcgaacacag 22440

aatgggagta ggtaatattg tcaaagtgct ctttaacggg aggcccgaag tcacgtgggt 22500

aggcagtgta gattgcttca atttcatagt tagtaatatc cctacctcta gtgtggggtt 22560

tatccattca gatatagaga ccttgcctga caaagatact atagagaagc tagaggaatt 22620

ggcagccatc ttatcgatgg ctctgctcct gggcaaaata ggatcaatac tggtgattaa 22680

gcttatgcct ttcagcgggg attttgttca gggatttata agttatgtag ggtctcatta 22740

tagagaagtg aaccttgtat accctagata cagcaacttc atctctactg aatcttattt 22800

ggttatgaca gatctcaagg ctaaccggct aatgaatcct gaaaagatta agcagcagat 22860

aattgaatca tctgtgagga cttcacctgg acttataggt cacatcctat ccattaagca 22920

actaagctgc atacaagcaa ttgtgggaga cgcagttagt agaggtgata tcaatcctac 22980

tctgaaaaaa cttacaccta tagagcaggt gctgatcaat tgcgggttgg caattaacgg 23040

acctaagctg tgcaaagaat tgatccacca tgatgttgcc tcagggcaag atggattgct 23100

taattctata ctcatcctct acagggagtt ggcaagattc aaagacaacc aaagaagtca 23160

acaagggatg ttccacgctt accccgtatt ggtaagtagc aggcaacgag aacttatatc 23220

taggatcacc cgcaaattct gggggcacat tcttctttac tccgggaaca aaaagttgat 23280

aaataagttt atccagaatc tcaagtccgg ctatctgata ctagacttac accagaatat 23340

cttcgttaag aatctatcca agtcagagaa acagattatt atgacggggg gtttgaaacg 23400

tgagtgggtt tttaaggtaa cagtcaagga gaccaaagaa tggtataagt tagtcggata 23460

cagtgccctg attaaggact aattggttga actccggaac cctaatcctg ccctaggtgg 23520

ttaggcatta tttgcaatat attaaagaaa actttgaaaa tacgaagttt ctattcccag 23580

ctttgtctgg tggccggcat ggtcccagcc tcctcgctgg cgccggctgg gcaacattcc 23640

gaggggaccg tcccctcggt aatggcgaat gggacgcggc cgatccggct gctaacaaag 23700

cccgaaagga agctgagttg gctgctgcca ccgctgagca ataactagca taaccccttg 23760

gggcctctaa acgggtcttg aggggttttt tgctgaaagg aggaactata tccggatgcg 23820

gccgcttcta tagtgtcacc taaatactag tgactccagc gtaactggac tggccacagt 23880

taggccgcaa atgtaatcac actggctcac cttcgggtgg gcctttctgc gttcgcgagt 23940

ggacccgata agctcatgga gcggcgtaac cgtcgcacag gaaggacaga gaaagcgcgg 24000

atctgggaag tgacggacag aacggtcagg acctggattg gggaggcggt tgccgccgct 24060

gctgctgacg gtgtgacgtt ctctgttccg gtcacaccac atacgttccg ccattcctat 24120

gcgatgcaca tgctgtatgc cggtataccg ctgaaagttc tgcaaagcct gatgggacat 24180

aagtccatca gttcaacgga agtctacacg aaggtttttg cgctggatgt ggctgcccgg 24240

caccgggtgc agtttgcgat gccggagtct gatgcggttg cgatgctgaa acaattatcc 24300

tgagaataaa tgccttggcc tttatatgga aatgtggaac tgagtggata tgctgttttt 24360

gtctgttaaa cagagaagct ggctgttatc cactgagaag cgaacgaaac agtcgggaaa 24420

atctcccatt atcgtagaga tccgcattat taatctcagg agcctgtgta gcgtttatag 24480

gaagtagtgt tctgtcatga tgcctgcaag cggtaacgaa aacgatttga atatgccttc 24540

aggaacaata gaaatcttcg tgcggtgtta cgttgaagtg gagcggatta tgtcagcaat 24600

ggacagaaca acctaatgaa cacagaacca tgatgtggtc tgtcctttta cagccagtag 24660

tgctcgccgc agtcgagcga cagggcgaag ccctcggctg gttgccctcg ccgctgggct 24720

ggcggccgtc tatggccctg caaacgcgcc agaaacgccg tcgaagccgt gtgcgagaca 24780

ccgcggcccg ccgccggcgt tgtggatacc tcgcggaaaa cttggccctc actgacagat 24840

gaggggcgga cgttgacact tgaggggccg actcacccgg cgcggcgttg acagatgagg 24900

ggcaggctcg atttcggccg gcgacgtgga gctggccagc ctcgcaaatc ggcgaaaacg 24960

cctgatttta cgcgagtttc ccacagatga tgtggacaag cctggggata agtgccctgc 25020

ggtattgaca cttgaggggc gcgactactg acagatgagg ggcgcgatcc ttgacacttg 25080

aggggcagag tgctgacaga tgaggggcgc acctattgac atttgagggg ctgtccacag 25140

gcagaaaatc cagcatttgc aagggtttcc gcccgttttt cggccaccgc taacctgtct 25200

tttaacctgc ttttaaacca atatttataa accttgtttt taaccagggc tgcgccctgt 25260

gcgcgtgacc gcgcacgccg aaggggggtg cccccccttc tcgaaccctc ccggtcgagt 25320

gagcgaggaa gcaccaggga acagcactta tatattctgc ttacacacga tgcctgaaaa 25380

aacttccctt ggggttatcc acttatccac ggggatattt ttataattat tttttttata 25440

gtttttagat cttctttttt agagcgcctt gtaggccttt atccatgctg gttctagaga 25500

aggtgttgtg acaaattgcc ctttcagtgt gacaaatcac cctcaaatga cagtcctgtc 25560

tgtgacaaat tgcccttaac cctgtgacaa attgccctca gaagaagctg ttttttcaca 25620

aagttatccc tgcttattga ctctttttta tttagtgtga caatctaaaa acttgtcaca 25680

cttcacatgg atctgtcatg gcggaaacag cggttatcaa tcacaagaaa cgtaaaaata 25740

gcccgcgaat cgtccagtca aacgacctca ctgaggcggc atatagtctc tcccgggatc 25800

aaaaacgtat gctgtatctg ttcgttgacc agatcagaaa atctgatggc accctacagg 25860

aacatgacgg tatctgcgag atccatgttg ctaaatatgc tgaaatattc ggattgacct 25920

ctgcggaagc cagtaaggat atacggcagg cattgaagag tttcgcgggg aaggaagtgg 25980

ttttttatcg ccctgaagag gatgccggcg atgaaaaagg ctatgaatct tttccttggt 26040

ttatcaaacg tgcgcacagt ccatccagag ggctttacag tgtacatatc aacccatatc 26100

tcattccctt ctttatcggg ttacagaacc ggtttacgca gtttcggctt agtgaaacaa 26160

aagaaatcac caatccgtat gccatgcgtt tatacgaatc cctgtgtcag tatcgtaagc 26220

cggatggctc aggcatcgtc tctctgaaaa tcgactggat catagagcgt taccagctgc 26280

ctcaaagtta ccagcgtatg cctgacttcc gccgccgctt cctgcaggtc tgtgttaatg 26340

agatcaacag cagaactcca atgcgcctct catacattga gaaaaagaaa ggccgccaga 26400

cgactcatat cgtattttcc ttccgcgata tcacttccat gacgacagga tagtctgagg 26460

gttatctgtc acagatttga gggtggttcg tcacatttgt tctgacctac tgagggtaat 26520

ttgtcacagt tttgctgttt ccttcagcct gcatggattt tctcatactt tttgaactgt 26580

aatttttaag gaagccaaat ttgagggcag tttgtcacag ttgatttcct tctctttccc 26640

ttcgtcatgt gacctgatat cgggggttag ttcgtcatca ttgatgaggg ttgattatca 26700

cagtttatta ctctgaattg gctatccgcg tgtgtacctc tacctggagt ttttcccacg 26760

gtggatattt cttcttgcgc tgagcgtaag agctatctga cagaacagtt cttctttgct 26820

tcctcgccag ttcgctcgct atgctcggtt acacggctgc ggcgagcgct agtgataata 26880

agtgactgag gtatgtgctc ttcttatctc cttttgtagt gttgctctta ttttaaacaa 26940

ctttgcggtt ttttgatgac tttgcgattt tgttgttgct ttgcagtaaa ttgcaagatt 27000

taataaaaaa acgcaaagca atgattaaag gatgttcaga atgaaactca tggaaacact 27060

taaccagtgc ataaacgctg gtcatgaaat gacgaaggct atcgccattg cacagtttaa 27120

tgatgacagc ccggaagcga ggaaaataac ccggcgctgg agaataggtg aagcagcgga 27180

tttagttggg gtttcttctc aggctatcag agatgccgag aaagcagggc gactaccgca 27240

cccggatatg gaaattcgag gacgggttga gcaacgtgtt ggttatacaa ttgaacaaat 27300

taatcatatg cgtgatgtgt ttggtacgcg attgcgacgt gctgaagacg tatttccacc 27360

ggtgatcggg gttgctgccc ataaaggagg cgtttacaaa acctcagttt ctgttcatct 27420

tgctcaggat ctggctctga aggggctacg tgttttgctc gtggaaggta acgaccccca 27480

gggaacagcc tcaatgtatc acggatgggt accagatctt catattcatg cagaagacac 27540

tctcctgcct ttctatcttg gggaaaagga cgatgtcact tatgcaataa agcccacttg 27600

ctggccgggg cttgacatta ttccttcctg tctggctctg caccgtattg aaactgagtt 27660

aatgggcaaa tttgatgaag gtaaactgcc caccgatcca cacctgatgc tccgactggc 27720

cattgaaact gttgctcatg actatgatgt catagttatt gacagtgcgc ctaacctggg 27780

tatcggcacg attaatgtcg tatgtgctgc tgatgtgctg attgttccca cgcctgctga 27840

gttgtttgac tacacctccg cactgcagtt tttcgatatg cttcgtgatc tgctcaagaa 27900

cgttgatctt aaagggttcg agcctgatgt acgtattttg cttaccaaat acagcaatag 27960

taatggctct cagtccccgt ggatggagga gcaaattcgg gatgcctggg gaagcatggt 28020

tctaaaaaat gttgtacgtg aaacggatga agttggtaaa ggtcagatcc ggatgagaac 28080

tgtttttgaa caggccattg atcaacgctc ttcaactggt gcctggagaa atgctctttc 28140

tatttgggaa cctgtctgca atgaaatttt cgatcgtctg attaaaccac gctgggagat 28200

tagataatga agcgtgcgcc tgttattcca aaacatacgc tcaatactca accggttgaa 28260

gatacttcgt tatcgacacc agctgccccg atggtggatt cgttaattgc tcgcgtagga 28320

gtaatggctc gcggtaatgc cattactttg cctgtatgtg gtcgggatgt gaagtttact 28380

cttgaagtgc tccggggtga tagtgttgag aagacctctc gggtatggtc aggtaatgaa 28440

cgtgaccagg agctgcttac tgaggacgca ctggatgatc tcatcccttc ttttctactg 28500

actggtcaac agacaccggc gttcggtcga agagtatctg gtgtcataga aattgccgat 28560

gggagtcgcc gtcgtaaagc tgctgcactt accgaaagtg attatcgtgt tctggttggc 28620

gagctggatg atgagcagat ggctgcatta tccagattgg gtaacgatta tcgcccaaca 28680

agtgcttatg aacgtggtca gcgttatgca agccgattgc agaatgaatt tgctggaaat 28740

atttctgcgc tggctgatgc ggaaaatatt tcacgtaaga ttattacccg ctgtatcaac 28800

accgccaaat tgcctaaatc agttgttgct cttttttctc accccggtga actatctgcc 28860

cggtcaggtg atgcacttca aaaagccttt acagataaag aggaattact taagcagcag 28920

gcatctaacc ttcatgagca gaaaaaagct ggggtgatat ttgaagctga agaagttatc 28980

actcttttaa cttctgtgct taaaacgtca tctgcatcaa gaactagttt aagctcacga 29040

catcagtttg ctcctggagc gacagtattg tataagggcg ataaaatggt gcttaacctg 29100

gacaggtctc gtgttccaac tgagtgtata gagaaaattg aggccattct taaggaactt 29160

gaaaagccag caccctgatg cgaccacgtt ttagtctacg tttatctgtc tttacttaat 29220

gtcctttgtt acaggccaga aagcataact ggcctgaata ttctctctgg gcccactgtt 29280

ccacttgtat cgtcggtctg ataatcagac tgggaccacg gtcccactcg tatcgtcggt 29340

ctgattatta gtctgggacc acggtcccac tcgtatcgtc ggtctgatta ttagtctggg 29400

accacggtcc cactcgtatc gtcggtctga taatcagact gggaccacgg tcccactcgt 29460

atcgtcggtc tgattattag tctgggacca tggtcccact cgtatcgtcg gtctgattat 29520

tagtctggga ccacggtccc actcgtatcg tcggtctgat tattagtctg gaaccacggt 29580

cccactcgta tcgtcggtct gattattagt ctgggaccac ggtcccactc gtatcgtcgg 29640

tctgattatt agtctgggac cacgatccca ctcgtgttgt cggtctgatt atcggtctgg 29700

gaccacggtc ccacttgtat tgtcgatcag actatcagcg tgagactacg attccatcaa 29760

tgcctgtcaa gggcaagtat tgacatggtc gtcgtaacct gtagaacgga gtaacctcgg 29820

tgtgcggttg tatgcctgct gtggattgct gctgtgtcct gcttatccac aacattttgc 29880

gcacggttat gtggacaaaa tacctggtta cccaggccgt gccggcacgt taaccgggct 29940

gcatccgatg caagtgtgtc gctgacgacg agctcgcgag ctcggacatg aggttgcccc 30000

gtattcagtg tcgctgattt gtattgtctg aagttgcttt tacgttaagt tgatgcagat 30060

caattaatac gatacctgcg tcataattga ttatttgacg tggtttgatg gcctccacgc 30120

acgttgtgat atgtagatga taatcattat cactttacgg gtcctttccg gtgatccgac 30180

aggttacggg gcggcgacct cgcgggtttt cgctatttat gaaaattttc cggtttaagg 30240

cgtttccgtt cttcttcgtc ataacttaat gtttttattt aaaataccct ctgaaaagaa 30300

aggaaacgac aggtgctgaa agcgagcttt ttggcctctg tcgtttcctt tctctgtttt 30360

tgtccgtgga atgaacaatg gaagtccgag ctcatcgcta ataacttcgt atagcataca 30420

ttatacgaag ttatattcga tgcggccgat ctagcagaaa gtcaaaagcc tccgaccgga 30480

ggcttttgac ttctgtcacc taggttacgc cccgccctgc cactcatcgc agtactgttg 30540

taattcatta agcattctgc cgacatggaa gccatcacaa acggcatgat gaacctgaat 30600

cgccagcggc atcagcacct tgtcgccttg cgtataatat ttgcccatgg tgaaaacggg 30660

ggcgaagaag ttgtccatat tggccacgtt taaatcaaaa ctggtgaaac tcacccaggg 30720

attggctgag acgaaaaaca tattctcaat aaacccttta gggaaatagg ccaggttttc 30780

accgtaacac gccacatctt gcgaatatat gtgtagaaac tgccggaaat cgtcgtggta 30840

ttcactccag agcgatgaaa acgtttcagt ttgctcatgg aaaacggtgt aacaagggtg 30900

aacactatcc catatcacca gctcaccgtc tttcattgcc atacgaaatt ccggatgagc 30960

attcatcagg cgggcaagaa tgtgaataaa ggccggataa aacttgtgct tatttttctt 31020

tacggtcttt aaaaaggccg taatatccag ctgaacggtc tggttatagg tacattgagc 31080

aactgactga aatgcctcaa aatgttcttt acgatgccat tgggatatat caacggtggt 31140

atatccagtg atttttttct ccattttagc ttccttagct cctgaaaatc tcgataactc 31200

aaaaaatacg cccggtagtg atcttatttc attatggtga aagttggaac ctcttacgtg 31260

ccgatcagat taaaacgaaa ggcccagtct ttcgactgag cctttcgttt tatttgacca 31320

tgttggtatg atttaaattc gtttagtgaa ccgtcagatc actagaagct ttattgcggt 31380

agtttatcac agttaaattg ctaacgcagt cagtgcttct gacacaacag tctcgaactt 31440

aagctgcagt gactctctta aggtagcctt gcagaagttg gtcgtgaggc actgggcagg 31500

taagtatcaa ggttacaaga caggtttaag gagaccaata gaaactgggc ttgtcgagac 31560

agagaagact cttgcgtttc tgataggcac ctattggtct tactgacatc cactttgcct 31620

ttctctccac aggtgtccac tcccagttca attacagctc ttaaggctag agtact 31676

<210> 13
<211> 1248

<212>  PRT
<213>  artificial sequence

<220>
<223>  Synthetic sequence

<400>  13

Met Glu Phe Ile Pro Thr Gln Thr Phe Tyr Asn Arg Arg Tyr Gln Pro
1               5                   10                  15

Arg Pro Trp Thr Pro Arg Pro Thr Ile Gln Val Ile Arg Pro Arg Pro
            20                  25                  30

Arg Pro Gln Arg Gln Ala Gly Gln Leu Ala Gln Leu Ile Ser Ala Val
        35                  40                  45

Asn Lys Leu Thr Met Arg Ala Val Pro Gln Gln Lys Pro Arg Arg Asn
    50                  55                  60

Arg Lys Asn Lys Lys Gln Lys Gln Lys Gln Gln Ala Pro Gln Asn Asn
65                  70                  75                  80

Thr Asn Gln Lys Lys Gln Pro Pro Lys Lys Lys Pro Ala Gln Lys Lys
                85                  90                  95

Lys Lys Pro Gly Arg Arg Glu Arg Met Cys Met Lys Ile Glu Asn Asp
            100                 105                 110

Cys Ile Phe Glu Val Lys His Glu Gly Lys Val Thr Gly Tyr Ala Cys
        115                 120                 125

Leu Val Gly Asp Lys Val Met Lys Pro Ala His Val Lys Gly Thr Ile
    130                 135                 140

Asp Asn Ala Asp Leu Ala Lys Leu Ala Phe Lys Arg Ser Ser Lys Tyr
145                 150                 155                 160

Asp Leu Glu Cys Ala Gln Ile Pro Val His Met Lys Ser Asp Ala Ser
                165                 170                 175

Lys Phe Thr His Glu Lys Pro Glu Gly Tyr Tyr Asn Trp His His Gly
            180                 185                 190

Ala Val Gln Tyr Ser Gly Gly Arg Phe Thr Ile Pro Thr Gly Ala Gly
        195                 200                 205

Lys Pro Gly Asp Ser Gly Arg Pro Ile Phe Asp Asn Lys Gly Arg Val
    210                 215                 220

Val Ala Ile Val Leu Gly Gly Ala Asn Glu Gly Ala Arg Thr Ala Leu
225                 230                 235                 240

Ser Val Val Thr Trp Asn Lys Asp Ile Val Thr Lys Ile Thr Pro Glu
                245                 250                 255

Gly Ala Glu Glu Trp Ser Leu Ala Ile Pro Val Met Cys Leu Leu Ala
            260                 265                 270

Asn Thr Thr Phe Pro Cys Ser Gln Pro Pro Cys Thr Pro Cys Cys Tyr
        275                 280                 285

Glu Lys Glu Pro Glu Glu Thr Leu Arg Met Leu Glu Asp Asn Val Met
    290                 295                 300

Arg Pro Gly Tyr Tyr Gln Leu Leu Gln Ala Ser Leu Thr Cys Ser Pro
305                 310                 315                 320

His Arg Gln Arg Arg Ser Thr Lys Asp Asn Phe Asn Val Tyr Lys Ala
                325                 330                 335

Thr Arg Pro Tyr Leu Ala His Cys Pro Asp Cys Gly Glu Gly His Ser
            340                 345                 350

Cys His Ser Pro Val Ala Leu Glu Arg Ile Arg Asn Glu Ala Thr Asp
        355                 360                 365

Gly Thr Leu Lys Ile Gln Val Ser Leu Gln Ile Gly Ile Lys Thr Asp
    370                 375                 380

Asp Ser His Asp Trp Thr Lys Leu Arg Tyr Met Asp Asn His Met Pro
385                 390                 395                 400

Ala Asp Ala Glu Arg Ala Gly Leu Phe Val Arg Thr Ser Ala Pro Cys
                405                 410                 415

Thr Ile Thr Gly Thr Met Gly His Phe Ile Leu Ala Arg Cys Pro Lys
            420                 425                 430

Gly Glu Thr Leu Thr Val Gly Phe Thr Asp Ser Arg Lys Ile Ser His
        435                 440                 445

Ser Cys Thr His Pro Phe His His Asp Pro Pro Val Ile Gly Arg Glu
    450                 455                 460

Lys Phe His Ser Arg Pro Gln His Gly Lys Glu Leu Pro Cys Ser Thr
465                 470                 475                 480

Tyr Val Gln Ser Thr Ala Ala Thr Thr Glu Glu Ile Glu Val His Met
                485                 490                 495

Pro Pro Asp Thr Pro Asp Arg Thr Leu Met Ser Gln Gln Ser Gly Asn
            500                 505                 510

Val Lys Ile Thr Val Asn Gly Gln Thr Val Arg Tyr Lys Cys Asn Cys
        515                 520                 525

Gly Gly Ser Asn Glu Gly Leu Thr Thr Thr Asp Lys Val Ile Asn Asn
    530                 535                 540

Cys Lys Val Asp Gln Cys His Ala Ala Val Thr Asn His Lys Lys Trp
545                 550                 555                 560

Gln Tyr Asn Ser Pro Leu Val Pro Arg Asn Ala Glu Leu Gly Asp Arg
                565                 570                 575

Lys Gly Lys Ile His Ile Pro Phe Pro Leu Ala Asn Val Thr Cys Arg
            580                 585                 590

Val Pro Lys Ala Arg Asn Pro Thr Val Thr Tyr Gly Lys Asn Gln Val
        595                 600                 605

Ile Met Leu Leu Tyr Pro Asp His Pro Thr Leu Leu Ser Tyr Arg Asn
    610                 615                 620

Met Gly Glu Glu Pro Asn Tyr Gln Glu Glu Trp Val Met His Lys Lys
625                 630                 635                 640

Glu Val Val Leu Thr Val Pro Thr Glu Gly Leu Glu Val Thr Trp Gly
                645                 650                 655

Asn Asn Glu Pro Tyr Lys Tyr Trp Pro Gln Leu Ser Thr Asn Gly Thr
            660                 665                 670

Ala His Gly His Pro His Glu Ile Ile Leu Tyr Tyr Tyr Glu Leu Tyr
        675                 680                 685

Pro Thr Met Thr Val Val Val Val Ser Val Ala Thr Phe Ile Leu Leu
    690                 695                 700

Ser Met Val Gly Met Ala Ala Gly Met Cys Met Cys Ala Arg Arg Arg
705                 710                 715                 720

Cys Ile Thr Pro Tyr Glu Leu Thr Pro Gly Ala Thr Val Pro Phe Leu

                725                 730                 735

Leu Ser Leu Ile Cys Cys Ile Arg Thr Ala Lys Ala Ala Thr Tyr Gln
            740                 745                 750

Glu Ala Ala Ile Tyr Leu Trp Asn Glu Gln Gln Pro Leu Phe Trp Leu
        755                 760                 765

Gln Ala Leu Ile Pro Leu Ala Ala Leu Ile Val Leu Cys Asn Cys Leu
    770                 775                 780

Arg Leu Leu Pro Cys Cys Cys Lys Thr Leu Ala Phe Leu Ala Val Met
785                 790                 795                 800

Ser Val Gly Ala His Thr Val Ser Ala Tyr Glu His Val Thr Val Ile
                805                 810                 815

Pro Asn Thr Val Gly Val Pro Tyr Lys Thr Leu Val Asn Arg Pro Gly
            820                 825                 830

Tyr Ser Pro Met Val Leu Glu Met Glu Leu Leu Ser Val Thr Leu Glu
        835                 840                 845

Pro Thr Leu Ser Leu Asp Tyr Ile Thr Cys Glu Tyr Lys Thr Val Ile
    850                 855                 860

Pro Ser Pro Tyr Val Lys Cys Cys Gly Thr Ala Glu Cys Lys Asp Lys
865                 870                 875                 880

Asn Leu Pro Asp Tyr Ser Cys Lys Val Phe Thr Gly Val Tyr Pro Phe
                885                 890                 895

Met Trp Gly Gly Ala Tyr Cys Phe Cys Asp Ala Glu Asn Thr Gln Leu
            900                 905                 910

Ser Glu Ala His Val Glu Lys Ser Glu Ser Cys Lys Thr Glu Phe Ala
        915                 920                 925

Ser Ala Tyr Arg Ala His Thr Ala Ser Ala Ser Ala Lys Leu Arg Val
    930                 935                 940

Leu Tyr Gln Gly Asn Asn Ile Thr Val Thr Ala Tyr Ala Asn Gly Asp
945                 950                 955                 960

His Ala Val Thr Val Lys Asp Ala Lys Phe Ile Val Gly Pro Met Ser
                965                 970                 975

Ser Ala Trp Thr Pro Phe Asp Asn Lys Ile Val Val Tyr Lys Gly Asp
            980                 985                 990

Val Tyr Asn Met Asp Tyr Pro Pro Phe Gly Ala Gly Arg Pro Gly Gln
        995                 1000                1005

Phe Gly Asp Ile Gln Ser Arg Thr Pro Glu Ser Lys Asp Val Tyr
    1010                1015                1020

Ala Asn Thr Gln Leu Val Leu Gln Arg Pro Ala Val Gly Thr Val
    1025                1030                1035

His Val Pro Tyr Ser Gln Ala Pro Ser Gly Phe Lys Tyr Trp Leu
    1040                1045                1050

Lys Glu Arg Gly Ala Ser Leu Gln His Thr Ala Pro Phe Gly Cys
    1055                1060                1065

Gln Ile Ala Thr Asn Pro Val Arg Ala Val Asn Cys Ala Val Gly
    1070                1075                1080

Asn Met Pro Ile Ser Ile Asp Ile Pro Glu Ala Ala Phe Thr Arg
    1085                1090                1095

Val Val Asp Ala Pro Ser Leu Thr Asp Met Ser Cys Glu Val Pro
    1100                1105                1110

Ala Cys Thr His Ser Ser Asp Phe Gly Gly Val Ala Ile Ile Lys
    1115                1120                1125

Tyr Ala Ala Ser Lys Lys Gly Lys Cys Ala Val His Ser Met Thr
    1130                1135                1140

Asn Ala Val Thr Ile Arg Glu Ala Glu Ile Glu Val Glu Gly Asn
    1145                1150                1155

Ser Gln Leu Gln Ile Ser Phe Ser Thr Ala Leu Ala Ser Ala Glu
    1160                1165                1170

Phe Arg Val Gln Val Cys Ser Thr Gln Val His Cys Ala Ala Glu
    1175                1180                1185

Cys His Pro Pro Lys Asp His Ile Val Asn Tyr Pro Ala Ser His
    1190                1195                1200

Thr Thr Leu Gly Val Gln Asp Ile Ser Ala Thr Ala Met Ser Trp
    1205                1210                1215

Val Gln Lys Ile Thr Gly Gly Val Gly Leu Val Val Ala Val Ala
    1220                1225                1230

Ala Leu Ile Leu Ile Val Val Leu Cys Val Ser Phe Ser Arg His
    1235                1240                1245

<210> 14
<211> 459
<212> PRT
<213> artificial sequence

<220>
<223> Synthetic sequence

<400> 14

Met Arg Val Val Phe Val Val Leu Leu Leu Leu Val Ala Pro Ala Tyr
1               5                   10                  15

Ser Phe Asn Cys Leu Gly Met Ser Asn Arg Asp Phe Leu Glu Gly Val
            20                  25                  30

Ser Gly Ala Thr Trp Val Asp Leu Val Leu Glu Gly Asp Ser Cys Val
        35                  40                  45

Thr Ile Met Ser Lys Asp Lys Pro Thr Ile Asp Val Lys Met Met Asn
    50                  55                  60

Met Glu Ala Val Asn Leu Ala Glu Val Arg Ser Tyr Cys Tyr Leu Ala
65                  70                  75                  80

Thr Val Ser Asp Leu Ser Thr Lys Ala Ala Cys Pro Thr Met Gly Glu
                85                  90                  95

Ala His Asn Asp Lys Arg Ala Asp Pro Ala Phe Val Cys Arg Gln Gly
            100                 105                 110

Val Val Asp Arg Gly Trp Gly Asn Gly Cys Gly Leu Phe Gly Lys Gly
        115                 120                 125

Ser Ile Asp Thr Cys Ala Lys Phe Ala Cys Ser Thr Lys Ala Ile Gly
    130                 135                 140

Arg Thr Ile Leu Lys Glu Asn Ile Lys Tyr Glu Val Ala Ile Phe Val
145                 150                 155                 160

His Gly Pro Thr Thr Val Glu Ser His Gly Asn Tyr Ser Thr Gln Val
                165                 170                 175

Gly Ala Thr Gln Ala Gly Arg Phe Ser Ile Thr Pro Ala Ala Pro Ser
        180                 185                 190

Tyr Thr Leu Lys Leu Gly Glu Tyr Gly Glu Val Thr Val Asp Cys Glu
    195                 200                 205

Pro Arg Ser Gly Ile Asp Thr Asn Ala Tyr Tyr Val Met Thr Val Gly
    210                 215                 220

Thr Lys Thr Phe Leu Val His Arg Glu Trp Phe Met Asp Leu Asn Leu
225                 230                 235                 240

Pro Trp Ser Ser Ala Gly Ser Thr Val Trp Arg Asn Arg Glu Thr Leu
                245                 250                 255

Met Glu Phe Glu Glu Pro His Ala Thr Lys Gln Ser Val Ile Ala Leu
            260                 265                 270

Gly Ser Gln Glu Gly Ala Leu His Gln Ala Leu Ala Gly Ala Ile Pro
        275                 280                 285

Val Glu Phe Ser Ser Asn Thr Val Lys Leu Thr Ser Gly His Leu Lys
    290                 295                 300

Cys Arg Val Lys Met Glu Lys Leu Gln Leu Lys Gly Thr Thr Tyr Gly
305                 310                 315                 320

Val Cys Ser Lys Ala Phe Lys Phe Leu Gly Thr Pro Ala Asp Thr Gly
                325                 330                 335

His Gly Thr Val Val Leu Glu Leu Gln Tyr Thr Gly Thr Asp Gly Pro
            340                 345                 350

Cys Lys Val Pro Ile Ser Ser Val Ala Ser Leu Asn Asp Leu Thr Pro
        355                 360                 365

Val Gly Arg Leu Val Thr Val Asn Pro Phe Val Ser Val Ala Thr Ala
    370                 375                 380

Asn Ala Lys Val Leu Ile Glu Leu Glu Pro Pro Phe Gly Asp Ser Tyr
385                 390                 395                 400

Ile Val Val Gly Arg Gly Glu Gln Gln Ile Asn His His Trp His Lys
                405                 410                 415

Ser Gly Ser Ser Ile Gly Lys Ala Phe Thr Thr Thr Leu Lys Gly Ala
            420                 425                 430

Gln Arg Leu Ala Ala Leu Gly Asp Thr Ala Trp Asp Phe Gly Ser Val
        435                 440                 445

Gly Gly Val Phe Thr Ser Val Gly Lys Ala Val
    450                 455

<210>  15
<211>  696
<212>  PRT
<213>  artificial sequence

<220>
<223>  Synthetic sequence

<400>  15

Met Glu Lys Lys Arg Arg Gly Ala Asp Thr Ser Val Gly Ile Val Gly
1               5                   10                  15

Leu Leu Leu Thr Thr Ala Met Ala Ala Glu Val Thr Arg Arg Gly Ser
            20                  25                  30

Ala Tyr Tyr Met Tyr Leu Asp Arg Asn Asp Ala Gly Glu Ala Ile Ser
        35                  40                  45

Phe Pro Thr Thr Leu Gly Met Asn Lys Cys Tyr Ile Gln Ile Met Asp
    50                  55                  60

Leu Gly His Met Cys Asp Ala Thr Met Ser Tyr Glu Cys Pro Met Leu
65                  70                  75                  80

Asp Glu Gly Val Glu Pro Asp Asp Val Asp Cys Trp Cys Asn Thr Thr
                85                  90                  95

Ser Thr Trp Val Val Tyr Gly Thr Cys His His Lys Lys Gly Glu Ala
            100                 105                 110

Arg Arg Ser Arg Arg Ala Val Thr Leu Pro Ser His Ser Thr Arg Lys
        115                 120                 125

Leu Gln Thr Arg Ser Gln Thr Trp Leu Glu Ser Arg Glu Tyr Thr Lys
    130                 135                 140

His Leu Ile Arg Val Glu Asn Trp Ile Phe Arg Asn Pro Gly Phe Ala
145                 150                 155                 160

Leu Ala Ala Ala Ala Ile Ala Trp Leu Leu Gly Ser Ser Thr Ser Gln
                165                 170                 175

Lys Val Ile Tyr Leu Val Met Ile Leu Leu Ile Ala Pro Ala Tyr Ser
            180                 185                 190

Ile Arg Cys Ile Gly Val Ser Asn Arg Asp Phe Val Glu Gly Met Ser
        195                 200                 205

Gly Gly Thr Trp Val Asp Val Val Leu Glu His Gly Gly Cys Val Thr
    210                 215                 220

Val Met Ala Gln Asp Lys Pro Thr Val Asp Ile Glu Leu Val Thr Thr
225                 230                 235                 240

Thr Val Ser Asn Met Ala Glu Val Arg Ser Tyr Cys Tyr Glu Ala Ser
                245                 250                 255

Ile Ser Asp Met Ala Ser Asp Ser Arg Cys Pro Thr Gln Gly Glu Ala
            260                 265                 270

Tyr Leu Asp Lys Gln Ser Asp Thr Gln Tyr Val Cys Lys Arg Thr Leu
        275                 280                 285

Val Asp Arg Gly Trp Gly Asn Gly Cys Gly Leu Phe Gly Lys Gly Ser
    290                 295                 300

Leu Val Thr Cys Ala Lys Phe Ala Cys Ser Lys Lys Met Thr Gly Lys
305                 310                 315                 320

Ser Ile Gln Pro Glu Asn Leu Glu Tyr Arg Ile Met Leu Ser Val His
                325                 330                 335

Gly Ser Gln His Ser Gly Met Ile Val Asn Asp Thr Gly His Glu Thr
            340                 345                 350

Asp Glu Asn Arg Ala Lys Val Glu Ile Thr Pro Asn Ser Pro Arg Ala
        355                 360                 365

Glu Ala Thr Leu Gly Gly Phe Gly Ser Leu Gly Leu Asp Cys Glu Pro
    370                 375                 380

Arg Thr Gly Leu Asp Phe Ser Asp Leu Tyr Tyr Leu Thr Met Asn Asn
385                 390                 395                 400

Lys His Trp Leu Val His Lys Glu Trp Phe His Asp Ile Pro Leu Pro
                405                 410                 415

Trp His Ala Gly Ala Asp Thr Gly Thr Pro His Trp Asn Asn Lys Glu
            420                 425                 430

Ala Leu Val Glu Phe Lys Asp Ala His Ala Lys Arg Gln Thr Val Val
        435                 440                 445

Val Leu Gly Ser Gln Glu Gly Ala Val His Thr Ala Leu Ala Gly Ala
    450                 455                 460

Leu Glu Ala Glu Met Asp Gly Ala Lys Gly Arg Leu Ser Ser Gly His
465                 470                 475                 480

Leu Lys Cys Arg Leu Lys Met Asp Lys Leu Arg Leu Lys Gly Val Ser
                485                 490                 495

Tyr Ser Leu Cys Thr Ala Ala Phe Thr Phe Thr Lys Ile Pro Ala Glu
            500                 505                 510

Thr Leu His Gly Thr Val Thr Val Glu Val Gln Tyr Ala Gly Thr Asp
        515                 520                 525

Gly Pro Cys Lys Val Pro Ala Gln Met Ala Val Asp Met Gln Thr Leu
    530                 535                 540

Thr Pro Val Gly Arg Leu Ile Thr Ala Asn Pro Val Ile Thr Glu Ser
545                 550                 555                 560

Thr Glu Asn Ser Lys Met Met Leu Glu Leu Asp Pro Pro Phe Gly Asp
                565                 570                 575

Ser Tyr Ile Val Ile Gly Val Gly Glu Lys Lys Ile Thr His His Trp
            580                 585                 590

His Arg Ser Gly Ser Thr Ile Gly Lys Ala Phe Glu Ala Thr Val Arg
        595                 600                 605

Gly Ala Lys Arg Met Ala Val Leu Gly Asp Thr Ala Trp Asp Phe Gly
    610                 615                 620

Ser Val Gly Gly Ala Leu Asn Ser Leu Gly Lys Gly Ile His Gln Ile
625                 630                 635                 640

Phe Gly Ala Ala Phe Lys Ser Leu Phe Gly Gly Met Ser Trp Phe Ser
                645                 650                 655

Gln Ile Leu Ile Gly Thr Leu Leu Met Trp Leu Gly Leu Asn Thr Lys
            660                 665                 670

Asn Gly Ser Ile Ser Leu Met Cys Leu Ala Leu Gly Gly Val Leu Ile
        675                 680                 685

Phe Leu Ser Thr Ala Val Ser Ala
    690                 695

<210>  16
<211>  58
<212>  DNA
<213>  artificial sequence

<220>
<223>  Synthetic sequence

<400>  16
ttataaaaaa cttaggaacc aggtccacac agccgccagc ccatcaacgc gtacgatg          58

<210>  17
<211>  84
<212>  DNA
<213>  artificial sequence

<220>
<223>  Synthetic sequence

<400>  17
taataggcgc gcagcgctta gacgtctcgc gatcgatact agtacaacct aaatccatta       60

taaaaaactt aggagcaaag tgat                                              84

<210>  18
<211>  8
<212>  DNA
<213>  artificial sequence

<220>
<223>  Synthetic sequence

<400>  18
gcggccgc                                                                 8

<210>  19
<211>  78
<212>  DNA
<213>  artificial sequence

<220>
<223>  Synthetic sequence

<400>  19
tataaaaaac ttaggaacca ggtccacaca gccgccagcc catcaaccat ccactcccac       60

gattggaggc cggccatg                                                     78

<210>  20
<211>  78
<212>  DNA
<213>  artificial sequence

<220>
<223>  Synthetic sequence

<400>  20
tataaaaaac ttaggaacca ggtccacaca gccgccagcc catcaaccat ccactcccac      60

gattggagcg gccgcatg                                                    78


<210>  21
<211>  31
<212>  DNA
<213>  artificial sequence

<220>
<223>  Synthetic sequence

<400>  21
cattactcct acgcgagcaa ttaacgaatc c                                     31


<210>  22
<211>  31
<212>  DNA
<213>  artificial sequence

<220>
<223>  Synthetic sequence

<400>  22
ggattcgtta attgctgccg taggagtaat g                                     31


<210>  23
<211>  31
<212>  DNA
<213>  artificial sequence

<220>
<223>  Synthetic sequence

<400>  23
acccaggtta ggcgcactgt caataactat g                                     31


<210>  24
<211>  31
<212>  DNA
<213>  artificial sequence

<220>
<223>  Synthetic sequence

<400>  24
catagttatt gacagtgcgc ctaacctggg t                                     31


<210>  25
<211>  32
<212>  DNA
<213>  artificial sequence

<220>

<223>  Synthetic sequence

<400>  25
tgatttaaat tcgtttagtg aaccgtcaga tc                                    32


<210>  26
<211>  32
<212>  DNA
<213>  artificial sequence

<220>
<223>  Synthetic sequence

<400>  26
ttgcggccgc agtactctag ccttaagagc tg                                    32


<210>  27
<211>  32
<212>  DNA
<213>  artificial sequence

<220>
<223>  Synthetic sequence

<400>  27
ttacgtacga tggagttcat cccaacccaa ac                                    32


<210>  28
<211>  96
<212>  DNA
<213>  artificial sequence

<220>
<223>  Synthetic sequence

<400>  28
ataatggatt taggttgtac tagttgggtc gactggcatg gggttggcag gtaagttgag      60

ctgtagttcg aactattagt gcctgctgaa cgacac                                96


<210>  29
<211>  106
<212>  DNA
<213>  artificial sequence

<220>
<223>  Synthetic sequence

<400>  29
aacctaaatc cattataaaa aacttaggaa ccaggtccac acagccgcca gcccatcaac      60

catccactcc cacgattgga cctgcaggat gagagttgtg tttgtc                    106


<210>  30
<211>  90
<212>  DNA
<213>  artificial sequence

<220>
<223>  Synthetic sequence

<400>  30
ataatggatt taggttgtac tagttgggtc gactggcatg gggttggcag gtaagttgag      60

ctgtagttcg aattagacag ccttcccaac                                        90


<210>  31
<211>  107
<212>  DNA
<213>  artificial sequence

<220>
<223>  Synthetic sequence

<400>  31
aacctaaatc cattataaaa aacttaggaa ccaggtccac acagccgcca gcccatcaac      60

catccactcc cacgattgga ggccggccat ggagaagaag cggagag                   107


<210>  32
<211>  27
<212>  DNA
<213>  artificial sequence

<220>
<223>  Synthetic sequence

<400>  32
tgagcgcgct tagacagcct tcccaac                                           27


<210>  33
<211>  25
<212>  DNA
<213>  artificial sequence

<220>
<223>  Synthetic sequence

<400>  33
ttagcgcgct catcaggcag acacg                                             25


<210>  34
<211>  29
<212>  DNA
<213>  artificial sequence

<220>
<223>  Synthetic sequence

<400>  34
attcctgcag gatgagagtt gtgtttgtc                                         29


<210>  35
<211>  30
<212>  DNA
<213>  artificial sequence

<220>
<223>  Synthetic sequence

<400>  35
ttacctgcag gatggagaag aagcggagag                                        30


<210>  36
<211>  28
<212>  DNA
<213>  artificial sequence

<220>
<223>  Synthetic sequence

<400>  36
ttacgtacga tggagaagaa gcggagag                                          28


<210>  37
<211>  97
<212>  DNA
<213>  artificial sequence

<220>
<223>  Synthetic sequence

<400>  37
ataatggatt taggttgtac tagttgggtc gactggcatg gggttggcag gtaagttgag      60

ctgtagttcg aatcatcagg cagacacggc ggtggac                               97


<210>  38
<211>  111
<212>  DNA
<213>  artificial sequence

<220>
<223>  Synthetic sequence

<400>  38
aacctaaatc cattataaaa aacttaggaa ccaggtccac acagccgcca gcccatcaac      60

catccactcc cacgattgga cctgcaggat ggagttcatc ccaacccaaa c              111


<210>  39
<211>  31
<212>  DNA
<213>  artificial sequence

<220>
<223>  Synthetic sequence

<400>  39
ttagcgcgcc tattagtgcc tgctgaacga c                                     31


<210>  40
<211>  36
<212>  DNA

<213>  artificial sequence

<220>
<223>  Synthetic sequence

<400>  40
cttaggattc aagatcctat tatcagggac aagagc                                36


<210>  41
<211>  36
<212>  DNA
<213>  artificial sequence

<220>
<223>  Synthetic sequence

<400>  41
cttaggaacc aggtccacac agccgccagc ccatca                                36


<210>  42
<211>  35
<212>  DNA
<213>  artificial sequence

<220>
<223>  Synthetic sequence

<400>  42
cttaggagca aagtgattgc ctcccaaggt ccaca                                 35


<210>  43
<211>  34
<212>  DNA
<213>  artificial sequence

<220>
<223>  Synthetic sequence

<400>  43
cttagggcca aggaacatac acacccaaca gaac                                  34


<210>  44
<211>  23
<212>  DNA
<213>  artificial sequence

<220>
<223>  Synthetic sequence

<400>  44
cttagggtgc aagatcatcc aca                                              23


<210>  45
<211>  25
<212>  DNA
<213>  artificial sequence

<220>

<223>  Synthetic sequence

<400>  45
cgtagggtcc aagtggttcc ccgtt                                          25


<210>  46
<211>  33
<212>  DNA
<213>  artificial sequence

<220>
<223>  Synthetic sequence

<400>  46
ccgcctacca tccatcattg ttataaaaaa ctt                                 33


<210>  47
<211>  33
<212>  DNA
<213>  artificial sequence

<220>
<223>  Synthetic sequence

<400>  47
actagtacaa cctaaatcca ttataaaaaa ctt                                 33


<210>  48
<211>  33
<212>  DNA
<213>  artificial sequence

<220>
<223>  Synthetic sequence

<400>  48
gatcctctct ccccggcaaa ctaaacaaaa ctt                                 33


<210>  49
<211>  20
<212>  DNA
<213>  artificial sequence

<220>
<223>  Synthetic sequence

<400>  49
tcggtagtta atcaaaactt                                                20


<210>  50
<211>  33
<212>  DNA
<213>  artificial sequence

<220>
<223>  Synthetic sequence

<400>  50

gtgtgaaata gacatcagaa ttaagaaaaa cgt                                    33

<210>  51
<211>  33
<212>  DNA
<213>  artificial sequence

<220>
<223>  Synthetic sequence

<400>  51
taggcattat ttgcaatata ttaaagaaaa ctt                                    33

<210>  52
<211>  116
<212>  DNA
<213>  artificial sequence

<220>
<223>  Synthetic sequence

<220>
<221>  misc_feature
<222>  (49)..(49)
<223>  n is a, c, g, or t

<400>  52
cttaggattc aagatcctat tatcagggac aagagcacgc gtacgatgnt aggcgcgcag      60

cgcttagacg tctcgcgatc gatccgccta ccatccatca ttgttataaa aaactt         116

<210>  53
<211>  116
<212>  DNA
<213>  artificial sequence

<220>
<223>  Synthetic sequence

<220>
<221>  misc_feature
<222>  (49)..(49)
<223>  n is a, c, g, or t

<400>  53
cttaggaacc aggtccacac agccgccagc ccatcaacgc gtacgatgnt aggcgcgcag      60

cgcttagacg tctcgcgatc gatactagta caacctaaat ccattataaa aaactt         116

<210>  54
<211>  115
<212>  DNA
<213>  artificial sequence

<220>
<223>  Synthetic sequence

<220>
<221>  misc_feature
<222>  (48)..(48)
<223>  n is a, c, g, or t

<400>  54
cttaggagca aagtgattgc ctcccaaggt ccacaacgcg tacgatgnta ggcgcgcagc      60

gcttagacgt ctcgcgatcg atgatcctct ctccccggca aactaaacaa aactt          115


<210>  55
<211>  101
<212>  DNA
<213>  artificial sequence

<220>
<223>  Synthetic sequence


<220>
<221>  misc_feature
<222>  (47)..(47)
<223>  n is a, c, g, or t

<400>  55
cttagggcca aggaacatac acacccaaca gaacacgcgt acgatgntag gcgcgcagcg      60

cttagacgtc tcgcgatcga ttcggtagtt aatcaaaact t                         101


<210>  56
<211>  103
<212>  DNA
<213>  artificial sequence

<220>
<223>  Synthetic sequence


<220>
<221>  misc_feature
<222>  (36)..(36)
<223>  n is a, c, g, or t

<400>  56
cttagggtgc aagatcatcc acaacgcgta cgatgntagg cgcgcagcgc ttagacgtct      60

cgcgatcgat gtgtgaaata gacatcagaa ttaagaaaaa cgt                       103


<210>  57
<211>  105
<212>  DNA
<213>  artificial sequence

<220>
<223>  Synthetic sequence


<220>
<221>  misc_feature

<222>  (38)..(38)
<223>  n is a, c, g, or t

<400>  57
cgtagggtcc aagtggttcc ccgttacgcg tacgatgnta ggcgcgcagc gcttagacgt      60

ctcgcgatcg attaggcatt atttgcaata tattaaagaa aactt                     105


<210>  58
<211>  15894
<212>  DNA
<213>  artificial sequence

<220>
<223>  Synthetic sequence

<400>  58
accaaacaaa gttgggtaag gatagttcaa tcaatgatca tcttctagtg cacttaggat      60

tcaagatcct attatcaggg acaagagcag gattagggat atccgagatg gccacacttt     120

taaggagctt agcattgttc aaaagaaaca aggacaaacc acccattaca tcaggatccg     180

gtggagccat cagaggaatc aaacacatta ttatagtacc aatccctgga gattcctcaa     240

ttaccactcg atccagactt ctggaccggt tggtcaggtt aattggaaac ccggatgtga     300

gcgggcccaa actaacaggg gcactaatag gtatattatc cttatttgtg gagtctccag     360

gtcaattgat tcagaggatc accgatgacc ctgacgttag cataaggctg ttagaggttg     420

tccagagtga ccagtcacaa tctggcctta ccttcgcatc aagaggtacc aacatggagg     480

atgaggcgga caaatacttt tcacatgatg atccaattag tagtgatcaa tccaggttcg     540

gatggttcga gaacaaggaa atctcagata ttgaagtgca agaccctgag ggattcaaca     600

tgattctggg taccatccta gcccaaattt gggtcttgct cgcaaaggcg gttacggccc     660

cagacacggc agctgattcg gagctaagaa ggtggataaa gtacacccaa caaagaaggg     720

tagttggtga atttagattg gagagaaaat ggttggatgt ggtgaggaac aggattgccg     780

aggacctctc cttacgccga ttcatggtcg ctctaatcct ggatatcaag agaacacccg     840

gaaacaaacc caggattgct gaaatgatat gtgacattga tacatatatc gtagaggcag     900

gattagccag ttttatcctg actattaagt ttgggataga aactatgtat cctgctcttg     960

gactgcatga atttgctggt gagttatcca cacttgagtc cttgatgaac ctttaccagc    1020

aaatggggga aactgcaccc tacatggtaa tcctggagaa ctcaattcag aacaagttca    1080

gtgcaggatc ataccctctg ctctggagct atgccatggg agtaggagtg gaacttgaaa    1140

actccatggg aggtttgaac tttggccgat cttactttga tccagcatat tttagattag    1200

ggcaagagat ggtaaggagg tcagctggaa aggtcagttc cacattggca tctgaactcg    1260

gtatcactgc cgaggatgca aggcttgttt cagagattgc aatgcatact actgaggaca    1320

agatcagtag agcggttgga cccagacaag cccaagtatc atttctacac ggtgatcaaa    1380

gtgagaatga gctaccgaga ttggggggca aggaagatag gagggtcaaa cagagtcgag 1440
gagaagccag ggagagctac agagaaaccg ggcccagcag agcaagtgat gcgagagctg 1500
cccatcttcc aaccggcaca cccctagaca ttgacactgc atcggagtcc agccaagatc 1560
cgcaggacag tcgaaggtca gctgacgccc tgcttaggct gcaagccatg gcaggaatct 1620
cggaagaaca aggctcagac acggacaccc ctatagtgta caatgacaga aatcttctag 1680
actaggtgcg agaggccgag gaccagaaca acatccgcct accctccatc attgttataa 1740
aaaacttagg aaccaggtcc acacagccgc cagcccatca accatccact cccacgattg 1800
gagccgatgg cagaagagca ggcacgccat gtcaaaaacg gactggaatg catccgggct 1860
ctcaaggccg agcccatcgg ctcactggcc atcgaggaag ctatggcagc atggtcagaa 1920
atatcagaca acccaggaca ggagcgagcc acctgcaggg aagagaaggc aggcagttcg 1980
ggtctcagca aaccatgcct ctcagcaatt ggatcaactg aaggcggtgc acctcgcatc 2040
cgcggtcagg gacctggaga gagcgatgac gacgctgaaa ctttgggaat ccccccaaga 2100
aatctccagg catcaagcac tgggttacag tgttattatg tttatgatca cagcggtgaa 2160
gcggttaagg gaatccaaga tgctgactct atcatggttc aatcaggcct tgatggtgat 2220
agcaccctat caggaggaga caatgaatct gaaaacagcg atgtggatat tggcgaacct 2280
gataccgagg gatatgctat cactgaccgg ggatctgctc ccatctctat ggggttcagg 2340
gcttctgatg ttgaaactgc agaaggaggg gagatccacg agctcctgag actccaatcc 2400
agaggcaaca actttccgaa gcttgggaaa actctcaatg ttcctccgcc cccggacccc 2460
ggtagggcca gcacttccgg gacacccatt aaaaagggca cagacgcgag attagcctca 2520
tttggaacgg agatcgcgtc tttattgaca ggtggtgcaa cccaatgtgc tcgaaagtca 2580
ccctcggaac catcagggcc aggtgcacct gcggggaatg tccccgagta tgtgagcaat 2640
gccgcactga tacaggagtg gacacccgaa tctggtacca caatctcccc gagatcccag 2700
aataatgaag aagggggaga ctattatgat gatgagctgt tctctgatgt ccaagatatt 2760
aaaacagcct tggccaaaat acacgaggat aatcagaaga taatctccaa gctagaatca 2820
ctgctgttat tgaagggaga agttgagtca attaagaagc agatcaacag gcaaaatatc 2880
agcatatcca ccctggaagg acacctctca agcatcatga tcgccattcc tggacttggg 2940
aaggatccca acgaccccac tgcagatgtc gaaatcaatc ccgacttgaa acccatcata 3000
ggcagagatt caggccgagc actggccgaa gttctcaaga aacccgttgc cagccgacaa 3060
ctccaaggaa tgacaaatgg acggaccagt tccagaggac agctgctgaa ggaatttcag 3120
ccaaagccga tcgggaaaaa gatgagctca gccgtcgggt ttgttcctga caccggccct 3180
gcatcacgca gtgtaatccg ctccattata aaatccagcc ggctagagga ggatcggaag 3240

cgttacctga tgactctcct tgatgatatc aaaggagcca atgatcttgc caagttccac 3300

cagatgctga tgaagataat aatgaagtag ctacagctca acttacctgc caaccccatg 3360

ccagtcgacc caactagtac aacctaaatc cattataaaa aacttaggag caaagtgatt 3420

gcctcccaag ttccacaatg acagagatct acgacttcga caagtcggca tgggacatca 3480

aagggtcgat cgctccgata caaccgacca cctacagtga tggcaggctg gtgccccagg 3540

tcagagtcat agatcctggt ctaggcgaca ggaaggatga atgctttatg tacatgtctc 3600

tgctgggggt tgttgaggac agcgatcccc tagggcctcc aatcgggcga gcatttgggt 3660

ccctgccctt aggtgttggc agatccacag caaagcccga aaaactcctc aaagaggcca 3720

ctgagcttga catagttgtt agacgtacag cagggctcaa tgaaaaactg gtgttctaca 3780

acaacacccc actaactctc ctcacacctt ggagaaaggt cctaacaaca gggagtgtct 3840

tcaacgcaaa ccaagtgtgc aatgcggtta atctgatacc gctcgatacc ccgcagaggt 3900

tccgtgttgt ttatatgagc atcacccgtc tttcggataa cgggtattac accgttccta 3960

gaagaatgct ggaattcaga tcggtcaatg cagtggcctt caacctgctg gtgaccctta 4020

ggattgacaa ggcgataggc cctgggaaga tcatcgacaa tacagagcaa cttcctgagg 4080

caacatttat ggtccacatc gggaacttca ggagaaagaa gagtgaagtc tactctgccg 4140

attattgcaa aatgaaaatc gaaaagatgg gcctggtttt tgcacttggt gggatagggg 4200

gcaccagtct tcacattaga agcacaggca aaatgagcaa gactctccat gcacaactcg 4260

ggttcaagaa gaccttatgt tacccgctga tggatatcaa tgaagacctt aatcgattac 4320

tctggaggag cagatgcaag atagtaagaa tccaggcagt tttgcagcca tcagttcctc 4380

aagaattccg catttacgac gacgtgatca taaatgatga ccaaggacta ttcaaagttc 4440

tgtagaccgt agtgcccagc aatgcccgaa aacgaccccc ctcacaatga cagccagaag 4500

gcccggacaa aaaagccccc tccgaaagac tccactgacc aagcgagagg ccagccagca 4560

gccgacggca agcacgaaca ccaggcggcc ccagcacaga acagccctga tacaaggcca 4620

ccaccagcca ccccaatctg catcctcctc gtgggacccc cgaggaccaa cccccaaggc 4680

tgcccccgat ccaaaccacc aaccgcatcc ccaccacccc cgggaaagaa acccccagca 4740

attggaaggc ccctccccct cttcctcaac acaagaactc cacaaccgaa ccgcacaagc 4800

gaccgaggtg acccaaccgc aggcatccga ctccctagac agatcctctc tccccggcaa 4860

actaaacaaa acttagggcc aaggaacata cacacccaac agaacccaga ccccggccca 4920

cggcgccgcg cccccaaccc ccgacaacca gagggagccc ccaaccaatc ccgccggctc 4980

ccccggtgcc cacaggcagg gacaccaacc cccgaacaga cccagcacct aaccatcgac 5040

aatccaagac gggggggccc ccccaaaaaa aggcccccag gggccgacag ccagcaccgc 5100

gaggaagccc acccacccca cacacgacca cggcaaccaa accagaaccc agaccaccct 5160

gggccaccag ctcccagact cggccatcac cccgcagaaa ggaaaggcca caacccgcgc 5220

accccagccc cgatccggcg gggagccacc caacccgaac cagcacccaa gagcgatccc 5280

cgaaggaccc ccgaaccgca aaggacatca gtatcccaca gcctctccaa gtcccccggt 5340

ctcctcctct tctcgaaggg accaaaagat caatccacca cacccgacga cactcaactc 5400

cccaccccta aaggagacac cgggaatccc agaatcaaga ctcatccaat gtccatcatg 5460

ggtctcaagg tgaacgtctc tgccatattc atggcagtac tgttaactct ccaaacaccc 5520

accggtcaaa tccattgggg caatctctct aagatagggg tggtaggaat aggaagtgca 5580

agctacaaag ttatgactcg ttccagccat caatcattag tcataaaatt aatgcccaat 5640

ataactctcc tcaataactg cacgagggta gagattgcag aatacaggag actactgaga 5700

acagttttgg aaccaattag agatgcactt aatgcaatga cccagaatat aagaccggtt 5760

cagagtgtag cttcaagtag gagacacaag agatttgcgg gagtagtcct ggcaggtgcg 5820

gccctaggcg ttgccacagc tgctcagata acagccggca ttgcacttca ccagtccatg 5880

ctgaactctc aagccatcga caatctgaga gcgagcctgg aaactactaa tcaggcaatt 5940

gaggcaatca gacaagcagg gcaggagatg atattggctg ttcagggtgt ccaagactac 6000

atcaataatg agctgatacc gtctatgaac caactatctt gtgatttaat cggccagaag 6060

ctcgggctca aattgctcag atactataca gaaatcctgt cattatttgg ccccagctta 6120

cgggacccca tatctgcgga gatatctatc caggctttga gctatgcgct tggaggagac 6180

atcaataagg tgttagaaaa gctcggatac agtggaggtg atttactggg catcttagag 6240

agcagaggaa taaaggcccg gataactcac gtcgacacag agtcctactt cattgtcctc 6300

agtatagcct atccgacgct gtccgagatt aagggggtga ttgtccaccg gctagagggg 6360

gtctcgtaca acataggctc tcaagagtgg tataccactg tgcccaagta tgttgcaacc 6420

caagggtacc ttatctcgaa ttttgatgag tcatcgtgta ctttcatgcc agaggggact 6480

gtgtgcagcc aaaatgcctt gtacccgatg agtcctctgc tccaagaatg cctccggggg 6540

tacaccaagt cctgtgctcg tacactcgta tccgggtctt ttgggaaccg gttcatttta 6600

tcacaaggga acctaatagc caattgtgca tcaatccttt gcaagtgtta cacaacagga 6660

acgatcatta atcaagaccc tgacaagatc ctaacataca ttgctgccga taactgcccg 6720

gtagtcgagg tgaacggcgt gaccatccaa gtcgggagca ggaggtatcc agacgctgtg 6780

tacttgcaca gaattgacct cggtcctccc atattattgg agaggttgga cgtagggaca 6840

aatctgggga atgcaattgc taagttggag gatgccaagg aattgttgga gtcatcggac 6900

cagatattga ggagtatgaa aggtttatcg agcacttgca tagtctacat cctgattgca 6960

gtgtgtcttg gagggttgat agggatcccc gctttaatat gttgctgcag ggggcgttgt 7020

aacaaaaagg gagaacaagt tggtatgtca agaccaggcc taaagcctga tcttacggga 7080

acatcaaaat cctatgtaag gtcgctctga tcctctacaa ctcttgaaac acaaatgtcc 7140

cacaagtctc ctcttcgtca tcaagcaacc accgcaccca gcatcaagcc cacctgaaat 7200

tatctccggc ttccctctgg ccgaacaata tcggtagtta attaaaactt agggtgcaag 7260

atcatccaca atgtcaccac aacgagaccg gataaatgcc ttctacaaag ataaccccca 7320

tcccaaggga agtaggatag tcattaacag agaacatctt atgattgata gaccttatgt 7380

tttgctggct gttctgtttg tcatgtttct gagcttgatc gggttgctag ccattgcagg 7440

cattagactt catcgggcag ccatctacac cgcagagatc cataaaagcc tcagcaccaa 7500

tctagatgta actaactcaa tcgagcatca ggtcaaggac gtgctgacac cactcttcaa 7560

aatcatcggt gatgaagtgg gcctgaggac acctcagaga ttcactgacc tagtgaaatt 7620

catctctgac aagattaaat tccttaatcc ggatagggag tacgacttca gagatctcac 7680

ttggtgtatc aacccgccag agagaatcaa attggattat gatcaatact gtgcagatgt 7740

ggctgctgaa gagctcatga atgcattggt gaactcaact ctactggaga ccagaacaac 7800

caatcagttc ctagctgtct caaagggaaa ctgctcaggg cccactacaa tcagaggtca 7860

attctcaaac atgtcgctgt ccctgttaga cttgtattta ggtcgaggtt acaatgtgtc 7920

atctatagtc actatgacat cccagggaat gtatggggga acttacctag tggaaaagcc 7980

taatctgagc agcaaaaggt cagagttgtc acaactgagc atgtaccgag tgtttgaagt 8040

aggtgttatc agaaatccgg gtttgggggc tccggtgttc catatgacaa actatcttga 8100

gcaaccagtc agtaatgatc tcagcaactg tatggtggct ttgggggagc tcaaactcgc 8160

agccctttgt caccgggaag attctatcac aattccctat cagggatcag ggaaaggtgt 8220

cagcttccag ctcgtcaagc taggtgtctg gaaatcccca accgacatgc aatcctgggt 8280

caccttatca acggatgatc cagtgataga caggctttac ctctcatctc acagaggtgt 8340

tatcgctgac aatcaagcaa aatgggctgt cccgacaaca cgaacagatg acaagttgcg 8400

aatggagaca tgcttccaac aggcgtgtaa gggtaaaatc caagcactct gcgagaatcc 8460

cgagtgggca ccattgaagg ataacaggat tccttcatac ggggtcttgt ctgttgatct 8520

gagtctgaca gttgagctta aaatcaaaat tgcttcggga ttcgggccat tgatcacaca 8580

cggttcaggg atggacctat acaaatccaa ccacaacaat gtgtattggc tgactatccc 8640

accaatgaag aacctagcct taggtgtaat caacacattg gagtggatac cgagattcaa 8700

ggttagtccc tacctcttca atgtcccaat taaggaagca ggcgaagact gccatgcccc 8760

aacataccta cctgcggagg tggatggtga tgtcaaactc agttccaatc tggtgattct 8820

acctggtcaa gatctccaat atgttttggc aacctacgat acttccaggg ttgaacatgc 8880

tgtggtttat tacgtttaca gcccaagccg ctcattttct tacttttatc cttttaggtt 8940

gcctataaag ggggtcccca tcgaattaca agtggaatgc ttcacatggg accaaaaact 9000

ctggtgccgt cacttctgtg tgcttgcgga ctcagaatct ggtggacata tcactcactc 9060

tgggatggtg ggcatgggag tcagctgcac agtcacccgg gaagatggaa ccaatcgcag 9120

atagggctgc tagtgaacta atctcatgat gtcacccaga catcaggcat acccactagt 9180

gtgaaataga catcagaatt aagaaaaacg tagggtccaa gtggttcccc gttatggact 9240

cgctatctgt caaccagatc ttataccctg aagttcacct agatagcccg atagttacca 9300

ataagatagt agccatcctg gagtatgctc gagtccctca cgcttacagc ctggaggacc 9360

ctacactgtg tcagaacatc aagcaccgcc taaaaaacgg attttccaac caaatgatta 9420

taaacaatgt ggaagttggg aatgtcatca agtccaagct taggagttat ccggcccact 9480

ctcatattcc atatccaaat tgtaatcagg atttatttaa catagaagac aaagagtcaa 9540

cgaggaagat ccgtgaactc ctcaaaaagg ggaattcgct gtactccaaa gtcagtgata 9600

aggttttcca atgcttaagg gacactaact cacggcttgg cctaggctcc gaattgaggg 9660

aggacatcaa ggagaaagtt attaacttgg gagtttacat gcacagctcc cagtggtttg 9720

agccctttct gttttggttt acagtcaaga ctgagatgag gtcagtgatt aaatcacaaa 9780

cccatacttg ccataggagg agacacacac ctgtattctt cactggtagt tcagttgagt 9840

tgctaatctc tcgtgacctt gttgctataa tcagtaaaga gtctcaacat gtatattacc 9900

tgacatttga actggttttg atgtattgtg atgtcataga ggggaggtta atgacagaga 9960

ccgctatgac tattgatgct aggtatacag agcttctagg aagagtcaga tacatgtgga 10020

aactgataga tggtttcttc cctgcactcg ggaatccaac ttatcaaatt gtagccatgc 10080

tggagcctct ttcacttgct tacctgcagc tgagggatat aacagtagaa ctcagaggtg 10140

ctttccttaa ccactgcttt actgaaatac atgatgttct tgaccaaaac gggttttctg 10200

atgaaggtac ttatcatgag ttaattgaag ctctagatta cattttcata actgatgaca 10260

tacatctgac aggggagatt ttctcatttt tcagaagttt cggccacccc agacttgaag 10320

cagtaacggc tgctgaaaat gttaggaaat acatgaatca gcctaaagtc attgtgtatg 10380

agactctgat gaaaggtcat gccatatttt gtggaatcat aatcaacggc tatcgtgaca 10440

ggcacggagg cagttggcca ccgctgaccc tccccctgca tgctgcagac acaatccgga 10500

atgctcaagc ttcaggtgaa gggttaacac atgagcagtg cgttgataac tggaaatctt 10560

ttgctggagt gaaatttggc tgctttatgc ctcttagcct ggatagtgat ctgacaatgt 10620

acctaaagga caaggcactt gctgctctcc aaagggaatg ggattcagtt tacccgaaag 10680

agttcctgcg ttacgaccct cccaagggaa ccgggtcacg gaggcttgta gatgttttcc 10740

ttaatgattc gagctttgac ccatatgatg tgataatgta tgttgtaagt ggagcttacc 10800

tccatgaccc tgagttcaac ctgtcttaca gcctgaaaga aaaggagatc aaggaaacag 10860

gtagactttt tgctaaaatg acttacaaaa tgagggcatg ccaagtgatt gctgaaaatc 10920

taatctcaaa cgggattggc aaatatttta aggacaatgg gatggccaag gatgagcacg 10980

atttgactaa ggcactccac actctagctg tctcaggagt ccccaaagat ctcaaagaaa 11040

gtcacagggg ggggccagtc ttaaaaacct actcccgaag cccagtccac acaagtacca 11100

ggaacgtgag agcagcaaaa gggtttatag ggttccctca agtaattcgg caggaccaag 11160

acactgatca tccggagaat atggaagctt acgagacagt cagtgcattt atcacgactg 11220

atctcaagaa gtactgcctt aattggagat atgagaccat cagcttgttt gcacagaggc 11280

taaatgagat ttacggattg ccctcatttt tccagtggct gcataagagg cttgagacct 11340

ctgtcctgta tgtaagtgac cctcattgcc cccccgacct tgacgcccat atcccgttat 11400

ataaagtccc caatgatcaa atcttcatta agtaccctat gggaggtata gaagggtatt 11460

gtcagaagct gtggaccatc agcaccattc cctatctata cctggctgct tatgagagcg 11520

gagtaaggat tgcttcgtta gtgcaagggg acaatcagac catagccgta acaaaaaggg 11580

tacccagcac atggccctac aaccttaaga aacgggaagc tgctagagta actagagatt 11640

actttgtaat tcttaggcaa aggctacatg atattggcca tcacctcaag gcaaatgaga 11700

caattgtttc atcacatttt tttgtctatt caaaaggaat atattatgat gggctacttg 11760

tgtcccaatc actcaagagc atcgcaagat gtgtattctg gtcagagact atagttgatg 11820

aaacaagggc agcatgcagt aatattgcta caacaatggc taaaagcatc gagagaggtt 11880

atgaccgtta ccttgcatat tccctgaacg tcctaaaagt gatacagcaa attctgatct 11940

ctcttggctt cacaatcaat tcaaccatga cccgggatgt agtcataccc ctcctcacaa 12000

acaacgacct cttaataagg atggcactgt tgcccgctcc tattgggggg atgaattatc 12060

tgaatatgag caggctgttt gtcagaaaca tcggtgatcc agtaacatca tcaattgctg 12120

atctcaagag aatgattctc gcctcactaa tgcctgaaga gaccctccat caagtaatga 12180

cacaacaacc gggggactct tcattcctag actgggctag cgacccttac tcagcaaatc 12240

ttgtatgtgt ccagagcatc actagactcc tcaagaacat aactgcaagg tttgtcctga 12300

tccatagtcc aaacccaatg ttaaaaggat tattccatga tgacagtaaa gaagaggacg 12360

agggactggc ggcattcctc atggacaggc atattatagt acctagggca gctcatgaaa 12420

tcctggatca tagtgtcaca ggggcaagag agtctattgc aggcatgctg gataccacaa 12480

aaggcctgat tcgagccagc atgaggaagg gggggttaac ctctcgagtg ataaccagat 12540

tgtccaatta tgactatgaa caattcagag cagggatggt gctattgaca ggaagaaaga 12600

gaaatgtcct cattgacaaa gagtcatgtt cagtgcagct ggcgagagct ctaagaagcc 12660

atatgtgggc gaggctagct cgaggacggc ctatttacgg ccttgaggtc cctgatgtac 12720

tagaatctat gcgaggccac cttattcggc gtcatgagac atgtgtcatc tgcgagtgtg 12780

gatcagtcaa ctacggatgg ttttttgtcc cctcgggttg ccaactggat gatattgaca 12840

aggaaacatc atccttgaga gtcccatata ttggttctac cactgatgag agaacagaca 12900

tgaagcttgc cttcgtaaga gccccaagtc gatccttgcg atctgctgtt agaatagcaa 12960

cagtgtactc atgggcttac ggtgatgatg atagctcttg gaacgaagcc tggttgttgg 13020

ctaggcaaag ggccaatgtg agcctggagg agctaagggt gatcactccc atctcaactt 13080

cgactaattt agcgcatagg ttgagggatc gtagcactca agtgaaatac tcaggtacat 13140

cccttgtccg agtggcgagg tataccacaa tctccaacga caatctctca tttgtcatat 13200

cagataagaa ggttgatact aactttatat accaacaagg aatgcttcta gggttgggtg 13260

ttttagaaac attgtttcga ctcgagaaag ataccggatc atctaacacg gtattacatc 13320

ttcacgtcga aacagattgt tgcgtgatcc cgatgataga tcatcccagg atacccagct 13380

cccgcaagct agagctgagg gcagagctat gtaccaaccc attgatatat gataatgcac 13440

ctttaattga cagagataca acaaggctat acacccagag ccataggagg caccttgtgg 13500

aatttgttac atggtccaca ccccaactat atcacatttt agctaagtcc acagcactat 13560

ctatgattga cctggtaaca aaatttgaga aggaccatat gaatgaaatt tcagctctca 13620

taggggatga cgatatcaat agtttcataa ctgagtttct gctcatagag ccaagattat 13680

tcactatcta cttgggccag tgtgcggcca tcaattgggc atttgatgta cattatcata 13740

gaccatcagg gaaatatcag atgggtgagc tgttgtcatc gttcctttct agaatgagca 13800

aaggagtgtt taaggtgctt gtcaatgctc taagccaccc aaagatctac aagaaattct 13860

ggcattgtgg tattatagag cctatccatg gtccttcact tgatgctcaa aacttgcaca 13920

caactgtgtg caacatggtt tacacatgct atatgaccta cctcgacctg ttgttgaatg 13980

aagagttaga agagttcaca tttctcttgt gtgaaagcga cgaggatgta gtaccggaca 14040

gattcgacaa catccaggca aaacacttat gtgttctggc agatttgtac tgtcaaccag 14100

gggcctgccc accaattcga ggtctaagac cggtagagaa atgtgcagtt ctaaccgacc 14160

atatcaaggc agaggctagg ttatctccag caggatcttc gtggaacata aatccaatta 14220

ttgtagacca ttactcatgc tctctgactt atctccggcg aggatcgatc aaacagataa 14280

gattgagagt tgatccagga ttcattttcg acgccctcgc tgaggtaaat gtcagtcagc 14340

caaagatcgg cagcaacaac atctcaaata tgagcatcaa ggctttcaga cccccacacg 14400

atgatgttgc aaaattgctc aaagatatca acacaagcaa gcacaatctt cccatttcag 14460

ggggcaatct cgccaattat gaaatccatg ctttccgcag aatcgggttg aactcatctg 14520

cttgctacaa agctgttgag atatcaacat taattaggag atgccttgag ccaggggagg 14580

acggcttgtt cttgggtgag ggatcgggtt ctatgttgat cacttataag gagatactta 14640

aactaaacaa gtgcttctat aatagtgggg tttccgccaa ttctagatct ggtcaaaggg 14700

aattagcacc ctatccctcc gaagttggcc ttgtcgaaca cagaatggga gtaggtaata 14760

ttgtcaaagt gctctttaac gggaggcccg aagtcacgtg ggtaggcagt gtagattgct 14820

tcaatttcat agttagtaat atccctacct ctagtgtggg gtttatccat tcagatatag 14880

agaccttgcc taacaaagat actatagaga agctagagga attggcagcc atcttatcga 14940

tggctctgct cctgggcaaa ataggatcaa tactggtgat taagcttatg cctttcagcg 15000

gggattttgt tcagggattt ataagttatg tagggtctta ttatagagaa gtgaaccttg 15060

tataccctag atacagcaac ttcatatcta ctgaatctta tttggttatg acagatctca 15120

aggctaaccg gctaatgaat cctgaaaaga ttaagcagca gataattgaa tcatctgtga 15180

ggacttcacc tggacttata ggtcacatcc tatccattaa gcaactaagc tgcatacaag 15240

caattgtggg agacgcagtt agtagaggtg atatcaatcc tactctgaaa aaacttacac 15300

ctatagagca ggtgctgatc aattgcgggt tggcaattaa cggacctaag ctgtgcaaag 15360

aattgatcca ccatgatgtt gcctcagggc aagatggatt gcttaattct atactcatcc 15420

tctacaggga gttggcaaga ttcaaagaca accgaagaag tcaacaaggg atgttccacg 15480

cttaccccgt attggtaagt agcaggcaac gagaacttat atctaggatc acccgcaaat 15540

tttgggggca cattcttctt tactccggga acagaaagtt gataaataag tttatccaga 15600

atctcaagtc cggctatctg atactagact tacaccagaa tatcttcgtt aagaatctat 15660

ccaagtcaga gaaacagatt attatgacgg ggggtttgaa acgtgagtgg gtttttaagg 15720

taacagtcaa ggagaccaaa gaatggtata agttagtcgg atacagtgcc ctgattaagg 15780

actaattgat tgaactccgg aaccctaatc ctgccctagg tggttaggca ttatttgcaa 15840

tatattaaag aaaactttga aaatacgaag tttctattcc cagctttgtc tggt 15894

<210> 59
<211> 15894
<212> DNA
<213> artificial sequence

<220>
<223> Synthetic sequence

<400> 59
accaaacaaa gttgggtaag gatagttcaa tcaatgatca ttttctagtg cacttaggat 60

tcaagatcct attatcaggg acaagagcag gattaaggat atccgagatg gccacacttt 120

taaggagctt agcattgttc aaaagaaaca aggacaaacc acccattaca tcaggatccg 180

gtggagccat cagaggaatc aaacacatta ttatagtacc aatccctgga gattcctcaa 240

ttaccactcg atccagactt ctggaccggt tggtcaggtt aattggaaac ccggatgtga 300

gcgggcccaa actaacaggg gcactaatag gtatattatc cttatttgtg gagtctccag 360

gtcaattgat tcagaggatc accgatgacc ctgacgttag cataaggctg ttagaggttg 420

tccagagtga ccagtcacaa tctggcctta ccttcgcatc aagaggtacc aacatggagg 480

atgaggcgga ccaatacttt tcacatgatg atccaattag tagtgatcaa tccaggttcg 540

gatggttcga gaacaaggaa atctcagata ttgaagtgca agaccctgag ggattcaaca 600

tgattctggg taccatccta gctcaaattt gggtcttgct cgcaaaggcg gttacggccc 660

cagacacggc agctgattcg gagctaagaa ggtggataaa gtacacccaa caaagaaggg 720

tagttggtga atttagattg gagagaaaat ggttggatgt ggtgaggaac aggattgccg 780

aggacctctc cttacgccga ttcatggtcg ctctaatcct ggatatcaag agaacacccg 840

gaaacaaacc caggattgct gaaatgatat gtgacattga tacatatatc gtagaggcag 900

gattagccag ttttatcctg actattaagt ttgggataga aactatgtat cctgctcttg 960

gactgcatga atttgctggt gagttatcca cacttgagtc cttgatgaac ctttaccagc 1020

aaatggggga aactgcaccc tacatggtaa tcctggagaa ctcaattcag aacaagttca 1080

gtgcaggatc ataccctctg ctctggagct atgccatggg agtaggagtg gaacttgaaa 1140

actccatggg aggtttgaac tttggccgat cttactttga tccagcatat tttagattag 1200

ggcaagagat ggtaaggagg tcagctggaa aggtcagttc cacattggca tctgaactcg 1260

gtatcactgc cgaggatgca aggcttgttt cagagattgc aatgcatact actgaggaca 1320

agatcagtag agcggttgga cccagacaag cccaagtatc atttctacac ggtgatcaaa 1380

gtgagaatga gctaccgaga ttggggggca aggaagatag gagggtcaaa cagagtcgag 1440

gagaagccag ggagagctac agagaaaccg ggcccagcag agcaagtgat gcgagagctg 1500

cccatcttcc aaccggcaca cccctagaca ttgacactgc atcggagtcc agccaagatc 1560

cgcaggacag tcgaaggtca gctgacgccc tgcttaggct gcaagccatg gcaggaatct 1620

cggaagaaca aggctcagac acggacaccc ctatagtgta caatgacaga aatcttctag 1680

actaggtgcg agaggccgag ggccagaaca acatccgcct accctccatc attgttataa 1740

aaaacttagg aaccaggtcc acacagccgc cagcccatca accatccact cccacgattg 1800

gagccgatgg cagaagagca ggcacgccat gtcaaaaacg gactggaatg catccgggct 1860

ctcaaggccg agcccatcgg ctcactggcc atcgaggaag ctatggcagc atggtcagaa 1920

atatcagaca acccaggaca ggagcgagcc acctgcaggg aagagaaggc aggcagttcg 1980

ggtctcagca aaccatgcct ctcagcaatt ggatcaactg aaggcggtgc acctcgcatc 2040

cgcggtcagg gacctggaga gagcgatgac gacgctgaaa ctttgggaat ccccccaaga 2100

aatctccagg catcaagcac tgggttacag tgttattatg tttatgatca cagcggtgaa 2160

gcggttaagg gaatccaaga tgctgactct atcatggttc aatcaggcct tgatggtgat 2220

agcaccctct caggaggaga caatgaatct gaaaacagcg atgtggatat tggcgaacct 2280

gataccgagg gatatgctat cactgaccgg ggatctgctc ccatctctat ggggttcagg 2340

gcttctgatg ttgaaactgc agaaggaggg gagatccacg agctcctgag actccaatcc 2400

agaggcaaca actttccgaa gcttgggaaa actctcaatg ttcctccgcc tccggacccc 2460

ggtagggcca gcacttccgg gacacccatt aaaaagggca cagacgcgag attagcctca 2520

tttggaacgg agatcgcgtc tttattgaca ggtggtgcaa cccaatgtgc tcgaaagtca 2580

ccctcggaac catcagggcc aggtgcacct gcggggaatg tccccgagtg tgtgagcaat 2640

gccgcactga tacaggagtg gacacccgaa tctggtacca caatctcccc gagatcccag 2700

aataatgaag aagggggaga ctattatgat gatgagctgt tctctgatgt ccaagatatt 2760

aaaacagcct tggccaaaat acacgaggat aatcagaaga taatctccaa gctagaatca 2820

ctgctgttat tgaagggaga agttgagtca attaagaagc agatcaacag gcaaaatatc 2880

agcatatcca ccctggaagg acacctctca agcatcatga tcgccattcc tggacttggg 2940

aaggatccca acgaccccac tgcagatgtc gaaatcaatc ccgacttgaa acccatcata 3000

ggcagagatt caggccgagc actggccgaa gttctcaaga aacccgttgc cagccgacaa 3060

ctccaaggaa tgacaaatgg acggaccagt tccagaggac agctgctgaa ggaatttcag 3120

ctaaagccga tcgggaaaaa gatgagctca gccgtcgggt ttgttcctga caccggccct 3180

gcatcacgca gtgtaatccg ctccattata aaatccagcc ggctagagga ggatcggaag 3240

cgttacctga tgactctcct tgatgatatc aaaggagcca atgatcttgc caagttccac 3300

cagatgctga tgaagataat aatgaagtag ctacagctca acttacctgc caaccccatg 3360

ccagtcgacc caactagtac aacctaaatc cattataaaa aacttaggag caaagtgatt 3420

gcctcccaag ttccacaatg acagagatct acgacttcga caagtcggca tgggacatca 3480

aagggttgat cgctccgata caacccacca cctacagtga tggcaggctg gtgccccagg 3540

tcagagtcat agatcctggt ctaggcgaca ggaaggatga atgctttatg tacatgtttc 3600

tgctgggggt tgttgaggac agcgatcccc tagggcctcc aatcgggcga gcatttgggt 3660

ccctgccctt aggtgttggc aaatccacag caaagcccga aaaactcctc aaagaggcca 3720

ctgagcttga catagttgtt agacgtacag cagggctcaa tgaaaaactg gtgttctaca 3780

acaacacccc actaactctc ctcacacctt ggagaaaggt cctaacaaca gggagtgtct 3840

tcaacgcaaa ccaagtgtgc agtgcggtta atctgatacc gctcgatacc ccgcagaggt 3900

tccgtgttgt ttatatgagc atcacccgtc tttcggataa cgggtattac accgttccta 3960

gaagaatgct ggaattcaga tcggtcaatg cagtggcctt caacctgctg gtgaccctta 4020

ggattgacaa ggcgataggc cctgggaaga tcatcgacaa tacagagcaa cttcctgagg 4080

caacatttat ggtccacatc gggaacttca ggagaaagaa gagtgaagtc tactctgccg 4140

attattgcaa aatgaaaatc gaaaagatgg gcctggtttt tgcacttggt gggatagggg 4200

gcaccagtct tcacattaga agcacaggca aaatgagcaa gactctccat gcacaactcg 4260

ggttcaagaa gaccttatgt tacccgctga tagatatcaa tgaagacctt aatcgattac 4320

tctggaggag cagatgcaag atagtaagaa tccaggcagt tttgcagcca tcagttcctc 4380

aagaattccg catttacgac gacgtgatca taaatgatga ccaaggacta ttcaaagttc 4440

tgtagaccgt agtgcccagc aatgcccgaa aacgaccccc ctcacaatga cagccagaag 4500

gcccggacaa aaaagccccc tccgaaagac tccacggacc aagcgagagg ccagccagca 4560

gccgacggca agcgcgaaca ccaggcggcc ccagcacaga acagccctga tacaaggcca 4620

ccaccagcca ccccaatctg catcctcctc gtgggacccc cgaggaccaa cccccaaggc 4680

tgcccccgat ccaaaccacc aaccgcatcc ccaccacccc cgggaaagaa acccccagca 4740

attggaaggc ccctccccct cttcctcaac acaagaactc cacaaccgaa ccgcacaagc 4800

gaccgaggtg acccaaccgc aggcatccga ctccctagac agatcctctc tccccggcaa 4860

actaaacaaa acttagggcc aaggaacata cacacccaac agaacccaga ccccggccca 4920

cggcgccgcg cccccaaccc ccgacaacca gagggagccc ccaaccaatc ccgccggctc 4980

ccccggtgcc cacaggcagg gacaccaacc cccgaacaga cccagcaccc aaccatcgac 5040

aatccaagac gggggggccc ccccaaaaaa aggcccccag gggccgacag ccagcaccgc 5100

gaggaagccc acccacccca cacacgacca cggcaaccaa accagaaccc agaccaccct 5160

gggccaccag ctcccagact cggccatcac cccgcagaaa ggaaaggcca caacccgcgc 5220

accccagccc cgatccggcg gggagccacc caacccgaac cagcacccaa gagcgatccc 5280

cgaaggaccc ccgaaccgca aaggacatca gtatcccaca gcctctccaa gtcccccggt 5340

ctcctcccct tctcgaaggg accaaaagat caatccacca cacccgacga cactcaactc 5400

cccaccccta aaggagacac cgggaatccc agaatcaaga ctcatccaat gtccatcatg 5460

ggtctcaagg tgaacgtctc tgccatattc atggcagtac tgttaactct ccaaacaccc 5520

accggtcaaa tccattgggg caatctctct aagatagggg tggtaggaat aggaagtgca 5580

agctacaaag ttatgactcg ttccagccat caatcattag tcataaaatt aatgcccaat 5640

ataactctcc tcaataactg cacgagggta gagattgcag aatacaggag actactgaga 5700

acagttttgg aaccaattag agatgcactt aatgcaatga cccagaatat aagaccggtt 5760

cagagtgtag cttcaagtag gagacacaag agatttgcgg gagtagtcct ggcaggtgcg 5820

gccctaggcg ttgccacagc tgctcagata acagccggca ttgcacttca ccagtccatg 5880

ctgaactctc aagccatcga caatctgaga gcgagcctgg aaactactaa tcaggcaatt 5940

gaggcaatca gacaagcagg gcaggagatg atattggctg ttcagggtgt ccaagactac 6000

atcaataatg agctgatacc gtctatgaac caactatctt gtgatttaat cggccagaag 6060

ctcgggctca aattgctcag atactataca gaaatcctgt cattatttgg ccccagctta 6120

cgggacccca tatctgcgga gatatctatc caggctttga gctatgcgct tggaggagac 6180

atcaataagg tgttagaaaa gctcggatac agtggaggtg atttactggg catcttagag 6240

agcagaggaa taaaggcccg gataactcac gtcgacacag agtcctactt cattgtcctc 6300

agtatagcct atccgacgct gtccgagatt aagggggtga ttgtccaccg gctagagggg 6360

gtctcgtaca acataggctc tcaagagtgg tataccactg tgcccaagta tgttgcaacc 6420

caagggtacc ttatctcgaa ttttgatgag tcatcgtgta ctttcatgcc agaggggact 6480

gtgtgcagcc aaaatgcctt gtacccgatg agtcctctgc tccaagaatg cctccggggg 6540

tccaccaagt cctgtgctcg tacactcgta tccgggtctt ttgggaaccg gttcatttta 6600

tcacaaggga acctaatagc caattgtgca tcaatccttt gcaagtgtta cacaacagga 6660

acgatcatta atcaagaccc tgacaagatc ctaacataca ttgctgccga tcactgcccg 6720

gtagtcgagg tgaacggcgt gaccatccaa gtcgggagca ggaggtatcc agatgctgtg 6780

tacttgcaca gaattgacct cggtcctccc atatcattgg agaggttgga cgtagggaca 6840

aatctgggga atgcaattgc taagttggag gatgccaagg aattgttgga gtcatcggac 6900

cagatattga ggagtatgaa aggtttatcg agcactagca tagtctacat cctgattgca 6960

gtgtgtcttg gagggttgat agggatcccc gctttaatat gttgctgcag ggggcgttgt 7020

aacaaaaagg gagaacaagt tggtatgtca agaccaggcc taaagcctga tcttacggga 7080

acatcaaaat cctatgtaag gtcgctctga tcctctacaa ctcttgaaac acaaatgtcc 7140

cacaagtctc ctcttcgtca tcaagcaacc accgcaccca gcatcaagcc cacctgaaat 7200

tatctccggc ttccctctgg ccgaacaata tcggtagtta attaaaactt agggtgcaag 7260

atcatccaca atgtcaccac aacgagaccg gataaatgcc ttctacaaag ataaccccca 7320

tcccaaggga agtaggatag tcattaacag agaacatctt atgattgata gaccttatgt 7380

tttgctggct gttctgtttg tcatgtttct gagcttgatc gggttgctag ccattgcagg 7440

cattagactt catcgggcag ccatctacac cgcagagatc cataaaagcc tcagcaccaa 7500

tctagatgta actaactcaa tcgagcatca ggtcaaggac gtgctgacac cactcttcaa 7560

aatcatcggt gatgaagtgg gcctgaggac acctcagaga ttcactgacc tagtgaaatt 7620

catctctgac aagattaaat tccttaatcc ggatagggag tacgacttca gagatctcac 7680

ttggtgtatc aacccgccag agagaatcaa attggattat gatcaatact gtgcagatgt 7740

ggctgctgaa gagctcatga atgcattggt gaactcaact ctactggaga ccagaacaac 7800

caatcagttc ctagctgtct caaagggaaa ctgctcaggg cccactacaa tcagaggtca 7860

attctcaaac atgtcgctgt ccctgttaga cttgtattta ggtcgaggtt acaatgtgtc 7920

atctatagtc actatgacat cccagggaat gtatggggga acttacctag tggaaaagcc 7980

taatctgagc agcaaaaggt cagagttgtc acaactgagc atgtaccgag tgtttgaagt 8040

aggtgttatc agaaatccgg gtttgggggc tccggtgttc catatgacaa actatcttga 8100

gcaaccagcc agtaatgatc tcagcaactg tatggtggct ttgggggagc tcaaactcgc 8160

agccctttgt cacggggaag attctatcac aattccctat cagggatcag ggaaaggtgt 8220

cagcttccag ctcgtcaagc taggtgtctg gaaatcccca accgacatgc aatcctgggt 8280

ccccttatca acggatgatc cagtgataga caggctttac ctctcatctc acagaggtgt 8340

tatcgctgac aatcaagcaa aatgggctgt cccgacaaca cgaacagatg acaagttgcg 8400

aatggagaca tgcttccaac aggcgtgtaa gggtaaaatc caagcactct gcgagaatcc 8460

cgagtgggca ccattgaagg ataacaggat tccttcatac ggggtcttgt ctgttgatct 8520

gagtctgaca gttgagctta aaatcaaaat tgcttcggga ttcgggccat tgatcacaca 8580

cggttcaggg atggacctat acaaatccaa ccacaacaat gtgtattggc tgactatccc 8640

gccaatgaag aacctagcct taggtgtaat caacacattg gagtggatac cgagattcaa 8700

ggttagtccc tacctcttca atgtcccaat taaggaagca ggcgaagact gccatgcccc 8760

aacataccta cctgcggagg tggatggtga tgtcaaactc agttccaatc tggtgattct 8820

acctggtcaa gatctccaat atgttttggc aacctacgat acttccaggg ttgaacatgc 8880

tgtggtttat tacgtttaca gcccaggccg ctcattttct tacttttatc cttttaggtt 8940

gcctataaag ggggtcccca tcgaattaca agtggaatgc ttcacatggg accaaaaact 9000

ctggtgccgt cacttctgtg tgcttgcgga ctcagaatct ggtggacata tcactcactc 9060

tgggatggtg ggcatgggag tcagctgcac agtcacccgg gaagatggaa ccaatcgcag 9120

atagggctgc tagtgaacca atctcatgat gtcacccaga catcaggcat acccactagt 9180

gtgaaataga catcagaatt aagaaaaacg tagggtccaa gtggttcccc gttatggact 9240

cgctatctgt caaccagatc ttataccctg aagttcacct agatagcccg atagttacca 9300

ataagatagt agccatcctg gagtatgctc gagtccctca cgcttacagc ctggaggacc 9360

ctacactgtg tcagaacatc aagcaccgcc taaaaaacgg attttccaac caaatgatta 9420

taaacaatgt ggaagttggg aatgtcatca agtccaagct taggagttat ccggcccact 9480

ctcatattcc atatccaaat tgtaatcagg atttatttaa catagaagac aaagagtcaa 9540

cgaggaagat ccgtgaactc ctcaaaaagg ggaattcgct gtactccaaa gtcagtgata 9600

aggttttcca atgcttaagg gacactaact cacggcttgg cctaggctcc gaattgaggg 9660

aggacatcaa ggagaaagtt attaacttgg gagtttacat gcacagctcc cagtggtttg 9720

agccctttct gttttggttt acagtcaaga ctgagatgag gtcagtgatt aaatcacaaa 9780

cccatacttg ccataggagg agacacacac ctgtattctt cactggtagt tcagttgagt 9840

tgctaatctc tcgtgacctt gttgctataa tcagtaaaga gtctcaacat gtatattacc 9900

tgacatttga actggttttg atgtattgtg atgtcataga ggggaggtta atgacagaga 9960

ccgctatgac tattgatgct aggtatacag agcttctagg aagagtcaga tacatgtgga 10020

aactgataga tggtttcttc cctgcactcg ggaatccaac ttatcaaatt gtagccatgc 10080

tggagcctct ttcacttgct tacctgcagc tgagggatat aacagtagaa ctcagaggtg 10140

ctttccttaa ccactgcttt actgaaatac atgatgttct tgaccaaaac gggttttctg 10200

atgaaggtac ttatcatgag ttaattgaag ctctagatta cattttcata actgatgaca 10260

tacatctgac aggggagatt ttctcatttt tcagaagttt cggccacccc agacttgaag 10320

cagtaacggc tgctgaaaat gttaggaaat acatgaatca gcctaaagtc attgtgtatg 10380

agactctgat gaaaggtcat gccatatttt gtggaatcat aatcaacggc tatcgtgaca 10440

ggcacggagg cagttggcca ccgctgaccc tccccctgca tgctgcagac acaatccgga 10500

atgctcaagc ttcaggtgaa gggttaacac atgagcagtg cgttgataac tggaaatctt 10560

ttgctggagt gaaatttggc tgctttatgc ctcttagcct ggatagtgat ctgacaatgt 10620

acctaaagga caaggcactt gctgctctcc aaagggaatg ggattcagtt tacccgaaag 10680

agttcctgcg ttacgaccct cccaagggaa ccgggtcacg gaggcttgta gatgttttcc 10740

ttaatgattc gagctttgac ccatatgatg tgataatgta tgttgtaagt ggagcttacc 10800

tccatgaccc tgagttcaac ctgtcttaca gcctgaaaga aaaggagatc aaggaaacag 10860

gtagactttt tgctaaaatg acttacaaaa tgagggcatg ccaagtgatt gctgaaaatc 10920

taatctcaaa cgggattggc aaatatttta aggacaatgg gatggccaag gatgagcacg 10980

atttgactaa ggcactccac actctagctg tctcaggagt ccccaaagat ctcaaagaaa 11040

gtcacagggg ggggccagtc ttaaaaacct actcccgaag cccagtccac acaagtacca 11100

ggaacgtgag agcagcaaaa gggtttatag ggttccctca agtaattcgg caggaccaag 11160

acactgatca tccggagaat atggaagctt acgagacagt cagtgcattt atcacgactg 11220

atctcaagaa gtactgcctt aattggagat atgagaccat cagcttgttt gcacagaggc 11280

taaatgagat ttacggattg ccctcatttt tccagtggct gcataagagg cttgagacct 11340

ctgtcctgta tgtaagtgac cctcattgcc cccccgacct tgacgcccat atcccgttat 11400

ataaagtccc caatgatcaa atcttcatta agtaccctat gggaggtata gaagggtatt 11460

gtcagaagct gtggaccatc agcaccattc cctatctata cctggctgct tatgagagcg 11520

gagtaaggat tgcttcgtta gtgcaagggg acaatcagac catagccgta acaaaaaggg 11580

tacccagcac atggccctac aaccttaaga aacgggaagc tgctagagta actagagatt 11640

actttgtaat tcttaggcaa aggctacatg atattggcca tcacctcaag gcaaatgaga 11700
caattgtttc atcacatttt tttgtctatt caaaaggaat atattatgat gggctacttg 11760
tgtcccaatc actcaagagc atcgcaagat gtgtattctg gtcagagact atagttgatg 11820
aaacaagggc agcatgcagt aatattgcta caacaatggc taaaagcatc gagagaggtt 11880
atgaccgtta ccttgcatat tccctgaacg tcctaaaagt gatacagcaa attctgatct 11940
ctcttggctt cacaatcaat tcaaccatga cccgggatgt agtcataccc ctcctcacaa 12000
acaacgacct cttaataagg atggcactgt tgcccgctcc tattgggggg atgaattatc 12060
tgaatatgag caggctgttt gtcagaaaca tcggtgatcc agtaacatca tcaattgctg 12120
atctcaagag aatgattctc gcctcactaa tgcctgaaga gaccctccat caagtaatga 12180
cacaacaacc ggggactctt tcattcctag actgggctag cgacccttac tcagcaaatc 12240
ttgtatgtgt ccagagcatc actagactcc tcaagaacat aactgcaagg tttgtcctga 12300
tccatagtcc aaacccaatg ttaaaaggat tattccatga tgacagtaaa gaagaggacg 12360
agggactggc ggcattcctc atggacaggc atattatagt acctagggca gctcatgaaa 12420
tcctggatca tagtgtcaca ggggcaagag agtctattgc aggcatgctg gataccacaa 12480
aaggcctgat tcgagccagc atgaggaagg gggggttaac ctctcgagtg ataaccagat 12540
tgtccaatta tgactatgaa caattcagag cagggatggt gctattgaca ggaagaaaga 12600
gaaatgtcct cattgacaaa gagtcatgtt cagtgcagct ggcgagagct ctaagaagcc 12660
atatgtgggc gaggctagct cgaggacggc ctatttacgg ccttgaggtc cctgatgtac 12720
tagaatctat gcgaggccac cttattcggc gtcatgagac atgtgtcatc tgcgagtgtg 12780
gatcagtcaa ctacggatgg ttttttgtcc cctcgggttg ccaactggat gatattgaca 12840
aggaaacatc atccttgaga gtcccatata ttggttctac cactgatgag agaacagaca 12900
tgaagcttgc cttcgtaaga gccccaagtc gatccttgcg atctgctgtt agaatagcaa 12960
cagtgtactc atgggcttac ggtgatgatg atagctcttg gaacgaagcc tggttgttgg 13020
ctaggcaaag ggccaatgtg agcctggagg agctaagggt gatcactccc atctcaactt 13080
cgactaattt agcgcatagg ttgagggatc gtagcactca agtgaaatac tcaggtacat 13140
cccttgtccg agtggcgagg tataccacaa tctccaacga caatctctca tttgtcatat 13200
cagataagaa ggttgatact aactttatat accaacaagg aatgcttcta gggttgggtg 13260
ttttagaaac attgtttcga ctcgagaaag ataccggatc atctaacacg gtattacatc 13320
ttcacgtcga aacagattgt tgcgtgatcc cgatgataga tcatcccagg atacccagct 13380
cccgcaagct agagctgagg gcagagctat gtaccaaccc attgatatat gataatgcac 13440
ctttaattga cagagataca acaaggctat acacccagag ccataggagg caccttgtgg 13500

aatttgttac atggtccaca ccccaactat atcacatttt agctaagtcc acagcactat 13560

ctatgattga cctggtaaca aaatttgaga aggaccatat gaatgaaatt tcagctctca 13620

taggggatga cgatatcaat agtttcataa ctgagtttct gctcatagag ccaagattat 13680

tcactatcta cttgggccag tgtgcggcca tcaattgggc atttgatgta cattatcata 13740

gaccatcagg gaaatatcag atgggtgagc tgttgtcatc gttcctttct agaatgagca 13800

aaggagtgtt taaggtgctt gtcaatgctc taagccaccc aaagatctac aagaaattct 13860

ggcattgtgg tattatagag cctatccatg gtccttcact tgatgctcaa aacttgcaca 13920

caactgtgtg caacatggtt tacacatgct atatgaccta cctcgacctg ttgttgaatg 13980

aagagttaga agagttcaca tttctcttgt gtgaaagcga cgaggatgta gtaccggaca 14040

gattcgacaa catccaggca aaacacttat gtgttctggc agatttgtac tgtcaaccag 14100

ggacctgccc accaattcga ggtctaagac cggtagagaa atgtgcagtt ctaaccgacc 14160

atatcaaggc agaggctagg ttatctccag caggatcttc gtggaacata aatccaatta 14220

ttgtagacca ttactcatgc tctctgactt atctccggcg aggatcgatc aaacagataa 14280

gattgagagt tgatccagga ttcattttcg acgccctcgc tgaggtaaat gtcagtcagc 14340

caaagatcgg cagcaacaac atctcaaata tgagcatcaa ggctttcaga cccccacacg 14400

atgatgttgc aaaattgctc aaagatatca acacaagcaa gcacaatctt cccatttcag 14460

ggggcaatct cgccaattat gaaatccatg ctttccgcag aatcgggttg aactcatctg 14520

cttgctacaa agctgttgag atatcaacat taattaggag atgccttgag ccaggggagg 14580

acggcttgtt cttgggtgag ggatcgggtt ctatgttgat cacttataag gagatactta 14640

aactaaacaa gtgcttctat aatagtgggg tttccgccaa ttctagatct ggtcaaaggg 14700

aattagcacc ctatccctcc gaagttggcc ttgtcgaaca cagaatggga gtaggtaata 14760

ttgtcaaagt gctctttaac gggaggcccg aagtcacgtg ggtaggcagt gtagattgct 14820

tcaatttcat agttagtaat atccctacct ctagtgtggg gtttatccat tcagatatag 14880

agaccttgcc taacaaagat actatagaga agctagagga attggcagcc atcttatcga 14940

tggctctgct cctgggcaaa ataggatcaa tactggtgat taagcttatg cctttcagcg 15000

gggattttgt tcagggattt ataagttatg tagggtccca ttatagagaa gtgaaccttg 15060

tataccctag atacagcaac ttcatatcta ctgaatctta tttggttatg acagatctca 15120

aggctaaccg gctaatgaat cctgaaaaga ttaagcagca gataattgaa tcatctgtga 15180

ggacttcacc tggacttata ggtcacatcc tatccattaa gcaactaagc tgcatacaag 15240

caattgtggg agacgcagtt agtagaggtg atatcaatcc tactctgaaa aaacttacac 15300

ctatagagca ggtgctgatc aattgcgggt tggcaattaa cggacctaag ctgtgcaaag 15360

aattgatcca ccatgatgtt gcctcagggc aagatggatt gcttaattct atactcatcc 15420

tctacaggga gttggcaaga ttcaaagaca accaaagaag tcaacaaggg atgttccacg 15480

cttaccccgt attggtaagt agcaggcaac gagaacttat atctaggatc acccgcaaat 15540

tttgggggca cattcttctt tactccggga acagaaagtt gataaataag tttatccaga 15600

atctcaagtc cggctatctg atactagact tacaccagaa tatcttcgtt aagaatctat 15660

ccaagtcaga gaaacagatt attatgacgg ggggtttgaa acgtgagtgg gtttttaagg 15720

taacagtcaa ggagaccaaa gaatggtata agttagtcgg atacagtgcc ctgattaagg 15780

actaattggt tgaactccgg aaccctaatc ctgccctagg tggttaggca ttatttgcaa 15840

tagattaaag aaaactttga aaatacgaag tttctattcc cagctttgtc tggt 15894

<210> 60
<211> 24061
<212> DNA
<213> artificial sequence

<220>
<223> Synthetic sequence

<400> 60
gcggccgcta atacgactca ctatagggcc aactttgttt ggtctgatga gtccgtgagg 60

acgaaacccg gagtcccggg tcaccaaaca aagttgggta aggatagttc aatcaatgat 120

catcttctag tgcacttagg attcaagatc ctattatcag ggacaagagc aggattaggg 180

atatccgaga tggccacact tttaaggagc ttagcattgt tcaaaagaaa caaggacaaa 240

ccacccatta catcaggatc cggtggagcc atcagaggaa tcaaacacat tattatagta 300

ccaatccctg gagattcctc aattaccact cgatccagac ttctggaccg gttggtcagg 360

ttaattggaa acccggatgt gagcgggccc aaactaacag gggcactaat aggtatatta 420

tccttatttg tggagtctcc aggtcaattg attcagagga tcaccgatga ccctgacgtt 480

agcataaggc tgttagaggt tgtccagagt gaccagtcac aatctggcct taccttcgca 540

tcaagaggta ccaacatgga ggatgaggcg gacaaatact tttcacatga tgatccaatt 600

agtagtgatc aatccaggtt cggatggttc gagaacaagg aaatctcaga tattgaagtg 660

caagaccctg agggattcaa catgattctg ggtaccatcc tagcccaaat ttgggtcttg 720

ctcgcaaagg cggttacggc cccagacacg gcagctgatt cggagctaag aaggtggata 780

aagtacaccc aacaaagaag ggtagttggt gaatttagat tggagagaaa atggttggat 840

gtggtgagga acaggattgc cgaggacctc tccttacgcc gattcatggt cgctctaatc 900

ctggatatca agagaacacc cggaaacaaa cccaggattg ctgaaatgat atgtgacatt 960

gatacatata tcgtagaggc aggattagcc agttttatcc tgactattaa gtttgggata 1020

gaaactatgt atcctgctct tggactgcat gaatttgctg gtgagttatc cacacttgag 1080

tccttgatga acctttacca gcaaatgggg gaaactgcac cctacatggt aatcctggag 1140

aactcaattc agaacaagtt cagtgcagga tcataccctc tgctctggag ctatgccatg 1200

ggagtaggag tggaacttga aaactccatg ggaggtttga actttggccg atcttacttt 1260

gatccagcat attttagatt agggcaagag atggtaagga ggtcagctgg aaaggtcagt 1320

tccacattgg catctgaact cggtatcact gccgaggatg caaggcttgt ttcagagatt 1380

gcaatgcata ctactgagga caagatcagt agagcggttg gacccagaca agcccaagta 1440

tcatttctac acggtgatca aagtgagaat gagctaccga gattgggggg caaggaagat 1500

aggagggtca aacagagtcg aggagaagcc agggagagct acagagaaac cgggcccagc 1560

agagcaagtg atgcgagagc tgcccatctt ccaaccggca cacccctaga cattgacact 1620

gcatcggagt ccagccaaga tccgcaggac agtcgaaggt cagctgacgc cctgcttagg 1680

ctgcaagcca tggcaggaat ctcggaagaa caaggctcag acacggacac ccctatagtg 1740

tacaatgaca gaaatcttct agactaggtg cgagaggccg aggaccagaa caacatccgc 1800

ctaccctcca tcattgttat aaaaaactta ggaaccaggt ccacacagcc gccagcccat 1860

caaccatcca ctcccacgat tggagccgat ggcagaagag caggcacgcc atgtcaaaaa 1920

cggactggaa tgcatccggg ctctcaaggc cgagcccatc ggctcactgg ccatcgagga 1980

agctatggca gcatggtcag aaatatcaga caacccagga caggagcgag ccacctgcag 2040

ggaagagaag gcaggcagtt cgggtctcag caaaccatgc ctctcagcaa ttggatcaac 2100

tgaaggcggt gcacctcgca tccgcggtca gggacctgga gagagcgatg acgacgctga 2160

aactttggga atccccccaa gaaatctcca ggcatcaagc actgggttac agtgttatta 2220

tgtttatgat cacagcggtg aagcggttaa gggaatccaa gatgctgact ctatcatggt 2280

tcaatcaggc cttgatggtg atagcaccct atcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cccccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tatgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agccaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaactagt acaacctaaa tccattataa 3480

aaaacttagg agcaaagtga ttgcctccca agttccacaa tgacagagat ctacgacttc 3540

gacaagtcgg catgggacat caaagggtcg atcgctccga tacaaccgac cacctacagt 3600

gatggcaggc tggtgcccca ggtcagagtc atagatcctg gtctaggcga caggaaggat 3660

gaatgcttta tgtacatgtc tctgctgggg gttgttgagg acagcgatcc cctagggcct 3720

ccaatcgggc gagcatttgg gtccctgccc ttaggtgttg gcagatccac agcaaagccc 3780

gaaaaactcc tcaaagaggc cactgagctt gacatagttg ttagacgtac agcagggctc 3840

aatgaaaaac tggtgttcta caacaacacc ccactaactc tcctcacacc ttggagaaag 3900

gtcctaacaa cagggagtgt cttcaacgca aaccaagtgt gcaatgcggt taatctgata 3960

ccgctcgata ccccgcagag gttccgtgtt gtttatatga gcatcacccg tctttcggat 4020

aacgggtatt acaccgttcc tagaagaatg ctggaattca gatcggtcaa tgcagtggcc 4080

ttcaacctgc tggtgaccct taggattgac aaggcgatag gccctgggaa gatcatcgac 4140

aatacagagc aacttcctga ggcaacattt atggtccaca tcgggaactt caggagaaag 4200

aagagtgaag tctactctgc cgattattgc aaaatgaaaa tcgaaaagat gggcctggtt 4260

tttgcacttg gtgggatagg gggcaccagt cttcacatta gaagcacagg caaaatgagc 4320

aagactctcc atgcacaact cgggttcaag aagaccttat gttacccgct gatggatatc 4380

aatgaagacc ttaatcgatt actctggagg agcagatgca agatagtaag aatccaggca 4440

gttttgcagc catcagttcc tcaagaattc cgcatttacg acgacgtgat cataaatgat 4500

gaccaaggac tattcaaagt tctgtagacc gtagtgccca gcaatgcccg aaaacgaccc 4560

ccctcacaat gacagccaga aggcccggac aaaaaagccc cctccgaaag actccactga 4620

ccaagcgaga ggccagccag cagccgacgg caagcacgaa caccaggcgg ccccagcaca 4680

gaacagccct gatacaaggc caccaccagc caccccaatc tgcatcctcc tcgtgggacc 4740

cccgaggacc aacccccaag gctgcccccg atccaaacca ccaaccgcat ccccaccacc 4800

cccgggaaag aaacccccag caattggaag gcccctcccc ctcttcctca acacaagaac 4860

tccacaaccg aaccgcacaa gcgaccgagg tgacccaacc gcaggcatcc gactccctag 4920

acagatcctc tctccccggc aaactaaaca aaacttaggg ccaaggaaca tacacaccca 4980

acagaaccca gaccccggcc cacggcgccg cgcccccaac ccccgacaac cagagggagc 5040

ccccaaccaa tcccgccggc tcccccggtg cccacaggca gggacaccaa cccccgaaca 5100

gacccagcac ctaaccatcg acaatccaag acgggggggc ccccccaaaa aaaggccccc 5160

aggggccgac agccagcacc gcgaggaagc ccacccaccc cacacacgac cacggcaacc 5220

aaaccagaac ccagaccacc ctgggccacc agctcccaga ctcggccatc accccgcaga 5280

aaggaaaggc cacaacccgc gcaccccagc cccgatccgg cggggagcca cccaacccga 5340

accagcaccc aagagcgatc cccgaaggac ccccgaaccg caaaggacat cagtatccca 5400

cagcctctcc aagtcccccg gtctcctcct cttctcgaag ggaccaaaag atcaatccac 5460

cacacccgac gacactcaac tccccacccc taaaggagac accgggaatc ccagaatcaa 5520

gactcatcca atgtccatca tgggtctcaa ggtgaacgtc tctgccatat tcatggcagt 5580

actgttaact ctccaaacac ccaccggtca aatccattgg ggcaatctct ctaagatagg 5640

ggtggtagga ataggaagtg caagctacaa agttatgact cgttccagcc atcaatcatt 5700

agtcataaaa ttaatgccca atataactct cctcaataac tgcacgaggg tagagattgc 5760

agaatacagg agactactga gaacagtttt ggaaccaatt agagatgcac ttaatgcaat 5820

gacccagaat ataagaccgg ttcagagtgt agcttcaagt aggagacaca agagatttgc 5880

gggagtagtc ctggcaggtg cggccctagg cgttgccaca gctgctcaga taacagccgg 5940

cattgcactt caccagtcca tgctgaactc tcaagccatc gacaatctga gagcgagcct 6000

ggaaactact aatcaggcaa ttgaggcaat cagacaagca gggcaggaga tgatattggc 6060

tgttcagggt gtccaagact acatcaataa tgagctgata ccgtctatga accaactatc 6120

ttgtgattta atcggccaga agctcgggct caaattgctc agatactata cagaaatcct 6180

gtcattattt ggccccagct tacgggaccc catatctgcg gagatatcta tccaggcttt 6240

gagctatgcg cttggaggag acatcaataa ggtgttagaa aagctcggat acagtggagg 6300

tgatttactg ggcatcttag agagcagagg aataaaggcc cggataactc acgtcgacac 6360

agagtcctac ttcattgtcc tcagtatagc ctatccgacg ctgtccgaga ttaagggggt 6420

gattgtccac cggctagagg gggtctcgta caacataggc tctcaagagt ggtataccac 6480

tgtgcccaag tatgttgcaa cccaagggta ccttatctcg aattttgatg agtcatcgtg 6540

tactttcatg ccagagggga ctgtgtgcag ccaaaatgcc ttgtacccga tgagtcctct 6600

gctccaagaa tgcctccggg ggtacaccaa gtcctgtgct cgtacactcg tatccgggtc 6660

ttttgggaac cggttcattt tatcacaagg gaacctaata gccaattgtg catcaatcct 6720

ttgcaagtgt tacacaacag gaacgatcat taatcaagac cctgacaaga tcctaacata 6780

cattgctgcc gataactgcc cggtagtcga ggtgaacggc gtgaccatcc aagtcgggag 6840

caggaggtat ccagacgctg tgtacttgca cagaattgac ctcggtcctc ccatattatt 6900

ggagaggttg gacgtaggga caaatctggg gaatgcaatt gctaagttgg aggatgccaa 6960

ggaattgttg gagtcatcgg accagatatt gaggagtatg aaaggtttat cgagcacttg 7020

catagtctac atcctgattg cagtgtgtct tggagggttg atagggatcc ccgctttaat 7080

atgttgctgc agggggcgtt gtaacaaaaa gggagaacaa gttggtatgt caagaccagg 7140

cctaaagcct gatcttacgg gaacatcaaa atcctatgta aggtcgctct gatcctctac 7200

aactcttgaa acacaaatgt cccacaagtc tcctcttcgt catcaagcaa ccaccgcacc 7260

cagcatcaag cccacctgaa attatctccg gcttccctct ggccgaacaa tatcggtagt 7320

taattaaaac ttagggtgca agatcatcca caatgtcacc acaacgagac cggataaatg 7380

ccttctacaa agataacccc catcccaagg gaagtaggat agtcattaac agagaacatc 7440

ttatgattga tagaccttat gttttgctgg ctgttctgtt tgtcatgttt ctgagcttga 7500

tcgggttgct agccattgca ggcattagac ttcatcgggc agccatctac accgcagaga 7560

tccataaaag cctcagcacc aatctagatg taactaactc aatcgagcat caggtcaagg 7620

acgtgctgac accactcttc aaaatcatcg gtgatgaagt gggcctgagg acacctcaga 7680

gattcactga cctagtgaaa ttcatctctg acaagattaa attccttaat ccggataggg 7740

agtacgactt cagagatctc acttggtgta tcaacccgcc agagagaatc aaattggatt 7800

atgatcaata ctgtgcagat gtggctgctg aagagctcat gaatgcattg gtgaactcaa 7860

ctctactgga gaccagaaca accaatcagt tcctagctgt ctcaaaggga aactgctcag 7920

ggcccactac aatcagaggt caattctcaa acatgtcgct gtccctgtta gacttgtatt 7980

taggtcgagg ttacaatgtg tcatctatag tcactatgac atcccaggga atgtatgggg 8040

gaacttacct agtggaaaag cctaatctga gcagcaaaag gtcagagttg tcacaactga 8100

gcatgtaccg agtgtttgaa gtaggtgtta tcagaaatcc gggtttgggg gctccggtgt 8160

tccatatgac aaactatctt gagcaaccag tcagtaatga tctcagcaac tgtatggtgg 8220

ctttggggga gctcaaactc gcagcccttt gtcaccggga agattctatc acaattccct 8280

atcagggatc agggaaaggt gtcagcttcc agctcgtcaa gctaggtgtc tggaaatccc 8340

caaccgacat gcaatcctgg gtcaccttat caacggatga tccagtgata gacaggcttt 8400

acctctcatc tcacagaggt gttatcgctg acaatcaagc aaaatgggct gtcccgacaa 8460

cacgaacaga tgacaagttg cgaatggaga catgcttcca acaggcgtgt aagggtaaaa 8520

tccaagcact ctgcgagaat cccgagtggg caccattgaa ggataacagg attccttcat 8580

acggggtctt gtctgttgat ctgagtctga cagttgagct taaaatcaaa attgcttcgg 8640

gattcgggcc attgatcaca cacggttcag ggatggacct atacaaatcc aaccacaaca 8700

atgtgtattg gctgactatc ccaccaatga agaacctagc cttaggtgta atcaacacat 8760

tggagtggat accgagattc aaggttagtc cctacctctt caatgtccca attaaggaag 8820

caggcgaaga ctgccatgcc ccaacatacc tacctgcgga ggtggatggt gatgtcaaac 8880

tcagttccaa tctggtgatt ctacctggtc aagatctcca atatgttttg gcaacctacg 8940

atacttccag ggttgaacat gctgtggttt attacgttta cagcccaagc cgctcatttt 9000

cttactttta tccttttagg ttgcctataa agggggtccc catcgaatta caagtggaat 9060

gcttcacatg ggaccaaaaa ctctggtgcc gtcacttctg tgtgcttgcg gactcagaat 9120

ctggtggaca tatcactcac tctgggatgg tgggcatggg agtcagctgc acagtcaccc 9180

gggaagatgg aaccaatcgc agatagggct gctagtgaac taatctcatg atgtcaccca 9240

gacatcaggc atacccacta gtgtgaaata gacatcagaa ttaagaaaaa cgtagggtcc 9300

aagtggttcc ccgttatgga ctcgctatct gtcaaccaga tcttataccc tgaagttcac 9360

ctagatagcc cgatagttac caataagata gtagccatcc tggagtatgc tcgagtccct 9420

cacgcttaca gcctggagga ccctacactg tgtcagaaca tcaagcaccg cctaaaaaac 9480

ggattttcca accaaatgat tataaacaat gtggaagttg ggaatgtcat caagtccaag 9540

cttaggagtt atccggccca ctctcatatt ccatatccaa attgtaatca ggatttattt 9600

aacatagaag acaaagagtc aacgaggaag atccgtgaac tcctcaaaaa ggggaattcg 9660

ctgtactcca aagtcagtga taaggttttc caatgcttaa gggacactaa ctcacggctt 9720

ggcctaggct ccgaattgag ggaggacatc aaggagaaag ttattaactt gggagtttac 9780

atgcacagct cccagtggtt tgagcccttt ctgttttggt ttacagtcaa gactgagatg 9840

aggtcagtga ttaaatcaca aacccatact tgccatagga ggagacacac acctgtattc 9900

ttcactggta gttcagttga gttgctaatc tctcgtgacc ttgttgctat aatcagtaaa 9960

gagtctcaac atgtatatta cctgacattt gaactggttt tgatgtattg tgatgtcata 10020

gaggggaggt taatgacaga gaccgctatg actattgatg ctaggtatac agagcttcta 10080

ggaagagtca gatacatgtg gaaactgata gatggtttct tccctgcact cgggaatcca 10140

acttatcaaa ttgtagccat gctggagcct ctttcacttg cttacctgca gctgagggat 10200

ataacagtag aactcagagg tgctttcctt aaccactgct ttactgaaat acatgatgtt 10260

cttgaccaaa acgggttttc tgatgaaggt acttatcatg agttaattga agctctagat 10320

tacattttca taactgatga catacatctg acaggggaga ttttctcatt tttcagaagt 10380

ttcggccacc ccagacttga agcagtaacg gctgctgaaa atgttaggaa atacatgaat 10440

cagcctaaag tcattgtgta tgagactctg atgaaaggtc atgccatatt ttgtggaatc 10500

ataatcaacg gctatcgtga caggcacgga ggcagttggc caccgctgac cctccccctg 10560

catgctgcag acacaatccg gaatgctcaa gcttcaggtg aagggttaac acatgagcag 10620
tgcgttgata actggaaatc ttttgctgga gtgaaatttg gctgctttat gcctcttagc 10680
ctggatagtg atctgacaat gtacctaaag gacaaggcac ttgctgctct ccaaagggaa 10740
tgggattcag tttacccgaa agagttcctg cgttacgacc ctcccaaggg aaccgggtca 10800
cggaggcttg tagatgtttt ccttaatgat tcgagctttg acccatatga tgtgataatg 10860
tatgttgtaa gtggagctta cctccatgac cctgagttca acctgtctta cagcctgaaa 10920
gaaaaggaga tcaaggaaac aggtagactt tttgctaaaa tgacttacaa aatgagggca 10980
tgccaagtga ttgctgaaaa tctaatctca aacgggattg gcaaatattt taaggacaat 11040
gggatggcca aggatgagca cgatttgact aaggcactcc acactctagc tgtctcagga 11100
gtccccaaag atctcaaaga aagtcacagg ggggggccag tcttaaaaac ctactcccga 11160
agcccagtcc acacaagtac caggaacgtg agagcagcaa aagggtttat agggttccct 11220
caagtaattc ggcaggacca agacactgat catccggaga atatggaagc ttacgagaca 11280
gtcagtgcat ttatcacgac tgatctcaag aagtactgcc ttaattggag atatgagacc 11340
atcagcttgt ttgcacagag gctaaatgag atttacggat tgccctcatt tttccagtgg 11400
ctgcataaga ggcttgagac ctctgtcctg tatgtaagtg accctcattg cccccccgac 11460
cttgacgccc atatcccgtt atataaagtc cccaatgatc aaatcttcat taagtaccct 11520
atgggaggta tagaagggta ttgtcagaag ctgtggacca tcagcaccat tccctatcta 11580
tacctggctg cttatgagag cggagtaagg attgcttcgt tagtgcaagg ggacaatcag 11640
accatagccg taacaaaaag ggtacccagc acatggccct acaaccttaa gaaacgggaa 11700
gctgctagag taactagaga ttactttgta attcttaggc aaaggctaca tgatattggc 11760
catcacctca aggcaaatga gacaattgtt tcatcacatt tttttgtcta ttcaaaagga 11820
atatattatg atgggctact tgtgtcccaa tcactcaaga gcatcgcaag atgtgtattc 11880
tggtcagaga ctatagttga tgaaacaagg gcagcatgca gtaatattgc tacaacaatg 11940
gctaaaagca tcgagagagg ttatgaccgt taccttgcat attccctgaa cgtcctaaaa 12000
gtgatacagc aaattctgat ctctcttggc ttcacaatca attcaaccat gacccgggat 12060
gtagtcatac ccctcctcac aaacaacgac ctcttaataa ggatggcact gttgcccgct 12120
cctattgggg ggatgaatta tctgaatatg agcaggctgt ttgtcagaaa catcggtgat 12180
ccagtaacat catcaattgc tgatctcaag agaatgattc tcgcctcact aatgcctgaa 12240
gagaccctcc atcaagtaat gacacaacaa ccgggggact cttcattcct agactgggct 12300
agcgaccctt actcagcaaa tcttgtatgt gtccagagca tcactagact cctcaagaac 12360
ataactgcaa ggtttgtcct gatccatagt ccaaacccaa tgttaaaagg attattccat 12420

gatgacagta aagaagagga cgagggactg gcggcattcc tcatggacag gcatattata 12480

gtacctaggg cagctcatga aatcctggat catagtgtca caggggcaag agagtctatt 12540

gcaggcatgc tggataccac aaaaggcctg attcgagcca gcatgaggaa gggggggtta 12600

acctctcgag tgataaccag attgtccaat tatgactatg aacaattcag agcagggatg 12660

gtgctattga caggaagaaa gagaaatgtc ctcattgaca aagagtcatg ttcagtgcag 12720

ctggcgagag ctctaagaag ccatatgtgg gcgaggctag ctcgaggacg gcctatttac 12780

ggccttgagg tccctgatgt actagaatct atgcgaggcc accttattcg gcgtcatgag 12840

acatgtgtca tctgcgagtg tggatcagtc aactacggat ggttttttgt cccctcgggt 12900

tgccaactgg atgatattga caaggaaaca tcatccttga gagtcccata tattggttct 12960

accactgatg agagaacaga catgaagctt gccttcgtaa gagccccaag tcgatccttg 13020

cgatctgctg ttagaatagc aacagtgtac tcatgggctt acggtgatga tgatagctct 13080

tggaacgaag cctggttgtt ggctaggcaa agggccaatg tgagcctgga ggagctaagg 13140

gtgatcactc ccatctcaac ttcgactaat ttagcgcata ggttgaggga tcgtagcact 13200

caagtgaaat actcaggtac atcccttgtc cgagtggcga ggtataccac aatctccaac 13260

gacaatctct catttgtcat atcagataag aaggttgata ctaactttat ataccaacaa 13320

ggaatgcttc tagggttggg tgttttagaa acattgtttc gactcgagaa agataccgga 13380

tcatctaaca cggtattaca tcttcacgtc gaaacagatt gttgcgtgat cccgatgata 13440

gatcatccca ggatacccag ctcccgcaag ctagagctga gggcagagct atgtaccaac 13500

ccattgatat atgataatgc acctttaatt gacagagata caacaaggct atacacccag 13560

agccatagga ggcaccttgt ggaatttgtt acatggtcca caccccaact atatcacatt 13620

ttagctaagt ccacagcact atctatgatt gacctggtaa caaaatttga gaaggaccat 13680

atgaatgaaa tttcagctct cataggggat gacgatatca atagtttcat aactgagttt 13740

ctgctcatag agccaagatt attcactatc tacttgggcc agtgtgcggc catcaattgg 13800

gcatttgatg tacattatca tagaccatca gggaaatatc agatgggtga gctgttgtca 13860

tcgttccttt ctagaatgag caaaggagtg tttaaggtgc ttgtcaatgc tctaagccac 13920

ccaaagatct acaagaaatt ctggcattgt ggtattatag agcctatcca tggtccttca 13980

cttgatgctc aaaacttgca cacaactgtg tgcaacatgg tttacacatg ctatatgacc 14040

tacctcgacc tgttgttgaa tgaagagtta gaagagttca catttctctt gtgtgaaagc 14100

gacgaggatg tagtaccgga cagattcgac aacatccagg caaaacactt atgtgttctg 14160

gcagatttgt actgtcaacc aggggcctgc ccaccaattc gaggtctaag accggtagag 14220

aaatgtgcag ttctaaccga ccatatcaag gcagaggcta ggttatctcc agcaggatct 14280

tcgtggaaca taaatccaat tattgtagac cattactcat gctctctgac ttatctccgg 14340

cgaggatcga tcaaacagat aagattgaga gttgatccag gattcatttt cgacgccctc 14400

gctgaggtaa atgtcagtca gccaaagatc ggcagcaaca acatctcaaa tatgagcatc 14460

aaggctttca gacccccaca cgatgatgtt gcaaaattgc tcaaagatat caacacaagc 14520

aagcacaatc ttcccatttc agggggcaat ctcgccaatt atgaaatcca tgctttccgc 14580

agaatcgggt tgaactcatc tgcttgctac aaagctgttg agatatcaac attaattagg 14640

agatgccttg agccagggga ggacggcttg ttcttgggtg agggatcggg ttctatgttg 14700

atcacttata aggagatact taaactaaac aagtgcttct ataatagtgg ggtttccgcc 14760

aattctagat ctggtcaaag ggaattagca ccctatccct ccgaagttgg ccttgtcgaa 14820

cacagaatgg gagtaggtaa tattgtcaaa gtgctcttta acgggaggcc cgaagtcacg 14880

tgggtaggca gtgtagattg cttcaatttc atagttagta atatccctac ctctagtgtg 14940

gggtttatcc attcagatat agagaccttg cctaacaaag atactataga gaagctagag 15000

gaattggcag ccatcttatc gatggctctg ctcctgggca aaataggatc aatactggtg 15060

attaagctta tgcctttcag cggggatttt gttcagggat ttataagtta tgtagggtct 15120

tattatagag aagtgaacct tgtataccct agatacagca acttcatatc tactgaatct 15180

tatttggtta tgacagatct caaggctaac cggctaatga atcctgaaaa gattaagcag 15240

cagataattg aatcatctgt gaggacttca cctggactta taggtcacat cctatccatt 15300

aagcaactaa gctgcataca agcaattgtg ggagacgcag ttagtagagg tgatatcaat 15360

cctactctga aaaaacttac acctatagag caggtgctga tcaattgcgg gttggcaatt 15420

aacggaccta agctgtgcaa agaattgatc caccatgatg ttgcctcagg gcaagatgga 15480

ttgcttaatt ctatactcat cctctacagg gagttggcaa gattcaaaga caaccgaaga 15540

agtcaacaag ggatgttcca cgcttacccc gtattggtaa gtagcaggca acgagaactt 15600

atatctagga tcacccgcaa attttggggg cacattcttc tttactccgg gaacagaaag 15660

ttgataaata agtttatcca gaatctcaag tccggctatc tgatactaga cttacaccag 15720

aatatcttcg ttaagaatct atccaagtca gagaaacaga ttattatgac ggggggtttg 15780

aaacgtgagt gggtttttaa ggtaacagtc aaggagacca aagaatggta taagttagtc 15840

ggatacagtg ccctgattaa ggactaattg attgaactcc ggaaccctaa tcctgcccta 15900

ggtggttagg cattatttgc aatatattaa agaaaacttt gaaaatacga agtttctatt 15960

cccagctttg tctggtggcc ggcatggtcc cagcctcctc gctggcgccg gctgggcaac 16020

attccgaggg gaccgtcccc tcggtaatgg cgaatgggac gcggccgatc cggctgctaa 16080

caaagcccga aaggaagctg agttggctgc tgccaccgct gagcaataac tagcataacc 16140

ccttggggcc tctaaacggg tcttgagggg ttttttgctg aaaggaggaa ctatatccgg 16200

atgcggccgc ttctatagtg tcacctaaat actagtgact ccagcgtaac tggactggcc 16260

acagttaggc cgcaaatgta atcacactgg ctcaccttcg ggtgggcctt tctgcgttcg 16320

cgagtggacc cgataagctc atggagcggc gtaaccgtcg cacaggaagg acagagaaag 16380

cgcggatctg ggaagtgacg gacagaacgg tcaggacctg gattggggag gcggttgccg 16440

ccgctgctgc tgacggtgtg acgttctctg ttccggtcac accacatacg ttccgccatt 16500

cctatgcgat gcacatgctg tatgccggta taccgctgaa agttctgcaa agcctgatgg 16560

gacataagtc catcagttca acggaagtct acacgaaggt ttttgcgctg gatgtggctg 16620

cccggcaccg ggtgcagttt gcgatgccgg agtctgatgc ggttgcgatg ctgaaacaat 16680

tatcctgaga ataaatgcct tggcctttat atggaaatgt ggaactgagt ggatatgctg 16740

tttttgtctg ttaaacagag aagctggctg ttatccactg agaagcgaac gaaacagtcg 16800

ggaaaatctc ccattatcgt agagatccgc attattaatc tcaggagcct gtgtagcgtt 16860

tataggaagt agtgttctgt catgatgcct gcaagcggta acgaaaacga tttgaatatg 16920

ccttcaggaa caatagaaat cttcgtgcgg tgttacgttg aagtggagcg gattatgtca 16980

gcaatggaca gaacaaccta atgaacacag aaccatgatg tggtctgtcc ttttacagcc 17040

agtagtgctc gccgcagtcg agcgacaggg cgaagccctc ggctggttgc cctcgccgct 17100

gggctggcgg ccgtctatgg ccctgcaaac gcgccagaaa cgccgtcgaa gccgtgtgcg 17160

agacaccgcg gcccgccgcc ggcgttgtgg atacctcgcg gaaaacttgg ccctcactga 17220

cagatgaggg gcggacgttg acacttgagg ggccgactca cccggcgcgg cgttgacaga 17280

tgaggggcag gctcgatttc ggccggcgac gtggagctgg ccagcctcgc aaatcggcga 17340

aaacgcctga ttttacgcga gtttcccaca gatgatgtgg acaagcctgg ggataagtgc 17400

cctgcggtat tgacacttga ggggcgcgac tactgacaga tgaggggcgc gatccttgac 17460

acttgagggg cagagtgctg acagatgagg ggcgcaccta ttgacatttg aggggctgtc 17520

cacaggcaga aaatccagca tttgcaaggg tttccgcccg tttttcggcc accgctaacc 17580

tgtcttttaa cctgctttta aaccaatatt tataaacctt gtttttaacc agggctgcgc 17640

cctgtgcgcg tgaccgcgca cgccgaaggg gggtgccccc ccttctcgaa ccctcccggt 17700

cgagtgagcg aggaagcacc agggaacagc acttatatat tctgcttaca cacgatgcct 17760

gaaaaaactt cccttggggt tatccactta tccacgggga tatttttata attatttttt 17820

ttatagtttt tagatcttct tttttagagc gccttgtagg cctttatcca tgctggttct 17880

agagaaggtg ttgtgacaaa ttgccctttc agtgtgacaa atcaccctca aatgacagtc 17940

ctgtctgtga caaattgccc ttaaccctgt gacaaattgc cctcagaaga agctgttttt 18000

tcacaaagtt atccctgctt attgactctt ttttatttag tgtgacaatc taaaaacttg 18060

tcacacttca catggatctg tcatggcgga aacagcggtt atcaatcaca agaaacgtaa 18120

aaatagcccg cgaatcgtcc agtcaaacga cctcactgag gcggcatata gtctctcccg 18180

ggatcaaaaa cgtatgctgt atctgttcgt tgaccagatc agaaaatctg atggcaccct 18240

acaggaacat gacggtatct gcgagatcca tgttgctaaa tatgctgaaa tattcggatt 18300

gacctctgcg gaagccagta aggatatacg gcaggcattg aagagtttcg cggggaagga 18360

agtggttttt tatcgccctg aagaggatgc cggcgatgaa aaaggctatg aatcttttcc 18420

ttggtttatc aaacgtgcgc acagtccatc cagagggctt tacagtgtac atatcaaccc 18480

atatctcatt cccttcttta tcgggttaca gaaccggttt acgcagtttc ggcttagtga 18540

aacaaaagaa atcaccaatc cgtatgccat gcgtttatac gaatccctgt gtcagtatcg 18600

taagccggat ggctcaggca tcgtctctct gaaaatcgac tggatcatag agcgttacca 18660

gctgcctcaa agttaccagc gtatgcctga cttccgccgc cgcttcctgc aggtctgtgt 18720

taatgagatc aacagcagaa ctccaatgcg cctctcatac attgagaaaa agaaaggccg 18780

ccagacgact catatcgtat tttccttccg cgatatcact tccatgacga caggatagtc 18840

tgagggttat ctgtcacaga tttgagggtg gttcgtcaca tttgttctga cctactgagg 18900

gtaatttgtc acagttttgc tgtttccttc agcctgcatg gattttctca tactttttga 18960

actgtaattt ttaaggaagc caaatttgag ggcagtttgt cacagttgat ttccttctct 19020

ttcccttcgt catgtgacct gatatcgggg gttagttcgt catcattgat gagggttgat 19080

tatcacagtt tattactctg aattggctat ccgcgtgtgt acctctacct ggagtttttc 19140

ccacggtgga tatttcttct tgcgctgagc gtaagagcta tctgacagaa cagttcttct 19200

ttgcttcctc gccagttcgc tcgctatgct cggttacacg gctgcggcga gcgctagtga 19260

taataagtga ctgaggtatg tgctcttctt atctcctttt gtagtgttgc tcttatttta 19320

aacaactttg cggttttttg atgactttgc gattttgttg ttgctttgca gtaaattgca 19380

agatttaata aaaaaacgca aagcaatgat taaaggatgt tcagaatgaa actcatggaa 19440

acacttaacc agtgcataaa cgctggtcat gaaatgacga aggctatcgc cattgcacag 19500

tttaatgatg acagcccgga agcgaggaaa ataacccggc gctggagaat aggtgaagca 19560

gcggatttag ttggggtttc ttctcaggct atcagagatg ccgagaaagc agggcgacta 19620

ccgcacccgg atatggaaat tcgaggacgg gttgagcaac gtgttggtta tacaattgaa 19680

caaattaatc atatgcgtga tgtgtttggt acgcgattgc gacgtgctga agacgtattt 19740

ccaccggtga tcggggttgc tgcccataaa ggaggcgttt acaaaacctc agtttctgtt 19800

catcttgctc aggatctggc tctgaagggg ctacgtgttt tgctcgtgga aggtaacgac 19860

ccccagggaa cagcctcaat gtatcacgga tgggtaccag atcttcatat tcatgcagaa 19920

gacactctcc tgcctttcta tcttggggaa aaggacgatg tcacttatgc aataaagccc 19980

acttgctggc cggggcttga cattattcct tcctgtctgg ctctgcaccg tattgaaact 20040

gagttaatgg gcaaatttga tgaaggtaaa ctgcccaccg atccacacct gatgctccga 20100

ctggccattg aaactgttgc tcatgactat gatgtcatag ttattgacag tgcgcctaac 20160

ctgggtatcg gcacgattaa tgtcgtatgt gctgctgatg tgctgattgt tcccacgcct 20220

gctgagttgt ttgactacac ctccgcactg cagtttttcg atatgcttcg tgatctgctc 20280

aagaacgttg atcttaaagg gttcgagcct gatgtacgta ttttgcttac caaatacagc 20340

aatagtaatg gctctcagtc cccgtggatg gaggagcaaa ttcgggatgc ctggggaagc 20400

atggttctaa aaaatgttgt acgtgaaacg gatgaagttg gtaaaggtca gatccggatg 20460

agaactgttt ttgaacaggc cattgatcaa cgctcttcaa ctggtgcctg gagaaatgct 20520

ctttctattt gggaacctgt ctgcaatgaa attttcgatc gtctgattaa accacgctgg 20580

gagattagat aatgaagcgt gcgcctgtta ttccaaaaca tacgctcaat actcaaccgg 20640

ttgaagatac ttcgttatcg acaccagctg ccccgatggt ggattcgtta attgctcgcg 20700

taggagtaat ggctcgcggt aatgccatta ctttgcctgt atgtggtcgg gatgtgaagt 20760

ttactcttga agtgctccgg ggtgatagtg ttgagaagac ctctcgggta tggtcaggta 20820

atgaacgtga ccaggagctg cttactgagg acgcactgga tgatctcatc ccttcttttc 20880

tactgactgg tcaacagaca ccggcgttcg gtcgaagagt atctggtgtc atagaaattg 20940

ccgatgggag tcgccgtcgt aaagctgctg cacttaccga aagtgattat cgtgttctgg 21000

ttggcgagct ggatgatgag cagatggctg cattatccag attgggtaac gattatcgcc 21060

caacaagtgc ttatgaacgt ggtcagcgtt atgcaagccg attgcagaat gaatttgctg 21120

gaaatatttc tgcgctggct gatgcggaaa atatttcacg taagattatt acccgctgta 21180

tcaacaccgc caaattgcct aaatcagttg ttgctctttt ttctcacccc ggtgaactat 21240

ctgcccggtc aggtgatgca cttcaaaaag cctttacaga taaagaggaa ttacttaagc 21300

agcaggcatc taaccttcat gagcagaaaa aagctggggt gatatttgaa gctgaagaag 21360

ttatcactct tttaacttct gtgcttaaaa cgtcatctgc atcaagaact agtttaagct 21420

cacgacatca gtttgctcct ggagcgacag tattgtataa gggcgataaa atggtgctta 21480

acctggacag gtctcgtgtt ccaactgagt gtatagagaa aattgaggcc attcttaagg 21540

aacttgaaaa gccagcaccc tgatgcgacc acgttttagt ctacgtttat ctgtctttac 21600

ttaatgtcct ttgttacagg ccagaaagca taactggcct gaatattctc tctgggccca 21660

ctgttccact tgtatcgtcg gtctgataat cagactggga ccacggtccc actcgtatcg 21720

tcggtctgat tattagtctg ggaccacggt cccactcgta tcgtcggtct gattattagt 21780

ctgggaccac ggtcccactc gtatcgtcgg tctgataatc agactgggac cacggtccca 21840

ctcgtatcgt cggtctgatt attagtctgg gaccatggtc ccactcgtat cgtcggtctg 21900

attattagtc tgggaccacg gtcccactcg tatcgtcggt ctgattatta gtctggaacc 21960

acggtcccac tcgtatcgtc ggtctgatta ttagtctggg accacggtcc cactcgtatc 22020

gtcggtctga ttattagtct gggaccacga tcccactcgt gttgtcggtc tgattatcgg 22080

tctgggacca cggtcccact tgtattgtcg atcagactat cagcgtgaga ctacgattcc 22140

atcaatgcct gtcaagggca agtattgaca tggtcgtcgt aacctgtaga acggagtaac 22200

ctcggtgtgc ggttgtatgc ctgctgtgga ttgctgctgt gtcctgctta tccacaacat 22260

tttgcgcacg gttatgtgga caaaatacct ggttacccag gccgtgccgg cacgttaacc 22320

gggctgcatc cgatgcaagt gtgtcgctga cgacgagctc gcgagctcgg acatgaggtt 22380

gccccgtatt cagtgtcgct gatttgtatt gtctgaagtt gcttttacgt taagttgatg 22440

cagatcaatt aatacgatac ctgcgtcata attgattatt tgacgtggtt tgatggcctc 22500

cacgcacgtt gtgatatgta gatgataatc attatcactt tacgggtcct ttccggtgat 22560

ccgacaggtt acggggcggc gacctcgcgg gttttcgcta tttatgaaaa ttttccggtt 22620

taaggcgttt ccgttcttct tcgtcataac ttaatgtttt tatttaaaat accctctgaa 22680

aagaaaggaa acgacaggtg ctgaaagcga gctttttggc ctctgtcgtt tcctttctct 22740

gtttttgtcc gtggaatgaa caatggaagt ccgagctcat cgctaataac ttcgtatagc 22800

atacattata cgaagttata ttcgatgcgg ccgatctagc agaaagtcaa aagcctccga 22860

ccggaggctt ttgacttctg tcacctaggt tacgccccgc cctgccactc atcgcagtac 22920

tgttgtaatt cattaagcat tctgccgaca tggaagccat cacaaacggc atgatgaacc 22980

tgaatcgcca gcggcatcag caccttgtcg ccttgcgtat aatatttgcc catggtgaaa 23040

acgggggcga agaagttgtc catattggcc acgtttaaat caaaactggt gaaactcacc 23100

cagggattgg ctgagacgaa aaacatattc tcaataaacc ctttagggaa ataggccagg 23160

ttttcaccgt aacacgccac atcttgcgaa tatatgtgta gaaactgccg gaaatcgtcg 23220

tggtattcac tccagagcga tgaaaacgtt tcagtttgct catggaaaac ggtgtaacaa 23280

gggtgaacac tatcccatat caccagctca ccgtctttca ttgccatacg aaattccgga 23340

tgagcattca tcaggcgggc aagaatgtga ataaaggccg gataaaactt gtgcttattt 23400

ttctttacgg tctttaaaaa ggccgtaata tccagctgaa cggtctggtt ataggtacat 23460

tgagcaactg actgaaatgc ctcaaaatgt tctttacgat gccattggga tatatcaacg 23520

gtggtatatc cagtgatttt tttctccatt ttagcttcct tagctcctga aaatctcgat 23580

aactcaaaaa atacgcccgg tagtgatctt atttcattat ggtgaaagtt ggaacctctt 23640

acgtgccgat cagattaaaa cgaaaggccc agtctttcga ctgagccttt cgttttattt 23700

gaccatgttg gtatgattta aattcgttta gtgaaccgtc agatcactag aagctttatt 23760

gcggtagttt atcacagtta aattgctaac gcagtcagtg cttctgacac aacagtctcg 23820

aacttaagct gcagtgactc tcttaaggta gccttgcaga agttggtcgt gaggcactgg 23880

gcaggtaagt atcaaggtta caagacaggt ttaaggagac caatagaaac tgggcttgtc 23940

gagacagaga agactcttgc gtttctgata ggcacctatt ggtcttactg acatccactt 24000

tgcctttctc tccacaggtg tccactccca gttcaattac agctcttaag gctagagtac 24060

t 24061

<210> 61
<211> 24061
<212> DNA
<213> artificial sequence

<220>
<223> Synthetic sequence

<400> 61
gcggccgcta atacgactca ctatagggcc aactttgttt ggtctgatga gtccgtgagg 60

acgaaacccg gagtcccggg tcaccaaaca aagttgggta aggatagttc aatcaatgat 120

cattttctag tgcacttagg attcaagatc ctattatcag ggacaagagc aggattaagg 180

atatccgaga tggccacact tttaaggagc ttagcattgt tcaaaagaaa caaggacaaa 240

ccacccatta catcaggatc cggtggagcc atcagaggaa tcaaacacat tattatagta 300

ccaatccctg gagattcctc aattaccact cgatccagac ttctggaccg gttggtcagg 360

ttaattggaa acccggatgt gagcgggccc aaactaacag gggcactaat aggtatatta 420

tccttatttg tggagtctcc aggtcaattg attcagagga tcaccgatga ccctgacgtt 480

agcataaggc tgttagaggt tgtccagagt gaccagtcac aatctggcct taccttcgca 540

tcaagaggta ccaacatgga ggatgaggcg gaccaatact tttcacatga tgatccaatt 600

agtagtgatc aatccaggtt cggatggttc gagaacaagg aaatctcaga tattgaagtg 660

caagaccctg agggattcaa catgattctg ggtaccatcc tagctcaaat ttgggtcttg 720

ctcgcaaagg cggttacggc cccagacacg gcagctgatt cggagctaag aaggtggata 780

aagtacaccc aacaaagaag ggtagttggt gaatttagat tggagagaaa atggttggat 840

gtggtgagga acaggattgc cgaggacctc tccttacgcc gattcatggt cgctctaatc 900

ctggatatca agagaacacc cggaaacaaa cccaggattg ctgaaatgat atgtgacatt 960

gatacatata tcgtagaggc aggattagcc agttttatcc tgactattaa gtttgggata 1020

gaaactatgt atcctgctct tggactgcat gaatttgctg gtgagttatc cacacttgag 1080

tccttgatga acctttacca gcaaatgggg gaaactgcac cctacatggt aatcctggag 1140

aactcaattc agaacaagtt cagtgcagga tcataccctc tgctctggag ctatgccatg 1200

ggagtaggag tggaacttga aaactccatg ggaggtttga actttggccg atcttacttt 1260

gatccagcat attttagatt agggcaagag atggtaagga ggtcagctgg aaaggtcagt 1320

tccacattgg catctgaact cggtatcact gccgaggatg caaggcttgt ttcagagatt 1380

gcaatgcata ctactgagga caagatcagt agagcggttg gacccagaca agcccaagta 1440

tcatttctac acggtgatca aagtgagaat gagctaccga gattgggggg caaggaagat 1500

aggagggtca aacagagtcg aggagaagcc agggagagct acagagaaac cgggcccagc 1560

agagcaagtg atgcgagagc tgcccatctt ccaaccggca cacccctaga cattgacact 1620

gcatcggagt ccagccaaga tccgcaggac agtcgaaggt cagctgacgc cctgcttagg 1680

ctgcaagcca tggcaggaat ctcggaagaa caaggctcag acacggacac ccctatagtg 1740

tacaatgaca gaaatcttct agactaggtg cgagaggccg agggccagaa caacatccgc 1800

ctaccctcca tcattgttat aaaaaactta ggaaccaggt ccacacagcc gccagcccat 1860

caaccatcca ctcccacgat tggagccgat ggcagaagag caggcacgcc atgtcaaaaa 1920

cggactggaa tgcatccggg ctctcaaggc cgagcccatc ggctcactgg ccatcgagga 1980

agctatggca gcatggtcag aaatatcaga caacccagga caggagcgag ccacctgcag 2040

ggaagagaag gcaggcagtt cgggtctcag caaaccatgc ctctcagcaa ttggatcaac 2100

tgaaggcggt gcacctcgca tccgcggtca gggacctgga gagagcgatg acgacgctga 2160

aactttggga atccccccaa gaaatctcca ggcatcaagc actgggttac agtgttatta 2220

tgtttatgat cacagcggtg aagcggttaa gggaatccaa gatgctgact ctatcatggt 2280

tcaatcaggc cttgatggtg atagcaccct ctcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cctccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tgtgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agctaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaactagt acaacctaaa tccattataa 3480

aaaacttagg agcaaagtga ttgcctccca agttccacaa tgacagagat ctacgacttc 3540

gacaagtcgg catgggacat caaagggttg atcgctccga tacaacccac cacctacagt 3600

gatggcaggc tggtgcccca ggtcagagtc atagatcctg gtctaggcga caggaaggat 3660

gaatgcttta tgtacatgtt tctgctgggg gttgttgagg acagcgatcc cctagggcct 3720

ccaatcgggc gagcatttgg gtccctgccc ttaggtgttg gcaaatccac agcaaagccc 3780

gaaaaactcc tcaaagaggc cactgagctt gacatagttg ttagacgtac agcagggctc 3840

aatgaaaaac tggtgttcta caacaacacc ccactaactc tcctcacacc ttggagaaag 3900

gtcctaacaa cagggagtgt cttcaacgca aaccaagtgt gcagtgcggt taatctgata 3960

ccgctcgata ccccgcagag gttccgtgtt gtttatatga gcatcacccg tctttcggat 4020

aacgggtatt acaccgttcc tagaagaatg ctggaattca gatcggtcaa tgcagtggcc 4080

ttcaacctgc tggtgaccct taggattgac aaggcgatag gccctgggaa gatcatcgac 4140

aatacagagc aacttcctga ggcaacattt atggtccaca tcgggaactt caggagaaag 4200

aagagtgaag tctactctgc cgattattgc aaaatgaaaa tcgaaaagat gggcctggtt 4260

tttgcacttg gtgggatagg gggcaccagt cttcacatta gaagcacagg caaaatgagc 4320

aagactctcc atgcacaact cgggttcaag aagaccttat gttacccgct gatagatatc 4380

aatgaagacc ttaatcgatt actctggagg agcagatgca agatagtaag aatccaggca 4440

gttttgcagc catcagttcc tcaagaattc cgcatttacg acgacgtgat cataaatgat 4500

gaccaaggac tattcaaagt tctgtagacc gtagtgccca gcaatgcccg aaaacgaccc 4560

ccctcacaat gacagccaga aggcccggac aaaaaagccc cctccgaaag actccacgga 4620

ccaagcgaga ggccagccag cagccgacgg caagcgcgaa caccaggcgg ccccagcaca 4680

gaacagccct gatacaaggc caccaccagc caccccaatc tgcatcctcc tcgtgggacc 4740

cccgaggacc aacccccaag gctgcccccg atccaaacca ccaaccgcat ccccaccacc 4800

cccgggaaag aaacccccag caattggaag gcccctcccc ctcttcctca acacaagaac 4860

tccacaaccg aaccgcacaa gcgaccgagg tgacccaacc gcaggcatcc gactccctag 4920

acagatcctc tctccccggc aaactaaaca aaacttaggg ccaaggaaca tacacaccca 4980

acagaaccca gaccccggcc cacggcgccg cgcccccaac cccgacaacc cagagggagc 5040

ccccaaccaa tcccgccggc tcccccggtg cccacaggca gggacaccaa cccccgaaca 5100

gacccagcac ccaaccatcg acaatccaag acgggggggc ccccccaaaa aaaggccccc 5160

aggggccgac agccagcacc gcgaggaagc ccacccaccc cacacacgac cacggcaacc 5220

aaaccagaac ccagaccacc ctgggccacc agctcccaga ctcggccatc accccgcaga 5280

aaggaaaggc cacaacccgc gcaccccagc cccgatccgg cggggagcca cccaacccga 5340

accagcaccc aagagcgatc cccgaaggac ccccgaaccg caaaggacat cagtatccca 5400

cagcctctcc aagtcccccg gtctcctccc cttctcgaag ggaccaaaag atcaatccac 5460

cacacccgac gacactcaac tccccacccc taaaggagac accgggaatc ccagaatcaa 5520

gactcatcca atgtccatca tgggtctcaa ggtgaacgtc tctgccatat tcatggcagt 5580

actgttaact ctccaaacac ccaccggtca aatccattgg ggcaatctct ctaagatagg 5640

ggtggtagga ataggaagtg caagctacaa agttatgact cgttccagcc atcaatcatt 5700

agtcataaaa ttaatgccca atataactct cctcaataac tgcacgaggg tagagattgc 5760

agaatacagg agactactga gaacagtttt ggaaccaatt agagatgcac ttaatgcaat 5820

gacccagaat ataagaccgg ttcagagtgt agcttcaagt aggagacaca agagatttgc 5880

gggagtagtc ctggcaggtg cggccctagg cgttgccaca gctgctcaga taacagccgg 5940

cattgcactt caccagtcca tgctgaactc tcaagccatc gacaatctga gagcgagcct 6000

ggaaactact aatcaggcaa ttgaggcaat cagacaagca gggcaggaga tgatattggc 6060

tgttcagggt gtccaagact acatcaataa tgagctgata ccgtctatga accaactatc 6120

ttgtgattta atcggccaga agctcgggct caaattgctc agatactata cagaaatcct 6180

gtcattattt ggccccagct tacgggaccc catatctgcg gagatatcta tccaggcttt 6240

gagctatgcg cttggaggag acatcaataa ggtgttagaa aagctcggat acagtggagg 6300

tgatttactg ggcatcttag agagcagagg aataaaggcc cggataactc acgtcgacac 6360

agagtcctac ttcattgtcc tcagtatagc ctatccgacg ctgtccgaga ttaagggggt 6420

gattgtccac cggctagagg gggtctcgta caacataggc tctcaagagt ggtataccac 6480

tgtgcccaag tatgttgcaa cccaagggta ccttatctcg aattttgatg agtcatcgtg 6540

tactttcatg ccagagggga ctgtgtgcag ccaaaatgcc ttgtacccga tgagtcctct 6600

gctccaagaa tgcctccggg ggtccaccaa gtcctgtgct cgtacactcg tatccgggtc 6660

ttttgggaac cggttcattt tatcacaagg gaacctaata gccaattgtg catcaatcct 6720

ttgcaagtgt tacacaacag gaacgatcat taatcaagac cctgacaaga tcctaacata 6780

cattgctgcc gatcactgcc cggtagtcga ggtgaacggc gtgaccatcc aagtcgggag 6840

caggaggtat ccagatgctg tgtacttgca cagaattgac ctcggtcctc ccatatcatt 6900

ggagaggttg gacgtaggga caaatctggg gaatgcaatt gctaagttgg aggatgccaa 6960

ggaattgttg gagtcatcgg accagatatt gaggagtatg aaaggtttat cgagcactag 7020

catagtctac atcctgattg cagtgtgtct tggagggttg atagggatcc ccgctttaat 7080

atgttgctgc agggggcgtt gtaacaaaaa gggagaacaa gttggtatgt caagaccagg 7140

cctaaagcct gatcttacgg gaacatcaaa atcctatgta aggtcgctct gatcctctac 7200

aactcttgaa acacaaatgt cccacaagtc tcctcttcgt catcaagcaa ccaccgcacc 7260

cagcatcaag cccacctgaa attatctccg gcttccctct ggccgaacaa tatcggtagt 7320

taattaaaac ttagggtgca agatcatcca caatgtcacc acaacgagac cggataaatg 7380

ccttctacaa agataacccc catcccaagg gaagtaggat agtcattaac agagaacatc 7440

ttatgattga tagaccttat gttttgctgg ctgttctgtt tgtcatgttt ctgagcttga 7500

tcgggttgct agccattgca ggcattagac ttcatcgggc agccatctac accgcagaga 7560

tccataaaag cctcagcacc aatctagatg taactaactc aatcgagcat caggtcaagg 7620

acgtgctgac accactcttc aaaatcatcg gtgatgaagt gggcctgagg acacctcaga 7680

gattcactga cctagtgaaa ttcatctctg acaagattaa attccttaat ccggataggg 7740

agtacgactt cagagatctc acttggtgta tcaacccgcc agagagaatc aaattggatt 7800

atgatcaata ctgtgcagat gtggctgctg aagagctcat gaatgcattg gtgaactcaa 7860

ctctactgga gaccagaaca accaatcagt tcctagctgt ctcaaaggga aactgctcag 7920

ggcccactac aatcagaggt caattctcaa acatgtcgct gtccctgtta gacttgtatt 7980

taggtcgagg ttacaatgtg tcatctatag tcactatgac atcccaggga atgtatgggg 8040

gaacttacct agtggaaaag cctaatctga gcagcaaaag gtcagagttg tcacaactga 8100

gcatgtaccg agtgtttgaa gtaggtgtta tcagaaatcc gggtttgggg gctccggtgt 8160

tccatatgac aaactatctt gagcaaccag ccagtaatga tctcagcaac tgtatggtgg 8220

ctttggggga gctcaaactc gcagcccttt gtcacgggga agattctatc acaattccct 8280

atcagggatc agggaaaggt gtcagcttcc agctcgtcaa gctaggtgtc tggaaatccc 8340

caaccgacat gcaatcctgg gtccccttat caacggatga tccagtgata gacaggcttt 8400

acctctcatc tcacagaggt gttatcgctg acaatcaagc aaaatgggct gtcccgacaa 8460

cacgaacaga tgacaagttg cgaatggaga catgcttcca acaggcgtgt aagggtaaaa 8520

tccaagcact ctgcgagaat cccgagtggg caccattgaa ggataacagg attccttcat 8580

acggggtctt gtctgttgat ctgagtctga cagttgagct taaaatcaaa attgcttcgg 8640

gattcgggcc attgatcaca cacggttcag ggatggacct atacaaatcc aaccacaaca 8700

atgtgtattg gctgactatc ccgccaatga agaacctagc cttaggtgta atcaacacat 8760

tggagtggat accgagattc aaggttagtc cctacctctt caatgtccca attaaggaag 8820

caggcgaaga ctgccatgcc ccaacatacc tacctgcgga ggtggatggt gatgtcaaac 8880

tcagttccaa tctggtgatt ctacctggtc aagatctcca atatgttttg gcaacctacg 8940

atacttccag ggttgaacat gctgtggttt attacgttta cagcccaggc cgctcatttt 9000

cttactttta tccttttagg ttgcctataa agggggtccc catcgaatta caagtggaat 9060

gcttcacatg ggaccaaaaa ctctggtgcc gtcacttctg tgtgcttgcg gactcagaat 9120

ctggtggaca tatcactcac tctgggatgg tgggcatggg agtcagctgc acagtcaccc 9180

gggaagatgg aaccaatcgc agatagggct gctagtgaac caatctcatg atgtcaccca 9240

gacatcaggc atacccacta gtgtgaaata gacatcagaa ttaagaaaaa cgtagggtcc 9300

aagtggttcc ccgttatgga ctcgctatct gtcaaccaga tcttataccc tgaagttcac 9360

ctagatagcc cgatagttac caataagata gtagccatcc tggagtatgc tcgagtccct 9420

cacgcttaca gcctggagga ccctacactg tgtcagaaca tcaagcaccg cctaaaaaac 9480

ggattttcca accaaatgat tataaacaat gtggaagttg ggaatgtcat caagtccaag 9540

cttaggagtt atccggccca ctctcatatt ccatatccaa attgtaatca ggatttattt 9600

aacatagaag acaaagagtc aacgaggaag atccgtgaac tcctcaaaaa ggggaattcg 9660

ctgtactcca aagtcagtga taaggttttc caatgcttaa gggacactaa ctcacggctt 9720

ggcctaggct ccgaattgag ggaggacatc aaggagaaag ttattaactt gggagtttac 9780

atgcacagct cccagtggtt tgagcccttt ctgttttggt ttacagtcaa gactgagatg 9840

aggtcagtga ttaaatcaca aacccatact tgccatagga ggagacacac acctgtattc 9900

ttcactggta gttcagttga gttgctaatc tctcgtgacc ttgttgctat aatcagtaaa 9960

gagtctcaac atgtatatta cctgacattt gaactggttt tgatgtattg tgatgtcata 10020

gaggggaggt taatgacaga gaccgctatg actattgatg ctaggtatac agagcttcta 10080

ggaagagtca gatacatgtg gaaactgata gatggtttct tccctgcact cgggaatcca 10140

acttatcaaa ttgtagccat gctggagcct ctttcacttg cttacctgca gctgagggat 10200

ataacagtag aactcagagg tgctttcctt aaccactgct ttactgaaat acatgatgtt 10260

cttgaccaaa acgggttttc tgatgaaggt acttatcatg agttaattga agctctagat 10320

tacattttca taactgatga catacatctg acaggggaga ttttctcatt tttcagaagt 10380

ttcggccacc ccagacttga agcagtaacg gctgctgaaa atgttaggaa atacatgaat 10440

cagcctaaag tcattgtgta tgagactctg atgaaaggtc atgccatatt ttgtggaatc 10500

ataatcaacg gctatcgtga caggcacgga ggcagttggc caccgctgac cctccccctg 10560

catgctgcag acacaatccg gaatgctcaa gcttcaggtg aagggttaac acatgagcag 10620

tgcgttgata actggaaatc ttttgctgga gtgaaatttg gctgctttat gcctcttagc 10680

ctggatagtg atctgacaat gtacctaaag gacaaggcac ttgctgctct ccaaagggaa 10740

tgggattcag tttacccgaa agagttcctg cgttacgacc ctcccaaggg aaccgggtca 10800

cggaggcttg tagatgtttt ccttaatgat tcgagctttg acccatatga tgtgataatg 10860

tatgttgtaa gtggagctta cctccatgac cctgagttca acctgtctta cagcctgaaa 10920

gaaaaggaga tcaaggaaac aggtagactt tttgctaaaa tgacttacaa aatgagggca 10980

tgccaagtga ttgctgaaaa tctaatctca aacgggattg gcaaatattt taaggacaat 11040

gggatggcca aggatgagca cgatttgact aaggcactcc acactctagc tgtctcagga 11100

gtccccaaag atctcaaaga aagtcacagg ggggggccag tcttaaaaac ctactcccga 11160

agcccagtcc acacaagtac caggaacgtg agagcagcaa aagggtttat agggttccct 11220

caagtaattc ggcaggacca agacactgat catccggaga atatggaagc ttacgagaca 11280

gtcagtgcat ttatcacgac tgatctcaag aagtactgcc ttaattggag atatgagacc 11340

atcagcttgt ttgcacagag gctaaatgag atttacggat tgccctcatt tttccagtgg 11400

ctgcataaga ggcttgagac ctctgtcctg tatgtaagtg accctcattg cccccccgac 11460

cttgacgccc atatcccgtt atataaagtc cccaatgatc aaatcttcat taagtaccct 11520

atgggaggta tagaagggta ttgtcagaag ctgtggacca tcagcaccat tccctatcta 11580

tacctggctg cttatgagag cggagtaagg attgcttcgt tagtgcaagg ggacaatcag 11640

accatagccg taacaaaaag ggtacccagc acatggccct acaaccttaa gaaacgggaa 11700

gctgctagag taactagaga ttactttgta attcttaggc aaaggctaca tgatattggc 11760

catcacctca aggcaaatga gacaattgtt tcatcacatt tttttgtcta ttcaaaagga 11820

atatattatg atgggctact tgtgtcccaa tcactcaaga gcatcgcaag atgtgtattc 11880

tggtcagaga ctatagttga tgaaacaagg gcagcatgca gtaatattgc tacaacaatg 11940

gctaaaagca tcgagagagg ttatgaccgt taccttgcat attccctgaa cgtcctaaaa 12000

gtgatacagc aaattctgat ctctcttggc ttcacaatca attcaaccat gacccgggat 12060

gtagtcatac ccctcctcac aaacaacgac ctcttaataa ggatggcact gttgcccgct 12120

cctattgggg ggatgaatta tctgaatatg agcaggctgt ttgtcagaaa catcggtgat 12180

ccagtaacat catcaattgc tgatctcaag agaatgattc tcgcctcact aatgcctgaa 12240

gagaccctcc atcaagtaat gacacaacaa ccgggggact cttcattcct agactgggct 12300

agcgaccctt actcagcaaa tcttgtatgt gtccagagca tcactagact cctcaagaac 12360

ataactgcaa ggtttgtcct gatccatagt ccaaacccaa tgttaaaagg attattccat 12420

gatgacagta aagaagagga cgagggactg gcggcattcc tcatggacag gcatattata 12480

gtacctaggg cagctcatga aatcctggat catagtgtca caggggcaag agagtctatt 12540

gcaggcatgc tggataccac aaaaggcctg attcgagcca gcatgaggaa gggggggtta 12600

acctctcgag tgataaccag attgtccaat tatgactatg aacaattcag agcagggatg 12660

gtgctattga caggaagaaa gagaaatgtc ctcattgaca aagagtcatg ttcagtgcag 12720

ctggcgagag ctctaagaag ccatatgtgg gcgaggctag ctcgaggacg gcctatttac 12780

ggccttgagg tccctgatgt actagaatct atgcgaggcc accttattcg gcgtcatgag 12840

acatgtgtca tctgcgagtg tggatcagtc aactacggat ggttttttgt cccctcgggt 12900

tgccaactgg atgatattga caaggaaaca tcatccttga gagtcccata tattggttct 12960

accactgatg agagaacaga catgaagctt gccttcgtaa gagccccaag tcgatccttg 13020

cgatctgctg ttagaatagc aacagtgtac tcatgggctt acggtgatga tgatagctct 13080

tggaacgaag cctggttgtt ggctaggcaa agggccaatg tgagcctgga ggagctaagg 13140

gtgatcactc ccatctcaac ttcgactaat ttagcgcata ggttgaggga tcgtagcact 13200

caagtgaaat actcaggtac atcccttgtc cgagtggcga ggtataccac aatctccaac 13260

gacaatctct catttgtcat atcagataag aaggttgata ctaactttat ataccaacaa 13320

ggaatgcttc tagggttggg tgttttagaa acattgtttc gactcgagaa agataccgga 13380

tcatctaaca cggtattaca tcttcacgtc gaaacagatt gttgcgtgat cccgatgata 13440

gatcatccca ggatacccag ctcccgcaag ctagagctga gggcagagct atgtaccaac 13500

ccattgatat atgataatgc acctttaatt gacagagata caacaaggct atacacccag 13560

agccatagga ggcaccttgt ggaatttgtt acatggtcca caccccaact atatcacatt 13620

ttagctaagt ccacagcact atctatgatt gacctggtaa caaaatttga gaaggaccat 13680

atgaatgaaa tttcagctct cataggggat gacgatatca atagtttcat aactgagttt 13740

ctgctcatag agccaagatt attcactatc tacttgggcc agtgtgcggc catcaattgg 13800

gcatttgatg tacattatca tagaccatca gggaaatatc agatgggtga gctgttgtca 13860

tcgttccttt ctagaatgag caaaggagtg tttaaggtgc ttgtcaatgc tctaagccac 13920

ccaaagatct acaagaaatt ctggcattgt ggtattatag agcctatcca tggtccttca 13980

cttgatgctc aaaacttgca cacaactgtg tgcaacatgg tttacacatg ctatatgacc 14040

tacctcgacc tgttgttgaa tgaagagtta gaagagttca catttctctt gtgtgaaagc 14100

gacgaggatg tagtaccgga cagattcgac aacatccagg caaaacactt atgtgttctg 14160

gcagatttgt actgtcaacc agggacctgc ccaccaattc gaggtctaag accggtagag 14220

aaatgtgcag ttctaaccga ccatatcaag gcagaggcta ggttatctcc agcaggatct 14280

tcgtggaaca taaatccaat tattgtagac cattactcat gctctctgac ttatctccgg 14340

cgaggatcga tcaaacagat aagattgaga gttgatccag gattcatttt cgacgccctc 14400

gctgaggtaa atgtcagtca gccaaagatc ggcagcaaca acatctcaaa tatgagcatc 14460

aaggctttca gacccccaca cgatgatgtt gcaaaattgc tcaaagatat caacacaagc 14520

aagcacaatc ttcccatttc agggggcaat ctcgccaatt atgaaatcca tgctttccgc 14580

agaatcgggt tgaactcatc tgcttgctac aaagctgttg agatatcaac attaattagg 14640

agatgccttg agccagggga ggacggcttg ttcttgggtg agggatcggg ttctatgttg 14700

atcacttata aggagatact taaactaaac aagtgcttct ataatagtgg ggtttccgcc 14760

aattctagat ctggtcaaag ggaattagca ccctatccct ccgaagttgg ccttgtcgaa 14820

cacagaatgg gagtaggtaa tattgtcaaa gtgctcttta acgggaggcc cgaagtcacg 14880

tgggtaggca gtgtagattg cttcaatttc atagttagta atatccctac ctctagtgtg 14940

gggtttatcc attcagatat agagaccttg cctaacaaag atactataga gaagctagag 15000

gaattggcag ccatcttatc gatggctctg ctcctgggca aaataggatc aatactggtg 15060

attaagctta tgcctttcag cggggatttt gttcagggat ttataagtta tgtagggtcc 15120

cattatagag aagtgaacct tgtataccct agatacagca acttcatatc tactgaatct 15180

tatttggtta tgacagatct caaggctaac cggctaatga atcctgaaaa gattaagcag 15240

cagataattg aatcatctgt gaggacttca cctggactta taggtcacat cctatccatt 15300

aagcaactaa gctgcataca agcaattgtg ggagacgcag ttagtagagg tgatatcaat 15360

cctactctga aaaaacttac acctatagag caggtgctga tcaattgcgg gttggcaatt 15420

aacggaccta agctgtgcaa agaattgatc caccatgatg ttgcctcagg gcaagatgga 15480

ttgcttaatt ctatactcat cctctacagg gagttggcaa gattcaaaga caaccaaaga 15540

agtcaacaag ggatgttcca cgcttacccc gtattggtaa gtagcaggca acgagaactt 15600

atatctagga tcacccgcaa attttggggg cacattcttc tttactccgg gaacagaaag 15660

ttgataaata agtttatcca gaatctcaag tccggctatc tgatactaga cttacaccag 15720

aatatcttcg ttaagaatct atccaagtca gagaaacaga ttattatgac ggggggtttg 15780

aaacgtgagt gggtttttaa ggtaacagtc aaggagacca aagaatggta taagttagtc 15840

ggatacagtg ccctgattaa ggactaattg gttgaactcc ggaaccctaa tcctgcccta 15900

ggtggttagg cattatttgc aatagattaa agaaaacttt gaaaatacga agtttctatt 15960

cccagctttg tctggtggcc ggcatggtcc cagcctcctc gctggcgccg gctgggcaac 16020

attccgaggg gaccgtcccc tcggtaatgg cgaatgggac gcggccgatc cggctgctaa 16080

caaagcccga aaggaagctg agttggctgc tgccaccgct gagcaataac tagcataacc 16140

ccttggggcc tctaaacggg tcttgagggg ttttttgctg aaaggaggaa ctatatccgg 16200

atgcggccgc ttctatagtg tcacctaaat actagtgact ccagcgtaac tggactggcc 16260

acagttaggc cgcaaatgta atcacactgg ctcaccttcg ggtgggcctt tctgcgttcg 16320

cgagtggacc cgataagctc atggagcggc gtaaccgtcg cacaggaagg acagagaaag 16380

cgcggatctg ggaagtgacg gacagaacgg tcaggacctg gattggggag gcggttgccg 16440
ccgctgctgc tgacggtgtg acgttctctg ttccggtcac accacatacg ttccgccatt 16500
cctatgcgat gcacatgctg tatgccggta taccgctgaa agttctgcaa agcctgatgg 16560
gacataagtc catcagttca acggaagtct acacgaaggt ttttgcgctg gatgtggctg 16620
cccggcaccg ggtgcagttt gcgatgccgg agtctgatgc ggttgcgatg ctgaaacaat 16680
tatcctgaga ataaatgcct tggcctttat atggaaatgt ggaactgagt ggatatgctg 16740
tttttgtctg ttaaacagag aagctggctg ttatccactg agaagcgaac gaaacagtcg 16800
ggaaaatctc ccattatcgt agagatccgc attattaatc tcaggagcct gtgtagcgtt 16860
tataggaagt agtgttctgt catgatgcct gcaagcggta acgaaaacga tttgaatatg 16920
ccttcaggaa caatagaaat cttcgtgcgg tgttacgttg aagtggagcg gattatgtca 16980
gcaatggaca gaacaaccta atgaacacag aaccatgatg tggtctgtcc ttttacagcc 17040
agtagtgctc gccgcagtcg agcgacaggg cgaagccctc ggctggttgc cctcgccgct 17100
gggctggcgg ccgtctatgg ccctgcaaac gcgccagaaa cgccgtcgaa gccgtgtgcg 17160
agacaccgcg gcccgccgcc ggcgttgtgg atacctcgcg gaaaacttgg ccctcactga 17220
cagatgaggg gcggacgttg acacttgagg ggccgactca cccggcgcgg cgttgacaga 17280
tgaggggcag gctcgatttc ggccggcgac gtggagctgg ccagcctcgc aaatcggcga 17340
aaacgcctga ttttacgcga gtttcccaca gatgatgtgg acaagcctgg ggataagtgc 17400
cctgcggtat tgacacttga ggggcgcgac tactgacaga tgaggggcgc gatccttgac 17460
acttgagggg cagagtgctg acagatgagg ggcgcaccta ttgacatttg aggggctgtc 17520
cacaggcaga aaatccagca tttgcaaggg tttccgcccg tttttcggcc accgctaacc 17580
tgtcttttaa cctgctttta aaccaatatt tataaacctt gtttttaacc agggctgcgc 17640
cctgtgcgcg tgaccgcgca cgccgaaggg gggtgccccc ccttctcgaa ccctcccggt 17700
cgagtgagcg aggaagcacc agggaacagc acttatatat tctgcttaca cacgatgcct 17760
gaaaaaactt cccttggggt tatccactta tccacgggga tatttttata attatttttt 17820
ttatagtttt tagatcttct tttttagagc gccttgtagg cctttatcca tgctggttct 17880
agagaaggtg ttgtgacaaa ttgccctttc agtgtgacaa atcaccctca aatgacagtc 17940
ctgtctgtga caaattgccc ttaaccctgt gacaaattgc cctcagaaga agctgttttt 18000
tcacaaagtt atccctgctt attgactctt ttttatttag tgtgacaatc taaaaacttg 18060
tcacacttca catggatctg tcatggcgga aacagcggtt atcaatcaca agaaacgtaa 18120
aaatagcccg cgaatcgtcc agtcaaacga cctcactgag gcggcatata gtctctcccg 18180
ggatcaaaaa cgtatgctgt atctgttcgt tgaccagatc agaaaatctg atggcaccct 18240

acaggaacat gacggtatct gcgagatcca tgttgctaaa tatgctgaaa tattcggatt 18300

gacctctgcg gaagccagta aggatatacg gcaggcattg aagagtttcg cggggaagga 18360

agtggttttt tatcgccctg aagaggatgc cggcgatgaa aaaggctatg aatcttttcc 18420

ttggtttatc aaacgtgcgc acagtccatc cagagggctt tacagtgtac atatcaaccc 18480

atatctcatt cccttcttta tcgggttaca gaaccggttt acgcagtttc ggcttagtga 18540

aacaaaagaa atcaccaatc cgtatgccat gcgtttatac gaatccctgt gtcagtatcg 18600

taagccggat ggctcaggca tcgtctctct gaaaatcgac tggatcatag agcgttacca 18660

gctgcctcaa agttaccagc gtatgcctga cttccgccgc cgcttcctgc aggtctgtgt 18720

taatgagatc aacagcagaa ctccaatgcg cctctcatac attgagaaaa agaaaggccg 18780

ccagacgact catatcgtat tttccttccg cgatatcact tccatgacga caggatagtc 18840

tgagggttat ctgtcacaga tttgagggtg gttcgtcaca tttgttctga cctactgagg 18900

gtaatttgtc acagttttgc tgtttccttc agcctgcatg gattttctca tacttttgaa 18960

actgtaattt ttaaggaagc caaatttgag ggcagtttgt cacagttgat ttccttctct 19020

ttcccttcgt catgtgacct gatatcgggg gttagttcgt catcattgat gagggttgat 19080

tatcacagtt tattactctg aattggctat ccgcgtgtgt acctctacct ggagtttttc 19140

ccacggtgga tatttcttct tgcgctgagc gtaagagcta tctgacagaa cagttcttct 19200

ttgcttcctc gccagttcgc tcgctatgct cggttacacg gctgcggcga gcgctagtga 19260

taataagtga ctgaggtatg tgctcttctt atctcctttt gtagtgttgc tcttatttta 19320

aacaactttg cggttttttg atgactttgc gattttgttg ttgctttgca gtaaattgca 19380

agatttaata aaaaaacgca aagcaatgat taaaggatgt tcagaatgaa actcatggaa 19440

acacttaacc agtgcataaa cgctggtcat gaaatgacga aggctatcgc cattgcacag 19500

tttaatgatg acagcccgga agcgaggaaa ataacccggc gctggagaat aggtgaagca 19560

gcggatttag ttggggtttc ttctcaggct atcagagatg ccgagaaagc agggcgacta 19620

ccgcacccgg atatggaaat tcgaggacgg gttgagcaac gtgttggtta tacaattgaa 19680

caaattaatc atatgcgtga tgtgtttggt acgcgattgc gacgtgctga agacgtattt 19740

ccaccggtga tcggggttgc tgcccataaa ggaggcgttt acaaaacctc agtttctgtt 19800

catcttgctc aggatctggc tctgaagggg ctacgtgttt tgctcgtgga aggtaacgac 19860

ccccagggaa cagcctcaat gtatcacgga tgggtaccag atcttcatat tcatgcagaa 19920

gacactctcc tgcctttcta tcttggggaa aaggacgatg tcacttatgc aataaagccc 19980

acttgctggc cggggcttga cattattcct tcctgtctgg ctctgcaccg tattgaaact 20040

gagttaatgg gcaaatttga tgaaggtaaa ctgcccaccg atccacacct gatgctccga 20100

ctggccattg aaactgttgc tcatgactat gatgtcatag ttattgacag tgcgcctaac 20160

ctgggtatcg gcacgattaa tgtcgtatgt gctgctgatg tgctgattgt tcccacgcct 20220

gctgagttgt ttgactacac ctccgcactg cagtttttcg atatgcttcg tgatctgctc 20280

aagaacgttg atcttaaagg gttcgagcct gatgtacgta ttttgcttac caaatacagc 20340

aatagtaatg gctctcagtc cccgtggatg gaggagcaaa ttcgggatgc ctggggaagc 20400

atggttctaa aaaatgttgt acgtgaaacg gatgaagttg gtaaaggtca gatccggatg 20460

agaactgttt ttgaacaggc cattgatcaa cgctcttcaa ctggtgcctg gagaaatgct 20520

ctttctattt gggaacctgt ctgcaatgaa attttcgatc gtctgattaa accacgctgg 20580

gagattagat aatgaagcgt gcgcctgtta ttccaaaaca tacgctcaat actcaaccgg 20640

ttgaagatac ttcgttatcg acaccagctg ccccgatggt ggattcgtta attgctcgcg 20700

taggagtaat ggctcgcggt aatgccatta ctttgcctgt atgtggtcgg gatgtgaagt 20760

ttactcttga agtgctccgg ggtgatagtg ttgagaagac ctctcgggta tggtcaggta 20820

atgaacgtga ccaggagctg cttactgagg acgcactgga tgatctcatc ccttcttttc 20880

tactgactgg tcaacagaca ccggcgttcg gtcgaagagt atctggtgtc atagaaattg 20940

ccgatgggag tcgccgtcgt aaagctgctg cacttaccga aagtgattat cgtgttctgg 21000

ttggcgagct ggatgatgag cagatggctg cattatccag attgggtaac gattatcgcc 21060

caacaagtgc ttatgaacgt ggtcagcgtt atgcaagccg attgcagaat gaatttgctg 21120

gaaatatttc tgcgctggct gatgcggaaa atatttcacg taagattatt acccgctgta 21180

tcaacaccgc caaattgcct aaatcagttg ttgctctttt ttctcacccc ggtgaactat 21240

ctgcccggtc aggtgatgca cttcaaaaag cctttacaga taaagaggaa ttacttaagc 21300

agcaggcatc taaccttcat gagcagaaaa aagctggggt gatatttgaa gctgaagaag 21360

ttatcactct tttaacttct gtgcttaaaa cgtcatctgc atcaagaact agtttaagct 21420

cacgacatca gtttgctcct ggagcgacag tattgtataa gggcgataaa atggtgctta 21480

acctggacag gtctcgtgtt ccaactgagt gtatagagaa aattgaggcc attcttaagg 21540

aacttgaaaa gccagcaccc tgatgcgacc acgttttagt ctacgtttat ctgtctttac 21600

ttaatgtcct ttgttacagg ccagaaagca taactggcct gaatattctc tctgggccca 21660

ctgttccact tgtatcgtcg gtctgataat cagactggga ccacggtccc actcgtatcg 21720

tcggtctgat tattagtctg ggaccacggt cccactcgta tcgtcggtct gattattagt 21780

ctgggaccac ggtcccactc gtatcgtcgg tctgataatc agactgggac cacggtccca 21840

ctcgtatcgt cggtctgatt attagtctgg gaccatggtc ccactcgtat cgtcggtctg 21900

attattagtc tgggaccacg gtcccactcg tatcgtcggt ctgattatta gtctggaacc 21960

acggtcccac tcgtatcgtc ggtctgatta ttagtctggg accacggtcc cactcgtatc 22020

gtcggtctga ttattagtct gggaccacga tcccactcgt gttgtcggtc tgattatcgg 22080

tctgggacca cggtcccact tgtattgtcg atcagactat cagcgtgaga ctacgattcc 22140

atcaatgcct gtcaagggca agtattgaca tggtcgtcgt aacctgtaga acggagtaac 22200

ctcggtgtgc ggttgtatgc ctgctgtgga ttgctgctgt gtcctgctta tccacaacat 22260

tttgcgcacg gttatgtgga caaaatacct ggttacccag gccgtgccgg cacgttaacc 22320

gggctgcatc cgatgcaagt gtgtcgctga cgacgagctc gcgagctcgg acatgaggtt 22380

gccccgtatt cagtgtcgct gatttgtatt gtctgaagtt gcttttacgt taagttgatg 22440

cagatcaatt aatacgatac ctgcgtcata attgattatt tgacgtggtt tgatggcctc 22500

cacgcacgtt gtgatatgta gatgataatc attatcactt tacgggtcct ttccggtgat 22560

ccgacaggtt acggggcggc gacctcgcgg gttttcgcta tttatgaaaa ttttccggtt 22620

taaggcgttt ccgttcttct tcgtcataac ttaatgtttt tatttaaaat accctctgaa 22680

aagaaaggaa acgacaggtg ctgaaagcga gctttttggc ctctgtcgtt tcctttctct 22740

gtttttgtcc gtggaatgaa caatggaagt ccgagctcat cgctaataac ttcgtatagc 22800

atacattata cgaagttata ttcgatgcgg ccgatctagc agaaagtcaa aagcctccga 22860

ccggaggctt ttgacttctg tcacctaggt tacgccccgc cctgccactc atcgcagtac 22920

tgttgtaatt cattaagcat tctgccgaca tggaagccat cacaaacggc atgatgaacc 22980

tgaatcgcca gcggcatcag caccttgtcg ccttgcgtat aatatttgcc catggtgaaa 23040

acgggggcga agaagttgtc catattggcc acgtttaaat caaaactggt gaaactcacc 23100

cagggattgg ctgagacgaa aaacatattc tcaataaacc ctttagggaa ataggccagg 23160

ttttcaccgt aacacgccac atcttgcgaa tatatgtgta gaaactgccg gaaatcgtcg 23220

tggtattcac tccagagcga tgaaaacgtt tcagtttgct catggaaaac ggtgtaacaa 23280

gggtgaacac tatcccatat caccagctca ccgtctttca ttgccatacg aaattccgga 23340

tgagcattca tcaggcgggc aagaatgtga ataaaggccg gataaaactt gtgcttattt 23400

ttctttacgg tctttaaaaa ggccgtaata tccagctgaa cggtctggtt ataggtacat 23460

tgagcaactg actgaaatgc ctcaaaatgt tctttacgat gccattggga tatatcaacg 23520

gtggtatatc cagtgatttt tttctccatt ttagcttcct tagctcctga aaatctcgat 23580

aactcaaaaa atacgcccgg tagtgatctt atttcattat ggtgaaagtt ggaacctctt 23640

acgtgccgat cagattaaaa cgaaaggccc agtctttcga ctgagccttt cgttttattt 23700

gaccatgttg gtatgattta aattcgttta gtgaaccgtc agatcactag aagctttatt 23760

gcggtagttt atcacagtta aattgctaac gcagtcagtg cttctgacac aacagtctcg 23820

aacttaagct gcagtgactc tcttaaggta gccttgcaga agttggtcgt gaggcactgg 23880

gcaggtaagt atcaaggtta caagacaggt ttaaggagac caatagaaac tgggcttgtc 23940

gagacagaga agactcttgc gtttctgata ggcacctatt ggtcttactg acatccactt 24000

tgcctttctc tccacaggtg tccactccca gttcaattac agctcttaag gctagagtac 24060

t 24061

<210> 62
<211> 15894
<212> DNA
<213> artificial sequence

<220>
<223> Synthetic sequence

<400> 62
accaaacaaa gttgggtaag gatagttcaa tcaatgatca tcttctagtg cacttaggat 60

tcaagatcct attatcaggg acaagagcag gattagggat atccgagatg gccacacttt 120

taaggagctt agcattgttc aaaagaaaca aggacaaacc acccattaca tcaggatccg 180

gtggagccat cagaggaatc aaacacatta ttatagtacc aatccctgga gattcctcaa 240

ttaccactcg atccagactt ctggaccggt tggtgaggtt aattggaaac ccggatgtga 300

gcgggcccaa actaacaggg gcactaatag gtatattatc cttatttgtg gagtctccag 360

gtcaattgat tcagaggatc accgatgacc ctgacgttag cataaggctg ttagaggttg 420

tccagagtga ccagtcacaa tctggcctta ccttcgcatc aagaggtacc aacatggagg 480

atgaggcgga ccaatacttt tcacatgatg atccaattag tagtgatcaa tccaggttcg 540

gatggttcgg gaacaaggaa atctcagata ttgaagtgca agaccctgag ggattcaaca 600

tgattctggg taccatccta gcccaaattt gggtcttgct cgcaaaggcg gttacggccc 660

cagacacggc agctgattcg gagctaagaa ggtggataaa gtacacccaa caaagaaggg 720

tagttggtga atttagattg gagagaaaat ggttggatgt ggtgaggaac aggattgccg 780

aggacctctc cttacgccga ttcatggtcg ctctaatcct ggatatcaag agaacacccg 840

gaaacaaacc caggattgct gaaatgatat gtgacattga tacatatatc gtagaggcag 900

gattagccag ttttatcctg actattaagt ttgggataga aactatgtat cctgctcttg 960

gactgcatga atttgctggt gagttatcca cacttgagtc cttgatgaac ctttaccagc 1020

aaatggggga aactgcaccc tacatggtaa tcctggagaa ctcaattcag aacaagttca 1080

gtgcaggatc ataccctctg ctctggagct atgccatggg agtaggagtg gaacttgaaa 1140

actccatggg aggtttgaac tttggccgat cttactttga tccagcatat tttagattag 1200

ggcaagagat ggtaaggagg tcagctggaa aggtcagttc cacattggca tctgaactcg 1260

gtatcactgc cgaggatgca aggcttgttt cagagattgc aatgcatact actgaggaca 1320

agatcagtag agcggttgga cccagacaag cccaagtatc atttctacac ggtgatcaaa 1380

gtgagaatga gctaccgaga ttggggggca aggaagatag gagggtcaaa cagagtcgag 1440

gagaagccag ggagagctac agagaaaccg ggcccagcag agcaagtgat gcgagagctg 1500

cccatcttcc aaccggcaca cccctagaca ttgacactgc aacggagtcc agccaagatc 1560

cgcaggacag tcgaaggtca gctgacgccc tgcttaggct gcaagccatg gcaggaatct 1620

cggaagaaca aggctcagac acggacaccc ctatagtgta caatgacaga aatcttctag 1680

actaggtgcg agaggccgag ggccagaaca acatccgcct accatccatc attgttataa 1740

aaaacttagg aaccaggtcc acacagccgc cagcccatca accatccact cccacgattg 1800

gagccaatgg cagaagagca ggcacgccat gtcaaaaacg gactggaatg catccgggct 1860

ctcaaggccg agcccatcgg ctcactggcc atcgaggaag ctatggcagc atggtcagaa 1920

atatcagaca acccaggaca ggagcgagcc acctgcaggg aagagaaggc aggcagttcg 1980

ggtctcagca aaccatgcct ctcagcaatt ggatcaactg aaggcggtgc acctcgcatc 2040

cgcggtcagg gacctggaga gagcgatgac gacgctgaaa ctttgggaat ccccccaaga 2100

aatctccagg catcaagcac tgggttacag tgttattacg tttatgatca cagcggtgaa 2160

gcggttaagg gaatccaaga tgctgactct atcatggttc aatcaggcct tgatggtgat 2220

agcaccctct caggaggaga caatgaatct gaaaacagcg atgtggatat tggcgaacct 2280

gataccgagg gatatgctat cactgaccgg ggatctgctc ccatctctat ggggttcagg 2340

gcttctgatg ttgaaactgc agaaggaggg gagatccacg agctcctgag actccaatcc 2400

agaggcaaca actttccgaa gcttgggaaa actctcaatg ttcctccgcc cccggacccc 2460

ggtagggcca gcacttccgg gacacccatt aaaaagggca cagacgcgag attagcctca 2520

tttggaacgg agatcgcgtc tttattgaca ggtggtgcaa cccaatgtgc tcgaaagtca 2580

ccctcggaac catcagggcc aggtgcacct gcggggaatg tccccgagtg tgtgagcaat 2640

gccgcactga tacaggagtg gacacccgaa tctggtacca caatctcccc gagatcccag 2700

aataatgaag aagggggaga ctattatgat gatgagctgt tctctgatgt ccaagatatt 2760

aaaacagcct tggccaaaat acacgaggat aatcagaaga taatctccaa gctagaatca 2820

ctgctgttat tgaagggaga agttgagtca attaagaagc agatcaacag gcaaaatatc 2880

agcatatcca ccctggaagg acacctctca agcatcatga tcgccattcc tggacttggg 2940

aaggatccca acgaccccac tgcagatgtc gaaatcaatc ccgacttgaa acccatcata 3000

ggcagagatt caggccgagc actggccgaa gttctcaaga aacccgttgc cagccgacaa 3060

ctccaaggaa tgacaaatgg acggaccagt tccagaggac agctgctgaa ggaatttcag 3120

ctaaagccga tcgggaaaaa gatgagctca gccgtcgggt ttgttcctga caccggccct 3180

gcatcacgca gtgtaatccg ctccattata aaatccagcc ggctagagga ggatcggaag 3240

cgttacctga tgactctcct tgatgatatc aaaggagcca atgatcttgc caagttccac 3300

cagatgctga tgaagataat aatgaagtag ctacagctca acttacctgc caaccccatg 3360

ccagtcgacc caactagtac aacctaaatc cattataaaa aacttaggag caaagtgatt 3420

gcctcccaag gtccacaatg acagagacct acgacttcga caagtcggca tgggacatca 3480

aagggtcgat cgctccgata caacccacca cctacagtga tggcaggctg gtgccccagg 3540

tcagagtcat agatcctggt ctaggcgaca ggaaggatga atgctttatg tacatgtttc 3600

tgctgggggt tgttgaggac agcgattccc tagggcctcc aatcgggcga gcatttgggt 3660

tcctgccctt aggtgttggc agatccacag caaagcccga aaaactcctc aaagaggcca 3720

ctgagcttga catagttgtt agacgtacag cagggctcaa tgaaaaactg gtgttctaca 3780

acaacacccc actaactctc ctcacacctt ggagaaaggt cctaacaaca gggagtgtct 3840

tcaacgcaaa ccaagtgtgc aatgcggtta atctgatacc gctcgatacc ccgcagaggt 3900

tccgtgttgt ttatatgagc atcacccgtc tttcggataa cgggtattac accgttccta 3960

gaagaatgct ggaattcaga tcggtcaatg cagtggcctt caacctgctg gtgaccctta 4020

ggattgacaa ggcgataggc cctgggaaga tcatcgacaa tacagagcaa cttcctgagg 4080

caacatttat ggtccacatc gggaacttca ggagaaagaa gagtgaagtc tactctgccg 4140

attattgcaa aatgaaaatc gaaaagatgg gcctggtttt tgcacttggt gggatagggg 4200

gcaccagtct tcacattaga agcacaggca aaatgagcaa gactctccat gcacaactcg 4260

ggttcaagaa gaccttatgt tacccgctga tggatatcaa tgaagacctt aatcgattac 4320

tctggaggag cagatgcaag atagtaagaa tccaggcagt tttgcagcca tcagttcctc 4380

aagaattccg catttacgac gacgtgatca taaatgatga ccaaggacta ttcaaagttc 4440

tgtagaccgt agtgcccagc aatgcccgaa aacgaccccc ctcacaatga cagccagaag 4500

gcccggacaa aaaagccccc tccgaaagac tccacggacc aagcgagagg ccagccagca 4560

gccgacggca agcgcgaaca ccaggcggcc ccagcacaga acagccctga cacaaggcca 4620

ccaccagcca ccccaatctg catcctcctc gtgggacccc cgaggaccaa cccccaaggc 4680

tgcccccgat ccaaaccacc aaccgcatcc ccaccacccc cgggaaagaa acccccagca 4740

attggaaggc ccctccccct cttcctcaac acaagaactc cacaaccgaa ccgcacaagc 4800

gaccgaggtg acccaaccgc aggcatccga ctccctagac agatcctctc tccccggcaa 4860

actaaacaaa acttagggcc aaggaacata cacacccaac agaacccaga ccccggccca 4920

cggcgccgcg cccccaaccc ccgacaacca gagggagccc ccaaccaatc ccgccggctc 4980

ccccggtgcc cacaggcagg gacaccaacc cccgaacaga cccagcaccc aaccatcgac 5040

aatccaagac gggggggccc ccccaaaaaa aggcccccag gggccgacag ccagcaccgc 5100

gaggaagccc acccacccca cacacgacca cggcaaccaa accagaaccc agaccaccct 5160

gggccaccag ctcccagact cggccatcac cccgcagaaa ggaaaggcca caacccgcgc 5220

accccagccc cgatccggcg gggagccacc caacccgaac cagcacccaa gagcgatccc 5280

cgaaggaccc ccgaaccgca aaggacatca gtatcccaca gcctctccaa gtcccccggt 5340

ctcctcctct tctcgaaggg accaaaagat caatccacca cacccgacga cactcaactc 5400

cccaccccta aaggagacac cgggaatccc agaatcaaga ctcatccaat gtccatcatg 5460

ggtctcaagg tgaacgtctc tgccatattc atggcagtac tgttaactct ccaaacaccc 5520

accggtcaaa tccattgggg caatctctct aagatagggg tggtaggaat aggaagtgca 5580

agctacaaag ttatgactcg ttccagccat caatcattag tcataaaatt aatgcccaat 5640

ataactctcc tcaataactg cacgagggta gagattgcag aatacaggag actactgaga 5700

acagttttgg aaccaattag agatgcactt aatgcaatga cccagaatat aagaccggtt 5760

cagagtgtag cttcaagtag gagacacaag agatttgcgg gagtagtcct ggcaggtgcg 5820

gccctaggcg ttgccacagc tgctcagata acagccggca ttgcacttca ccagtccatg 5880

ctgaactctc aagccatcga caatctgaga gcgagcctgg aaactactaa tcaggcaatt 5940

gagacaatca gacaagcagg gcaggagatg atattggctg ttcagggtgt ccaagactac 6000

atcaataatg agctgatacc gtctatgaac caactatctt gtgatttaat cggccagaag 6060

ctcgggctca aattgctcag atactataca gaaatcctgt cattatttgg ccccagttta 6120

cgggacccca tatctgcgga gatatctatc caggctttga gctatgcgct tggaggagac 6180

atcaataagg tgttagaaaa gctcggatac agtggaggtg atttactggg catcttagag 6240

agcggaggaa taaaggcccg gataactcac gtcgacacag agtcctactt cattgtcctc 6300

agtatagcct atccgacgct gtccgagatt aagggggtga ttgtccaccg gctagagggg 6360

gtctcgtaca acataggctc tcaagagtgg tataccactg tgcccaagta tgttgcaacc 6420

caagggtacc ttatctcgaa ttttgatgag tcatcgtgta ctttcatgcc agaggggact 6480

gtgtgcagcc aaaatgcctt gtacccgatg agtcctctgc tccaagaatg cctccggggg 6540

tacaccaagt cctgtgctcg tacactcgta tccgggtctt ttgggaaccg gttcatttta 6600

tcacaaggga acctaatagc caattgtgca tcaatccttt gcaagtgtta cacaacagga 6660

acgatcatta atcaagaccc tgacaagatc ctaacataca ttgctgccga tcactgcccg 6720

gtagtcgagg tgaacggcgt gaccatccaa gtcgggagca ggaggtatcc agacgctgtg 6780

tacttgcaca gaattgacct cggtcctccc atatcattgg agaggttgga cgtagggaca 6840

aatctgggga atgcaattgc taagttggag gatgccaagg aattgttgga gtcatcggac 6900

cagatattga ggagtatgaa aggtttatcg agcactagca tagtctacat cctgattgca 6960

gtgtgtcttg gagggttgat agggatcccc gctttaatat gttgctgcag ggggcgttgt 7020

aacaaaaagg gagaacaagt tggtatgtca agaccaggcc taaagcctga tcttacggga 7080

acatcaaaat cctatgtaag gtcgctctga tcctctacaa ctcttgaaac acaaatgtcc 7140

cacaagtctc ctcttcgtca tcaagcaacc accgcaccca gcatcaagcc cacctgaaat 7200

tatctccggc ttccctctgg ccgaacaata tcggtagtta atcaaaactt agggtgcaag 7260

atcatccaca atgtcaccac aacgagaccg gataaatgcc ttctacaaag ataaccccca 7320

tcccaaggga agtaggatag tcattaacag agaacatctt atgattgata gaccttatgt 7380

tttgctggct gttctgtttg tcatgtttct gagcttgatc gggttgctag ccattgcagg 7440

cattagactt catcgggcag ccatctacac cgcagagatc cataaaagcc tcagcaccaa 7500

tctagatgta actaactcaa tcgagcatca ggtcaaggac gtgctgacac cactcttcaa 7560

aatcatcggt gatgaagtgg gcctgaggac acctcagaga ttcactgacc tagtgaaatt 7620

aatctctgac aagattaaat tccttaatcc ggatagggag tacgacttca gagatctcac 7680

ttggtgtatc aacccgccag agagaatcaa attggattat gatcaatact gtgcagatgt 7740

ggctgctgaa gagctcatga atgcattggt gaactcaact ctactggaga ccagaacaac 7800

caatcagttc ctagctgtct caaagggaaa ctgctcaggg cccactacaa tcagaggtca 7860

attctcaaac atgtcgctgt ccctgttaga cttgtattta ggtcgaggtt acaatgtgtc 7920

atctatagtc actatgacat cccagggaat gtatggggga acttacctag tggaaaagcc 7980

taatctgagc agcaaaaggt cagagttgtc acaactgagc atgtaccgag tgtttgaagt 8040

aggtgttatc agaaatccgg gtttgggggc tccggtgttc catatgacaa actatcttga 8100

gcaaccagtc agtaatgatc tcagcaactg tatggtggct ttgggggagc tcaaactcgc 8160

agccctttgt cacggggaag attctatcac aattccctat cagggatcag ggaaaggtgt 8220

cagcttccag ctcgtcaagc taggtgtctg gaaatcccca accgacatgc aatcctgggt 8280

ccccttatca acggatgatc cagtgataga caggctttac ctctcatctc acagaggtgt 8340

tatcgctgac aatcaagcaa aatgggctgt cccgacaaca cgaacagatg acaagttgcg 8400

aatggagaca tgcttccaac aggcgtgtaa gggtaaaatc caagcactct gcgagaatcc 8460

cgagtgggca ccattgaagg ataacaggat tccttcatac ggggtcttgt ctgttgatct 8520

gagtctgaca gttgagctta aaatcaaaat tgcttcggga ttcgggccat tgatcacaca 8580

cggttcaggg atggacctat acaaatccaa ccacaacaat gtgtattggc tgactatccc 8640

gccaatgaag aacctagcct taggtgtaat caacacattg gagtggatac cgagattcaa 8700

ggttagtccc tacctcttca ctgtcccaat taaggaagca ggcgaagact gccatgcccc 8760

aacataccta cctgcggagg tggatggtga tgtcaaactc agttccaatc tggtgattct 8820

acctggtcaa gatctccaat atgttttggc aacctacgat acttccaggg ttgaacatgc 8880

tgtggtttat tacgtttaca gcccaagccg ctcattttct tacttttatc cttttaggtt 8940

gcctataaag ggggtcccca tcgaattaca agtggaatgc ttcacatggg accaaaaact 9000

ctggtgccgt cacttctgtg tgcttgcgga ctcagaatct ggtggacata tcactcactc 9060

tgggatggtg ggcatgggag tcagctgcac agtcacccgg gaagatggaa ccaatcgcag 9120

atagggctgc tagtgaacca atcacatgat gtcacccaga catcaggcat acccactagt 9180

gtgaaataga catcagaatt aagaaaaacg tagggtccaa gtggttcccc gttatggact 9240

cgctatctgt caaccagatc ttataccctg aagttcacct agatagcccg atagttacca 9300

ataagatagt agccatcctg gagtatgctc gagtccctca cgcttacagc ctggaggacc 9360

ctacactgtg tcagaacatc aagcaccgcc taaaaaacgg attttccaac caaatgatta 9420

taaacaatgt ggaagttggg aatgtcatca agtccaagct taggagttat ccggcccact 9480

ctcatattcc atatccaaat tgtaatcagg atttatttaa catagaagac aaagagtcaa 9540

cgaggaagat ccgtgaactc ctcaaaaagg ggaattcgct gtactccaaa gtcagtgata 9600

aggttttcca atgcttaagg gacactaact cacggcttgg cctaggctcc gaattgaggg 9660

aggacatcaa ggagaaagtt attaacttgg gagtttacat gcacagctcc cagtggtttg 9720

agccctttct gttttggttt acagtcaaga ctgagatgag gtcagtgatt aaatcacaaa 9780

cccatacttg ccataggagg agacacacac ctgtattctt cactggtagt tcagttgagt 9840

tgctaatctc tcgtgacctt gttgctataa tcagtaaaga gtctcaacat gtatattacc 9900

tgacatttga actggttttg atgtattgtg atgtcataga ggggaggtta atgacagaga 9960

ccgctatgac tattgatgct aggtatacag agcttctagg aagagtcaga tacatgtgga 10020

aactgataga tggtttcttc cctgcactcg ggaatccaac ttatcaaatt gtagccatgc 10080

tggagcctct ttcacttgct tacctgcagc tgagggatat aacagtagaa ctcagaggtg 10140

ctttccttaa ccactgcttt actgaaatac atgatgttct tgaccaaaac gggttttctg 10200

atgaaggtac ttatcatgag ttaactgaag ctctagatta cattttcata actgatgaca 10260

tacatctgac aggggagatt ttctcatttt tcagaagttt cggccacccc agacttgaag 10320

cagtaacggc tgctgaaaat gttaggaaat acatgaatca gcctaaagtc attgtgtatg 10380

agactctgat gaaaggtcat gccatatttt gtggaatcat aatcaacggc tatcgtgaca 10440

ggcacggagg cagttggcca ccgctgaccc tccccctgca tgctgcagac acaatccgga 10500

atgctcaagc ttcaggtgaa gggttaacac atgagcagtg cgttgataac tggaaatctt 10560

ttgctggagt gaaatttggc tgctttatgc ctcttagcct ggatagtgat ctgacaatgt 10620

acctaaagga caaggcactt gctgctctcc aaagggaatg ggattcagtt tacccgaaag 10680

agttcctgcg ttacgaccct cccaagggaa ccgggtcacg gaggcttgta gatgttttcc 10740

ttaatgattc gagctttgac ccatatgatg tgataatgta tgttgtaagt ggagcttacc 10800

tccatgaccc tgagttcaac ctgtcttaca gcctgaaaga aaaggagatc aaggaaacag 10860

gtagactttt tgctaaaatg acttacaaaa tgagggcatg ccaagtgatt gctgaaaatc 10920

taatctcaaa cgggattggc aaatatttta aggacaatgg gatggccaag gatgagcacg 10980

atttgactaa ggcactccac actctagctg tctcaggagt ccccaaagat ctcaaagaaa 11040

gtcacagggg ggggccagtc ttaaaaacct actcccgaag cccagtccac acaagtacca 11100

ggaacgtgag agcagcaaaa gggtttatag ggttccctca agtaattcgg caggaccaag 11160

acactgatca tccggagaat atggaagctt acgagacagt cagtgcattt atcacgactg 11220

atctcaagaa gtactgcctt aattggagat atgagaccat cagcttgttt gcacagaggc 11280

taaatgagat ttacggattg ccctcatttt tccagtggct gcataagagg cttgagacct 11340

ctgtcctgta tgtaagtgac cctcattgcc cccccgacct tgacgcccat atcccgttat 11400

ataaagtccc caatgatcaa atcttcatta agtaccctat gggaggtata gaagggtatt 11460

gtcagaagct gtggaccatc agcaccattc cctatctata cctggctgct tatgagagcg 11520

gagtaaggat tgcttcgtta gtgcaagggg acaatcagac catagccgta acaaaaaggg 11580

tacccagcac atggccctac aaccttaaga aacgggaagc tgctagagta actagagatt 11640

actttgtaat tcttaggcaa aggctacatg atattggcca tcacctcaag gcaaatgaga 11700

caattgtttc atcacatttt tttgtctatt caaaaggaat atattatgat gggctacttg 11760

tgtcccaatc actcaagagc atcgcaagat gtgtattctg gtcagagact atagttgatg 11820

aaacaagggc agcatgcagt aatattgcta caacaatggc taaaagcatc gagagaggtt 11880

atgaccgtta ccttgcatat tccctgaacg tcctaaaagt gatacagcaa attctgatct 11940

ctcttggctt cacaatcaat tcaaccatga cccgggatgt agtcataccc ctcctcacaa 12000

acaacgacct cttaataagg atggcactgt tgcccgctcc tattgggggg atgaattatc 12060

tgaatatgag caggctgttt gtcagaaaca tcggtgatcc agtaacatca tcaattgctg 12120

atctcaagag aatgattctc gcctcactaa tgcctgaaga gaccctccat caagtaatga 12180

cacaacaacc gggggactct tcattcctag actgggctag cgacccttac tcagcaaatc 12240

ttgtatgtgt ccagagcatc actagactcc tcaagaacat aactgcaagg tttgtcctga 12300

tccatagtcc aaacccaatg ttaaaaggat tattccatga tgacagtaaa gaagaggacg 12360

agggactggc ggcattcctc atggacaggc atattatagt acctagggca gctcatgaaa 12420

tcctggatca tagtgtcaca ggggcaagag agtctattgc aggcatgctg gataccacaa 12480

aaggcttgat tcgagccagc atgaggaagg gggggttaac ctctcgagtg ataaccagat 12540

tgtccaatta tgactatgaa caattcagag cagggatggt gctattgaca ggaagaaaga 12600

gaaatgtcct cattgacaaa gagtcatgtt cagtgcagct ggcgagagct ctaagaagcc 12660

atatgtgggc gaggctagct cgaggacggc ctatttacgg ccttgaggtc cctgatgtac 12720

tagaatctat gcgaggccac cttattcggc gtcatgagac atgtgtcatc tgcgagtgtg 12780

gatcagtcaa ctacggatgg ttttttgtcc cctcgggttg ccaactggat gatattgaca 12840

aggaaacatc atccttgaga gtcccatata ttggttctac cactgatgag agaacagaca 12900

tgaagcttgc cttcgtaaga gccccaagtc gatccttgcg atctgctgtt agaatagcaa 12960

cagtgtactc atgggcttac ggtgatgatg atagctcttg gaacgaagcc tggttgttgg 13020

ctaggcaaag ggccaatgtg agcctggagg agctaagggt gatcactccc atctcaactt 13080

cgactaattt agcgcatagg ttgagggatc gtagcactca agtgaaatac tcaggtacat 13140

cccttgtccg agtggcgagg tataccacaa tctccaacga caatctctca tttgtcatat 13200

cagataagaa ggttgatact aactttatat accaacaagg aatgcttcta gggttgggtg 13260

ttttagaaac attgtttcga ctcgagaaag ataccggatc atctaacacg gtattacatc 13320

ttcacgtcga aacagattgt tgcgtgatcc cgatgataga tcatcccagg atacccagct 13380

cccgcaagct agagctgagg gcagagctat gtaccaaccc attgatatat gataatgcac 13440

ctttaattga cagagatgca acaaggctat acacccagag ccataggagg caccttgtgg 13500

aatttgttac atggtccaca ccccaactat atcacatttt agctaagtcc acagcactat 13560

ctatgattga cctggtaaca aaatttgaga aggaccatat gaatgaaatt tcagctctca 13620

taggggatga cgatatcaat agtttcataa ctgagtttct gctcatagag ccaagattat 13680

tcactatcta cttgggccag tgtgcggcca tcaattgggc atttgatgta cattatcata 13740

gaccatcagg gaaatatcag atgggtgagc tgttgtcatc gttcctttct agaatgagca 13800

aaggagtgtt taaggtgctt gtcaatgctc taagccaccc aaagatctac aagaaattct 13860

ggcattgtgg tattatagag cctatccatg gtccttcact tgatgctcaa aacttgcaca 13920

caactgtgtg caacatggtt tacacatgct atatgaccta cctcgacctg ttgttgaatg 13980

aagagttaga agagttcaca tttctcttgt gtgaaagcga cgaggatgta gtaccggaca 14040

gattcgacaa catccaggca aaacacttat gtgttctggc agatttgtac tgtcaaccag 14100

ggacctgccc accaattcga ggtctaagac cggtagagaa atgtgcagtt ctaaccgacc 14160

atatcaaggc agaggctatg ttatctccag caggatcttc gtggaacata aatccaatta 14220

ttgtagacca ttactcatgc tctctgactt atctccggcg aggatcgatc aaacagataa 14280

gattgagagt tgatccagga ttcattttcg acgccctcgc tgaggtaaat gtcagtcagc 14340

caaagatcgg cagcaacaac atctcaaata tgagcatcaa ggctttcaga cccccacacg 14400

atgatgttgc aaaattgctc aaagatatca acacaagcaa gcacaatctt cccatttcag 14460

ggggcaatct cgccaattat gaaatccatg ctttccgcag aatcgggttg aactcatctg 14520

cttgctacaa agctgttgag atatcaacat taattaggag atgccttgag ccaggggagg 14580

acggcttgtt cttgggtgag ggatcgggtt ctatgttgat cacttataaa gagatactta 14640

aactaaacaa gtgcttctat aatagtgggg tttccgccaa ttctagatct ggtcaaaggg 14700

aattagcacc ctatccctcc gaagttggcc ttgtcgaaca cagaatggga gtaggtaata 14760

ttgtcaaagt gctctttaac gggaggcccg aagtcacgtg ggtaggcagt gtagattgct 14820

tcaatttcat agttagtaat atccctacct ctagtgtggg gtttatccat tcagatatag 14880

agaccttgcc tgacaaagat actatagaga agctagagga attggcagcc atcttatcga 14940

tggctctgct cctgggcaaa ataggatcaa tactggtgat taagcttatg cctttcagcg 15000

gggattttgt tcagggattt ataagttatg tagggtctca ttatagagaa gtgaaccttg 15060

tataccctag atacagcaac ttcatctcta ctgaatctta tttggttatg acagatctca 15120

aggctaaccg gctaatgaat cctgaaaaga ttaagcagca gataattgaa tcatctgtga 15180

ggacttcacc tggacttata ggtcacatcc tatccattaa gcaactaagc tgcatacaag 15240

caattgtggg agacgcagtt agtagaggtg atatcaatcc tactctgaaa aaacttacac 15300

ctatagagca ggtgctgatc aattgcgggt tggcaattaa cggacctaag ctgtgcaaag 15360

aattgatcca ccatgatgtt gcctcagggc aagatggatt gcttaattct atactcatcc 15420

tctacaggga gttggcaaga ttcaaagaca accaaagaag tcaacaaggg atgttccacg 15480

cttaccccgt attggtaagt agcaggcaac gagaacttat atctaggatc acccgcaaat 15540

tctgggggca cattcttctt tactccggga acaaaaagtt gataaataag tttatccaga 15600

atctcaagtc cggctatctg atactagact tacaccagaa tatcttcgtt aagaatctat 15660

ccaagtcaga gaaacagatt attatgacgg ggggtttgaa acgtgagtgg gtttttaagg 15720

taacagtcaa ggagaccaaa gaatggtata agttagtcgg atacagtgcc ctgattaagg 15780

actaattggt tgaactccgg aaccctaatc ctgccctagg tggttaggca ttatttgcaa 15840

tatattaaag aaaactttga aaatacgaag tttctattcc cagctttgtc tggt 15894